# EUROPEAN PATENT APPLICATION

(11) **EP 2 587 263 A1**
(43) Date of publication of application: **01.05.2013**
(21) Application number: 13152898.6
(22) Date of filing: 25.07.2008
(51) Int. Cl.: G01N 33/532

(54) **Self coupling recombinant antibody fusion proteins**

(30) Priority: 25.07.2007 DE 102007035160
(62) Divisional of application: 08775358.8
(71) Applicant: Fraunhofer-Gesellschaft zur Förderung der angewandten Forschung e.V., 80686 München (DE); Rheinisch-Westfälisch-Technische Hochschule Aachen, 52062 Aachen (DE)
(72) Inventor: The designation of the inventor has not yet been filed
(74) Representative: von Kreisler Selting Werner

(57) **Abstract**

A compound comprising three components A, B, and C, which components are covalently bound forming the compound having the structure A-B-C wherein
component A has a specific binding affinity for antigens,
component B is covalently linked to component A
component C is a compound having an alkylated purin or pyrimidin moiety such as guanin, cytosin or a Coenzyme A moiety and linked thereto a moiety having a physiological effect with the proviso that
component B has an catalytical or acceptor activity to couple component C with covalently coupled components A-B.

SEQUENCE LISTING

<110>  Fraunhofer Gesellschaft, RWTH Aachen

<120>  Self Coupling Recombinant Antibody Fusion Proteins

<130>  130252EP

<150>  08775358.8
<151>  2008-07-25

<160>  70

<170>  PatentIn version 3.3

<210>  1
<211>  1484
<212>  DNA
<213>  Homo sapiens

<400>  1
atggagacag acacactcct gctatgggta ctgctgctct gggttccagg ttccactggt      60

gacgcggccc agccggccat ggcccaggtg cagctggtgg agagcggtgg aggtgttgtg     120

caacctggcc ggtccctgcg cctgtcctgc tcctcgtctg gcttcatttt cagtgacaat     180

tacatgtatt gggtgagaca ggcacctgga aaaggtcttg agtgggttgc aaccattagt     240

gatggtggta gttacaccta ctatccagac agtgtgaagg gaagatttac aatatcgaga     300

gacaacagca agaacacatt gttcctgcaa atggacagcc tgagacccga agacaccggg     360

gtctattttt gtgcaagagg ctactatagg tacgaggggg ctatggacta ctggggccaa     420

gggaccccgg tcaccgtgag ctcaggaggt ggcggctccg gaggtggagg cagcggaggg     480

ggcggatccg acatccagct gacccagagc ccaagcagcc tgagcgccag cgtgggtgac     540

agagtgacca tcacctgtaa gtccagtcaa agtgttttat acagttcaaa tcagaagaac     600

tacttggcct ggtaccagca gaagccaggt aaggctccaa agctgctgat ctactgggca     660

tccactaggg aatctggtgt gccaagcaga ttcagcggta gcggtagcgg taccgacttc     720

accttcacca tcagcagcct ccagccagag gacatcgcca cctactactg ccatcaatac     780

ctctcctcgt ggacgttcgg ccaagggacc aagctggaga tcaaagcggc cgcactcgag     840

tctagaatgg acaaagactg cgaaatgaag cgcaccaccc tggatagccc tctgggcaag     900

ctggaactgt ctgggtgcga acagggcctg cacgagatca agctgctggg caaaggaaca     960

tctgccgccg acgccgtgga agtgcctgcc ccagccgccg tgctgggcgg accagagcca    1020

ctgatgcagg ccaccgcctg gctcaacgcc tactttcacc agcctgaggc catcgaggag    1080

ttccctgtgc cagccctgca ccacccagtg ttccagcagg agagctttac ccgccaggtg    1140

ctgtggaaac tgctgaaagt ggtgaagttc ggagaggtca tcagctacca gcagctggcc    1200

gccctggccg gcaatcccgc cgccaccgcc gccgtgaaaa ccgccctgag cggaaatccc    1260

gtgcccattc tgatcccctg ccaccgggtg gtgtctagct ctggcgccgt ggggggctac    1320

gagggcgggc tcgccgtgaa agagtggctg ctggcccacg agggccacag actgggcaag 1380

cctgggctgg gcgctgagca cgaatttcga ggagggcccg aacaaaaact catctcagaa 1440

gaggatctga atagcgccgt cgaccatcat catcatcatc attg 1484

<210> 2
<211> 1200
<212> DNA
<213> Homo sapiens

<400> 2
atggagacag acacactcct gctatgggta ctgctgctct gggttccagg ttccactggt 60

gactctagaa tggacaaaga ctgcgaaatg aagcgcacca ccctggatag ccctctgggc 120

aagctggaac tgtctgggtg cgaacagggc ctgcacgaga tcaagctgct gggcaaagga 180

acatctgccg ccgacgccgt ggaagtgcct gccccagccg ccgtgctggg cggaccagag 240

ccactgatgc aggccaccgc ctggctcaac gcctactttc accagcctga ggccatcgag 300

gagttccctg tgccagccct gcaccaccca gtgttccagc aggagagctt tacccgccag 360

gtgctgtgga aactgctgaa agtggtgaag ttcggagagg tcatcagcta ccagcagctg 420

gccgccctgg ccggcaatcc cgccgccacc gccgccgtga aaaccgccct gagcggaaat 480

cccgtgccca ttctgatccc ctgccaccgg gtggtgtcta gctctggcgc cgtggggggc 540

tacgagggcg ggctcgccgt gaaagagtgg ctgctggccc acgagggcca cagactgggc 600

aagcctgggc tgggcgctga gcacgaaggt gacgcggccc agccggccca gaggacggac 660

tccattccca actcacctga caacgtcccc ctcaaaggag gaaattgctc agaagacctc 720

ttatgtatcc tgaaaagagc tccattcaag aagtcatggg cctacctcca agtggcaaag 780

catctgaaca aaaccaagtt gtcttggaac aaagatggca ttctccatgg agtcagatat 840

caggatggga atctggtgat ccaattccct ggtttgtact tcatcatttg ccaactgcag 900

tttcttgtac aatgcccaaa taattctgtc gatctgaagt tggagcttct catcaacaag 960

catatcaaaa aacaggccct ggtgacagtg tgtgagtctg gaatgcaaac gaaacacgta 1020

taccagaatc tctctcaatt cttgctggat tacctgcagg tcaacaccac catatcagtc 1080

aatgtggata cattccagta catagataca agcacctttc ctcttgagaa tgtgttgtcc 1140

atcttcttat acagtaattc agacgcggcc gcagggcccc atcatcatca tcatcattga 1200

<210> 3
<211> 1491
<212> DNA
<213> Mus sp.

<400> 3
atggagacag acacactcct gctatgggta ctgctgctct gggttccagg ttccactggt 60

gacgcggccc agccggccat ggcccaggtc aagctgcagg agtcagggac tgaactggca 120

aagcctgggg ccgcagtgaa gatgtcctgc aaggcttctg gctacacctt tactgactac 180

tggatgcact gggttaaaca gaggcctgga cagggtctgg aatggattgg atacattaat 240

cctaacactg cttatactga ctacaatcag aaattcaagg acaaggccac attgactgca 300

gacaaatcct ccagcacagc ctacatgcaa ctgcgcagcc tgacctctga ggattctgca 360

gtctattact gtgcaaaaaa gacaactcag actacgtggg ggtttccttt ttggggccaa 420

gggaccacgg tcaccgtctc ctcaggtgga ggcggttcag gcggaggtgg ctctggcggt 480

ggcggatcgg acattgtgct gacccagtct ccaaaatcca tggccatgtc agtcggagag 540

agggtcacct tgagctgcaa ggccagtgag aatgtggatt cttttgtttc ctggtatcaa 600

cagaaaccag gccagtctcc taaactgctg atatacgggg cctccaaccg gtacactggg 660

gtccccgatc gcttcgcagg cagtggatct ggaagagatt tcactctgac catcagcagt 720

gtgcaggctg aagaccttgc agattatcac tgtggacaga attacaggta tccgctcacg 780

ttcggtgctg gcaccaagct ggaaatcaaa cgggcggccg catctggcgg tggcggatcg 840

ctcgagtcta gaatggacaa agactgcgaa atgaagcgca ccaccctgga tagccctctg 900

ggcaagctgg aactgtctgg gtgcgaacag ggcctgcacg agatcaagct gctgggcaaa 960

ggaacatctg ccgccgacgc cgtggaagtg cctgccccag ccgccgtgct gggcggacca 1020

gagccactga tgcaggccac cgcctggctc aacgcctact ttcaccagcc tgaggccatc 1080

gaggagttcc ctgtgccagc cctgcaccac ccagtgttcc agcaggagag ctttacccgc 1140

caggtgctgt ggaaactgct gaaagtggtg aagttcggag aggtcatcag ctaccagcag 1200

ctggccgccc tggccggcaa tcccgccgcc accgccgccg tgaaaaccgc cctgagcgga 1260

aatcccgtgc ccattctgat cccctgccac cgggtggtgt ctagctctgg cgccgtgggg 1320

ggctacgagg gcgggctcgc cgtgaaagag tggctgctgg cccacgaggg ccacagactg 1380

ggcaagcctg ggctgggcgc tgagcacgaa tttcgaggag ggcccgaaca aaaactcatc 1440

tcagaagagg atctgaatag cgccgtcgac catcatcatc atcatcattg a 1491

<210> 4
<211> 1473
<212> DNA
<213> Mus sp.

<400> 4
atggagacag acacactcct gctatgggta ctgctgctct gggttccagg ttccactggt 60

gacgcggccc agccggccat ggccgaggtg caactgcagc agtctggggc tgaactggtg 120

aagcctgggg cttcagtgaa gttgtcctgc aaggcttccg gctacacctt caccagccac 180

tggatgcact gggtgaagca gagggctgga caaggccttg agtggatcgg agagtttaat 240

cccagcaacg gccgtactaa ctacaatgag aaattcaaga gcaaggccac actgactgta 300

gacaaatcct ccagcacagc ctacatgcaa ctcagcagcc tgacatctga ggactctgcg 360

gtctattact gtgccagtcg ggactatgat tacgacggac ggtactttga ctactggggc 420

caagggacca cggtcaccgt ctcctcaggt ggcggtggct cgggcggtgg tgggtcgggt 480

ggtggcggat ctgacatcga gctcacccag tctccagcaa tcatgtctgc atctccaggg 540

gagaaggtca ctatgacctg cagtgccagc tcaagtgtaa cttacatgta ttggtaccag 600

cagaagccag gatcctcccc cagactcctg atttatgaca catccaacct ggcttctgga 660

gtccctgttc gtttcagtgg cagtgggtct gggacctctt actctctcac aatcagccga 720

atggaggctg aagatgctgc cacttattac tgccagcagt ggagtagtca catattcacg 780

ttcggctcgg ggacagaact cgagatcaaa cgggcggccg cactcgagtc tagaatggac 840

aaagactgcg aaatgaagcg caccaccctg gatagccctc tgggcaagct ggaactgtct 900

gggtgcgaac agggcctgca cgagatcaag ctgctgggca aaggaacatc tgccgccgac 960

gccgtggaag tgcctgcccc agccgccgtg ctgggcggac cagagccact gatgcaggcc 1020

accgcctggc tcaacgccta ctttcaccag cctgaggcca tcgaggagtt ccctgtgcca 1080

gccctgcacc acccagtgtt ccagcaggag agctttaccc gccaggtgct gtggaaactg 1140

ctgaaagtgg tgaagttcgg agaggtcatc agctaccagc agctggccgc cctggccggc 1200

aatcccgccg ccaccgccgc cgtgaaaacc gccctgagcg gaaatcccgt gcccattctg 1260

atcccctgcc accgggtggt gtctagctct ggcgccgtgg ggggctacga gggcgggctc 1320

gccgtgaaag agtggctgct ggcccacgag ggccacagac tgggcaagcc tgggctgggc 1380

gctgagcacg aatttcgagg agggcccgaa caaaaactca tctcagaaga ggatctgaat 1440

agcgccgtcg accatcatca tcatcatcat tga 1473

<210> 5
<211> 843
<212> DNA
<213> Homo sapiens

<400> 5
atggagacag acacactcct gctatgggta ctgctgctct gggttccagg ttccactggt 60

gactctagaa tggacaaaga ctgcgaaatg aagcgcacca ccctggatag ccctctgggc 120

aagctggaac tgtctgggtg cgaacagggc ctgcacgaga tcaagctgct gggcaaagga 180

acatctgccg ccgacgccgt ggaagtgcct gccccagccg ccgtgctggg cggaccagag 240

ccactgatgc aggccaccgc ctggctcaac gcctactttc accagcctga ggccatcgag 300

gagttccctg tgccagccct gcaccaccca gtgttccagc aggagagctt tacccgccag 360

gtgctgtgga aactgctgaa agtggtgaag ttcggagagg tcatcagcta ccagcagctg 420

gccgccctgg ccggcaatcc cgccgccacc gccgccgtga aaaccgccct gagcggaaat 480

cccgtgccca ttctgatccc ctgccaccgg gtggtgtcta gctctggcgc cgtggggggc 540

tacgagggcg ggctcgccgt gaaagagtgg ctgctggccc acgagggcca cagactgggc 600

aagcctgggc tgggcgctga gcacgaaggt gacgcggccc agccggccaa tagtgactct 660

gaatgtcccc tgtcccacga tgggtactgc ctccatgatg gtgtgtgcat gtatattgaa 720

gcattggaca agtatgcatg caactgtgtt gttggctaca tcggggagcg atgtcagtac 780

cgagacctga agtggtggga actgcgcgcg gccgcagggc cccatcatca tcatcatcat 840

tga 843

<210> 6
<211> 1551
<212> DNA
<213> Mus sp.

<400> 6
atggagacag acacactcct gctatgggta ctgctgctct gggttccagg ttccactggt 60

gacgcggccc agccggccat ggccgaggtg caactgcagc agtctggggc tgaactggtg 120

aagcctgggg cttcagtgaa gttgtcctgc aaggcttccg gctacacctt caccagccac 180

tggatgcact gggtgaagca gaggcctgga caaggccttg agtggatcgg agagtttaat 240

cccagcaacg gccgtactaa ctacaatgag aaattcaaga gcaaggccac actgactgta 300

gacaaatcct ccagcacagc ctacatgcaa ctcagcagcc tgacatctga ggactctgcg 360

gtctattact gtgccagtcg ggactatgat tacgacggac ggtactttga ctactggggc 420

caagggacca cggtcaccgt ctcctcaggt ggcggtggct cgggcggtgg tgggtcgggt 480

ggtggcggat ctgacatcga gctcacccag tctccagcaa tcatgtctgc atctccaggg 540

gagaaggtca ctatgacctg cagtgccagc tcaagtgtaa cttacatgta ttggtaccag 600

cagaagccag gatcctcccc cagactcctg atttatgaca catccaacct ggcttctgga 660

gtccctgttc gtttcagtgg cagtgggtct gggacctctt actctctcac aatcagccga 720

atggaggctg aagatgctgc cacttattac tgccagcagt ggagtagtca catattcacg 780

ttcggctcgg ggacagaact cgagatcaaa cgggcggccg ctagccgtca tcgccagccg 840

cgcggcaatc gtgtccgacg ctcacatatg cccttatcgt caatcttctc gcgcattggg 900

gacccttcta gaatggacaa agactgcgaa atgaagcgca ccaccctgga tagccctctg 960

ggcaagctgg aactgtctgg gtgcgaacag ggcctgcacg agatcaagct gctgggcaaa 1020

ggaacatctg ccgccgacgc cgtggaagtg cctgccccag ccgccgtgct gggcggacca 1080

gagccactga tgcaggccac cgcctggctc aacgcctact ttcaccagcc tgaggccatc 1140

gaggagttcc ctgtgccagc cctgcaccac ccagtgttcc agcaggagag ctttacccgc 1200

caggtgctgt ggaaactgct gaaagtggtg aagttcggag aggtcatcag ctaccagcag 1260

ctggccgccc tggccggcaa tcccgccgcc accgccgccg tgaaaaccgc cctgagcgga 1320

aatcccgtgc ccattctgat cccctgccac cgggtggtgt ctagctctgg cgccgtgggg 1380

ggctacgagg gcgggctcgc cgtgaaagag tggctgctgg cccacgaggg ccacagactg 1440

ggcaagcctg ggctgggcgc tgagcacgaa tttcgaggag ggcccgaaca aaaactcatc 1500

tcagaagagg atctgaatag cgccgtcgac catcatcatc atcatcattg a 1551

<210> 7
<211> 1458
<212> DNA
<213> Mus sp.

<400> 7
atggagacag acacactcct gctatgggta ctgctgctct gggttccagg ttccactggt 60

gacgcggccc agccggccat ggcccaggtg cagctggagc agtcgggggg aggcttagtg 120

aagcctggag ggtccctgaa actctcctgt gcagcctctg gattcacttt cagtagctat 180

gccatgtctt gggttcgcca gactccggag aagaggctgg agtgggtcgc aaccattaat 240

agtggtggta gttacaccta ctattcggac agtgtgaagg gtcgattcac catctccaga 300

gacaatgcca ggaataccct gtatctacaa atgagcagtc tgaggtctga ggacacggcc 360

atgtattact gtgcaagaat ctactatgct atggactact ggggcgcagg aaccccggtc 420

accgtctcct caggtggcgg tggctcgggc ggtggtgggt cgggtggcgg cggatcagac 480

attgtgatga cccagactcc aaaatccatg tcaacattag taggagacag ggtcagcatc 540

acctgcaagg ccagtcagga tgtgagcact gctgtagcct ggtatcaaca gaaaccagga 600

caatctccta aactactgat ttactcggca tcctaccggt acactggagt ccctgatcgc 660

ttcactggca gtggatctgg gacggatttc actttcacca tcagcaatgt gcagtctgaa 720

gacttggcag agtatttctg tcagcaatat aacagctatc ctctgacgtt cgccgcaggc 780

accaagctgg agatcaaagc ggccgcactc gagtctagaa tggacaaaga ctgcgaaatg 840

aagcgcacca ccctggatag ccctctgggc aagctggaac tgtctgggtg cgaacagggc 900

ctgcacgaga tcaagctgct gggcaaagga acatctgccg ccgacgccgt ggaagtgcct 960

gccccagccg ccgtgctggg cggaccagag ccactgatgc aggccaccgc ctggctcaac 1020

gcctactttc accagcctga ggccatcgag gagttccctg tgccagccct gcaccaccca 1080

gtgttccagc aggagagctt tacccgccag gtgctgtgga aactgctgaa agtggtgaag 1140

ttcggagagg tcatcagcta ccagcagctg gcggccctgg cgggcaatcc cgccgccacc 1200

gccgccgtga aaaccgccct gagcggaaat cccgtgccca ttctgatccc ctgccaccgg 1260

gtggtgtcta gctctggcgc cgtggggggc tacgagggcg ggctcgccgt gaaagagtgg 1320

ctgctggccc acgagggcca cagactgggc aagcctgggc tgggcgctga gcacgaattt 1380

cgaggagggc ccgaacaaaa actcatctca gaagaggatc tgaatagcgc cgtcgaccat 1440

catcatcatc atcattga 1458

<210> 8
<211> 1107
<212> DNA
<213> Homo sapiens

<400>  8
atggagacag acacactcct gctatgggta ctgctgctct gggttccagg ttccactggt      60

gactctagaa tggacaaaga ctgcgaaatg aagcgcacca ccctggatag ccctctgggc     120

aagctggaac tgtctgggtg cgaacagggc ctgcacgaga tcaagctgct gggcaaagga     180

acatctgccg ccgacgccgt ggaagtgcct gccccagccg ccgtgctggg cggaccagag     240

ccactgatgc aggccaccgc ctggctcaac gcctactttc accagcctga ggccatcgag     300

gagttccctg tgccagccct gcaccaccca gtgttccagc aggagagctt tacccgccag     360

gtgctgtgga aactgctgaa agtggtgaag ttcggagagg tcatcagcta ccagcagctg     420

gccgccctgg ccggcaatcc cgccgccacc gccgccgtga aaaccgccct gagcggaaat     480

cccgtgccca ttctgatccc ctgccaccgg gtggtgtcta gctctggcgc cgtggggggc     540

tacgagggcg ggctcgccgt gaaagagtgg ctgctggccc acgagggcca cagactgggc     600

aagcctgggc tgggcgctga gcacgaaggt gacgcggccc agccggccgg acaattcaga     660

gtgataggac cagggtatcc catccgggct ttagttgggg atgaagcaga gctgccgtgc     720

cgcatctctc ctgggaaaaa tgccacgggc atggaggtgg gttggtaccg ttctcccttc     780

tcaagagtgg ttcacctcta ccgaaatggc aaggaccaag atgcagagca agcacctgaa     840

taccggggac gcacagagct tctgaaagag actatcagtg agggaaaggt tacccttagg     900

attcagaacg tgagattctc agatgaagga ggctacacct gcttcttcag agaccactct     960

taccaagaag aggcagcaat ggagttgaaa gtggaagatc ccttctattg ggtcaacccc    1020

ggtgtggcgg ccgcagggcc cgaacaaaaa ctcatctcag aagaggatct gaatagcgcc    1080

gtcgaccatc atcatcatca tcattga                                        1107


<210>  9
<211>  1428
<212>  DNA
<213>  Homo sapiens

<400>  9
atggagacag acacactcct gctatgggta ctgctgctct gggttccagg ttccactggt      60

gactctagaa tggacaaaga ctgcgaaatg aagcgcacca ccctggatag ccctctgggc     120

aagctggaac tgtctgggtg cgaacagggc ctgcacgaga tcaagctgct gggcaaagga     180

acatctgccg ccgacgccgt ggaagtgcct gccccagccg ccgtgctggg cggaccagag     240

ccactgatgc aggccaccgc ctggctcaac gcctactttc accagcctga ggccatcgag     300

gagttccctg tgccagccct gcaccaccca gtgttccagc aggagagctt tacccgccag     360

gtgctgtgga aactgctgaa agtggtgaag ttcggagagg tcatcagcta ccagcagctg     420

gccgccctgg ccggcaatcc cgccgccacc gccgccgtga aaaccgccct gagcggaaat     480

cccgtgccca ttctgatccc ctgccaccgg gtggtgtcta gctctggcgc cgtggggggc     540

tacgagggcg ggctcgccgt gaaagagtgg ctgctggccc acgagggcca cagactgggc 600

aagcctgggc tgggcgctga gcacgaaggt gacgcggccc agccggccgt gatgacccag 660

tctccatcct ccctgtctgc atctgtagga gacagagtca ccatcgcttg ccgggcaagt 720

cagaccatta gcaactattt aaattggtat cagcagaaac cagggaaagc ccctaagctc 780

ctgatctatg gtgcatccag tttgcaaagt ggggtcccat caaggttcag tggcagtgga 840

tctgggacag atttcactct caccatcagc agtctgcaac ctgaagattt tgcaacttac 900

tactgtcaac agagttacag tacccctccg acgtacactt ttggccaggg gaccaagctg 960

gagatcaaag gtggcggtgg ctcgggcggt ggtgggtcgg gtggcagcgg atcatcgggg 1020

ggcgacttgg tccagccggg ggggtccctg agagtctcct gtgtagcctc tggatttaca 1080

tttaggacct atgtgatgaa ctgggtccgc caggctccag gaaaggggct ggagtgggtg 1140

gcccacataa gtccagaggg aactgaagaa tactatgcgg accctgtgaa gggccgattt 1200

accgtctcca gagacaacgc gaagaattca gtatttctgc aaatgaatag tctgagaggc 1260

gaggacacgg ctgtgtatta ttgcgcgaga gtccgacgct atggtccctc tacgctcagt 1320

ccgttcacct ggaaggacaa tcactacgcc atggacgtct ggggccaagg gacaacggtc 1380

accgtctctc cagcggccgc agggccccat catcatcatc atcattga 1428

<210> 10
<211> 774
<212> DNA
<213> Human immunodeficiency virus

<400> 10
atggagacag acacactcct gctatgggta ctgctgctct gggttccagg ttccactggt 60

gacgcggccc agccggccta cggtcgtaaa aaacgtcgtc agcgtcgtcg tagggcggcc 120

gcactcgagt ctagaatgga caaagactgc gaaatgaagc gcaccaccct ggatagccct 180

ctgggcaagc tggaactgtc tgggtgcgaa cagggcctgc acgagatcaa gctgctgggc 240

aaaggaacat ctgccgccga cgccgtggaa gtgcctgccc cagccgccgt gctgggcgga 300

ccagagccac tgatgcaggc caccgcctgg ctcaacgcct actttcacca gcctgaggcc 360

atcgaggagt tccctgtgcc agccctgcac cacccagtgt tccagcagga gagctttacc 420

cgccaggtgc tgtggaaact gctgaaagtg gtgaagttcg gagaggtcat cagctaccag 480

cagctggccg ccctggccgg caatcccgcc gccaccgccg ccgtgaaaac cgccctgagc 540

ggaaatcccg tgcccattct gatcccctgc caccgggtgg tgtctagctc tggcgccgtg 600

gggggctacg agggcgggct cgccgtgaaa gagtggctgc tggcccacga gggccacaga 660

ctgggcaagc ctgggctggg cgctgagcac gaatttcgag gagggcccga acaaaaactc 720

atctcagaag aggatctgaa tagcgccgtc gaccatcatc atcatcatca ttga 774

<210> 11

<211>  1695
<212>  DNA
<213>  Homo sapiens

<400>  11
atggagacag acacactcct gctatgggta ctgctgctct gggttccagg ttccactggt      60

gacgcggccc agccggccgc acaggttctc agaggcactg tgactgactt ccctggattt     120

gatgagcggg ctgatgcaga aactcttcgg aaggctatga aaggcttggg cacagatgag     180

gagagcatcc tgactctgtt gacatcccga agtaatgctc agcgccagga aatctctgca     240

gcttttaaga ctctgtttgg cagggatctt ctggatgacc tgaaatcaga actaactgga     300

aaatttgaaa aattaattgt ggctctgatg aaaccctctc ggctttatga tgcttatgaa     360

ctgaaacatg ccttgaaggg agctggaaca aatgaaaaag tactgacaga aattattgct     420

tcaaggacac ctgaagaact gagagccatc aaacaagttt atgaagaaga atatggctca     480

agcctggaag atgacgtggt gggggacact tcagggtact accagcggat gttggtggtt     540

ctccttcagg ctaacagaga ccctgatgct ggaattgatg aagctcaagt tgaacaagat     600

gctcaggctt tatttcaggc tggagaactt aaatggggga cagatgaaga aaagtttatc     660

accatctttg gaacacgaag tgtgtctcat ttgagaaagg tgtttgacaa gtacatgact     720

atatcaggat ttcaaattga ggaaaccatt gaccgcgaga cttctggcaa tttagagcaa     780

ctactccttg ctgttgtgaa atctattcga agtatacctg cctaccttgc agagaccctc     840

tattatgcta tgaagggagc tgggacagat gatcataccc tcatcagagt catggtttcc     900

aggagtgaga ttgatctgtt taacatcagg aaggagttta ggaagaattt tgccacctct     960

ctttattcca tgattaaggg agatacatct ggggactata agaaagctct tctgctgctc    1020

tgtggagaag atgacgcggc cgcactcgag tctagaatgg acaaagactg cgaaatgaag    1080

cgcaccaccc tggatagccc tctgggcaag ctggaactgt ctgggtgcga acagggcctg    1140

cacgagatca agctgctggg caaaggaaca tctgccgccg acgccgtgga agtgcctgcc    1200

ccagccgccg tgctgggcgg accagagcca ctgatgcagg ccaccgcctg gctcaacgcc    1260

tactttcacc agcctgaggc catcgaggag ttccctgtgc cagccctgca ccacccagtg    1320

ttccagcagg agagctttac ccgccaggtg ctgtggaaac tgctgaaagt ggtgaagttc    1380

ggagaggtca tcagctacca gcagctggcg gccctggcgg gcaatcccgc cgccaccgcc    1440

gccgtgaaaa ccgccctgag cggaaatccc gtgcccattc tgatcccctg ccaccgggtg    1500

gtgtctagct ctggcgccgt ggggggctac gagggcgggc tcgccgtgaa agagtggctg    1560

ctggcccacg agggccacag actgggcaag cctgggctgg gcgctgagca cgaatttcga    1620

ggagggcccg aacaaaaact catctcagaa gaggatctga atagcgccgt cgaccatcat    1680

catcatcatc attga                                                     1695

<210>  12

<211>  1443
<212>  DNA
<213>  Mus sp.

<400>  12
atggagacag acacactcct gctatgggta ctgctgctct gggttccagg ttccactggt      60

gacgcggccc agccggccca gcagtctggg actgtgctgg caaggcctgg ggcttccgtg     120

aagatgtcct gcaaggcttc tggctacagg tttaccaact actggatgca ctgggtaaaa     180

cagaggcctg gacagggtct agaatggatt ggtgttattt atcctggaaa tagtgatact     240

agctacaacc agaagttcaa gggcaaggcc aaactgactg cagtcacatc cgccagcact     300

gcctacatgg agctcagcag cctgacaaat gaggactctg cggtctatta ctgtacaaga     360

gagggagaag gctctgacta ctggggccaa gggaccacgg tcaccgtctc ctcaggtgga     420

ggcggttcag gcggaggtgg ctctggcggt ggcggatcgc aaattgttct cacccagtct     480

ccagcaacca tggctgcatc tcccggggag aagatcacta tcacctgcag tgccagctca     540

agtataagtt ccaattactt gcattggtat cagcagaagc caggattctc ccctaaactc     600

ttgatttata ggacttccaa tctggcttct ggagtcccag ctcgcttcag tggcagtggg     660

tctgggacct cttactctct cacaattggc accatggagg ctgaagatgt tgccacttac     720

tactgccagc agggtagtag tataccgtac acgttcggag gggggaccaa gctggagctg     780

aaagcggccg cactcgagtc tagaatggac aaagactgcg aaatgaagcg caccaccctg     840

gatagccctc tgggcaagct ggaactgtct gggtgcgaac agggcctgca cgagatcaag     900

ctgctgggca aaggaacatc tgccgccgac gccgtggaag tgcctgcccc agccgccgtg     960

ctgggcggac cagagccact gatgcaggcc accgcctggc tcaacgccta ctttcaccag    1020

cctgaggcca tcgaggagtt ccctgtgcca gccctgcacc acccagtgtt ccagcaggag    1080

agctttaccc gccaggtgct gtggaaactg ctgaaagtgg tgaagttcgg agaggtcatc    1140

agctaccagc agctggcggc cctggcgggc aatcccgccg ccaccgccgc cgtgaaaacc    1200

gccctgagcg gaaatcccgt gcccattctg atcccctgcc accgggtggt gtctagctct    1260

ggcgccgtgg ggggctacga gggcgggctc gccgtgaaag agtggctgct ggcccacgag    1320

ggccacagac tgggcaagcc tgggctgggc gctgagcacg aatttcgagg agggcccgaa    1380

caaaaactca tctcagaaga ggatctgaat agcgccgtcg accatcatca tcatcatcat    1440

tga                                                                  1443


<210>  13
<211>  1467
<212>  DNA
<213>  Homo sapiens

<400>  13
atggagacag acacactcct gctatgggta ctgctgctct gggttccagg ttccactggt      60

gacgcggccc agccggccat ggcccaggtg cagctgcagg agtcgggggg aggcttggta     120

cagcctgggg ggtccctgag actctcctgt gcagcctctg gattcacctt tagcaactct     180

gccatgagct gggtccgcca ggctccaggg aaggggctgg agtgggtctc aagtatcagt     240

ggtagtggcg gtaacacata ctccgctgac tccgtgaagg gccgattcac catctccaga     300

gacaacgcca agaactcact gtatctgcaa atgaacagcc tgagagccga ggacacggcc     360

gtgtattact gtgcgagaga ttggtacggt atggacgtct ggggccaagg gaccacggtc     420

accgtctcct caggtggagg cggttcaggc ggaggtggct ctggcggtgg cggatcgtcc     480

tatgtgctga ctcaggaccc tgctgtgtct gtggccttgg gacagacagt caggatcaca     540

tgccaaggag acagcctcag aggctattat gcaagctggt accagcagaa gccaggacag     600

gcccctgtac ttgtcatcta tgctaaaacc aaccggccct cagggatccc agaccgattc     660

tctggctcca gctcaggaaa cacagcttcc ttgaccatca ctggggctca ggcggaagat     720

gaagctgact attactgtaa ctcccgggac aacagtggta cccatcttga agtattcggc     780

ggagggacca agctgaccgt cctaggtgcg gccgcactcg agtctagaat ggacaaagac     840

tgcgaaatga agcgcaccac cctggatagc cctctgggca agctggaact gtctgggtgc     900

gaacagggcc tgcacgagat caagctgctg ggcaaaggaa catctgccgc cgacgccgtg     960

gaagtgcctg ccccagccgc cgtgctgggc ggaccagagc cactgatgca ggccaccgcc    1020

tggctcaacg cctactttca ccagcctgag gccatcgagg agttccctgt gccagccctg    1080

caccacccag tgttccagca ggagagcttt acccgccagg tgctgtggaa actgctgaaa    1140

gtggtgaagt tcggagaggt catcagctac cagcagctgg cggccctggc gggcaatccc    1200

gccgccaccg ccgccgtgaa aaccgccctg agcggaaatc ccgtgcccat tctgatcccc    1260

tgccaccggg tggtgtctag ctctggcgcc gtggggggct acgagggcgg gctcgccgtg    1320

aaagagtggc tgctggccca cgagggccac agactgggca agcctgggct gggcgctgag    1380

cacgaatttc gaggagggcc cgaacaaaaa ctcatctcag aagaggatct gaatagcgcc    1440

gtcgaccatc atcatcatca tcattga                                        1467

<210>  14
<211>  1161
<212>  DNA
<213>  Mus sp.

<400>  14
atggagacag acacactcct gctatgggta ctgctgctct gggttccagg ttccactggt      60

gacgcggccc agccggccat ggccgaggtg caactgcagc agtctggggc tgaactggtg     120

aagcctgggg cttcagtgaa gttgtcctgc aaggcttccg gctacacctt caccagccac     180

tggatgcact gggtgaagca gaggcctgga caaggccttg agtggatcgg agagtttaat     240

cccagcaacg gccgtactaa ctacaatgag aaattcaaga gcaaggccac actgactgta     300

gacaaatcct ccagcacagc ctacatgcaa ctcagcagcc tgacatctga ggactctgcg     360

gtctattact gtgccagtcg ggactatgat tacgacggac ggtactttga ctactggggc 420

caagggacca cggtcaccgt ctcctcaggt ggcggtggct cgggcggtgg tgggtcgggt 480

ggtggcggat ctgacatcga gctcacccag tctccagcaa tcatgtctgc atctccaggg 540

gagaaggtca ctatgacctg cagtgccagc tcaagtgtaa cttacatgta ttggtaccag 600

cagaagccag gatcctcccc cagactcctg atttatgaca catccaacct ggcttctgga 660

gtccctgttc gtttcagtgg cagtgggtct gggacctctt actctctcac aatcagccga 720

atggaggctg aagatgctgc cacttattac tgccagcagt ggagtagtca catattcacg 780

ttcggctcgg ggacagaact cgagatcaaa cgggcggccg cactcgagtc tagaatgagc 840

actatcgaag aacgcgttaa gaaaattatc ggcgaacagc tgggcgttaa gcaggaagaa 900

gttaccaaca atgcttcttt cgttgaagac ctgggcgcgg attctcttga caccgttgag 960

ctggtaatgg ctctggaaga agagtttgat actgagattc cggacgaaga agctgagaaa 1020

atcaccaccg ttcaggctgc cattgattac atcaacggcc accaggcggc tgagcacgaa 1080

tttcgaggag ggcccgaaca aaaactcatc tcagaagagg atctgaatag cgccgtcgac 1140

catcatcatc atcatcattg a 1161

<210> 15
<211> 1179
<212> DNA
<213> Mus sp.

<400> 15
atggagacag acacactcct gctatgggta ctgctgctct gggttccagg ttccactggt 60

gacgcggccc agccggccat ggcccaggtc aagctgcagg agtcagggac tgaactggca 120

aagcctgggg ccgcagtgaa gatgtcctgc aaggcttctg gctacacctt tactgactac 180

tggatgcact gggttaaaca gaggcctgga cagggtctgg aatggattgg atacattaat 240

cctaacactg cttatactga ctacaatcag aaattcaagg acaaggccac attgactgca 300

gacaaatcct ccagcacagc ctacatgcaa ctgcgcagcc tgacctctga ggattctgca 360

gtctattact gtgcaaaaaa gacaactcag actacgtggg ggtttccttt ttggggccaa 420

gggaccacgg tcaccgtctc ctcaggtgga ggcggttcag gcggaggtgg ctctggcggt 480

ggcggatcgg acattgtgct gacccagtct ccaaaatcca tggccatgtc agtcggagag 540

agggtcacct tgagctgcaa ggccagtgag aatgtggatt cttttgtttc ctggtatcaa 600

cagaaaccag gccagtctcc taaactgctg atatacgggg cctccaaccg gtacactggg 660

gtccccgatc gcttcgcagg cagtggatct ggaagagatt tcactctgac catcagcagt 720

gtgcaggctg aagaccttgc agattatcac tgtggacaga attacaggta tccgctcacg 780

ttcggtgctg gcaccaagct ggaaatcaaa cgggcggccg catctggcgg tggcggatcg 840

ctcgagtcta gaatgagcac tatcgaagaa cgcgttaaga aaattatcgg cgaacagctg 900

ggcgttaagc aggaagaagt taccaacaat gcttctttcg ttgaagacct gggcgcggat 960

tctcttgaca ccgttgagct ggtaatggct ctggaagaag agtttgatac tgagattccg 1020

gacgaagaag ctgagaaaat caccaccgtt caggctgcca ttgattacat caacggccac 1080

caggcggctg agcacgaatt tcgaggaggg cccgaacaaa aactcatctc agaagaggat 1140

ctgaatagcg ccgtcgacca tcatcatcat catcattga 1179

<210> 16
<211> 1473
<212> DNA
<213> Mus sp.

<400> 16
atggagacag acacactcct gctatgggta ctgctgctct gggttccagg ttccactggt 60

gacgcggccc agccggccat ggccgaggtg caactgcagc agtctggggc tgaactggtg 120

aagcctgggg cttcagtgaa gttgtcctgc aaggcttccg gctacacctt caccagccac 180

tggatgcact gggtgaagca gaggcctgga caaggccttg agtggatcgg agagtttaat 240

cccagcaacg gccgtactaa ctacaatgag aaattcaaga gcaaggccac actgactgta 300

gacaaatcct ccagcacagc ctacatgcaa ctcagcagcc tgacatctga ggactctgcg 360

gtctattact gtgccagtcg ggactatgat tacgacggac ggtactttga ctactggggc 420

caagggacca cggtcaccgt ctcctcaggt ggcggtggct cgggcggtgg tgggtcgggt 480

ggtggcggat ctgacatcga gctcacccag tctccagcaa tcatgtctgc atctccaggg 540

gagaaggtca ctatgacctg cagtgccagc tcaagtgtaa cttacatgta ttggtaccag 600

cagaagccag gatcctcccc cagactcctg atttatgaca catccaacct ggcttctgga 660

gtccctgttc gtttcagtgg cagtgggtct gggacctctt actctctcac aatcagccga 720

atggaggctg aagatgctgc cacttattac tgccagcagt ggagtagtca catattcacg 780

ttcggctcgg ggacagaact cgagatcaaa cgggcggccg cactcgagtc tagaatggac 840

aaagactgcg aaatgaagcg caccaccctg gatagccctc tgggcaagct ggaactgtct 900

gggtgcgaac agggcctgca cgagatcatc ttcctgggca aaggaacatc tgccgccgac 960

gccgtggaag tgcctgcccc agccgccgtg ctgggcggac cagagccact gatccaggcc 1020

accgcctggc tcaacgccta ctttcaccag cctgaggcca tcgaggagtt ccctgtgcca 1080

gccctgcacc acccagtgtt ccagcaggag agctttaccc gccaggtgct gtggaaactg 1140

ctgaaagtgg tgaagttcgg agaggtcatc agcgagagcc acctggccgc cctggtgggc 1200

aatcccgccg ccaccgccgc cgtgaacacc gccctggacg gaaatcccgt gcccattctg 1260

atcccctgcc accgggtggt gcagggcgac agcgacgtgg ggccctacct gggcgggctc 1320

gccgtgaaag agtggctgct ggcccacgag ggccacagac tgggcaagcc tgggctgggt 1380

gctgagcacg aatttcgagg agggcccgaa caaaaactca tctcagaaga ggatctgaat 1440

agcgccgtcg accatcatca tcatcatcat tga 1473

<210> 17
<211> 1491
<212> DNA
<213> Mus sp.

<400> 17
atggagacag acacactcct gctatgggta ctgctgctct gggttccagg ttccactggt 60

gacgcggccc agccggccat ggcccaggtc aagctgcagg agtcagggac tgaactggca 120

aagcctgggg ccgcagtgaa gatgtcctgc aaggcttctg gctacacctt tactgactac 180

tggatgcact gggttaaaca gaggcctgga cagggtctgg aatggattgg atacattaat 240

cctaacactg cttatactga ctacaatcag aaattcaagg acaaggccac attgactgca 300

gacaaatcct ccagcacagc ctacatgcaa ctgcgcagcc tgacctctga ggattctgca 360

gtctattact gtgcaaaaaa gacaactcag actacgtggg ggtttccttt ttggggccaa 420

gggaccacgg tcaccgtctc ctcaggtgga ggcggttcag gcggaggtgg ctctggcggt 480

ggcggatcgg acattgtgct gacccagtct ccaaaatcca tggccatgtc agtcggagag 540

agggtcacct tgagctgcaa ggccagtgag aatgtggatt cttttgtttc ctggtatcaa 600

cagaaaccag gccagtctcc taaactgctg atatacgggg cctccaaccg gtacactggg 660

gtccccgatc gcttcgcagg cagtggatct ggaagagatt tcactctgac catcagcagt 720

gtgcaggctg aagaccttgc agattatcac tgtggacaga attacaggta tccgctcacg 780

ttcggtgctg gcaccaagct ggaaatcaaa cgggcggccg catctggcgg tggcggatcg 840

ctcgagtcta gaatggacaa agactgcgaa atgaagcgca ccaccctgga tagccctctg 900

ggcaagctgg aactgtctgg gtgcgaacag ggcctgcacg agatcatctt cctgggcaaa 960

ggaacatctg ccgccgacgc cgtggaagtg cctgccccag ccgccgtgct gggcggacca 1020

gagccactga tccaggccac cgcctggctc aacgcctact ttcaccagcc tgaggccatc 1080

gaggagttcc ctgtgccagc cctgcaccac ccagtgttcc agcaggagag ctttacccgc 1140

caggtgctgt ggaaactgct gaaagtggtg aagttcggag aggtcatcag cgagagccac 1200

ctggccgccc tggtgggcaa tcccgccgcc accgccgccg tgaacaccgc cctggacgga 1260

aatcccgtgc ccattctgat cccctgccac cgggtggtgc agggcgacag cgacgtgggg 1320

ccctacctgg gcgggctcgc cgtgaaagag tggctgctgg cccacgaggg ccacagactg 1380

ggcaagcctg ggctgggtgc tgagcacgaa tttcgaggag ggcccgaaca aaaactcatc 1440

tcagaagagg atctgaatag cgccgtcgac catcatcatc atcatcattg a 1491

<210> 18
<211> 1182
<212> DNA
<213> Homo sapiens

<400>  18
atggagacag acacactcct gctatgggta ctgctgctct gggttccagg ttccactggt      60

gacgcggccc agccggcccc ggtcatgagg ctgttccctt gcttcctgca gctcctggcc     120

gggctggcgc tgcctgctgt gcccccccag cagtgggcct tgtctgctgg gaacggctcg     180

tcagaggtgg aagtggtacc cttccaggaa gtgtggggcc gcagctactg ccgggcgctg     240

gagaggctgg tggacgtcgt gtccgagtac cccagcgagg tggagcacat gttcagccca     300

tcctgtgtct ccctgctgcg ctgcaccggc tgctgcggcg atgagaatct gcactgtgtg     360

ccggtggaga cggccaatgt caccatgcag ctcctaaaga tccgttctgg ggaccggccc     420

tcctacgtgg agctgacgtt ctctcagcac gttcgctgcg aatgccggcc tctgcgggag     480

aagatgaagc cggaaaggtg cggcgatgct gttccccgga gggcggccgc actcgagtct     540

agaatggaca aagactgcga aatgaagcgc accaccctgg atagccctct gggcaagctg     600

gaactgtctg ggtgcgaaca gggcctgcac gagatcaagc tgctgggcaa aggaacatct     660

gccgccgacg ccgtggaagt gcctgcccca gccgccgtgc tgggcggacc agagccactg     720

atgcaggcca ccgcctggct caacgcctac tttcaccagc ctgaggccat cgaggagttc     780

cctgtgccag ccctgcacca cccagtgttc cagcaggaga gctttacccg ccaggtgctg     840

tggaaactgc tgaaagtggt gaagttcgga gaggtcatca gctaccagca gctggcggcc     900

ctggcgggca atcccgccgc caccgccgcc gtgaaaaccg ccctgagcgg aaatcccgtg     960

cccattctga tcccctgcca ccgggtggtg tctagctctg gcgccgtggg gggctacgag    1020

ggcgggctcg ccgtgaaaga gtggctgctg gcccacgagg gccacagact gggcaagcct    1080

gggctgggcg ctgagcacga atttcgagga gggcccgaac aaaaactcat ctcagaagag    1140

gatctgaata gcgccgtcga ccatcatcat catcatcatt ga                       1182


<210>  19
<211>  1194
<212>  DNA
<213>  Homo sapiens

<400>  19
atggagacag acacactcct gctatgggta ctgctgctct gggttccagg ttccactggt      60

gacgcggccc agccggccta caggatgcaa ctcctgtctt gcattgcact aagtcttgca     120

cttgtcacaa acagtgcacc tacttcaagt tctacaaaga aaacacagct acaactggag     180

catttactgc tggatttaca gatgattttg aatggaatta ataattacaa gaatcccaaa     240

ctcaccagga tgctcacatt taagttttac atgcccaaga aggccacaga actgaaacat     300

cttcagtgtc tagaagaaga actcaaacct ctggaggaag tgctaaattt agctcaaagc     360

aaaaactttc acttaagacc cagggactta atcagcaata tcaacgtaat agttctggaa     420

ctaaagggat ctgaaacaac attcatgtgt gaatatgctg atgagacagc aaccattgta     480

gaatttctga acagatggat taccttttgt caaagcatca tctcaacact gactgcggcc 540

gcactcgagt ctagaatgga caaagactgc gaaatgaagc gcaccaccct ggatagccct 600

ctgggcaagc tggaactgtc tgggtgcgaa cagggcctgc acgagatcaa gctgctgggc 660

aaaggaacat ctgccgccga cgccgtggaa gtgcctgccc cagccgccgt gctgggcgga 720

ccagagccac tgatgcaggc caccgcctgg ctcaacgcct actttcacca gcctgaggcc 780

atcgaggagt tccctgtgcc agccctgcac cacccagtgt tccagcagga gagctttacc 840

cgccaggtgc tgtggaaact gctgaaagtg gtgaagttcg gagaggtcat cagctaccag 900

cagctggcgg ccctggcggg caatcccgcc gccaccgccg ccgtgaaaac cgccctgagc 960

ggaaatcccg tgcccattct gatcccctgc caccgggtgg tgtctagctc tggcgccgtg 1020

gggggctacg agggcgggct cgccgtgaaa gagtggctgc tggcccacga gggccacaga 1080

ctgggcaagc ctgggctggg cgctgagcac gaatttcgag gagggcccga acaaaaactc 1140

atctcagaag aggatctgaa tagcgccgtc gaccatcatc atcatcatca ttga 1194

<210> 20
<211> 1518
<212> DNA
<213> Homo sapiens

<400> 20
atggagacag acacactcct gctatgggta ctgctgctct gggttccagg ttccactggt 60

gacgcggccc agccggccat cgaaacatac aaccaaactt ctccccgatc tgcggccact 120

ggactgccca tcagcatgaa aatttttatg tatttactta ctgtttttct tatcacccag 180

atgattgggt cagcactttt tgctgtgtat cttcatagaa ggttggacaa gatagaagat 240

gaaaggaatc ttcatgaaga ttttgtattc atgaaaacga tacagagatg caacacagga 300

gaaagatcct tatccttact gaactgtgag gagattaaaa gccagtttga aggctttgtg 360

aaggatataa tgttaaacaa agaggagacg aagaaagaaa acagctttga aatgcaaaaa 420

ggtgatcaga atcctcaaat tgcggcacat gtcataagtg aggccagcag taaaacaaca 480

tctgtgttac agtgggctga aaaaggatac tacaccatga gcaacaactt ggtaaccctg 540

gaaaatggga aacagctgac cgttaaaaga caaggactct attatatcta tgcccaagtc 600

accttctgtt ccaatcggga agcttcgagt caagctccat ttatagccag cctctgccta 660

aagtcccccg gtagattcga gagaatctta ctcagagctg caaataccca cagttccgcc 720

aaaccttgcg ggcaacaatc cattcacttg ggaggagtat ttgaattgca accaggtgct 780

tcggtgtttg tcaatgtgac tgatccaagc caagtgagcc atggcactgg cttcacgtcc 840

tttggcttac tcaaactcgc ggccgcactc gagtctagaa tggacaaaga ctgcgaaatg 900

aagcgcacca ccctggatag ccctctgggc aagctggaac tgtctgggtg cgaacagggc 960

ctgcacgaga tcaagctgct gggcaaagga acatctgccg ccgacgccgt ggaagtgcct 1020

gccccagccg ccgtgctggg cggaccagag ccactgatgc aggccaccgc ctggctcaac 1080

gcctactttc accagcctga ggccatcgag gagttccctg tgccagccct gcaccaccca 1140

gtgttccagc aggagagctt tacccgccag gtgctgtgga aactgctgaa agtggtgaag 1200

ttcggagagg tcatcagcta ccagcagctg gcggccctgg cgggcaatcc cgccgccacc 1260

gccgccgtga aaaccgccct gagcggaaat cccgtgccca ttctgatccc ctgccaccgg 1320

gtggtgtcta gctctggcgc cgtggggggc tacgagggcg ggctcgccgt gaaagagtgg 1380

ctgctggccc acgagggcca cagactgggc aagcctgggc tgggcgctga gcacgaattt 1440

cgaggagggc ccgaacaaaa actcatctca gaagaggatc tgaatagcgc cgtcgaccat 1500

catcatcatc atcattga 1518

<210> 21
<211> 1308
<212> DNA
<213> Homo sapiens

<400> 21
atggagacag acacactcct gctatgggta ctgctgctct gggttccagg ttccactggt 60

gacgcggccc agccggccaa ctttctgctg tcttgggtgc attggagcct cgccttgctg 120

ctctacctcc accatgccaa gtggtcccag gctgcaccca tggcagaagg aggagggcag 180

aatcatcacg aagtggtgaa gttcatggat gtctatcagc gcagctactg ccatccaatc 240

gagaccctgg tggacatctt ccaggagtac cctgatgaga tcgagtacat cttcaagcca 300

tcctgtgtgc ccctgatgcg atgcgggggc tgctgcaatg acgagggcct ggagtgtgtg 360

cccactgagg agtccaacat caccatgcag attatgcgga tcaaacctca ccaaggccag 420

cacataggag agatgagctt cctacagcac aacaaatgtg aatgcagacc aaagaaagat 480

agagcaagac aagaaaatcc ctgtgggcct tgctcagagc ggagaaagca tttgtttgta 540

caagatccgc agacgtgtaa atgttcctgc aaaaacacag actcgcgttg caaggcgagg 600

cagcttgagt taaacgaacg tacttgcaga tgtgacaagc cgaggcgggc ggccgcactc 660

gagtctagaa tggacaaaga ctgcgaaatg aagcgcacca ccctggatag ccctctgggc 720

aagctggaac tgtctgggtg cgaacagggc ctgcacgaga tcaagctgct gggcaaagga 780

acatctgccg ccgacgccgt ggaagtgcct gccccagccg ccgtgctggg cggaccagag 840

ccactgatgc aggccaccgc ctggctcaac gcctactttc accagcctga ggccatcgag 900

gagttccctg tgccagccct gcaccaccca gtgttccagc aggagagctt tacccgccag 960

gtgctgtgga aactgctgaa agtggtgaag ttcggagagg tcatcagcta ccagcagctg 1020

gcggccctgg cgggcaatcc cgccgccacc gccgccgtga aaaccgccct gagcggaaat 1080

cccgtgccca ttctgatccc ctgccaccgg gtggtgtcta gctctggcgc cgtggggggc 1140

tacgagggcg ggctcgccgt gaaagagtgg ctgctggccc acgagggcca cagactgggc 1200

aagcctgggc tgggcgctga gcacgaattt cgaggagggc ccgaacaaaa actcatctca 1260

gaagaggatc tgaatagcgc cgtcgaccat catcatcatc atcattga 1308

<210> 22
<211> 1542
<212> DNA
<213> Homo sapiens

<400> 22
atggagacag acacactcct gctatgggta ctgctgctct gggttccagg ttccactggt 60

gacgcggccc agccggccgc agaagtacct gagctcgcca gtgaaatgat ggcttattac 120

agtggcaatg aggatgactt gttctttgaa gctgatggcc ctaaacagat gaagtgctcc 180

ttccaggacc tggacctctg ccctctggat ggcggcatcc agctacgaat ctccgaccac 240

cactacagca agggcttcag gcaggccgcg tcagttgttg tggccatgga caagctgagg 300

aagatgctgg ttccctgccc acagaccttc caggagaatg acctgagcac cttctttccc 360

ttcatctttg aagaagaacc tatcttcttc gacacatggg ataacgaggc ttatgtgcac 420

gatgcacctg tacgatcact gaactgcacg ctccgggact cacagcaaaa aagcttggtg 480

atgtctggtc catatgaact gaaagctctc cacctccagg gacaggatat ggagcaacaa 540

gtggtgttct ccatgtcctt tgtacaagga gaagaaagta atgacaaaat acctgtggcc 600

ttgggcctca aggaaaagaa tctgtacctg tcctgcgtgt tgaaagatga taagcccact 660

ctacagctgg agagtgtaga tcccaaaaat tacccaaaga agaagatgga aaagcgattt 720

gtcttcaaca agatagaaat caataacaag ctggaatttg agtctgccca gttccccaac 780

tggtacatca gcacctctca agcagaaaac atgcccgtct tcctgggagg gaccaaaggc 840

ggccaggata taactgactt caccatgcaa tttgtgtctt ccgcggccgc actcgagtct 900

agaatggaca aagactgcga aatgaagcgc accaccctgg atagccctct gggcaagctg 960

gaactgtctg ggtgcgaaca gggcctgcac gagatcaagc tgctgggcaa aggaacatct 1020

gccgccgacg ccgtggaagt gcctgcccca gccgccgtgc tgggcggacc agagccactg 1080

atgcaggcca ccgcctggct caacgcctac tttcaccagc ctgaggccat cgaggagttc 1140

cctgtgccag ccctgcacca cccagtgttc cagcaggaga gctttacccg ccaggtgctg 1200

tggaaactgc tgaaagtggt gaagttcgga gaggtcatca gctaccagca gctggcggcc 1260

ctggcgggca atcccgccgc caccgccgcc gtgaaaaccg ccctgagcgg aaatcccgtg 1320

cccattctga tcccctgcca ccgggtggtg tctagctctg gcgccgtggg gggctacgag 1380

ggcgggctcg ccgtgaaaga gtggctgctg gcccacgagg gccacagact gggcaagcct 1440

gggctgggcg ctgagcacga atttcgagga gggcccgaac aaaaactcat ctcagaagag 1500

gatctgaata gcgccgtcga ccatcatcat catcatcatt ga 1542

<210> 23

<211>  1191
<212>  DNA
<213>  Homo sapiens

<400>  23
atggagacag acacactcct gctatgggta ctgctgctct gggttccagg ttccactggt      60

gacgcggccc agccggccag ccgcctgccc gtcctgctcc tgctccaact cctggtccgc     120

cccggactcc aagctcccat gacccagaca acgtccttga agacaagctg ggttaactgc     180

tctaacatga tcgatgaaat tataacacac ttaaagcagc cacctttgcc tttgctggac     240

ttcaacagcc tcaatgggga agaccaagac attctgatgg aaaataacct tcgaaggcca     300

aacctggagg cattcaacag ggctgtcaag agtttacaga acgcatcagc aattgagagc     360

attcttaaaa atctcctgcc atgtctgccc ctggccacgg ccgcacccac gcgacatcca     420

atccatatca aggacggtga ctggaatgaa ttccggagga aactgacgtt ctatctgaaa     480

acccttgaga atgcgcaggc tcaacagacg actttgagcc tcgcgatctt tgcggccgca     540

ctcgagtcta gaatggacaa agactgcgaa atgaagcgca ccaccctgga tagccctctg     600

ggcaagctgg aactgtctgg gtgcgaacag ggcctgcacg agatcaagct gctgggcaaa     660

ggaacatctg ccgccgacgc cgtggaagtg cctgccccag ccgccgtgct gggcggacca     720

gagccactga tgcaggccac cgcctggctc aacgcctact ttcaccagcc tgaggccatc     780

gaggagttcc ctgtgccagc cctgcaccac ccagtgttcc agcaggagag ctttacccgc     840

caggtgctgt ggaaactgct gaaagtggtg aagttcggag aggtcatcag ctaccagcag     900

ctggcggccc tggcgggcaa tcccgccgcc accgccgccg tgaaaaccgc cctgagcgga     960

aatcccgtgc ccattctgat cccctgccac cgggtggtgt ctagctctgg cgccgtgggg    1020

ggctacgagg gcgggctcgc cgtgaaagag tggctgctgg cccacgaggg ccacagactg    1080

ggcaagcctg ggctgggcgc tgagcacgaa tttcgaggag ggcccgaaca aaaactcatc    1140

tcagaagagg atctgaatag cgccgtcgac catcatcatc atcatcattg a             1191


<210>  24
<211>  1194
<212>  DNA
<213>  Homo sapiens

<400>  24
atggagacag acacactcct gctatgggta ctgctgctct gggttccagg ttccactggt      60

gacgcggccc agccggccgg tctcacctcc caactgcttc cccctctgtt cttcctgcta     120

gcatgtgccg gcaactttgt ccacggacac aagtgcgata tcaccttaca ggagatcatc     180

aaaactttga acagcctcac agagcagaag actctgtgca ccgagttgac cgtaacagac     240

atctttgctg cctccaagaa cacaactgag aaggaaacct tctgcagggc tgcgactgtg     300

ctccggcagt tctacagcca ccatgagaag gacactcgct gcctgggtgc gactgcacag     360

cagttccaca ggcacaagca gctgatccga ttcctgaaac ggctcgacag gaacctctgg     420

ggcctggcgg gcttgaattc ctgtcctgtg aaggaagcca accagagtac gttggaaaac 480

ttcttggaaa ggctaaagac gatcatgaga gagaaatatt caaagtgttc gagcgcggcc 540

gcactcgagt ctagaatgga caaagactgc gaaatgaagc gcaccaccct ggatagccct 600

ctgggcaagc tggaactgtc tgggtgcgaa cagggcctgc acgagatcaa gctgctgggc 660

aaaggaacat ctgccgccga cgccgtggaa gtgcctgccc cagccgccgt gctgggcgga 720

ccagagccac tgatgcaggc caccgcctgg ctcaacgcct actttcacca gcctgaggcc 780

atcgaggagt tccctgtgcc agccctgcac cacccagtgt tccagcagga gagctttacc 840

cgccaggtgc tgtggaaact gctgaaagtg gtgaagttcg gagaggtcat cagctaccag 900

cagctggcgg ccctggcggg caatcccgcc gccaccgccg ccgtgaaaac cgccctgagc 960

ggaaatcccg tgcccattct gatcccctgc caccgggtgg tgtctagctc tggcgccgtg 1020

gggggctacg agggcgggct cgccgtgaaa gagtggctgc tggcccacga gggccacaga 1080

ctgggcaagc ctgggctggg cgctgagcac gaatttcgag gagggcccga acaaaaactc 1140

atctcagaag aggatctgaa tagcgccgtc gaccatcatc atcatcatca ttga 1194

<210> 25
<211> 1080
<212> DNA
<213> Homo sapiens

<400> 25
atggagacag acacactcct gctatgggta ctgctgctct gggttccagg ttccactggt 60

gacgcggccc agccggcccc cacagaaatt cccacaagtg cattggtgaa agagaccttg 120

gcactgcttt ctactcatcg aactctgctg atagccaatg agactctgag gattcctgtt 180

cctgtacata aaaatcacca actgtgcact gaagaaatct ttcagggaat aggcacactg 240

gagagtcaaa ctgtgcaagg gggtactgtg gaaagactat tcaaaaactt gtccttaata 300

aagaaataca ttgacggcca aaaaaaaaag tgtggagaag aaagacggag agtaaaccaa 360

ttcctagact acctgcaaga gtttcttggt gtaatgaaca ccgagtggat aatagaaagt 420

gcggccgcac tcgagtctag aatggacaaa gactgcgaaa tgaagcgcac caccctggat 480

agccctctgg gcaagctgga actgtctggg tgcgaacagg gcctgcacga gatcaagctg 540

ctgggcaaag gaacatctgc cgccgacgcc gtggaagtgc ctgccccagc cgccgtgctg 600

ggcggaccag agccactgat gcaggccacc gcctggctca acgcctactt tcaccagcct 660

gaggccatcg aggagttccc tgtgccagcc ctgcaccacc cagtgttcca gcaggagagc 720

tttacccgcc aggtgctgtg gaaactgctg aaagtggtga agttcggaga ggtcatcagc 780

taccagcagc tggcggccct ggcgggcaat cccgccgcca ccgccgccgt gaaaaccgcc 840

ctgagcggaa atcccgtgcc cattctgatc ccctgccacc gggtggtgtc tagctctggc 900

gccgtggggg gctacgaggg cgggctcgcc gtgaaagagt ggctgctggc ccacgagggc 960

cacagactgg gcaagcctgg gctgggcgct gagcacgaat ttcgaggagg gcccgaacaa 1020

aaactcatct cagaagagga tctgaatagc gccgtcgacc atcatcatca tcatcattga 1080

<210> 26
<211> 1371
<212> DNA
<213> Homo sapiens

<400> 26
atggagacag acacactcct gctatgggta ctgctgctct gggttccagg ttccactggt 60

gacgcggccc agccggccaa ctccttctcc acaagcgcct tcggtccagt tgccttctcc 120

ctggggctgc tcctggtgtt gcctgctgcc ttccctgccc cagtaccccc aggagaagat 180

tccaaagatg tagccgcccc acacagacag ccactcacct cttcagaacg aattgacaaa 240

caaattcggt acatcctcga cggcatctca gccctgagaa aggagacatg taacaagagt 300

aacatgtgtg aaagcagcaa agaggcactg gcagaaaaca acctgaacct tccaaagatg 360

gctgaaaaag atggatgctt ccaatctgga ttcaatgagg agacttgcct ggtgaaaatc 420

atcactggtc ttttggagtt tgaggtatac ctagagtacc tccagaacag atttgagagt 480

agtgaggaac aagccagagc tgtgcagatg agtacaaaag tcctgatcca gttcctgcag 540

aaaaaggcaa agaatctaga tgcaataacc acccctgacc caaccacaaa tgccagcctg 600

ctgacgaagc tgcaggcaca gaaccagtgg ctgcaggaca tgacaactca tctcattctg 660

cgcagcttta aggagttcct gcagtccagc ctgagggctc ttcggcaaat ggcggccgca 720

ctcgagtcta gaatggacaa agactgcgaa atgaagcgca ccaccctgga tagccctctg 780

ggcaagctgg aactgtctgg gtgcgaacag ggcctgcacg agatcaagct gctgggcaaa 840

ggaacatctg ccgccgacgc cgtggaagtg cctgccccag ccgccgtgct gggcggacca 900

gagccactga tgcaggccac cgcctggctc aacgcctact ttcaccagcc tgaggccatc 960

gaggagttcc ctgtgccagc cctgcaccac ccagtgttcc agcaggagag ctttacccgc 1020

caggtgctgt ggaaactgct gaaagtggtg aagttcggag aggtcatcag ctaccagcag 1080

ctggcggccc tggcgggcaa tcccgccgcc accgccgccg tgaaaaccgc cctgagcgga 1140

aatcccgtgc ccattctgat cccctgccac cgggtggtgt ctagctctgg cgccgtgggg 1200

ggctacgagg gcgggctcgc cgtgaaagag tggctgctgg cccacgaggg ccacagactg 1260

ggcaagcctg ggctgggcgc tgagcacgaa tttcgaggag ggcccgaaca aaaactcatc 1320

tcagaagagg atctgaatag cgccgtcgac catcatcatc atcatcattg a 1371

<210> 27
<211> 1194
<212> DNA
<213> Homo sapiens

<400> 27

atggagacag acacactcct gctatgggta ctgctgctct gggttccagg ttccactggt 60

gacgcggccc agccggccga ttgtgatatt gaaggtaaag atggcaaaca atatgagagt 120

gttctaatgg tcagcatcga tcaattattg gacagcatga aagaaattgg tagcaattgc 180

ctgaataatg aatttaactt ttttaaaaga catatctgtg atgctaataa ggaaggtatg 240

tttttattcc gtgctgctcg caagttgagg caatttctta aaatgaatag cactggtgat 300

tttgatctcc acttattaaa agtttcagaa ggcacaacaa tactgttgaa ctgcactggc 360

caggttaaag gaagaaaacc agctgccctg ggtgaagccc aaccaacaaa gagtttggaa 420

gaaaataaat ctttaaagga acagaaaaaa ctgaatgact tgtgtttcct aaagagacta 480

ttacaagaga taaaaacttg ttggaataaa attttgatgg gcactaaaga acacgcggcc 540

gcactcgagt ctagaatgga caaagactgc gaaatgaagc gcaccaccct ggatagccct 600

ctgggcaagc tggaactgtc tgggtgcgaa cagggcctgc acgagatcaa gctgctgggc 660

aaaggaacat ctgccgccga cgccgtggaa gtgcctgccc cagccgccgt gctgggcgga 720

ccagagccac tgatgcaggc caccgcctgg ctcaacgcct actttcacca gcctgaggcc 780

atcgaggagt tccctgtgcc agccctgcac cacccagtgt tccagcagga gagctttacc 840

cgccaggtgc tgtggaaact gctgaaagtg gtgaagttcg gagaggtcat cagctaccag 900

cagctggcgg ccctggcggg caatcccgcc gccaccgccg ccgtgaaaac cgccctgagc 960

ggaaatcccg tgcccattct gatcccctgc caccgggtgg tgtctagctc tggcgccgtg 1020

gggggctacg agggcgggct cgccgtgaaa gagtggctgc tggcccacga gggccacaga 1080

ctgggcaagc ctgggctggg cgctgagcac gaatttcgag gagggcccga acaaaaactc 1140

atctcagaag aggatctgaa tagcgccgtc gaccatcatc atcatcatca ttga 1194

<210> 28
<211> 975
<212> DNA
<213> Homo sapiens

<400> 28
atggagacag acacactcct gctatgggta ctgctgctct gggttccagg ttccactggt 60

gacgcggccc agccggccga aggtgcagtt ttgccaagga gtgctaaaga acttagatgt 120

cagtgcataa agacatactc caaacctttc caccccaaat ttatcaaaga actgagagtg 180

attgagagtg gaccacactg cgccaacaca gaaattattg taaagctttc tgatggaaga 240

gagctctgtc tggaccccaa ggaaaactgg gtgcagaggg ttgtggagaa gtttttgaag 300

agggctgaga attcagcggc cgcactcgag tctagaatgg acaaagactg cgaaatgaag 360

cgcaccaccc tggatagccc tctgggcaag ctggaactgt ctgggtgcga acagggcctg 420

cacgagatca agctgctggg caaaggaaca tctgccgccg acgccgtgga agtgcctgcc 480

ccagccgccg tgctgggcgg accagagcca ctgatgcagg ccaccgcctg gctcaacgcc 540

tactttcacc agcctgaggc catcgaggag ttccctgtgc cagccctgca ccacccagtg 600

ttccagcagg agagctttac ccgccaggtg ctgtggaaac tgctgaaagt ggtgaagttc 660

ggagaggtca tcagctacca gcagctggcg gccctggcgg gcaatcccgc cgccaccgcc 720

gccgtgaaaa ccgccctgag cggaaatccc gtgcccattc tgatcccctg ccaccgggtg 780

gtgtctagct ctggcgccgt ggggggctac gagggcgggc tcgccgtgaa agagtggctg 840

ctggcccacg agggccacag actgggcaag cctgggctgg gcgctgagca cgaatttcga 900

ggagggcccg aacaaaaact catctcagaa gaggatctga atagcgccgt cgaccatcat 960

catcatcatc attga 975

<210> 29
<211> 1115
<212> DNA
<213> Homo sapiens

<400> 29
atggagacag acacactcct gctatgggta ctgctgctct gggttccagg ttccactggt 60

gacgcggccc agccggccag gggtgtccaa ccttggcggg gatcctggac atcaacttcc 120

tcatcaacaa gatgcaggaa gatccagctt ccaagtgcca ctgcagtgct aatgtgacca 180

gttgtctctg tttgggcatt ccctctgaca actgcaccag accatgcttc agtgagagac 240

tgtctcagat gaccaatacc accatgcaaa caagataccc actgattttc agtcgggtga 300

aaaaatcagt tgaagtacta aagaacaaca agtgtccata tttttcctgt gaacagccat 360

gcaaccaaac cacggcaggc aacgcgctga catttctgaa gagtcttctg gaaattttcc 420

agaaagaaaa gatgagaggg atgagaggca agatagcggc cgcactcgag tctagaatgg 480

acaaagactg cgaaatgaag cgcaccaccc tggatagccc tctgggcaag ctggaactgt 540

ctgggtgcga acagggcctg cacgagatca agctgctggg caaaggaaca tctgccgccg 600

acgccgtgga agtgcctgcc ccagccgccg tgctgggcgg accagagcca ctgatgcagg 660

ccaccgcctg gctcaacgcc tactttcacc agcctgaggc catcgaggag ttccctgtgc 720

cagccctgca ccacccagtg ttccagcagg agagctttac ccgccaggtg ctgtggaaac 780

tgctgaaagt ggtgaagttc ggagaggtca tcagctacca gcagctggcg gccctggcgg 840

gcaatcccgc cgccaccgcc gccgtgaaaa ccgccctgag cggaaatccc gtgcccattc 900

tgatcccctg ccaccgggtg gtgtctagct ctggcgccgt ggggggctac gagggcgggc 960

tcgccgtgaa agagtggctg ctggcccacg agggccacag actgggcaag cctgggctgg 1020

gcgctgagca cgaatttcga ggagggcccg aacaaaaact catctcagaa gaggatctga 1080

atagcgccgt cgaccatcat catcatcatc attga 1115

<210> 30
<211> 1218
<212> DNA

<213>  Homo sapiens

<400>  30
atggagacag acacactcct gctatgggta ctgctgctct gggttccagg ttccactggt      60

gacgcggccc agccggccag cccaggccag ggcacccagt ctgagaacag ctgcacccac     120

ttcccaggca acctgcctaa catgcttcga gatctccgag atgccttcag cagagtgaag     180

actttctttc aaatgaagga tcagctggac aacttgttgt taaaggagtc cttgctggag     240

gactttaagg gttacctggg ttgccaagcc ttgtctgaga tgatccagtt ttacctggag     300

gaggtgatgc cccaagctga gaaccaagac ccagacatca aggcgcatgt gaactccctg     360

ggggagaacc tgaagaccct caggctgagg ctacggcgct gtcatcgatt tcttccctgt     420

gaaaacaaga gcaaggccgt ggagcaggtg aagaatgcct ttaataagct ccaagagaaa     480

ggcatctaca aagccatgag tgagtttgac atcttcatca actacataga agcctacatg     540

acaatgaaga tacgaaacgc ggccgcactc gagtctagaa tggacaaaga ctgcgaaatg     600

aagcgcacca ccctggatag ccctctgggc aagctggaac tgtctgggtg cgaacagggc     660

ctgcacgaga tcaagctgct gggcaaagga acatctgccg ccgacgccgt ggaagtgcct     720

gccccagccg ccgtgctggg cggaccagag ccactgatgc aggccaccgc ctggctcaac     780

gcctactttc accagcctga ggccatcgag gagttccctg tgccagccct gcaccaccca     840

gtgttccagc aggagagctt tacccgccag gtgctgtgga aactgctgaa agtggtgaag     900

ttcggagagg tcatcagcta ccagcagctg gcggccctgg cgggcaatcc cgccgccacc     960

gccgccgtga aaaccgccct gagcggaaat cccgtgccca ttctgatccc ctgccaccgg    1020

gtggtgtcta gctctggcgc cgtggggggc tacgagggcg ggctcgccgt gaaagagtgg    1080

ctgctggccc acgagggcca cagactgggc aagcctgggc tgggcgctga gcacgaattt    1140

cgaggagggc ccgaacaaaa actcatctca gaagaggatc tgaatagcgc cgtcgaccat    1200

catcatcatc atcattga                                                  1218


<210>  31
<211>  1272
<212>  DNA
<213>  Homo sapiens

<400>  31
atggagacag acacactcct gctatgggta ctgctgctct gggttccagg ttccactggt      60

gacgcggccc agccggcccc tgggccacca cctggccccc ctcgagtttc cccagaccct     120

cgggccgagc tggacagcac cgtgctcctg acccgctctc tcctggcgga cacgcggcag     180

ctggctgcac agctgaggga caaattccca gctgacgggg accacaacct ggattccctg     240

cccaccctgg ccatgagtgc gggggcactg ggagctctac agctcccagg tgtgctgaca     300

aggctgcgag cggacctact gtcctacctg cggcacgtgc agtggctgcg ccgggcaggt     360

ggctcttccc tgaagaccct ggagcccgag ctgggcaccc tgcaggcccg actggaccgg     420

ctgctgcgcc ggctgcagct cctgatgtcc cgcctggccc tgccccagcc acccccggac 480

ccgccggcgc ccccgctggc gcccccctcc tcagcctggg ggggcatcag ggccgcccac 540

gccatcctgg gggggctgca cctgacactt gactgggccg tgaggggact gctgctgctg 600

aagactcggc tggcggccgc actcgagtct agaatggaca aagactgcga aatgaagcgc 660

accaccctgg atagccctct gggcaagctg gaactgtctg ggtgcgaaca gggcctgcac 720

gagatcaagc tgctgggcaa aggaacatct gccgccgacg ccgtggaagt gcctgcccca 780

gccgccgtgc tgggcggacc agagccactg atgcaggcca ccgcctggct caacgcctac 840

tttcaccagc ctgaggccat cgaggagttc cctgtgccag ccctgcacca cccagtgttc 900

cagcaggaga gctttacccg ccaggtgctg tggaaactgc tgaaagtggt gaagttcgga 960

gaggtcatca gctaccagca gctggcggcc ctggcgggca atcccgccgc caccgccgcc 1020

gtgaaaaccg ccctgagcgg aaatcccgtg cccattctga tcccctgcca ccgggtggtg 1080

tctagctctg gcgccgtggg gggctacgag ggcgggctcg ccgtgaaaga gtggctgctg 1140

gcccacgagg gccacagact gggcaagcct gggctgggcg ctgagcacga atttcgagga 1200

gggcccgaac aaaaactcat ctcagaagag gatctgaata gcgccgtcga ccatcatcat 1260

catcatcatt ga 1272

<210> 32
<211> 1329
<212> DNA
<213> Homo sapiens

<400> 32
atggagacag acacactcct gctatgggta ctgctgctct gggttccagg ttccactggt 60

gacgcggccc agccggccag aaacctcccc gtggccactc cagacccagg aatgttccca 120

tgccttcacc actcccaaaa cctgctgagg gccgtcagca acatgctcca gaaggccaga 180

caaactctag aattttaccc ttgcacttct gaagagattg atcatgaaga tatcacaaaa 240

gataaaacca gcacagtgga ggcctgttta ccattggaat taaccaagaa tgagagttgc 300

ctaaattcca gagagacctc tttcataact aatgggagtt gcctggcctc cagaaagacc 360

tcttttatga tggccctgtg ccttagtagt atttatgaag acttgaagat gtaccaggtg 420

gagttcaaga ccatgaatgc aaagcttctg atggatccta agaggcagat ctttctagat 480

caaaacatgc tggcagttat tgatgagctg atgcaggccc tgaatttcaa cagtgagact 540

gtgccacaaa aatcctccct tgaagaaccg gatttttata aaactaaaat caagctctgc 600

atacttcttc atgctttcag aattcgggca gtgactattg atagagtgat gagctatctg 660

aatgcttccg cggccgcact cgagtctaga atggacaaag actgcgaaat gaagcgcacc 720

accctggata gccctctggg caagctggaa ctgtctgggt gcgaacaggg cctgcacgag 780

atcaagctgc tgggcaaagg aacatctgcc gccgacgccg tggaagtgcc tgccccagcc 840

gccgtgctgg gcggaccaga gccactgatg caggccaccg cctggctcaa cgcctacttt 900

caccagcctg aggccatcga ggagttccct gtgccagccc tgcaccaccc agtgttccag 960

caggagagct ttacccgcca ggtgctgtgg aaactgctga aagtggtgaa gttcggagag 1020

gtcatcagct accagcagct ggcggccctg gcgggcaatc ccgccgccac cgccgccgtg 1080

aaaaccgccc tgagcggaaa tcccgtgccc attctgatcc cctgccaccg ggtggtgtct 1140

agctctggcg ccgtgggggg ctacgagggc gggctcgccg tgaaagagtg gctgctggcc 1200

cacgagggcc acagactggg caagcctggg ctgggcgctg agcacgaatt tcgaggaggg 1260

cccgaacaaa aactcatctc agaagaggat ctgaatagcg ccgtcgacca tcatcatcat 1320

catcattga 1329

<210> 33
<211> 1074
<212> DNA
<213> Homo sapiens

<400> 33
atggagacag acacactcct gctatgggta ctgctgctct gggttccagg ttccactggt 60

gacgcggccc agccggccgg ccctgtgcct ccctctacag ccctcaggga gctcattgag 120

gagctggtca acatcaccca gaaccagaag gctccgctct gcaatggcag catggtatgg 180

agcatcaacc tgacagctgg catgtactgt gcagccctgg aatccctgat caacgtgtca 240

ggctgcagtg ccatcgagaa gacccagagg atgctgagcg gattctgccc gcacaaggtc 300

tcagctgggc agttttccag cttgcatgtc cgagacacca aaatcgaggt ggcccagttt 360

gtaaaggacc tgctcttaca tttaaagaaa ctttttcgcg agggacagtt caacgcggcc 420

gcactcgagt ctagaatgga caaagactgc gaaatgaagc gcaccaccct ggatagccct 480

ctgggcaagc tggaactgtc tgggtgcgaa cagggcctgc acgagatcaa gctgctgggc 540

aaaggaacat ctgccgccga cgccgtggaa gtgcctgccc cagccgccgt gctgggcgga 600

ccagagccac tgatgcaggc caccgcctgg ctcaacgcct actttcacca gcctgaggcc 660

atcgaggagt tccctgtgcc agccctgcac cacccagtgt tccagcagga gagctttacc 720

cgccaggtgc tgtggaaact gctgaaagtg gtgaagttcg gagaggtcat cagctaccag 780

cagctggcgg ccctggcggg caatcccgcc gccaccgccg ccgtgaaaac cgccctgagc 840

ggaaatcccg tgcccattct gatcccctgc caccgggtgg tgtctagctc tggcgccgtg 900

gggggctacg agggcgggct cgccgtgaaa gagtggctgc tggcccacga gggccacaga 960

ctgggcaagc ctgggctggg cgctgagcac gaatttcgag gagggcccga acaaaaactc 1020

atctcagaag aggatctgaa tagcgccgtc gaccatcatc atcatcatca ttga 1074

<210> 34
<211> 1080

<212>  DNA
<213>  Homo sapiens

<400>  34
atggagacag acacactcct gctatgggta ctgctgctct gggttccagg ttccactggt      60

gacgcggccc agccggccaa ctgggtgaat gtaataagtg atttgaaaaa aattgaagat     120

cttattcaat ctatgcatat tgatgctact ttatatacgg aaagtgatgt tcaccccagt     180

tgcaaagtaa cagcaatgaa gtgctttctc ttggagttac aagttatttc acttgagtcc     240

ggagatgcaa gtattcatga tacagtagaa aatctgatca tcctagcaaa caacagtttg     300

tcttctaatg ggaatgtaac agaatctgga tgcaaagaat gtgaggaact ggagaaaaaa     360

aatattaaag aatttttgca gagttttgta catattgtcc aaatgttcat caacacttct     420

gcggccgcac tcgagtctag aatggacaaa gactgcgaaa tgaagcgcac caccctggat     480

agccctctgg gcaagctgga actgtctggg tgcgaacagg gcctgcacga gatcaagctg     540

ctgggcaaag gaacatctgc cgccgacgcc gtggaagtgc ctgccccagc cgccgtgctg     600

ggcggaccag agccactgat gcaggccacc gcctggctca acgcctactt tcaccagcct     660

gaggccatcg aggagttccc tgtgccagcc ctgcaccacc cagtgttcca gcaggagagc     720

tttacccgcc aggtgctgtg gaaactgctg aaagtggtga agttcggaga ggtcatcagc     780

taccagcagc tggcggccct ggcgggcaat cccgccgcca ccgccgccgt gaaaaccgcc     840

ctgagcggaa atcccgtgcc cattctgatc ccctgccacc gggtggtgtc tagctctggc     900

gccgtggggg gctacgaggg cgggctcgcc gtgaaagagt ggctgctggc ccacgagggc     960

cacagactgg gcaagcctgg gctgggcgct gagcacgaat ttcgaggagg gcccgaacaa    1020

aaactcatct cagaagagga tctgaatagc gccgtcgacc atcatcatca tcatcattga    1080


<210>  35
<211>  2097
<212>  DNA
<213>  Homo sapiens

<400>  35
atggagacag acacactcct gctatgggta ctgctgctct gggttccagg ttccactggt      60

gacgcggccc agccggccga gtcgcacagc cgcgctggaa agagcagaaa atctgcaaaa     120

tttcggtcca tctccaggtc cctgatgctc tgtaatgcta agaccagtga tgatggctct     180

agccctgatg agaaatatcc tgatcccttt gagatttcct tggcccaggg caaggaggga     240

attttccact catctgtgca gctggcagac acatcggagg ctgggcccag cagtgttcct     300

gatctagcac tggcctcgga ggctgctcaa ctccaagcag ctgggaatga tcgaggcaag     360

acctgtagga ggatattctt catgaaggaa tcttccacag cttcctctcg agaaaagcct     420

ggaaaactag aagcacaaag tagtaacttc ctgtttccta aagcctgcca ccaaagggca     480

cgcagcaact caaccagtgt taatccctat tgcacaagag aaattgattt tccaatgacc     540

aagaaatctg cagcgcccac ggacaggcag ccttactctc tctgcagtaa caggaagtcc 600

ctctctcaac aattggactg tccagcagga aaggctgcgg gaacttcgag accaacacgg 660

tccctgagca cagctcagct cgtgcagcca tctgggggcc tccaggcttc agtcatctcc 720

aacatcgtgc tgatgaaggg ccaggctaag ggtctgggct tcagcatcgt tgggggaaaa 780

gacagcattt atggccccat tgggatttac gtcaaaacca tttttgcagg gggagcagca 840

gcagccgatg gaaggctaca ggaaggtgat gaaattctgg agctcaatgg tgaatcaatg 900

gctggactaa cacatcagga tgctttgcag aagttcaagc aagccaaaaa ggggctcctc 960

accctcaccg tgagaacccg cctgacggcg cctccttccc tgtgcagcca cctgtctccc 1020

ccactgtgcc gctccctgag ctccagcact tgtatcacca aggacagcag ctccttcgcc 1080

ttggaaagcc cctcggctcc catcagcacc gccaagccca attacagaat catggtggag 1140

gtttctctgc agaaagaggc cggcgtgggc ctgggcatcg gcctgtgcag cgttccctac 1200

ttccaatgca tctctggcat tttcgtccac acgctgtcac caggatccgt ggcgcacctg 1260

gacggacgtc tccggtgtgg ggacgagatt gtggaaatca gtgattcccc tgtgcactgc 1320

ctgacgctca atgaagtcta cacgatcctg agtcactgtg atcccggtcc agtctccatc 1380

attgttagcc gacatccaga cccacagaag gaagaaatgg aggctcagga ggttaaggcg 1440

gccgcactcg agtctagaat ggacaaagac tgcgaaatga agcgcaccac cctggatagc 1500

cctctgggca agctggaact gtctgggtgc gaacagggcc tgcacgagat caagctgctg 1560

ggcaaaggaa catctgccgc cgacgccgtg gaagtgcctg ccccagccgc cgtgctgggc 1620

ggaccagagc cactgatgca ggccaccgcc tggctcaacg cctactttca ccagcctgag 1680

gccatcgagg agttccctgt gccagccctg caccacccag tgttccagca ggagagcttt 1740

acccgccagg tgctgtggaa actgctgaaa gtggtgaagt tcggagaggt catcagctac 1800

cagcagctgg cggccctggc gggcaatccc gccgccaccg ccgccgtgaa aaccgccctg 1860

agcggaaatc ccgtgcccat tctgatcccc tgccaccggg tggtgtctag ctctggcgcc 1920

gtggggggct acgagggcgg gctcgccgtg aaagagtggc tgctggccca cgagggccac 1980

agactgggca agcctgggct gggcgctgag cacgaatttc gaggagggcc cgaacaaaaa 2040

ctcatctcag aagaggatct gaatagcgcc gtcgaccatc atcatcatca tcattga 2097

<210> 36
<211> 1203
<212> DNA
<213> Homo sapiens

<400> 36
atggagacag acacactcct gctatgggta ctgctgctct gggttccagg ttccactggt 60

gacgcggccc agccggccat gactcctggg aagacctcat tggtgtcact gctactgctg 120

ctgagcctgg aggccatagt gaaggcagga atcacaatcc cacgaaatcc aggatgccca 180

aattctgagg acaagaactt cccccggact gtgatggtca acctgaacat ccataaccgg 240

aataccaata ccaatcccaa aaggtcctca gattactaca accgatccac ctcaccttgg 300

aatctccacc gcaatgagga ccctgagaga tatccctctg tgatctggga ggcaaagtgc 360

cgccacttgg gctgcatcaa cgctgatggg aacgtggact accacatgaa ctctgtcccc 420

atccagcaag agatcctggt cctgcgcagg gagcctccac actgccccaa ctccttccgg 480

ctggagaaga tactggtgtc cgtgggctgc acctgtgtca ccccgattgt ccaccatgtg 540

gccgcggccg cactcgagtc tagaatggac aaagactgcg aaatgaagcg caccaccctg 600

gatagccctc tgggcaagct ggaactgtct gggtgcgaac agggcctgca cgagatcaag 660

ctgctgggca aaggaacatc tgccgccgac gccgtggaag tgcctgcccc agccgccgtg 720

ctgggcggac cagagccact gatgcaggcc accgcctggc tcaacgccta ctttcaccag 780

cctgaggcca tcgaggagtt ccctgtgcca gccctgcacc acccagtgtt ccagcaggag 840

agctttaccc gccaggtgct gtggaaactg ctgaaagtgg tgaagttcgg agaggtcatc 900

agctaccagc agctggcggc cctggcgggc aatcccgccg ccaccgccgc cgtgaaaacc 960

gccctgagcg gaaatcccgt gcccattctg atcccctgcc accgggtggt gtctagctct 1020

ggcgccgtgg ggggctacga gggcgggctc gccgtgaaag agtggctgct ggcccacgag 1080

ggccacagac tgggcaagcc tgggctgggc gctgagcacg aatttcgagg agggcccgaa 1140

caaaaactca tctcagaaga ggatctgaat agcgccgtcg accatcatca tcatcatcat 1200

tga 1203

<210> 37
<211> 1208
<212> DNA
<213> Homo sapiens

<400> 37
atggagacag acacactcct gctatgggta ctgctgctct gggttccagg ttccactggt 60

gacgcggccc agccggccac tttggcaagc ttgaatctaa attatcagtc ataagaaatt 120

tgaatgacca agttctcttc attgaccaag gaaatcggcc tctatttgaa gatatgactg 180

attctgactg tagagataat gcaccccgga ccatatttat tataagtatg tataaagata 240

gccagcctag aggtatggct gtaactatct ctgtgaagtg tgagaaaatt tcaactctct 300

cctgtgagaa caaaattatt tcctttaagg aaatgaatcc tcctgataac atcaaggata 360

caaaaagtga catcatattc tttcagagaa gtgtcccagg acatgataat aagatgcaat 420

ttgaatcttc atcatacgaa ggatactttc tagcttgtga aaaagagaga gaccttttta 480

aactcatttt gaaaaaagag gatgaattgg gggatagatc tataatgttc actgttcaaa 540

acgaagacgc ggccgcactc gagtctagaa tggacaaaga ctgcgaaatg aagcgcacca 600

ccctggatag ccctctgggc aagctggaac tgtctgggtg cgaacagggc ctgcacgaga 660

tcaagctgct gggcaaagga acatctgccg ccgacgccgt ggaagtgcct gccccagccg 720

ccgtgctggg cggaccagag ccactgatgc aggccaccgc ctggctcaac gcctactttc 780

accagcctga ggccatcgag gagttccctg tgccagccct gcaccaccca gtgttccagc 840

aggagagctt tacccgccag gtgctgtgga aactgctgaa agtggtgaag ttcggagagg 900

tcatcagcta ccagcagctg gcggccctgg cgggcaatcc cgccgccacc gccgccgtga 960

aaaccgccct gagcggaaat cccgtgccca ttctgatccc ctgccaccgg gtggtgtcta 1020

gctctggcgc cgtggggggc tacgagggcg ggctcgccgt gaaagagtgg ctgctggccc 1080

acgagggcca cagactgggc aagcctgggc tgggcgctga gcacgaattt cgaggagggc 1140

ccgaacaaaa actcatctca gaagaggatc tgaatagcgc cgtcgaccat catcatcatc 1200

atcattga 1208

<210> 38
<211> 1215
<212> DNA
<213> Homo sapiens

<400> 38
atggagacag acacactcct gctatgggta ctgctgctct gggttccagg ttccactggt 60

gacgcggccc agccggcctc agtagacaac cacggtctca ggagatgtct gatttccaca 120

gacatgcacc atatagaaga gagtttccaa gaaatcaaaa gagccatcca agctaaggac 180

accttcccaa atgtcactat cctgtccaca ttggagactc tgcagatcat taagccctta 240

gatgtgtgct gcgtgaccaa gaacctcctg gcgttctacg tggacagggt gttcaaggat 300

catcaggagc caaaccccaa aatcttgaga aaaatcagca gcattgccaa ctctttcctc 360

tacatgcaga aaactctgcg gcaatgtcag gaacagaggc agtgtcactg caggcaggaa 420

gccaccaatg ccaccagagt catccatgac aactatgatc agctggaggt ccacgctgct 480

gccattaaat ccctgggaga gctcgacgtc tttctagcct ggattaataa gaatcatgaa 540

gtaatgttct cagctgcggc cgcactcgag tctagaatgg acaaagactg cgaaatgaag 600

cgcaccaccc tggatagccc tctgggcaag ctggaactgt ctgggtgcga acagggcctg 660

cacgagatca agctgctggg caaaggaaca tctgccgccg acgccgtgga agtgcctgcc 720

ccagccgccg tgctgggcgg accagagcca ctgatgcagg ccaccgcctg gctcaacgcc 780

tactttcacc agcctgaggc catcgaggag ttccctgtgc cagccctgca ccacccagtg 840

ttccagcagg agagctttac ccgccaggtg ctgtggaaac tgctgaaagt ggtgaagttc 900

ggagaggtca tcagctacca gcagctggcg gccctggcgg gcaatcccgc cgccaccgcc 960

gccgtgaaaa ccgccctgag cggaaatccc gtgcccattc tgatcccctg ccaccgggtg 1020

gtgtctagct ctggcgccgt ggggggctac gagggcgggc tcgccgtgaa agagtggctg 1080

ctggcccacg agggccacag actgggcaag cctgggctgg gcgctgagca cgaatttcga 1140

ggagggcccg aacaaaaact catctcagaa gaggatctga atagcgccgt cgaccatcat 1200

catcatcatc attga 1215

<210> 39
<211> 1266
<212> DNA
<213> Homo sapiens

<400> 39
atggagacag acacactcct gctatgggta ctgctgctct gggttccagg ttccactggt 60

gacgcggccc agccggccat gaaagcctct agtcttgcct tcagccttct ctctgctgcg 120

ttttatctcc tatggactcc ttccactgga ctgaagacac tcaatttggg aagctgtgtg 180

atcgccacaa accttcagga aatacgaaat ggattttctg agatacgggg cagtgtgcaa 240

gccaaagatg gaaacattga catcagaatc ttaaggagga ctgagtcttt gcaagacaca 300

aagcctgcga atcgatgctg cctcctgcgc catttgctaa gactctatct ggacagggta 360

tttaaaaact accagacccc tgaccattat actctccgga agatcagcag cctcgccaat 420

tcctttctta ccatcaagaa ggacctccgg ctctgtcatg cccacatgac atgccattgt 480

ggggaggaag caatgaagaa atacagccag attctgagtc actttgaaaa gctggaacct 540

caggcagcag ttgtgaaggc tttgggggaa ctagacattc ttctgcaatg gatggaggag 600

acagaagcgg ccgcactcga gtctagaatg gacaaagact gcgaaatgaa gcgcaccacc 660

ctggatagcc ctctgggcaa gctggaactg tctgggtgcg aacagggcct gcacgagatc 720

aagctgctgg gcaaaggaac atctgccgcc gacgccgtgg aagtgcctgc cccagccgcc 780

gtgctgggcg gaccagagcc actgatgcag gccaccgcct ggctcaacgc ctactttcac 840

cagcctgagg ccatcgagga gttccctgtg ccagccctgc accacccagt gttccagcag 900

gagagcttta cccgccaggt gctgtggaaa ctgctgaaag tggtgaagtt cggagaggtc 960

atcagctacc agcagctggc ggccctggcg ggcaatcccg ccgccaccgc cgccgtgaaa 1020

accgccctga gcggaaatcc cgtgcccatt ctgatcccct gccaccgggt ggtgtctagc 1080

tctggcgccg tggggggcta cgagggcggg ctcgccgtga aagagtggct gctggcccac 1140

gagggccaca gactgggcaa gcctgggctg ggcgctgagc acgaatttcg aggagggccc 1200

gaacaaaaac tcatctcaga agaggatctg aatagcgccg tcgaccatca tcatcatcat 1260

cattga 1266

<210> 40
<211> 1221
<212> DNA
<213> Homo sapiens

<400> 40
atggagacag acacactcct gctatgggta ctgctgctct gggttccagg ttccactggt 60

gacgcggccc agccggccag atccagtcct ggcaacatgg agaggattgt catctgtctg 120

atggtcatct tcttggggac actggtccac aaatcaagct cccaaggtca agatcgccac 180

atgattagaa tgcgtcaact tatagatatt gttgatcagc tgaaaaatta tgtgaatgac 240

ttggtccctg aatttctgcc agctccagaa gatgtagaga caaactgtga gtggtcagct 300

ttttcctgct ttcagaaggc ccaactaaag tcagcaaata caggaaacaa tgaaaggata 360

atcaatgtat caattaaaaa gctgaagagg aaaccacctt ccacaaatgc agggagaaga 420

cagaaacaca gactaacatg cccttcatgt gattcttatg agaaaaaacc acccaaagaa 480

ttcctagaaa gattcaaatc acttctccaa aagatgattc atcagcatct gtcctctaga 540

acacacggaa gtgaagattc cgcggccgca ctcgagtcta gaatggacaa agactgcgaa 600

atgaagcgca ccaccctgga tagccctctg ggcaagctgg aactgtctgg gtgcgaacag 660

ggcctgcacg agatcaagct gctgggcaaa ggaacatctg ccgccgacgc cgtggaagtg 720

cctgccccag ccgccgtgct gggcggacca gagccactga tgcaggccac cgcctggctc 780

aacgcctact ttcaccagcc tgaggccatc gaggagttcc ctgtgccagc cctgcaccac 840

ccagtgttcc agcaggagag ctttacccgc caggtgctgt ggaaactgct gaaagtggtg 900

aagttcggag aggtcatcag ctaccagcag ctggcggccc tggcgggcaa tcccgccgcc 960

accgccgccg tgaaaaccgc cctgagcgga aatcccgtgc ccattctgat cccctgccac 1020

cgggtggtgt ctagctctgg cgccgtgggg ggctacgagg gcgggctcgc cgtgaaagag 1080

tggctgctgg cccacgaggg ccacagactg ggcaagcctg ggctgggcgc tgagcacgaa 1140

tttcgaggag ggcccgaaca aaaactcatc tcagaagagg atctgaatag cgccgtcgac 1200

catcatcatc atcatcattg a 1221

<210> 41
<211> 1272
<212> DNA
<213> Homo sapiens

<400> 41
atggagacag acacactcct gctatgggta ctgctgctct gggttccagg ttccactggt 60

gacgcggccc agccggccgc cgccctgcag aaatctgtga gctctttcct tatggggacc 120

ctggccacca gctgcctcct tctcttggcc ctcttggtac agggaggagc agctgcgccc 180

atcagctccc actgcaggct tgacaagtcc aacttccagc agccctatat caccaaccgc 240

accttcatgc tggctaagga ggctagcttg gctgataaca acacagacgt tcgtctcatt 300

ggggagaaac tgttccacgg agtcagtatg agtgagcgct gctatctgat gaagcaggtg 360

ctgaacttca cccttgaaga agtgctgttc cctcaatctg ataggttcca gccttatatg 420

caggaggtgg tgcccttcct ggccaggctc agcaacaggc taagcacatg tcatattgaa 480

ggtgatgacc tgcatatcca gaggaatgtg caaaagctga aggacacagt gaaaaagctt 540

ggagagagtg gagagatcaa agcaattgga gaactggatt tgctgtttat gtctctgaga 600

aatgcctgca ttgcggccgc actcgagtct agaatggaca aagactgcga aatgaagcgc 660

accaccctgg atagccctct gggcaagctg gaactgtctg ggtgcgaaca gggcctgcac 720

gagatcaagc tgctgggcaa aggaacatct gccgccgacg ccgtggaagt gcctgcccca 780

gccgccgtgc tgggcggacc agagccactg atgcaggcca ccgcctggct caacgcctac 840

tttcaccagc ctgaggccat cgaggagttc cctgtgccag ccctgcacca cccagtgttc 900

cagcaggaga gctttacccg ccaggtgctg tggaaactgc tgaaagtggt gaagttcgga 960

gaggtcatca gctaccagca gctggcggcc ctggcgggca atcccgccgc caccgccgcc 1020

gtgaaaaccg ccctgagcgg aaatcccgtg cccattctga tcccctgcca ccgggtggtg 1080

tctagctctg gcgccgtggg gggctacgag ggcgggctcg ccgtgaaaga gtggctgctg 1140

gcccacgagg gccacagact gggcaagcct gggctgggcg ctgagcacga atttcgagga 1200

gggcccgaac aaaaactcat ctcagaagag gatctgaata gcgccgtcga ccatcatcat 1260

catcatcatt ga 1272

<210> 42
<211> 1248
<212> DNA
<213> Homo sapiens

<400> 42
atggagacag acacactcct gctatgggta ctgctgctct gggttccagg ttccactggt 60

gacgcggccc agccggccag agctgtgcct gggggcagca gccctgcctg gactcagtgc 120

cagcagcttt cacagaagct ctgcacactg gcctggagtg cacatccact agtgggacac 180

atggatctaa gagaagaggg agatgaagag actacaaatg atgttcccca tatccagtgt 240

ggagatggct gtgaccccca aggactcagg gacaacagtc agttctgctt gcaaaggatc 300

caccagggtc tgatttttta tgagaagctg ctaggatcgg atattttcac aggggagcct 360

tctctgctcc ctgatagccc tgtgggccag cttcatgcct ccctactggg cctcagccaa 420

ctcctgcagc ctgagggtca ccactgggag actcagcaga ttccaagcct cagtcccagc 480

cagccatggc agcgtctcct tctccgcttc aaaatccttc gcagcctcca ggcctttgtg 540

gctgtagccg cccgggtctt tgcccatgga gcagcaaccc tgagtcccgc ggccgcactc 600

gagtctagaa tggacaaaga ctgcgaaatg aagcgcacca ccctggatag ccctctgggc 660

aagctggaac tgtctgggtg cgaacagggc ctgcacgaga tcaagctgct gggcaaagga 720

acatctgccg ccgacgccgt ggaagtgcct gccccagccg ccgtgctggg cggaccagag 780

ccactgatgc aggccaccgc ctggctcaac gcctactttc accagcctga ggccatcgag 840

gagttccctg tgccagccct gcaccaccca gtgttccagc aggagagctt tacccgccag 900

gtgctgtgga aactgctgaa agtggtgaag ttcggagagg tcatcagcta ccagcagctg 960

gcggccctgg cgggcaatcc cgccgccacc gccgccgtga aaaccgccct gagcggaaat 1020

cccgtgccca ttctgatccc ctgccaccgg gtggtgtcta gctctggcgc cgtggggggc 1080

tacgagggcg ggctcgccgt gaaagagtgg ctgctggccc acgagggcca cagactgggc 1140

aagcctgggc tgggcgctga gcacgaattt cgaggagggc ccgaacaaaa actcatctca 1200

gaagaggatc tgaatagcgc cgtcgaccat catcatcatc atcattga 1248

<210> 43
<211> 1212
<212> DNA
<213> Homo sapiens

<400> 43
atggagacag acacactcct gctatgggta ctgctgctct gggttccagg ttccactggt 60

gacgcggccc agccggccgc ccagggccaa gaattccact ttgggccctg ccaagtgaag 120

ggggttgttc cccagaaact gtgggaagcc ttctgggctg tgaaagacac tatgcaagct 180

caggataaca tcacgagtgc ccggctgctg cagcaggagg ttctgcagaa cgtctcggat 240

gctgagagct gttaccttgt ccacaccctg ctggagttct acttgaaaac tgttttcaaa 300

aactaccaca atagaacagt tgaagtcagg actctgaagt cattctctac tctggccaac 360

aactttgttc tcatcgtgtc acaactgcaa cccagtcaag aaaatgagat gttttccatc 420

agagacagtg cacacaggcg gtttctgcta ttccggagag cattcaaaca gttggacgta 480

gaagcagctc tgaccaaagc ccttggggaa gtggacattc ttctgacctg gatgcagaaa 540

ttctacaagc tcgcggccgc actcgagtct agaatggaca aagactgcga aatgaagcgc 600

accaccctgg atagccctct gggcaagctg gaactgtctg ggtgcgaaca gggcctgcac 660

gagatcaagc tgctgggcaa aggaacatct gccgccgacg ccgtggaagt gcctgcccca 720

gccgccgtgc tgggcggacc agagccactg atgcaggcca ccgcctggct caacgcctac 780

tttcaccagc ctgaggccat cgaggagttc cctgtgccag ccctgcacca cccagtgttc 840

cagcaggaga gctttacccg ccaggtgctg tggaaactgc tgaaagtggt gaagttcgga 900

gaggtcatca gctaccagca gctggcggcc ctggcgggca atcccgccgc caccgccgcc 960

gtgaaaaccg ccctgagcgg aaatcccgtg cccattctga tcccctgcca ccgggtggtg 1020

tctagctctg gcgccgtggg gggctacgag ggcgggctcg ccgtgaaaga gtggctgctg 1080

gcccacgagg gccacagact gggcaagcct gggctgggcg ctgagcacga atttcgagga 1140

gggcccgaac aaaaactcat ctcagaagag gatctgaata gcgccgtcga ccatcatcat 1200

catcatcatt ga 1212

<210> 44
<211> 1173
<212> DNA
<213> Homo sapiens

<400> 44

atggagacag acacactcct gctatgggta ctgctgctct gggttccagg ttccactggt 60

gacgcggccc agccggccta cagccactgg cccagctgct gccccagcaa agggcaggac 120

acctctgagg agctgctgag gtggagcact gtgcctgtgc ctcccctaga gcctgctagg 180

cccaaccgcc acccagagtc ctgtagggcc agtgaagatg gacccctcaa cagcagggcc 240

atctccccct ggagatatga gttggacaga gacttgaacc ggctccccca ggacctgtac 300

cacgcccgtt gcctgtgccc gcactgcgtc agcctacaga caggctccca catggacccc 360

cggggcaact cggagctgct ctaccacaac cagactgtct tctaccggcg gccatgccat 420

ggcgagaagg gcacccacaa gggctactgc ctggagcgca ggctgtaccg tgtttcctta 480

gcttgtgtgt gtgtgcggcc ccgtgtgatg ggcgcggccg cactcgagtc tagaatggac 540

aaagactgcg aaatgaagcg caccaccctg gatagccctc tgggcaagct ggaactgtct 600

gggtgcgaac agggcctgca cgagatcaag ctgctgggca aaggaacatc tgccgccgac 660

gccgtggaag tgcctgcccc agccgccgtg ctgggcggac cagagccact gatgcaggcc 720

accgcctggc tcaacgccta ctttcaccag cctgaggcca tcgaggagtt ccctgtgcca 780

gccctgcacc acccagtgtt ccagcaggag agctttaccc gccaggtgct gtggaaactg 840

ctgaaagtgg tgaagttcgg agaggtcatc agctaccagc agctggcggc cctggcgggc 900

aatcccgccg ccaccgccgc cgtgaaaacc gccctgagcg gaaatcccgt gcccattctg 960

atcccctgcc accgggtggt gtctagctct ggcgccgtgg ggggctacga gggcgggctc 1020

gccgtgaaag agtggctgct ggcccacgag ggccacagac tgggcaagcc tgggctgggc 1080

gctgagcacg aatttcgagg agggcccgaa caaaaactca tctcagaaga ggatctgaat 1140

agcgccgtcg accatcatca tcatcatcat tga 1173

<210> 45
<211> 1188
<212> DNA
<213> Homo sapiens

<400> 45
atggagacag acacactcct gctatgggta ctgctgctct gggttccagg ttccactggt 60

gacgcggccc agccggccaa gcacaagcaa tcttccttca ccaaaagttg ttacccaagg 120

ggaacattgt cccaagctgt tgacgctctc tatatcaaag cagcatggct caaagcaacg 180

attccagaag accgcataaa aaatatacga ttattaaaaa agaaaacaaa aaagcagttt 240

atgaaaaact gtcaatttca agaacagctt ctgtccttct tcatggaaga cgtttttggt 300

caactgcaat tgcaaggctg caagaaaata cgctttgtgg aggactttca tagccttagg 360

cagaaattga gccactgtat ttcctgtgct tcatcagcta gagagatgaa atccattacc 420

aggatgaaaa gaatatttta taggattgga aacaaaggaa tctacaaagc catcagtgaa 480

ctggatattc ttctttcctg gattaaaaaa ttattggaaa gcagtcaggc ggccgcactc 540

gagtctagaa tggacaaaga ctgcgaaatg aagcgcacca ccctggatag ccctctgggc 600

aagctggaac tgtctgggtg cgaacagggc ctgcacgaga tcaagctgct gggcaaagga 660

acatctgccg ccgacgccgt ggaagtgcct gccccagccg ccgtgctggg cggaccagag 720

ccactgatgc aggccaccgc ctggctcaac gcctactttc accagcctga ggccatcgag 780

gagttccctg tgccagccct gcaccaccca gtgttccagc aggagagctt tacccgccag 840

gtgctgtgga aactgctgaa agtggtgaag ttcggagagg tcatcagcta ccagcagctg 900

gcggccctgg cgggcaatcc cgccgccacc gccgccgtga aaaccgccct gagcggaaat 960

cccgtgccca ttctgatccc ctgccaccgg gtggtgtcta gctctggcgc cgtggggggc 1020

tacgagggcg ggctcgccgt gaaagagtgg ctgctggccc acgagggcca cagactgggc 1080

aagcctgggc tgggcgctga gcacgaattt cgaggagggc ccgaacaaaa actcatctca 1140

gaagaggatc tgaatagcgc cgtcgaccat catcatcatc atcattga 1188

<210> 46
<211> 1464
<212> DNA
<213> Homo sapiens

<400> 46
atggagacag acacactcct gctatgggta ctgctgctct gggttccagg ttccactggt 60

gacgcggccc agccggccgg ccagacggca ggcgaccttg gctggcggct cagcctgttg 120

ctgcttccct tgctcctggt tcaagctggt gtctggggat tcccaaggcc cccagggagg 180

ccccagctga gcctgcagga gctgcggagg gagttcacag tcagcctgca tctcgccagg 240

aagctgctcg ccgaggttcg gggccaggcc caccgctttg cggaatctca cctgccagga 300

gtgaacctgt acctcctgcc cctgggagag cagctccctg atgtttccct gaccttccag 360

gcctggcgcc gcctctctga cccggagcgt ctctgcttca tctccaccac gcttcagccc 420

ttccatgccc tgctgggagg gctggggacc cagggccgct ggaccaacat ggagaggatg 480

cagctgtggg ccatgaggct ggacctccgc gatctgcagc ggcacctccg cttccaggtg 540

ctggctgcag gattcaacct cccggaggag gaggaggagg aagaggagga ggaggaggag 600

gagaggaagg ggctgctccc aggggcactg ggcagcgcct tacagggccc ggcccaggtg 660

tcctggcccc agctcctctc cacctaccgc ctgctgcact ccttggagct cgtcttatct 720

cgggccgtgc gggagttgct gctgctgtcc aaggctgggc actcagtctg gcccttgggg 780

ttcccaacat tgagccccca gcccgcggcc gcactcgagt ctagaatgga caaagactgc 840

gaaatgaagc gcaccaccct ggatagccct ctgggcaagc tggaactgtc tgggtgcgaa 900

cagggcctgc acgagatcaa gctgctgggc aaaggaacat ctgccgccga cgccgtggaa 960

gtgcctgccc cagccgccgt gctgggcgga ccagagccac tgatgcaggc caccgcctgg 1020

ctcaacgcct actttcacca gcctgaggcc atcgaggagt tccctgtgcc agccctgcac 1080

cacccagtgt tccagcagga gagctttacc cgccaggtgc tgtggaaact gctgaaagtg 1140

gtgaagttcg gagaggtcat cagctaccag cagctggcgg ccctggcggg caatcccgcc 1200

gccaccgccg ccgtgaaaac cgccctgagc ggaaatcccg tgcccattct gatcccctgc 1260

caccgggtgg tgtctagctc tggcgccgtg gggggctacg agggcgggct cgccgtgaaa 1320

gagtggctgc tggcccacga gggccacaga ctgggcaagc ctgggctggg cgctgagcac 1380

gaatttcgag gagggcccga acaaaaactc atctcagaag aggatctgaa tagcgccgtc 1440

gaccatcatc atcatcatca ttga 1464

<210> 47
<211> 1335
<212> DNA
<213> Homo sapiens

<400> 47
atggagacag acacactcct gctatgggta ctgctgctct gggttccagg ttccactggt 60

gacgcggccc agccggccaa actagacatg actggggact gcacgccagt gctggtgctg 120

atggccgcag tgctgaccgt gactggagca gttcctgtcg ccaggctcca cggggctctc 180

ccggatgcaa ggggctgcca catagcccag ttcaagtccc tgtctccaca ggagctgcag 240

gcctttaaga gggccaaaga tgccttagaa gagtcgcttc tgctgaagga ctgcaggtgc 300

cactcccgcc tcttccccag gacctgggac ctgaggcagc tgcaggtgag ggagcgcccc 360

atggctttgg aggctgagct ggccctgacg ctgaaggttc tggaggccac cgctgacact 420

gacccagccc tggtggacgt cttggaccag ccccttcaca ccctgcacca tatcctctcc 480

cagttccggg cctgtatcca gcctcagccc acggcagggc ccaggacccg gggccgcctc 540

caccattggc tgtaccggct ccaggaggcc ccaaaaaagg agtcccctgg ctgcctcgag 600

gcctctgtca ccttcaacct cttccgcctc ctcacgcgag acctgaattg tgttgccagt 660

ggggacctgt gtgtcgcggc cgcactcgag tctagaatgg acaaagactg cgaaatgaag 720

cgcaccaccc tggatagccc tctgggcaag ctggaactgt ctgggtgcga acagggcctg 780

cacgagatca agctgctggg caaaggaaca tctgccgccg acgccgtgga agtgcctgcc 840

ccagccgccg tgctgggcgg accagagcca ctgatgcagg ccaccgcctg gctcaacgcc 900

tactttcacc agcctgaggc catcgaggag ttccctgtgc cagccctgca ccacccagtg 960

ttccagcagg agagctttac ccgccaggtg ctgtggaaac tgctgaaagt ggtgaagttc 1020

ggagaggtca tcagctacca gcagctggcg gccctggcgg gcaatcccgc cgccaccgcc 1080

gccgtgaaaa ccgccctgag cggaaatccc gtgcccattc tgatcccctg ccaccgggtg 1140

gtgtctagct ctggcgccgt ggggggctac gagggcgggc tcgccgtgaa agagtggctg 1200

ctggcccacg agggccacag actgggcaag cctgggctgg gcgctgagca cgaatttcga 1260

ggagggcccg aacaaaaact catctcagaa gaggatctga atagcgccgt cgaccatcat 1320

catcatcatc attga 1335

<210> 48
<211> 1323
<212> DNA
<213> Homo sapiens

<400> 48
atggagacag acacactcct gctatgggta ctgctgctct gggttccagg ttccactggt 60

gacgcggccc agccggccac cggggactgc atgccagtgc tggtgctgat ggccgcagtg 120

ctgaccgtga ctggagcagt tcctgtcgcc aggctccgcg gggctctccc ggatgcaagg 180

ggctgccaca tagcccagtt caagtccctg tctccacagg agctgcaggc ctttaagagg 240

gccaaagatg ccttagaaga gtcgcttctg ctgaaggact gcaagtgccg ctcccgcctc 300

ttccccagga cctgggacct gaggcagctg caggtgaggg agcgccccgt ggctttggag 360

gctgagctgg ccctgacgct gaaggttctg gaggccaccg ctgacactga cccagccctg 420

ggggatgtct tggaccagcc ccttcacacc ctgcaccata tcctctccca gctccgggcc 480

tgtatccagc ctcagcccac ggcagggccc aggacccggg gccgcctcca ccattggctg 540

caccggctcc aggaggcccc aaaaaaggag tcccctggct gcctcgaggc ctctgtcacc 600

ttcaacctct tccgcctcct cacgcgagac ctgaattgtg ttgccagcgg ggacctgtgt 660

gtcgcggccg cactcgagtc tagaatggac aaagactgcg aaatgaagcg caccaccctg 720

gatagccctc tgggcaagct ggaactgtct gggtgcgaac agggcctgca cgagatcaag 780

ctgctgggca aaggaacatc tgccgccgac gccgtggaag tgcctgcccc agccgccgtg 840

ctgggcggac cagagccact gatgcaggcc accgcctggc tcaacgccta ctttcaccag 900

cctgaggcca tcgaggagtt ccctgtgcca gccctgcacc acccagtgtt ccagcaggag 960

agctttaccc gccaggtgct gtggaaactg ctgaaagtgg tgaagttcgg agaggtcatc 1020

agctaccagc agctggcggc cctggcgggc aatcccgccg ccaccgccgc cgtgaaaacc 1080

gccctgagcg gaaatcccgt gcccattctg atcccctgcc accgggtggt gtctagctct 1140

ggcgccgtgg ggggctacga gggcgggctc gccgtgaaag agtggctgct ggcccacgag 1200

ggccacagac tgggcaagcc tgggctgggc gctgagcacg aatttcgagg agggcccgaa 1260

caaaaactca tctcagaaga ggatctgaat agcgccgtcg accatcatca tcatcatcat 1320

tga 1323

<210> 49
<211> 1250
<212> DNA
<213> Homo sapiens

<400> 49
atggagacag acacactcct gctatgggta ctgctgctct gggttccagg ttccactggt 60

gacgcggccc agccggccct gggaagggct gccacattgg caggttcaaa tctctgtcac 120

cacaggagct agcgagcttc aagaaggcca gggacgcctt ggaagagtca ctcaagctga 180

aaaactggag ttgcagctct cctgtcttcc ccgggaattg ggacctgagg cttctccagg 240

tgagggagcg ccctgtggcc ttggaggctg agctggccct gacgctgaag gtcctggagg 300

ccgctgctgg cccagccctg gaggacgtcc tagaccagcc ccttcacacc ctgcaccaca 360

tcctctccca gctccaggcc tgtatccagc ctcagcccac agcagggccc aggccccggg 420

gccgcctcca ccactggctg caccggctcc aggaggcccc caaaaaggag tccgctggct 480

gcctggaggc atctgtcacc ttcaacctct tccgcctcct cacgcgagac ctcaaatatg 540

tggccgatgg gaacctgtgt ctgagaacgt caacccaccc tgagtccacc gcggccgcac 600

tcgagtctag aatggacaaa gactgcgaaa tgaagcgcac caccctggat agccctctgg 660

gcaagctgga actgtctggg tgcgaacagg gcctgcacga gatcaagctg ctgggcaaag 720

gaacatctgc cgccgacgcc gtggaagtgc ctgccccagc cgccgtgctg ggcggaccag 780

agccactgat gcaggccacc gcctggctca acgcctactt tcaccagcct gaggccatcg 840

aggagttccc tgtgccagcc ctgcaccacc cagtgttcca gcaggagagc tttacccgcc 900

aggtgctgtg gaaactgctg aaagtggtga agttcggaga ggtcatcagc taccagcagc 960

tggcggccct ggcgggcaat cccgccgcca ccgccgccgt gaaaaccgcc ctgagcggaa 1020

atcccgtgcc cattctgatc ccctgccacc gggtggtgtc tagctctggc gccgtggggg 1080

gctacgaggg cgggctcgcc gtgaaagagt ggctgctggc ccacgagggc cacagactgg 1140

gcaagcctgg gctgggcgct gagcacgaat ttcgaggagg gcccgaacaa aaactcatct 1200

cagaagagga tctgaatagc gccgtcgacc atcatcatca tcatcattga 1250

<210> 50
<211> 1161
<212> DNA
<213> Homo sapiens

<400> 50
atggagacag acacactcct gctatgggta ctgctgctct gggttccagg ttccactggt 60

gacgcggccc agccggcctc ccacacgttg cccgtccgtt tactacgacc aagtgatgat 120

gtacagaaaa tagtcgagga attacagtcc ctctcgaaga tgcttttgaa agatgtggag 180

gaagagaagg gcgtgctcgt gtcccagaat tacacgctgc cgtgtctcag ccctgacgcc 240

cagccgccaa acaacatcca cagcccagcc atccgggcat atctcaagac aatcagacag 300

ctagacaaca aatctgttat tgatgagatc atagagcacc tcgacaaact catatttcaa 360

gatgcaccag aaacaaacat ttctgtgcca acagacaccc atgaatgtaa acgcttcatc 420

ctgactattt ctcaacagtt ttcagagtgc atggacctcg cactaaaatc attgacctct 480

ggagcccaac aggccaccac tgcggccgca ctcgagtcta gaatggacaa agactgcgaa 540

atgaagcgca ccaccctgga tagccctctg ggcaagctgg aactgtctgg gtgcgaacag 600

ggcctgcacg agatcaagct gctgggcaaa ggaacatctg ccgccgacgc cgtggaagtg 660

cctgccccag ccgccgtgct gggcggacca gagccactga tgcaggccac cgcctggctc 720

aacgcctact ttcaccagcc tgaggccatc gaggagttcc ctgtgccagc cctgcaccac 780

ccagtgttcc agcaggagag ctttacccgc caggtgctgt ggaaactgct gaaagtggtg 840

aagttcggag aggtcatcag ctaccagcag ctggcggccc tggcgggcaa tcccgccgcc 900

accgccgccg tgaaaaccgc cctgagcgga aatcccgtgc ccattctgat cccctgccac 960

cgggtggtgt ctagctctgg cgccgtgggg ggctacgagg gcgggctcgc cgtgaaagag 1020

tggctgctgg cccacgaggg ccacagactg ggcaagcctg ggctgggcgc tgagcacgaa 1080

tttcgaggag ggcccgaaca aaaactcatc tcagaagagg atctgaatag cgccgtcgac 1140

catcatcatc atcatcattg a 1161

<210> 51
<211> 1047
<212> DNA
<213> Homo sapiens

<400> 51
atggagacag acacactcct gctatgggta ctgctgctct gggttccagg ttccactggt 60

gacgcggccc agccggccac cccagtagtg agaaagggtc gctgttcctg catcagcacc 120

aaccaaggga ctatccacct acaatccttg aaagacctta aacaatttgc cccaagccct 180

tcctgcgaga aaattgaaat cattgctaca ctgaagaatg gagttcaaac atgtctaaac 240

ccagattcag cagatgtgaa ggaactgatt aaaaagtggg agaaacaggt cagccaaaag 300

aaaaagcaaa agaatgggaa aaaacatcaa aaaaagaaag ttctgaaagt tcgaaaatct 360

caacgttctc gtcaaaagaa gactacagcg gccgcactcg agtctagaat ggacaaagac 420

tgcgaaatga agcgcaccac cctggatagc cctctgggca agctggaact gtctgggtgc 480

gaacagggcc tgcacgagat caagctgctg ggcaaaggaa catctgccgc cgacgccgtg 540

gaagtgcctg ccccagccgc cgtgctgggc ggaccagagc cactgatgca ggccaccgcc 600

tggctcaacg cctactttca ccagcctgag gccatcgagg agttccctgt gccagccctg 660

caccacccag tgttccagca ggagagcttt acccgccagg tgctgtggaa actgctgaaa 720

gtggtgaagt tcggagaggt catcagctac cagcagctgg cggccctggc gggcaatccc 780

gccgccaccg ccgccgtgaa aaccgccctg agcggaaatc ccgtgcccat tctgatcccc 840

tgccaccggg tggtgtctag ctctggcgcc gtggggggct acgagggcgg gctcgccgtg 900

aaagagtggc tgctggccca cgagggccac agactgggca agcctgggct gggcgctgag 960

cacgaatttc gaggagggcc cgaacaaaaa ctcatctcag aagaggatct gaatagcgcc 1020

gtcgaccatc atcatcatca tcattga 1047

<210>  52
<211>  969
<212>  DNA
<213>  Homo sapiens

<400>  52
atggagacag acacactcct gctatgggta ctgctgctct gggttccagg ttccactggt      60

gacgcggccc agccggccgt acctctctct agaactgtac gctgtacctg catcagcatt     120

agtaatcaac ctgttaatcc aaggtcttta gaaaaacttg aaattattcc tgcaagccaa     180

ttttgtccac gtgttgagat cattgctaca atgaaaaaga agggtgagaa gagatgtctg     240

aatccagaat cgaaggccat caagaattta ctgaaagcag ttagcaagga aaggtctaaa     300

agatctcctg cggccgcact cgagtctaga atggacaaag actgcgaaat gaagcgcacc     360

accctggata gccctctggg caagctggaa ctgtctgggt gcgaacaggg cctgcacgag     420

atcaagctgc tgggcaaagg aacatctgcc gccgacgccg tggaagtgcc tgccccagcc     480

gccgtgctgg gcggaccaga gccactgatg caggccaccg cctggctcaa cgcctacttt     540

caccagcctg aggccatcga ggagttccct gtgccagccc tgcaccaccc agtgttccag     600

caggagagct ttacccgcca ggtgctgtgg aaactgctga aagtggtgaa gttcggagag     660

gtcatcagct accagcagct ggcggccctg gcgggcaatc ccgccgccac cgccgccgtg     720

aaaaccgccc tgagcggaaa tcccgtgccc attctgatcc cctgccaccg ggtggtgtct     780

agctctggcg ccgtgggggg ctacgagggc gggctcgccg tgaaagagtg gctgctggcc     840

cacgagggcc acagactggg caagcctggg ctgggcgctg agcacgaatt tcgaggaggg     900

cccgaacaaa aactcatctc agaagaggat ctgaatagcg ccgtcgacca tcatcatcat     960

catcattga                                                             969

<210>  53
<211>  1554
<212>  DNA
<213>  Homo sapiens

<400>  53
atggagacag acacactcct gctatgggta ctgctgctct gggttccagg ttccactggt      60

gacgcggccc agccggcctc tgggagtcag agcgaggtgg ctccatcccc gcagagtccg     120

cggagccccg agatgggacg ggacttgcgg cccgggtccc gcgtgctcct gctcctgctt     180

ctgctcctgc tggtgtacct gactcagcca ggcaatggca acgagggcag cgtcactgga     240

agttgttatt gtggtaaaag aatttcttcc gactccccgc catcggttca gttcatgaat     300

cgtctccgga aacacctgag agcttaccat cggtgtctat actacacgag gttccagctc     360

ctttcctgga gcgtgtgtgg gggcaacaag gacccatggg ttcaggaatt gatgagctgt     420

cttgatctca aagaatgtgg acatgcttac tcggggattg tggcccacca gaagcattta     480

cttcctacca gccccccaat ttctcaggcc tcagaggggg catcttcaga tatccacacc     540

cctgcccaga tgctcctgtc caccttgcag tccactcagc gccccaccct cccagtagga 600

tcactgtcct cggacaaaga gctcactcgt cccaatgaaa ccaccattca cactgcgggc 660

cacagtctgg cagctgggcc tgaggctggg gagaaccaga agcagccgga aaaaaatgct 720

ggtcccacag ccaggacatc agccacagtg ccagtcctgt gcctcctggc catcatcttc 780

atcctcaccg cagccctttc ctatgtgctg tgcaagagga ggagggggca gtcaccgcag 840

tcctctccag atctgccggt tcattatata cctgtggcac ctgactctaa taccgcggcc 900

gcactcgagt ctagaatgga caaagactgc gaaatgaagc gcaccaccct ggatagccct 960

ctgggcaagc tggaactgtc tgggtgcgaa cagggcctgc acgagatcaa gctgctgggc 1020

aaaggaacat ctgccgccga cgccgtggaa gtgcctgccc cagccgccgt gctgggcgga 1080

ccagagccac tgatgcaggc caccgcctgg ctcaacgcct actttcacca gcctgaggcc 1140

atcgaggagt tccctgtgcc agccctgcac cacccagtgt tccagcagga gagctttacc 1200

cgccaggtgc tgtggaaact gctgaaagtg gtgaagttcg gagaggtcat cagctaccag 1260

cagctggcgg ccctggcggg caatcccgcc gccaccgccg ccgtgaaaac cgccctgagc 1320

ggaaatcccg tgcccattct gatcccctgc caccgggtgg tgtctagctc tggcgccgtg 1380

gggggctacg agggcgggct cgccgtgaaa gagtggctgc tggcccacga gggccacaga 1440

ctgggcaagc ctgggctggg cgctgagcac gaatttcgag gagggcccga acaaaaactc 1500

atctcagaag aggatctgaa tagcgccgtc gaccatcatc atcatcatca ttga 1554

<210> 54
<211> 957
<212> DNA
<213> Homo sapiens

<400> 54
atggagacag acacactcct gctatgggta ctgctgctct gggttccagg ttccactggt 60

gacgcggccc agccggcctt ccccatgttc aaaagaggac gctgtctttg cataggccct 120

ggggtaaaag cagtgaaagt ggcagatatt gagaaagcct ccataatgta cccaagtaac 180

aactgtgaca aaatagaagt gattattacc ctgaaagaaa ataaaggaca acgatgccta 240

aatcccaaat cgaagcaagc aaggcttata atcaaaaaag ttgaaagaaa gaattttgcg 300

gccgcactcg agtctagaat ggacaaagac tgcgaaatga agcgcaccac cctggatagc 360

cctctgggca agctggaact gtctgggtgc gaacagggcc tgcacgagat caagctgctg 420

ggcaaaggaa catctgccgc cgacgccgtg gaagtgcctg ccccagccgc cgtgctgggc 480

ggaccagagc cactgatgca ggccaccgcc tggctcaacg cctactttca ccagcctgag 540

gccatcgagg agttccctgt gccagccctg caccacccag tgttccagca ggagagcttt 600

acccgccagg tgctgtggaa actgctgaaa gtggtgaagt tcggagaggt catcagctac 660

cagcagctgg cggccctggc gggcaatccc gccgccaccg ccgccgtgaa aaccgccctg 720

agcggaaatc ccgtgcccat tctgatcccc tgccaccggg tggtgtctag ctctggcgcc 780

gtggggggct acgagggcgg gctcgccgtg aaagagtggc tgctggccca cgagggccac 840

agactgggca agcctgggct gggcgctgag cacgaatttc gaggagggcc cgaacaaaaa 900

ctcatctcag aagaggatct gaatagcgcc gtcgaccatc atcatcatca tcattga 957

<210> 55
<211> 999
<212> DNA
<213> Homo sapiens

<400> 55
atggagacag acacactcct gctatgggta ctgctgctct gggttccagg ttccactggt 60

gacgcggccc agccggccgt tctggaggtc tattacacaa gcttgaggtg tagatgtgtc 120

caagagagct cagtctttat ccctagacgc ttcattgatc gaattcaaat cttgccccgt 180

gggaatggtt gtccaagaaa agaaatcata gtctggaaga agaacaagtc aattgtgtgt 240

gtggaccctc aagctgaatg gatacaaaga atgatggaag tattgagaaa aagaagttct 300

tcaactctac cagttccagt gtttaagaga aagattcccg cggccgcact cgagtctaga 360

atggacaaag actgcgaaat gaagcgcacc accctggata gccctctggg caagctggaa 420

ctgtctgggt gcgaacaggg cctgcacgag atcaagctgc tgggcaaagg aacatctgcc 480

gccgacgccg tggaagtgcc tgccccagcc gccgtgctgg gcggaccaga gccactgatg 540

caggccaccg cctggctcaa cgcctacttt caccagcctg aggccatcga ggagttccct 600

gtgccagccc tgcaccaccc agtgttccag caggagagct ttacccgcca ggtgctgtgg 660

aaactgctga aagtggtgaa gttcggagag gtcatcagct accagcagct ggcggccctg 720

gcgggcaatc ccgccgccac cgccgccgtg aaaaccgccc tgagcggaaa tcccgtgccc 780

attctgatcc cctgccaccg ggtggtgtct agctctggcg ccgtgggggg ctacgagggc 840

gggctcgccg tgaaagagtg gctgctggcc cacgagggcc acagactggg caagcctggg 900

ctgggcgctg agcacgaatt tcgaggaggg cccgaacaaa aactcatctc agaagaggat 960

ctgaatagcg ccgtcgacca tcatcatcat catcattga 999

<210> 56
<211> 960
<212> DNA
<213> Homo sapiens

<400> 56
atggagacag acacactcct gctatgggta ctgctgctct gggttccagg ttccactggt 60

gacgcggccc agccggccgg gccagcttct gtcccaacca cctgctgctt taacctggcc 120

aataggaaga taccccttca gcgactagag agctacagga gaatcaccag tggcaaatgt 180

ccccagaaag ctgtgatctt caagaccaaa ctggccaagg atatctgtgc cgaccccaag 240

aagaagtggg tgcaggattc catgaagtat ctggaccaaa aatctccaac tccaaagcca 300

gcggccgcac tcgagtctag aatggacaaa gactgcgaaa tgaagcgcac caccctggat 360

agccctctgg gcaagctgga actgtctggg tgcgaacagg gcctgcacga gatcaagctg 420

ctgggcaaag gaacatctgc cgccgacgcc gtggaagtgc ctgccccagc cgccgtgctg 480

ggcggaccag agccactgat gcaggccacc gcctggctca acgcctactt tcaccagcct 540

gaggccatcg aggagttccc tgtgccagcc ctgcaccacc cagtgttcca gcaggagagc 600

tttacccgcc aggtgctgtg gaaactgctg aaagtggtga agttcggaga ggtcatcagc 660

taccagcagc tggcggccct ggcgggcaat cccgccgcca ccgccgccgt gaaaaccgcc 720

ctgagcggaa atcccgtgcc cattctgatc ccctgccacc gggtggtgtc tagctctggc 780

gccgtggggg gctacgaggg cgggctcgcc gtgaaagagt ggctgctggc ccacgagggc 840

cacagactgg gcaagcctgg gctgggcgct gagcacgaat ttcgaggagg gcccgaacaa 900

aaactcatct cagaagagga tctgaatagc gccgtcgacc atcatcatca tcatcattga 960

<210> 57
<211> 960
<212> DNA
<213> Homo sapiens

<400> 57
atggagacag acacactcct gctatgggta ctgctgctct gggttccagg ttccactggt 60

gacgcggccc agccggccac caagactgaa tcctcctcac ggggacctta ccacccctca 120

gagtgctgct tcacctacac tacctacaag atcccgcgtc agcggattat ggattactat 180

gagaccaaca gccagtgctc caagcccgga attgtcttca tcaccaaaag gggccattcc 240

gtctgtacca accccagtga caagtgggtc caggactata tcaaggacat gaaggagaac 300

gcggccgcac tcgagtctag aatggacaaa gactgcgaaa tgaagcgcac caccctggat 360

agccctctgg gcaagctgga actgtctggg tgcgaacagg gcctgcacga gatcaagctg 420

ctgggcaaag gaacatctgc cgccgacgcc gtggaagtgc ctgccccagc cgccgtgctg 480

ggcggaccag agccactgat gcaggccacc gcctggctca acgcctactt tcaccagcct 540

gaggccatcg aggagttccc tgtgccagcc ctgcaccacc cagtgttcca gcaggagagc 600

tttacccgcc aggtgctgtg gaaactgctg aaagtggtga agttcggaga ggtcatcagc 660

taccagcagc tggcggccct ggcgggcaat cccgccgcca ccgccgccgt gaaaaccgcc 720

ctgagcggaa atcccgtgcc cattctgatc ccctgccacc gggtggtgtc tagctctggc 780

gccgtggggg gctacgaggg cgggctcgcc gtgaaagagt ggctgctggc ccacgagggc 840

cacagactgg gcaagcctgg gctgggcgct gagcacgaat ttcgaggagg gcccgaacaa 900

aaactcatct cagaagagga tctgaatagc gccgtcgacc atcatcatca tcatcattga 960

<210> 58
<211> 1029

<212>  DNA
<213>  Homo sapiens

<400>  58
atggagacag acacactcct gctatgggta ctgctgctct gggttccagg ttccactggt      60

gacgcggccc agccggccca gccaaaagtt cctgagtggg tgaacacccc atccacctgc     120

tgcctgaagt attatgagaa agtgttgcca aggagactag tggtgggata cagaaaggcc     180

ctcaactgtc acctgccagc aatcatcttc gtcaccaaga ggaaccgaga agtctgcacc     240

aaccccaatg acgactgggt ccaagagtac atcaaggatc ccaacctacc tttgctgcct     300

accaggaact tgtccacggt taaaattatt acagcaaaga atggtcaacc ccagctcctc     360

aactcccagg cggccgcact cgagtctaga atggacaaag actgcgaaat gaagcgcacc     420

accctggata gccctctggg caagctggaa ctgtctgggt gcgaacaggg cctgcacgag     480

atcaagctgc tgggcaaagg aacatctgcc gccgacgccg tggaagtgcc tgccccagcc     540

gccgtgctgg gcggaccaga gccactgatg caggccaccg cctggctcaa cgcctacttt     600

caccagcctg aggccatcga ggagttccct gtgccagccc tgcaccaccc agtgttccag     660

caggagagct ttacccgcca ggtgctgtgg aaactgctga aagtggtgaa gttcggagag     720

gtcatcagct accagcagct ggcggccctg gcgggcaatc ccgccgccac cgccgccgtg     780

aaaaccgccc tgagcggaaa tcccgtgccc attctgatcc cctgccaccg ggtggtgtct     840

agctctggcg ccgtgggggg ctacgagggc gggctcgccg tgaaagagtg gctgctggcc     900

cacgagggcc acagactggg caagcctggg ctgggcgctg agcacgaatt tcgaggaggg     960

cccgaacaaa aactcatctc agaagaggat ctgaatagcg ccgtcgacca tcatcatcat    1020

catcattga                                                            1029

<210>  59
<211>  945
<212>  DNA
<213>  Homo sapiens

<400>  59
atggagacag acacactcct gctatgggta ctgctgctct gggttccagg ttccactggt      60

gacgcggccc agccggccgc acaagttggt accaacaaag agctctgctg cctcgtctat     120

acctcctggc agattccaca aaagttcata gttgactatt ctgaaaccag cccccagtgc     180

cccaagccag gtgtcatcct cctaaccaag agaggccggc agatctgtgc tgaccccaat     240

aagaagtggg tccagaaata catcagcgac ctgaagctga atgccgcggc cgcactcgag     300

tctagaatgg acaaagactg cgaaatgaag cgcaccaccc tggatagccc tctgggcaag     360

ctggaactgt ctgggtgcga acagggcctg cacgagatca agctgctggg caaaggaaca     420

tctgccgccg acgccgtgga agtgcctgcc ccagccgccg tgctgggcgg accagagcca     480

ctgatgcagg ccaccgcctg gctcaacgcc tactttcacc agcctgaggc catcgaggag     540

ttccctgtgc cagccctgca ccacccagtg ttccagcagg agagctttac ccgccaggtg 600

ctgtggaaac tgctgaaagt ggtgaagttc ggagaggtca tcagctacca gcagctggcg 660

gccctggcgg gcaatcccgc cgccaccgcc gccgtgaaaa ccgccctgag cggaaatccc 720

gtgcccattc tgatcccctg ccaccgggtg gtgtctagct ctggcgccgt ggggggctac 780

gagggcgggc tcgccgtgaa agagtggctg ctggcccacg agggccacag actgggcaag 840

cctgggctgg gcgctgagca cgaatttcga ggagggcccg aacaaaaact catctcagaa 900

gaggatctga atagcgccgt cgaccatcat catcatcatc attga 945

<210> 60
<211> 1002
<212> DNA
<213> Homo sapiens

<400> 60
atggagacag acacactcct gctatgggta ctgctgctct gggttccagg ttccactggt 60

gacgcggccc agccggcctt cctactgcca cccagcactg cctgctgtac tcagctctac 120

cgaaagccac tctcagacaa gctactgagg aaggtcatcc aggtggaact gcaggaggct 180

gacggggact gtcacctcca ggctttcgtg cttcacctgg ctcaacgcag catctgcatc 240

cacccccaga accccagcct gtcacagtgg tttgagcacc aagagagaaa gctccatggg 300

actctgccca agctgaattt tgggatgcta aggaaaatgg gcgcggccgc actcgagtct 360

agaatggaca aagactgcga aatgaagcgc accaccctgg atagccctct gggcaagctg 420

gaactgtctg ggtgcgaaca gggcctgcac gagatcaagc tgctgggcaa aggaacatct 480

gccgccgacg ccgtggaagt gcctgcccca gccgccgtgc tgggcggacc agagccactg 540

atgcaggcca ccgcctggct caacgcctac tttcaccagc ctgaggccat cgaggagttc 600

cctgtgccag ccctgcacca cccagtgttc cagcaggaga gctttacccg ccaggtgctg 660

tggaaactgc tgaaagtggt gaagttcgga gaggtcatca gctaccagca gctggcggcc 720

ctggcgggca atcccgccgc caccgccgcc gtgaaaaccg ccctgagcgg aaatcccgtg 780

cccattctga tcccctgcca ccgggtggtg tctagctctg gcgccgtggg gggctacgag 840

ggcgggctcg ccgtgaaaga gtggctgctg gcccacgagg gccacagact gggcaagcct 900

gggctgggcg ctgagcacga atttcgagga gggcccgaac aaaaactcat ctcagaagag 960

gatctgaata gcgccgtcga ccatcatcat catcatcatt ga 1002

<210> 61
<211> 1083
<212> DNA
<213> Homo sapiens

<400> 61
atggagacag acacactcct gctatgggta ctgctgctct gggttccagg ttccactggt 60

gacgcggccc agccggccgt ctgtgcggcc ctacatgcct cagaagccat acttcccatt 120

gcctccagct gttgcacgga ggtttcacat catatttcca gaaggctcct ggaaagagtg 180

aatatgtgtc gcatccagag agctgatggg gattgtgact tggctgctgt catccttcat 240

gtcaagcgca gaagaatctg tgtcagcccg cacaaccata ctgttaagca gtggatgaaa 300

gtgcaagctg ccaagaaaaa tggtaaagga aatgtttgcc acaggaagaa acaccatggc 360

aagaggaaca gtaacagggc acatcagggg aaacacgaaa catacggcca taaaactcct 420

tatgcggccg cactcgagtc tagaatggac aaagactgcg aaatgaagcg caccaccctg 480

gatagccctc tgggcaagct ggaactgtct gggtgcgaac agggcctgca cgagatcaag 540

ctgctgggca aaggaacatc tgccgccgac gccgtggaag tgcctgcccc agccgccgtg 600

ctgggcggac cagagccact gatgcaggcc accgcctggc tcaacgccta ctttcaccag 660

cctgaggcca tcgaggagtt ccctgtgcca gccctgcacc acccagtgtt ccagcaggag 720

agctttaccc gccaggtgct gtggaaactg ctgaaagtgg tgaagttcgg agaggtcatc 780

agctaccagc agctggcggc cctggcgggc aatcccgccg ccaccgccgc cgtgaaaacc 840

gccctgagcg gaaatcccgt gcccattctg atcccctgcc accgggtggt gtctagctct 900

ggcgccgtgg ggggctacga gggcgggctc gccgtgaaag agtggctgct ggcccacgag 960

ggccacagac tgggcaagcc tgggctgggc gctgagcacg aatttcgagg agggcccgaa 1020

caaaaactca tctcagaaga ggatctgaat agcgccgtcg accatcatca tcatcatcat 1080

tga 1083

<210> 62
<211> 1017
<212> DNA
<213> Homo sapiens

<400> 62
atggagacag acacactcct gctatgggta ctgctgctct gggttccagg ttccactggt 60

gacgcggccc agccggccgt agggagtgaa gtctcagata agaggacctg tgtgagcctc 120

actacccagc gactgccggt tagcagaatc aagacctaca ccatcacgga aggctccttg 180

agagcagtaa tttttattac caaacgtggc ctaaaagtct gtgctgatcc acaagccaca 240

tgggtgagag acgtggtcag gagcatggac aggaaatcca acaccagaaa taacatgatc 300

cagaccaagc caacaggaac ccagcaatcg accaatacag ctgtgactct gactggcgcg 360

gccgcactcg agtctagaat ggacaaagac tgcgaaatga agcgcaccac cctggatagc 420

cctctgggca agctggaact gtctgggtgc gaacagggcc tgcacgagat caagctgctg 480

ggcaaaggaa catctgccgc cgacgccgtg gaagtgcctg ccccagccgc cgtgctgggc 540

ggaccagagc cactgatgca ggccaccgcc tggctcaacg cctactttca ccagcctgag 600

gccatcgagg agttccctgt gccagccctg caccacccag tgttccagca ggagagcttt 660

acccgccagg tgctgtggaa actgctgaaa gtggtgaagt tcggagaggt catcagctac 720

cagcagctgg cggccctggc gggcaatccc gccgccaccg ccgccgtgaa aaccgccctg 780

agcggaaatc ccgtgcccat tctgatcccc tgccaccggg tggtgtctag ctctggcgcc 840

gtggggggct acgagggcgg gctcgccgtg aaagagtggc tgctggccca cgagggccac 900

agactgggca agcctgggct gggcgctgag cacgaatttc gaggagggcc cgaacaaaaa 960

ctcatctcag aagaggatct gaatagcgcc gtcgaccatc atcatcatca tcattga 1017

<210> 63
<211> 1857
<212> DNA
<213> Homo sapiens

<400> 63
atggagacag acacactcct gctatgggta ctgctgctct gggttccagg ttccactggt 60

gacgcggccc agccggccca gcaccacggt gtgacgaaat gcaacatcac gtgcagcaag 120

atgacatcaa agatacctgt agctttgctc atccactatc aacagaacca ggcatcatgc 180

ggcaaacgcg caatcatctt ggagacgaga cagcacaggc tgttctgtgc cgacccgaag 240

gagcaatggg tcaaggacgc gatgcagcat ctggaccgcc aggctgctgc cctaactcga 300

aatggcggca ccttcgagaa gcagatcggc gaggtgaagc ccaggaccac ccctgccgcc 360

gggggaatgg acgagtctgt ggtcctggag cccgaagcca caggcgaaag cagtagcctg 420

gagccgactc cttcttccca ggaagcacag agggccctgg ggacctcccc agagctgccg 480

acgggcgtga ctggttcctc agggaccagg ctccccccga cgccaaaggc tcaggatgga 540

gggcctgtgg gcacggagct tttccgagtg cctcccgtct ccactgccgc cacgtggcag 600

agttctgctc cccaccaacc tgggcccagc ctctgggctg aggcaaagac ctctgaggcc 660

ccgtccaccc aggacccctc cacccaggcc tccactgcgt cctccccagc cccagaggag 720

aatgctccgt ctgaaggcca gcgtgtgtgg ggtcagggac agagccccag gccagagaac 780

tctctggagc gggaggagat gggtcccgtg ccagcgcaca cggatgcctt ccaggactgg 840

gggcctggca gcatggccca cgtctctgtg gtccctgtct cctcagaagg gacccccagc 900

agggagccag tggcttcagg cagctggacc cctaaggctg aggaacccat ccatgccacc 960

atggaccccc agaggctggg cgtccttatc actcctgtcc ctgacgccca ggctgccacc 1020

cggaggcagg cggtggggct gctggccttc cttggcctcc tcttctgcct gggggtggcc 1080

atgttcacct accagagcct ccagggctgc cctcgaaaga tggcaggaga gatggcggag 1140

ggccttcgct acatcccccg gagctgtggt agtaattcat atgtcctggt gcccgtggcg 1200

gccgcactcg agtctagaat ggacaaagac tgcgaaatga agcgcaccac cctggatagc 1260

cctctgggca agctggaact gtctgggtgc gaacagggcc tgcacgagat caagctgctg 1320

ggcaaaggaa catctgccgc cgacgccgtg gaagtgcctg ccccagccgc cgtgctgggc 1380

ggaccagagc cactgatgca ggccaccgcc tggctcaacg cctactttca ccagcctgag 1440

gccatcgagg agttccctgt gccagccctg caccacccag tgttccagca ggagagcttt 1500

acccgccagg tgctgtggaa actgctgaaa gtggtgaagt tcggagaggt catcagctac 1560

cagcagctgg cggccctggc gggcaatccc gccgccaccg ccgccgtgaa aaccgccctg 1620

agcggaaatc ccgtgcccat tctgatcccc tgccaccggg tggtgtctag ctctggcgcc 1680

gtggggggct acgagggcgg gctcgccgtg aaagagtggc tgctggccca cgagggccac 1740

agactgggca agcctgggct gggcgctgag cacgaatttc gaggagggcc cgaacaaaaa 1800

ctcatctcag aagaggatct gaatagcgcc gtcgaccatc atcatcatca tcattga 1857

<210> 64
<211> 1074
<212> DNA
<213> Homo sapiens

<400> 64
atggagacag acacactcct gctatgggta ctgctgctct gggttccagg ttccactggt 60

gacgcggccc agccggccgc cctggacacc aactattgct tcagctccac ggagaagaac 120

tgctgcgtgc ggcagctgta cattgacttc cgcaaggacc tcggctggaa gtggatccac 180

gagcccaagg gctaccatgc caacttctgc ctcgggccct gcccctacat ttggagcctg 240

gacacgcagt acagcaaggt cctggccctg tacaaccagc ataacccggg cgcctcggcg 300

gcgccgtgct gcgtgccgca ggcgctggag ccgctgccca tcgtgtacta cgtgggccgc 360

aagcccaagg tggagcagct gtccaacatg atcgtgcgct cctgcaagtg cagcgcggcc 420

gcactcgagt ctagaatgga caaagactgc gaaatgaagc gcaccaccct ggatagccct 480

ctgggcaagc tggaactgtc tgggtgcgaa cagggcctgc acgagatcaa gctgctgggc 540

aaaggaacat ctgccgccga cgccgtggaa gtgcctgccc cagccgccgt gctgggcgga 600

ccagagccac tgatgcaggc caccgcctgg ctcaacgcct actttcacca gcctgaggcc 660

atcgaggagt tccctgtgcc agccctgcac cacccagtgt tccagcagga gagctttacc 720

cgccaggtgc tgtggaaact gctgaaagtg gtgaagttcg gagaggtcat cagctaccag 780

cagctggcgg ccctggcggg caatcccgcc gccaccgccg ccgtgaaaac cgccctgagc 840

ggaaatcccg tgcccattct gatcccctgc caccgggtgg tgtctagctc tggcgccgtg 900

gggggctacg agggcgggct cgccgtgaaa gagtggctgc tggcccacga gggccacaga 960

ctgggcaagc ctgggctggg cgctgagcac gaatttcgag gagggcccga acaaaaactc 1020

atctcagaag aggatctgaa tagcgccgtc gaccatcatc atcatcatca ttga 1074

<210> 65
<211> 1269
<212> DNA
<213> Homo sapiens

<400> 65

atggagacag acacactcct gctatgggta ctgctgctct gggttccagg ttccactggt 60

gacgcggccc agccggccac ccccctgggc cctgccagct ccctgcccca gagcttcctg 120

ctcaagtgct tagagcaagt gaggaagatc cagggcgatg gcgcagcgct ccaggagaag 180

ctggtgagtg agtgtgccac ctacaagctg tgccaccccg aggagctggt gctgctcgga 240

cactctctgg gcatcccctg ggctcccctg agcagctgcc ccagccaggc cctgcagctg 300

gcaggctgct tgagccaact ccatagcggc cttttcctct accaggggct cctgcaggcc 360

ctggaaggga tctcccccga gttgggtccc accttggaca cactgcagct ggacgtcgcc 420

gactttgcca ccaccatctg gcagcagatg gaagaactgg gaatggcccc tgccctgcag 480

cccacccagg gtgccatgcc ggccttcgcc tctgctttcc agcgccgggc aggaggggtc 540

ctggttgcct cccatctgca gagcttcctg gaggtgtcgt accgcgttct acgccacctt 600

gcccagcccg cggccgcact cgagtctaga atggacaaag actgcgaaat gaagcgcacc 660

accctggata gccctctggg caagctggaa ctgtctgggt gcgaacaggg cctgcacgag 720

atcaagctgc tgggcaaagg aacatctgcc gccgacgccg tggaagtgcc tgccccagcc 780

gccgtgctgg gcggaccaga gccactgatg caggccaccg cctggctcaa cgcctacttt 840

caccagcctg aggccatcga ggagttccct gtgccagccc tgcaccaccc agtgttccag 900

caggagagct ttacccgcca ggtgctgtgg aaactgctga aagtggtgaa gttcggagag 960

gtcatcagct accagcagct ggcggccctg gcgggcaatc ccgccgccac cgccgccgtg 1020

aaaaccgccc tgagcggaaa tcccgtgccc attctgatcc cctgccaccg ggtggtgtct 1080

agctctggcg ccgtgggggg ctacgagggc gggctcgccg tgaaagagtg gctgctggcc 1140

cacgagggcc acagactggg caagcctggg ctgggcgctg agcacgaatt tcgaggaggg 1200

cccgaacaaa aactcatctc agaagaggat ctgaatagcg ccgtcgacca tcatcatcat 1260

catcattga 1269

<210> 66
<211> 1407
<212> DNA
<213> Homo sapiens

<400> 66
atggagacag acacactcct gctatgggta ctgctgctct gggttccagg ttccactggt 60

gacgcggccc agccggccga accacactca gagagcaatg tccctgcagg acacaccatc 120

ccccaagccc actggactaa acttcagcat tcccttgaca ctgcccttcg cagagcccgc 180

agcgccccgg cagcggcgat agctgcacgc gtggcggggc agacccgcaa cattactgtg 240

gaccccaggc tgtttaaaaa gcggcgactc cgttcacccc gtgtgctgtt tagcacccag 300

cctccccgtg aagctgcaga cactcaggat ctggacttcg aggtcggtgg tgctgccccc 360

ttcaacagga ctcacaggag caagcggtca tcatcccatc ccatcttcca caggggcgaa 420

ttctcggtgt gtgacagtgt cagcgtgtgg gttggggata agaccaccgc cacagacatc 480

aagggcaagg aggtgatggt gttgggagag gtgaacatta acaacagtgt attcaaacag 540

tacttttttg agaccaagtg ccgggaccca aatcccgttg acagcgggtg ccggggcatt 600

gactcaaagc actggaactc atattgtacc acgactcaca cctttgtcaa ggcgctgacc 660

atggatggca agcaggctgc ctggcggttt atccggatag atacggcctg tgtgtgtgtg 720

ctcagcagga aggctgtgag aagagccgcg gccgcactcg agtctagaat ggacaaagac 780

tgcgaaatga agcgcaccac cctggatagc cctctgggca agctggaact gtctgggtgc 840

gaacagggcc tgcacgagat caagctgctg ggcaaaggaa catctgccgc cgacgccgtg 900

gaagtgcctg ccccagccgc cgtgctgggc ggaccagagc cactgatgca ggccaccgcc 960

tggctcaacg cctactttca ccagcctgag gccatcgagg agttccctgt gccagccctg 1020

caccacccag tgttccagca ggagagcttt acccgccagg tgctgtggaa actgctgaaa 1080

gtggtgaagt tcggagaggt catcagctac cagcagctgg cggccctggc gggcaatccc 1140

gccgccaccg ccgccgtgaa aaccgccctg agcggaaatc ccgtgcccat tctgatcccc 1200

tgccaccggg tggtgtctag ctctggcgcc gtggggggct acgagggcgg gctcgccgtg 1260

aaagagtggc tgctggccca cgagggccac agactgggca agcctgggct gggcgctgag 1320

cacgaatttc gaggagggcc cgaacaaaaa ctcatctcag aagaggatct gaatagcgcc 1380

gtcgaccatc atcatcatca tcattga 1407

<210> 67
<211> 2814
<212> DNA
<213> Homo sapiens

<400> 67
atggagacag acacactcct gctatgggta ctgctgctct gggttccagg ttccactggt 60

gacgcggccc agccggccca aaggaaaaga agaaatacaa ttcatgaatt caaaaaatca 120

gcaaagacta ccctaatcaa aatagatcca gcactgaaga taaaaaccaa aaaagtgaat 180

actgcagacc aatgtgctaa tagatgtact aggaataaag gacttccatt cacttgcaag 240

gcttttgttt ttgataaagc aagaaaacaa tgcctctggt tccccttcaa tagcatgtca 300

agtggagtga aaaaagaatt tggccatgaa tttgacctct atgaaaacaa agactacatt 360

agaaactgca tcattggtaa aggacgcagc tacaagggaa cagtatctat cactaagagt 420

ggcatcaaat gtcagccctg gagttccatg ataccacacg aacacagcta tcggggtaaa 480

gacctacagg aaaactactg tcgaaatcct cgaggggaag aagggggacc ctggtgtttc 540

acaagcaatc cagaggtacg ctacgaagtc tgtgacattc ctcagtgttc agaagttgaa 600

tgcatgacct gcaatgggga gagttatcga ggtctcatgg atcatacaga atcaggcaag 660

atttgtcagc gctgggatca tcagacacca caccggcaca aattcttgcc tgaaagatat 720

cccgacaagg gctttgatga taattattgc cgcaatcccg atggccagcc gaggccatgg 780
tgctatactc ttgaccctca cacccgctgg gagtactgtg caattaaaac atgcgctgac 840
aatactatga atgacactga tgttcctttg gaaacaactg aatgcatcca aggtcaagga 900
gaaggctaca ggggcactgt caataccatt tggaatggaa ttccatgtca gcgttgggat 960
tctcagtatc ctcacgagca tgacatgact cctgaaaatt tcaagtgcaa ggacctacga 1020
gaaaattact gccgaaatcc agatgggtct gaatcaccct ggtgttttac cactgatcca 1080
aacatccgag ttggctactg ctcccaaatt ccaaactgtg atatgtcaca tggacaagat 1140
tgttatcgtg ggaatggcaa aaattatatg ggcaacttat cccaaacaag atctggacta 1200
acatgttcaa tgtgggacaa gaacatggaa gacttacatc gtcatatctt ctgggaacca 1260
gatgcaagta agctgaatga gaattactgc cgaaatccag atgatgatgc tcatggaccc 1320
tggtgctaca cgggaaatcc actcattcct tgggattatt gccctatttc tcgttgtgaa 1380
ggtgatacca cacctacaat agtcaattta gaccatcccg taatatcttg tgccaaaacg 1440
aaacaattgc gagttgtaaa tgggattcca acacgaacaa acataggatg gatggttagt 1500
ttgagataca gaaataaaca tatctgcgga ggatcattga taaaggagag ttgggttctt 1560
actgcacgac agtgtttccc ttctcgagac ttgaaagatt atgaagcttg gcttggaatt 1620
catgatgtcc acggaagagg agatgagaaa tgcaaacagg ttctcaatgt ttcccagctg 1680
gtatatggcc ctgaaggatc agatctggtt ttaatgaagc ttgccaggcc tgctgtcctg 1740
gatgattttg ttagtacgat tgatttacct aattatggat gcacaattcc tgaaaagacc 1800
agttgcagtg tttatggctg gggctacact ggattgatca actatgatgg cctattacga 1860
gtggcacatc tctatataat gggaaatgag aaatgcagcc agcatcatcg agggaaggtg 1920
actctgaatg agtctgaaat atgtgctggg gctgaaaaga ttggatcagg accatgtgag 1980
ggggattatg gtggcccact tgtttgtgag caacataaaa tgagaatggt tcttggtgtc 2040
attgttcctg gtcgtggatg tgccattcca aatcgtcctg gtatttttgt ccgagtagca 2100
tattatgcaa aatggataca caaaattatt ttaacatata aggtaccaca gtcagcggcc 2160
gcactcgagt ctagaatgga caaagactgc gaaatgaagc gcaccaccct ggatagccct 2220
ctgggcaagc tggaactgtc tgggtgcgaa cagggcctgc acgagatcaa gctgctgggc 2280
aaaggaacat ctgccgccga cgccgtggaa gtgcctgccc cagccgccgt gctgggcgga 2340
ccagagccac tgatgcaggc caccgcctgg ctcaacgcct actttcacca gcctgaggcc 2400
atcgaggagt tccctgtgcc agccctgcac cacccagtgt tccagcagga gagctttacc 2460
cgccaggtgc tgtggaaact gctgaaagtg gtgaagttcg gagaggtcat cagctaccag 2520
cagctggcgg ccctggcggg caatcccgcc gccaccgccg ccgtgaaaac cgccctgagc 2580
ggaaatcccg tgcccattct gatcccctgc caccgggtgg tgtctagctc tggcgccgtg 2640
gggggctacg agggcgggct cgccgtgaaa gagtggctgc tggcccacga gggccacaga 2700

ctgggcaagc ctgggctggg cgctgagcac gaatttcgag gagggcccga acaaaaactc 2760

atctcagaag aggatctgaa tagcgccgtc gaccatcatc atcatcatca ttga 2814

<210> 68
<211> 1558
<212> DNA
<213> Homo sapiens

<400> 68
atggagacag acacactcct gctatgggta ctgctgctct gggttccagg ttccactggt 60

gacgcggccc agccggccgg cagtgatcac tttgcagcct ccatgggtca gcgtgttcca 120

agaggaaacc gtaaccttgc actgtgaggt gctccatctg cctgggagca gctctacaca 180

gtggtttctc aatggcacag ccactcagac ctcgaccccc agctacagaa tcacctctgc 240

cagtgtcaat gacagtggtg aatacaggtg ccagagaggt ctctcagggc gaagtgaccc 300

catacagctg gaaatccaca gaggctggct actactgcag gtctccagca gagtcttcat 360

ggaaggagaa cctctggcct tgaggtgtca tgcgtggaag gataagctgg tgtacaatgt 420

gctttactat cgaaatggca aagcctttaa gtttttccac tggaattcta acctcaccat 480

tctgaaaacc aacataagtc acaatggcac ctaccattgc tcaggcatgg gaaagcatcg 540

ctacacatca gcaggaatat cacaatacac tgtgaaagag ctatttccag ctccagtgct 600

gaatgcatct gtgacatccc cactcctgga ggggaatctg gtcaccctga gctgtgaaac 660

aaagttgctc ttgcagaggc ctggtttgca gctttacttc tccttctaca tgggcagcaa 720

gaccctgcga ggcaggaaca catcctctga ataccaaata ctaactgcta gaagagaaga 780

ctctgggtta tactggtgcg aggctgccac agaggatgga aatgtcctta agcgcagccc 840

tgagttggag cttcaagtgc ttggcctccg gttaccaact cctgtctggt ttcatgtcgc 900

ggccgcactc gagtctagaa tggacaaaga ctgcgaaatg aagcgcacca ccctggatag 960

ccctctgggc aagctggaac tgtctgggtg cgaacagggc ctgcacgaga tcaagctgct 1020

gggcaaagga acatctgccg ccgacgccgt ggaagtgcct gccccagccg ccgtgctggg 1080

cggaccagag ccactgatgc aggccaccgc ctggctcaac gcctactttc accagcctga 1140

ggccatcgag gagttccctg tgccagccct gcaccaccca gtgttccagc aggagagctt 1200

tacccgccag gtgctgtgga aactgctgaa agtggtgaag ttcggagagg tcatcagcta 1260

ccagcagctg gcggccctgg cgggcaatcc cgccgccacc gccgccgtga aaaccgccct 1320

gagcggaaat cccgtgccca ttctgatccc ctgccaccgg gtggtgtcta gctctggcgc 1380

cgtggggggc tacgagggcg ggctcgccgt gaaagagtgg ctgctggccc acgagggcca 1440

cagactgggc aagcctgggc tgggcgctga gcacgaattt cgaggagggc ccgaacaaaa 1500

actcatctca gaagaggatc tgaatagcgc cgtcgaccat catcatcatc atcattga 1558

<210>  69
<211>  1491
<212>  DNA
<213>  Mus sp.

<400>  69
atggagacag acacactcct gctatgggta ctgctgctct gggttccagg ttccactggt      60

gacgcggccc agccggccat ggcccaggtc aagctgcagg agtcagggac tgaactggca     120

aagcctgggg ccgcagtgaa gatgtcctgc aaggcttctg gctacacctt tactgactac     180

tggatgcact gggttaaaca gaggcctgga cagggtctgg aatggattgg atacattaat     240

cctaacactg cttatactga ctacaatcag aaattcaagg acaaggccac attgactgca     300

gacaaatcct ccagcacagc ctacatgcaa ctgcgcagcc tgacctctga ggattctgca     360

gtctattact gtgcaaaaaa gacaactcag actacgtggg ggtttccttt ttggggccaa     420

gggaccacgg tcaccgtctc ctcaggtgga ggcggttcag gcggaggtgg ctctggcggt     480

ggcggatcgg acattgtgct gacccagtct ccaaaatcca tggccatgtc agtcggagag     540

agggtcacct tgagctgcaa ggccagtgag aatgtggatt cttttgtttc ctggtatcaa     600

cagaaaccag gccagtctcc taaactgctg atatacgggg cctccaaccg gtacactggg     660

gtccccgatc gcttcgcagg cagtggatct ggaagagatt tcactctgac catcagcagt     720

gtgcaggctg aagaccttgc agattatcac tgtggacaga attacaggta tccgctcacg     780

ttcggtgctg gcaccaagct ggaaatcaaa cgggcggccg catctggcgg tggcggatcg     840

ctcgagtcta gaatggacaa agactgcgaa atgaagcgca ccaccctgga tagccctctg     900

ggcaagctgg aactgtctgg gtgcgaacag ggcctgcacg agatcaagct gctgggcaaa     960

ggaacatctg ccgccgacgc cgtggaagtg cctgccccag ccgccgtgct gggcggacca    1020

gagccactga tgcaggccac cgcctggctc aacgcctact ttcaccagcc tgaggccatc    1080

gaggagttcc ctgtgccagc cctgcaccac ccagtgttcc agcaggagag ctttacccgc    1140

caggtgctgt ggaaactgct gaaagtggtg aagttcggag aggtcatcag ctaccagcag    1200

ctggccgccc tggccggcaa tcccgccgcc accgccgccg tgaaaaccgc cctgagcgga    1260

aatcccgtgc ccattctgat cccctgccac cgggtggtgt ctagctctgg cgccgtgggg    1320

ggctacgagg gcgggctcgc cgtgaaagag tggctgctgg cccacgaggg ccacagactg    1380

ggcaagcctg ggctgggcgc tgagcacgaa tttcgaggag ggcccgaaca aaaactcatc    1440

tcagaagagg atctgaatag cgccgtcgac catcatcatc atcatcattg a             1491


<210>  70
<211>  1500
<212>  DNA
<213>  Mus sp.

<400>  70
atggagacag acacactcct gctatgggta ctgctgctct gggttccagg ttccactggt      60

gactctagaa tggacaaaga ctgcgaaatg aagcgcacca ccctggatag ccctctgggc 120

aagctggaac tgtctgggtg cgaacagggc ctgcacgaga tcaagctgct gggcaaagga 180

acatctgccg ccgacgccgt ggaagtgcct gccccagccg ccgtgctggg cggaccagag 240

ccactgatgc aggccaccgc ctggctcaac gcctactttc accagcctga ggccatcgag 300

gagttccctg tgccagccct gcaccaccca gtgttccagc aggagagctt tacccgccag 360

gtgctgtgga aactgctgaa agtggtgaag ttcggagagg tcatcagcta ccagcagctg 420

gccgccctgg ccggcaatcc cgccgccacc gccgccgtga aaaccgccct gagcggaaat 480

cccgtgccca ttctgatccc ctgccaccgg gtggtgtcta gctctggcgc cgtggggggc 540

tacgagggcg ggctcgccgt gaaagagtgg ctgctggccc acgagggcca cagactgggc 600

aagcctgggc tgggcggatc caaactagct gagcacgaag gtgacgcggc ccagccggcc 660

atggcccagg ttcagcttca gcagtctgga gctgagctga tgaagcctgg ggcctcagtg 720

aagatatcct gcaaggctac tggctacaca ttcaataaca actggataga gtgggtaaag 780

cagaggcctg gacatggcct tgagtggatt ggagagattt tacctggaag tggtagtact 840

aagtacaatg agaagttcaa gggcaaggcc acattcactg cagatacatc ctccaagaca 900

gcctacatgc aactcagcag cctgacatct gaggactctg ccgtctatta ctgtacaagg 960

gtgtatggta actacaatgc tatggattac tggggtcaag gaacctcggt caccgtctcc 1020

tcaggctcca cctcaggctc cggtaaacct ggcccagggg agggatcaac taagggcgcg 1080

cctcaggttg ttctcactca gtcatctgca ctcaccacat cacctggtga aacagtcaca 1140

ctcacttgtc gctcaagtac tggggctgtt acaactagta actatgccaa ctgggtccaa 1200

gaaaaaccag atcatttatt cactggtcta ataggtggta ccaacaaccg agctccaggt 1260

gttcctgcca gattctcagg ctccctgatt ggagacaagg ctgccctcac catcacaggg 1320

gcacagactg aggatgaggc aatatatttc tgtgctctat ggtacagcaa ccattgggtg 1380

ttcggtggag gaaccaaagt gactgtccta ggccaggtcg acgcggccgc agggcccgaa 1440

caaaaactca tctcagaaga ggatctgaat agcgccgtcg accatcatca tcatcatcat 1500

## Description

### Field of the invention

The present invention relates to a complex formed from at least one component A and at least one component B. The present invention also relates to nucleic acids and/or vectors coding for such a complex. Furthermore an integral part of the complex comprises a component C which consists of an orthogonale substrate for component B which is chemically linked to a chemical or solid matter. Component C is added in a covalent coupling reaction to B through a substrate specific manner thereby transferring its inherent physico-chemical properties to the complex ABC.

### Background of the invention

Today there is a series of approved methods available for diagnosis and/or therapy of malignant disorders in man like cancer, chronic inflammatory diseases and allergy. The classical therapeutic approaches have, because of their relatively unselective nature (for example radio- and chemotherapy in cancer treatment), a lot of severe side effects.

In the field of diagnosis there exist a lot of new high resolution imaging technologies that render possible a very exact topographic localization of e.g. solid tumors (X-Ray/magnetic resonance imaging (MRI)). Despite the correct localization the tumor biology and physiology is of great importance for the kind of therapy being optimal.

Modern molecular biology approaches like antibody technology open new opportunities and way of diagnosis and therapy. As a selective component and fusion partner to therapeutics/diagnostics can target almost every desired cell /tissue specific marker. They also drastically improve the specificity of therapy and limit the incidence of false, false-positive/false-negative diagnosis.

Before their application as immunodiagnostic tool or as immunotherapeutic (e.g. immunotoxin) full length antibodies have to be modified with detectable agents or effector molecules. A gold standard is still the chemical methodology. However, the chemical modification of antibodies very often leads to complications like loss of binding activity or specificity. Chemical properties of the generic proteins like solubility are also affected negatively in some cases. Due to more and more sophisticated applications there is a strong demand for functional modificated antibodies.

A further development in this field is the genetic fusion of recombinant antibodies to effector molecules. Unfortunately each of the resulting fusion proteins is limited to one or a few applications. For each new application field the antibody has to be coupled to another suited effector molecule resulting in loborious proces optimizations for each case. Additionally this approach is limited to peptidic effector molecules.

An object of the invention is to avoid as far as possible the loss of binding activity and specificity due to chemical modification of full length antibodies in e.g. fusion proteins used for cell targeting.

A further object of the present invention is providing a compound which enables the skilled person to use a similar or same tool both for diagnosis and therapy of diseases. Still another object of the invention is providing a compound avoiding immunogenicity/strong side effects of of immunotherapeutics.

Yet another object of the invention is to provide a missing link between classical therapeutic approaches and more specific new technologies

### Summary of the invention

The invention relates to novel compounds, in particular fusion proteins, comprising at least one antigen specific binding moiety and at least one enzyme type protein which reacts covalently with a specific substrate.

The objects of the invention are solved by a compound comprising three components A, B, and C, which components are covalently bound forming the compound having the structure A-B-C wherein
component A has a specific binding affinity for antigens,
component B is covalently linked to component A
component C is a compound having an alkylated purin or pyrimidin moiety such as guanin, cytosin or a Coenzyme A moiety and linked thereto a moiety having a physiological effect with the proviso that
component B has an catalytical or acceptor activity to couple component C with covalently coupled components A-B.

In one particular embodiment of the invention the compound can be rearded as a complex with the generic structure:

### Antigen binding moiety (A) - enzyme type protein (B) - C

The compound of the invention is in particular a heterologous complex comprising at least one recombinant fusion protein comprising at least one specific binding component A in particular cell-specific binding component and one enzyme type protein B and at least one additional component C that is covalently coupled to B.

In another embodiment of the invention the compound is a heterologous complex comprising at least one recombinant fusion protein comprising at least one component A binding to a soluble antigen and one enzyme type protein B and at least one additional component C that is covalently coupled to B.

In a specific embodiment, the compound of the invention has a covalent modification of component A with component C through component B.

In a further embodiment of the invention component A of the compound of the invention is a chemical moiety having a polypeptidic antigen binding structure and component B is an enzyme type protein linked to component A.

In yet another embodiment, component B of the compound of the invention is capable of reacting with component C in a substrate specific manner, thereby connecting covalently the complex AB with component C.

In particular, component A of the compound of the invention belongs to the group of antigen binding polypeptides/proteins targeting celltype specific markers, in particular component A is directed against disease specific structures of pathogenic substances or pathogenic matter. Some representatives of component A comprise moieties which are affinity moieties from affinity substances or affinity substances in their entirety selected from the group consisting of antibodies, receptor/receptor ligands, including protein A/IgG, avidin/biotin and the like, enzyme substrates, lectins, interleukins, cytokines, chemokines, lymphokines, allergens, peptidic allergens, recombinant allergens, allergen-idiotypical antibodies, autoimmune-provoking structures, tissue-rejection-inducing structures, immunoglobulin constant regions and their derivatives, mutants or combinations thereof. Furthermore component A can bind to soluble markers of disease/environment/food and feed safety or biodefense (e.g. toxins).

Component A may have a specific binding affinity also to antigens, which are immunologically relevant only when coupled to immunogenic molecules. These compounds typically addressed as haptenes are for instance low molecular substances, which - as individuals - are not provoking an immune response, but when inked to an immunogenic compound such as a protein.

In an embodiment of the invention the component B of the compound of the invention is a polypeptide that reacts covalently with a specific substrate. In particular, component B may be a derivative of the human DNA repair protein O⁶-alkylguanine-DNA alkyltransferase (AGT). The component B can be derived from the Acyl Carrier Protein (ACP). A person skilled in the art recognizes that there may be multiple alterations and modifications on the DNA or the amino acid level which lead to components with functional equivalence.

In a specific embodiment of the invention the substrate for component B consists of 06-benzylguanine, 02-benzylcytosine or a coenzyme A (CoA).

Advantageously in the compound of the invention components A-B are covalently coupled polypeptides.

Component C holds physico-chemical or physiological properties to be transferred to the complex AB.

In an embodiment of the invention component C of the compound of the invention is a drug, a detectable label or other components mediating biological activity in a targeted cell or organism.

In another embodiment of the invention component C of the compound of the invention is a solid phase or a support.

In a further embodiment of the invention component C of the compound of the invention comprises a moiety which serves as a substrate for component B.

In particular, component C can have the structure

(X)ₙ₁-(Y) -(Z)ₙ₂

with X being a for component B specific substrate and n1 being one or more preferentially 1-3 and Z being a drug, a detectable label or other components mediating biological activity in a targeted cell or organism and n2 being 1 or more.

The structural element Y of component C may fullfill the following fuctions: a spacer mediating the desired flexibility between X and Z (and this way between B and C) ensuring the functionality of each component within the assembled complex.

Further the linker structural element may contain structures enabling the controlled release of Z under certain environmental conditions during interactions like chemical reactions (e.g. pH sensitive or reducable structures for release in endosomes or the cytosol).

The linker structural element Y may also have linear, branched, tree like or polymeric structure.

Subject matter of the invention is also a nucleic acid molecule coding for polypeptides of the invention.

In an additional embodiment of the present invention there are provided expression cassettes comprising a polynucleotide encoding the polypeptide, in particular a chimeric polypeptide, comprising components A and B in the order AB or BA. Further different versions are possible: AAB, ABB, AAAB, AAAAB, BAA; BBA, BAAA, BAAAA, BAB and ABA.

The nucleic acid molecule of the invention and expression cassette of the invention may further be a part of a vector or vector system suitable for expression of the complexes AB (BA) in a host cell. Therefor also the vector is subject matter of the invention.

In a further embodiment there are provided methods for the expression of the recombinant genes encoding the recombinant compounds of the invention.

In a further embodiment the present invention provides for a method using a host cell comprising an afore mentioned expression vector of the invention and culturing the host cell under conditions suitable for the expression of the invention related complexes.

The host cell is further defined as a procaryotic host cell or a eucaryotic host cell like mammalian, plant or yeast cells.

Moreover the invention relates to methods of reacting a complex AB (BA) with a compound C comprising one ore more enzyme substrates for which B is specific and further carrying one or more copies of a drug, a detectable label or other components mediating biological activity in a targeted cell or organism.

Furthermore the invention relates to methods of preparing and administering the invention related complexes to cells *in vitro* and *in vivo,* with C carrying one or more copies of a drug, a detectable label or other components mediating biological activity in a targeted cell or organism.

Applications of the invention related complex include in vitro and in vivo diagnostic approaches in the field of human and animal disorders as well as analytic approaches in the field of environmental monitoring, ecotoxicology and biosensor applications, with C being or containing a detectable label.

In a certain embodiment of the afore mentioned applications the complex will be used in therapy for human and animal disorders, with C being or containing a drug or components mediating biological activity in a targeted cell or organism.

In a specific embodiment component A is directly immobilized via component C to a given surface (planar, bead) allowing to enrich the marker at a distinct location.

In another specific embodiment, component A is used to detect an enriched marker (pref. soluble, e.g. soluble CD30/CEA/ PSA/ sIL-2R/ sFAS/ sCD23/sCD26/ sCD40L/ sCD40/ CRP/ sVCAM-1/ MCP1/ thrombomodulin/ plasma C4bBP/Protein C/ activated proteinC/ proteinS/ von willebrand factor/ TNFR / p55 / p75 / Fas (CD95) /Nerve growth factor R / CD27 / CD30 /Growth hormone R/ GM-CSF/ Erythropoietin-R/ Thrombopoietin /G-CSF/ IL-IRI / IL-IRII/ IL-2Ra (Tac, CD25) IL-4R / IL-5Ra/IL-7R/ IL/ CNTFR/ LIFR /Leptin R/ IL-11R / IL-12/ Stem cell factor R (c-kit)/ Interferon R/ Lipopolysaccharide R (CD14)/ Complement receptor Type I/ Hyaluronate R (CD44)/ CD58/ IgER (FceRII, CD23)/IgGR (FcgRII)/ ICAM-1 (CD54)/ ICAM-3 (CD50)/ Transforming growth factor bRIII/Epidermal growth factor R (c-erb B)/ Vascular endothelial growth factor R/Platelet derived growth factor R/ Fibroblast growth factor / Colony stimulating factor-1R (MCFR, c-fms)/ ARK/ Tie/ Insulin R / Insulin-like growth factor-IIR/mannose 6-phosphate R) at a distinct location (spot, bead) via component C having the following characteristics: optical including fluorescence, magnetic including resp. beads (e.g. FeOH-based), radiolabel including gamma ray emitting nuclides like Technetium-99m, Thallium-201, Gallium-67, Fluorine-18, Indium-111, ultrasound including resp. bubbles, electrochemical including enzymes like alkaline phosphatase, oligonucleotides like hybridization probes for PCR.

In vitro/in vivo detection of the distinct cells is via component C having the above-mentioned characteristics.

In an additional embodiment, the component A is binding to a cell surface marker being internalized (EGFR, CD30R, BCR, and the like); component C is an agent selected from the group of small molecules having cytotoxic/cytostatic activities.

In another specific embodiment, the component A is binding to a cell surface marker (MUC1, Syndecanl), not being internalized; component C is an agent delivering CpG motives, beta ray emitting nuclides like Iodine-131, Yttrium-90, Lutetium-177, or enzymes activating cytotoxic agents (directed enzyme prodrug therapy: DEPT using e.g. carboxypeptidase as enzyme).

In another embodiment component A contains or is composed of D-amino acids in an artificial process copying the above mentioned proteins/peptides which naturally are synthesized with L- amino acids.

In specific embodiments components A and B may be modified with or contain chemically modified azido and alkynyl monosaccharide precursors for labeling glycans, unnatural amino acids bearing azides and alkynes for residue-specific protein labeling or azido lipid substrates for probing lipidated proteins.

The process, called bioorthogonal labeling enables a site-specific modification of components A or B via click chemistry like described by Baskin, J. and Bertozzi C.(2007).

### Detailed description of the invention

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

The invention also relates to diagnostic, therapeutic or analytical compositions of the heterologous complex, methods of producing such complexes and methods of using the same *in vitro* and *in vivo.*

As used herein, the specification, "a" or "an" may mean one ore more. As used herein in the claim(s), when used in conjunction with the word "comprising", the words "a" and "an" may mean one or more than one. As used herein "another" may mean at least a second or more.

As used herein, the term "component A" of the complex represents the actively binding structure of the complex of present invention. The component A is selected from the group of actively binding structures consisting of antibodies or their derivatives or fragments thereof, synthetic peptides such as scFv, mimotopes, etc. or chemical molecules such as carbohydrates, lipids, nucleic acids, peptides, vitamins, etc., and/or small molecules with up to 100 atoms with receptor-binding activity like ligands, in particular single atoms, peptidic molecules, non-peptidic molecules, etc., and/or cell surface carbohydrate binding proteins and their ligands such as lectins, in particular calnexins, c-type lectins, I-type lectins, m-type lectins, p-type lectins, r-type lectins, galectins and their derivatives, and/or receptor binding molecules such as natural ligands to the cluster of differentiation (CD) antigens, like CD30, CD40, etc., cytokines such as chemokines, colony stimulating factors, type-1 cytokines, type-2 cytokines, interferons, interleukins, lymphokines, monokines, etc., and/or adhesion molecules including their derivatives and mutants, and/or derivatives or combinations of any of the above listed of actively binding structures, which bind to CD antigens, cytokine receptors, hormone receptors, growth factor receptors, ion pumps, channel-forming proteins. The component A may also be selected from the group of passively binding structures consisting of allergens, peptidic allergens, recombinant allergens, allergen-idiotypical antibodies, autoimmune-provoking structures, tissue-rejection-inducing structures, immunoglobulin constant regions and their derivatives, mutants or combinations thereof. Combining at least two identical or different binding structures selected from the above-mentioned groups may generate a component A with higher valency.

In an additional object of the present invention, component A is binding to a cell surface marker of a healthy or diseased cell belonging to the cluster of differentiation antigens (CD-antigens, Table 1).

In another specific embodiment, the component A is a chemokine or a specifically binding fragment thereof like those provided in table 2 binding to its specific cellular receptors.

In another embodiment, component A is an interleukin or a specifically binding fragment thereof like those provided in table 3 binding to its specific cellular receptor.

In another embodiment, component A is the extracellular or intracellular part of a cluster of differentiation antigens as listed in table 1 specifically binding to soluble factors and being used to detect a soluble antigen or a family of soluble antigens.

In another specific embodiment, the component A is an angiogenic factor modulating growth, chemotactic behavior and/or functional activities of vascular endothelial cells or a specifically-binding fragment thereof including AcSDKP, aFGF, ANF, Angiogenin, angiomodulin, Angiotropin, AtT20-ECGF, B61, bFGF, bFGF inducing activity, CAM-RF, ChDI, CLAF, ECGF, ECI, EDMF, EGF, EMAP-2, Neurothelin (see: EMMPRIN), Endostatin, Endothelial cell growth inhibitor, Endothelial cell-viability maintaining factor, Epo, FGF-5, IGF-2 (see: Growth-promoting activity for vascular endothelial cells), HBNF, HGF, HUAF, IFN-gamma, IL1, K-FGF, LIF, MD-ECI, MECIF, NPY, Oncostatin M, PD-ECGF, PDGF, PF4, PIGF, Prolactin, TNF-alpha, TNF-beta, Transferrin, VEGF. Some of these factors are protein factors detected initially due to some other biological activities and later shown to promote angiogenesis. The list of protein factors angiogenically active in vivo includes fibroblast growth factors (see: FGF), Angiogenin, Angiopoietin-1, EGF, HGF, NPY, VEGF, TNF-alpha, TGF-beta, PD-ECGF, PDGF, IGF, IL8, Growth hormone. Fibrin fragment E has been shown also to have angiogenic activity. In addition there are factors such as Angiopoietin-1, which do not behave as classical growth factors for endothelial cells but play a prominent role in vasculogenic and angiogenic processes.

In another embodiment, the component A is a virulence factor or the corresponding part of it binding to a subset of human cells such as 121R, 14.7 kDa orf virus protein, 145R, 16 kDa orf virus protein, 2C, 38K gene of Cowpox virus, 3a, 5EL, 5-HL, 7a, A224L, A238L, A39R, A41L, AcMNPV ORF32, Actinobacillus actinomycetem comitans Cytolethal distending toxin, Actinobacillus actinomycetem comitans leukotoxin, Adenovirus Death Protein, Adenovirus E1B 19kDa protein, Adenovirus E3 10.4K/14.5kDa protein, Adenovirus E3 14.7kDa protein, Adenovirus E3 19kDa protein, Aerolysin, AgMNPV IAP3, AHV-Sema, AIP56, Alpha-Hemolysin, alpha-HL, Alpha-toxin, Anti-cytokines, Apoptin, Apoptosis, B13R, B15R, B18R, B8R, Bacillus anthracis toxin, Bacteriokine, baculovirus p35 protein, baculovirus P49 protein, BAD1, BALF-1, BARF1, BCK, BCL2, BCRF-1, Beta-Hemolysin, Beta-toxin, BHRF-1, Bm-MIF, BmNPV FGF, Bordetella dermonecrotic toxin, BORFE2, BPV-1 E6, BZLF1, C12L, C21L, CADD, Campylobacter Cytolethal distending toxin, caspase-7-like protein, Caspases, CDT, Ce-MIF, Chemokines, Circovirus type 2 ORF3, CLAP, Clostridium perfringens alpha-toxin, Clostridium perfringens beta-toxin, CMV IL10, CMV RR1, CNF1, CNF2, COPE version 15.8, COPE version 8.7, COPE, crmA, crmB, crmC, crmD, crmE, Cytokine assays, Cytokine Inter-species Reactivities, Cytokines, D7L, Delta-hemolysin, Delta-toxin, E1.1, E1B-55K, E2, E3-6.7, E3L, E3L-like protein, E4orf4, E5, E6, E7, E8, Early response gene, EBNA-LP, ECRF-3, ectromelia poxvirus p13, Ectromelia virus p28 protein, EHV-2 E1 ORF, EHV-2 IL10, EP153R, EP402R, Erns, Escherichia coli Cytolethal distending toxin, F1L, FLIP, FPV016 protein, Fractalkine, Fumonisins, Fusobacterium necrophorum leukotoxin, G4R, G5R, GAM-1, Gamma-hemolysin, GIF, glycoprotein G, gp120, GPCMV-MIP, H3L, H4R, H83, Haemophilus ducreyi Cytolethal distending toxin, HBx, Helicobacter Cytolethal distending toxin, hemolysin BL, Herpesvirus saimiri BCL2, HJ1, HP1118, HP-NAP, HSGF-2, HVP IL10, HVS13, IAP, ICP0, ICP10PK, ICP22, ICP27, ICP34.5, IE1, IE2, IE2579aa, IMP, Influenza A virus NS1 protein, IpaB, ITA, K13, K2, K2R, K3R, K4.1, K6, KSHV ORF4, KSHV, L*, LANA-2, Leishmania mexicana cysteine protease CPB2.8, LMP2A, M11L, m131/129, M3, M33, M78, MALP-404, Mannheimia haemolytica leukotoxin, MC148R, MC159, MC53L, MC54L, MDM, MDV003, MDV078, MEQ, MGF, Microcystin-LR, Microkine, Modulins, M-T1, M-T7, MyD, N1R, Nipah virus P protein, Nipah virus V protein, Nipah virus W protein, Npro, NS1, NS2, NS5A, orf virus IL10, orf virus, ORF, ORF13, ORF152, ORF16, ORF390, ORF45, ORF50, ORF74, ORFK2, ORFK4.1, ORFK4, ORFK5, ORFK6, ORFK7, ORFK9, ORFV2-VEGF, p13, Panton-Valentine leukocidin, Pasteurella multocida toxin, PB1-F2, Poxvirus growth factor, PRGF, Pseudomonas aeruginosa exotoxin A, RK-BARFO, RRV ORF74, RSV Glycoprotein G, RTA, SARS coronavirus E protein, SARS coronavirus N protein, SARS coronavirus non-structural protein-1, SCMV IL10, SERP1, SERP2, SERP3, SFGF, Shigella Cytolethal distending toxin, sigmaC, SipB, sis, Sliap, Slp49, SPI-2, SPV146, Staphylococcus aureus alpha-toxin, Staphylococcus aureus delta-toxin, Staphylococcus aureus gamma-toxin, STI, Streptolysin O, SV40 large T antigen, SV5 V protein, swinepox virus SPV003/148 protein, T2, TAIP, Tanapoxvirus 2L protein, Tanapoxvirus 38 kDa protein, Thogoto virus ML protein, Trypanokine, U12, U51, U83, U83A, UL111.5A, UL111a, UL119-UL118, UL141, UL144, UL146, UL147, UL18, UL3 protein, UL36, UL37, UL69 protein, UL82, US27, US28, US3, Us5, V protein, VacA, Vaccinia 19 kDa protein, Vaccinia growth factor, Vaccinia virus growth factor, Vaccinia virus protein phosphatase VH1, vBCK, vBCL2, vC4bBP, vCCI, vCCL1, vCKBP, vCKBP-1, vCKBP-2, vCKBP-3, vCKBP-4, VCP, vCSF1BP, VEGF-E, vFGF, VG71, vGPCR, vICA, vIL17, vIL18BP, vIL6, vIL8, viral BCK, viral BCL2, viral C4b binding protein, viral CCL1, viral CD30, viral chemokine binding protein, viral chemokine binding protein-1, viral chemokine binding protein-2, viral chemokine binding protein-3, viral chemokine binding protein-4, viral chemokine inhibitor, viral CSF1 binding protein, viral cytokine receptors, viral cytokines, viral EGF, viral Fc-gamma R2, viral Fc-gamma R3, Viral FLICE-inhibitory proteins, viral G-protein-coupled receptor, viral IFN-gamma/IL2/IL5 binding protein, viral IL10, viral IL17, viral IL18 binding protein, viral IL6, viral IL8, viral inhibitor of apoptosis protein, viral inhibitor of caspase activation, viral interferon regulatory factor, viral interferon regulatory factor-1, viral interferon regulatory factor-2, viral interferon regulatory factor-3, viral M-CSF binding protein, viral MIP-1, viral MIP-1-alpha, viral MIP-1-beta, viral MIP-2, viral NGF-beta, viral OX2, viral semaphorin, viral TGF-beta, viral VEGF, vIRF, vIRF1, vIRF2, vIRF3, Viroceptor, Virokine, vMCC-1, vM-CSFBP, vMIA, vMIP-1, vMIP-1-alpha, vMIP-1-beta, vMIP-2, vMIP-3, vNGF-beta, vOX2, VP35 protein, VPS, vTGF-beta, vTNFR, VVGF, Y134R, Yaba monkey tumor virus 2L protein, YLDV IL10, YopJ, ZmpB, Zta.

As used herein, the term "antibody" refers to polyclonal antibodies, monoclonal antibodies, humanized antibodies, single-chain antibodies, and fragments thereof such as Fab, F(ab')2, Fv, and other fragments which retain the antigen binding function and specificity of the parent antibody.

As used herein, the term "monoclonal antibody" refers to an antibody composition having a homogeneous antibody population. The term is not limited regarding the species or source of the antibody, nor is it intended to be limited by the manner in which it is made. The term encompasses whole immunoglobulins as well as fragments such as Fab, F(ab')2, Fv, and others which retain the antigen binding function and specificity of the antibody. Monoclonal antibodies of any mammalian species can be used in this invention. In practice, however, the antibodies will typically be of rat or murine origin because of the availability of rat or murine cell lines for use in making the required hybrid cell lines or hybridomas to produce monoclonal antibodies.

As used herein, the term "human antibodies" means that the framework regions of an immunoglobulin are derived from human immunoglobulin sequences.

As used herein, the term "single chain antibody fragments" (scFv) refers to antibodies prepared by determining the binding domains (both heavy and light chains) of a binding antibody, and supplying a linking moiety, which permits preservation of the binding function. This forms, in essence, a radically abbreviated antibody, having only that part of the variable domain necessary for binding to the antigen. Determination and construction of single chain antibodies are described in US-A-4,946,778 to Ladner et al.

The component B is an enzyme like protein derived from the Alklguanine-DNA-alkyltransferase (AGT), which has a substrate specificity for O⁶ -benzylguanine or O⁶ heteroarylmethylguanine. The enzyme like protein is able to transfer a certain label from the substrate in a reaction previously described in WO/2005/085470.

In a specific embodiment the enzyme like protein has been modified to recognize 2-amino-4-benzyloxypyrimidines as described in WO/2006/114409.

The component B may also be an enzyme like protein derived from the protein Alkylcytosine transferase (ACT), which has the substrate specificity for O²-benzylcytosine derivatives and realted O² heteroarylmethyl-cytosine derivatives described previously in WO/2008/012296.

In an alternate embodiment of the invention B consists of an Acyl carrier protein or fragments thereof. Coenzym A derivatives are able to transfer their label to the ACP or part of the ACP in the presence of the modifying enzyme holo-acyl carrier protein (ACPS) or modification or mutants thereof as previously described in WO/2004/104588.

The DNA sequences of the invention may be engineered in order to alter a chimeric coding sequence for a variety of modifications, including but not limited to alterations, which modify processing, and expression of the gene product. For example, mutations my be introduced by techniques which are well known in the art, for example site directed mutagenesis or SOE-PCR to insert or remove restriction sites, to alter glycosylation or phosphorylation pattern or to alter the substrate specificity of the active center.

As used herein, the term "component C" of the complex represents a specific additional function added to the complex AB through covalent coupling. Component C is a drug, a detectable label or other components mediating biological activity in a targeted cell or organism. C can also be a solid phase.

C further contains a moiety which serves as a substrate for component B.

Component C can have the structure (X)ₙ₁-(Y) -(Z)ₙ₂ with X being a component B specific substrate and n1 being one or more preferentially 1-3 and Z being a drug, a detectable label or other components mediating biological activity in a targeted cell or organism and n2 being 1 or more.

Y is a linker structure designed to functionally connect X and Z. Y may fullfill the following functions: a spacer mediating the desired flexibility between X and Z (and this way between B and C) ensuring the functionality of each component within the assembled complex.

Further the linker may contain structures enabeling the controlled release of Z under certain environmental conditions (e.g. pH sensitive or reducable structures for release in endosomes or the cytosol or enzyme degradable linkers). Such linker structures may be e.g. cis-Aconityl linkages, linkers containing an ester bond, acid sensitive hydrazone linkers, lysosomally degradable peptide linkers, self eliminating spacers, sulphhydryl linkers, light sensitive linkers (reviewed in Dyba et al.)

Further the linker may contain chelating agents such as DOTA (1,4,7,10-tetraazacyclododecane-1,4,7,10-tetraacetic acid) or DTAP (Diethylene triamine pentaacetic acid) that can be used for complexing e.g. radioisotopes.

Y may have linear, branched, tree like or polymeric structure.

Drugs considered as component C include all kinds of substances that can display their mode of action on the targeted cell and that are likely to be more effective when transported to a particular site within the body. Preferentially these are compounds with proven efficacy e.g. as chemotherapeutical agents. They may be selected from the group of alkylating agents (e.g. cyclophosphamide, chlorambucil), anthracyclins (doxorubicin, daunomycin), maytansinoids (maytansinoid DM1), anti-metabolites, plant alkaloids and terpenoids as the Vinca alkaloids (vinblastine, vincristine vinorebline, vindesin) Podophyllotoxin and derivatives hereof and taxanes (paclitaxel, docetaxel, taxotere) or topoisomerase inhibitors (camptothecins), synthetic toxins as ellipticine analogs or synthetic analogs of tumor antibiotics as duocarmycin or CC1065, other tubulin binding agents as halichondrin B, hemiasterlins and dolastatins or analogs as monomethyl-auristatin E; component C may also be selected from the group of small molecules having cytotoxic/cytostatic activities like alkylating agents (like Cyclophosphamide, Mechlorethamine, Chlorambucil, Melphalan) or anthracyclines (like Danorubicin, Doxorubicin, Epirubicin, Idarubicin, Mitoxantrone, Valrubicin) or cytoskeletal disruptors (like Paclitaxel, Docetaxel) or Epothilones (like) or Inhibitors of topoisomerase II (like Etoposide, Teniposide, Tafluposide) or nucleotide analogs and precursor analogs (like azacididine, azathioprine, capecitabine, cytarabine, doxofluridine, fluorouracil, gemcitabine, mercaptopurine, methotrexate, tioguanine) or peptide antibiotics (like bleomycin) or platinum-based agents (like carboplatin, cisplatin, oxaliplatin) or retinoids (like all-trans retinoic acid) or vinca alkaloids and derivatives (like vinblastine, vincristine, vindestine, vinorelbine), beta ray emiting nuclides like Iodine-131, Yttrium-90, Lutetium-177, from the group of Aromatase Inhibitors (like Aminoglutethimide, Anastrozole, Letrozole, Vorozole, Exemestane, 4-androstene-3,6,17-trione, 1,4,6-androstatrien-3,17-dione, Formestane, Testolactone), Carbonic Anhydrase Inhibitors (like Acetazolamide, Methazolamide, Dorzolamide, Topiramate), Cholinesterase Inhibitors (Organophosphates like Metrifonate, Carbamates like Physostigmine, Neostigmine, Pyridostigmine, Ambenonium, Demarcarium, Rivastigmine, Phananthrine like Galantamine, Piperidine like Donepezil, Tacrine, Edophonium, or Phenothiazines), Cyclooxygenase Inhibitors (like Celecoxib, Rofecoxib, Etoricoxib, Acetaminophen, Diclofenac, Ibuprofen), Folic Acid Antagonists (like Methotrexate), Hydroxymethylglutaryl-CoA Reductase Inhibitors (like Atorvastatin, Cerivastatin, Fluvastatin, Lovastatin, Mevastatin, Pitavastatin, Pravastatin, Rosuvastatin, Simvastatin, Vytorin, Advicor, Caduet), Integrase Inhibitors (like Raltegravir, Elvitegravir), Lipoxygenase Inhibitors (like Zileutron), Monoamine Oxidase Inhibitors (like Isocarboxazid, Moclobemide, Phenelzine, Tranylcypromine, Selegiline, Rasagiline, Nialamide, Iproniazid, Iproclozide, Toloxatone, Linezolid, Tryptamines, Dienolide, Detxtroamphetamine), Nucleic Acid Synthesis Inhibitors, Phosphodiesterase Inhibitors (like Caffeine, Theopyline, 3-isobutyl-1-methylxanthine, Vinpocetine, EHNA, Enoximone, Lirinone, PDE3, Mesembrine, Rolipram, Ibudilast, Sildenafil, Tadalafil, Vardenafil, Udenafil, Avanafil), Protease Inhibitors (like Saquinavir, Ritonavir, Idinavir, Nelfinavir, Amprenavir, Lopinavir, Atazanavir, Fosamprenavir, Tipranavir, Darunavir), Protein Kinase Inhibitors (like Imatinib, Geftinib, Pegaptanib, Sorafenib, Dasatinib, Sunitinib, Erlotinib, Nilotinib, Lapatinib), Protein Synthesis Inhibitors (like Anisomycin, Cycloheximide, Chloramphenicol, Tetracycline, Streptomycin, Erythromycin, Puromycin, etc.), Proton Pump Inhibitors (like Omeprazole, Lansoprazole, Esomeprazole, Pantoprazole, Rabeprazole), from the group of oligonucleotides nucleic acids like small interfering RNAs (siRNAs) or a short hairpin RNA (shRNA), an antisense DNA or RNA, a double stranded RNA (dsRNA) or a micro RNA (miRNA) might be used to down-regulate specific key elements of regulative pathways within a cell.

In a specific embodiment component C is a polymer or dendrimer carrying several cytostatic/cytotoxic agents as exemplified above like e.g. paclitaxel or methothrexat molecules carrying a Benzylguanine (BG)/Benzylcytosine(BC)-group and is modified to improve biocompatibility e.g. by pegylation.

Further the drug can be a radioisotope selected from the group of beta emiting isotopes that can be used for radiotherapy (e.g. iodine-131, lutetium-177, yttrium 90).

In another example the drug can be a nucleic acid or a nucleic acid analog, which can exert biological activity in the targeted cell. More specifically the nucleic acid molecule can be designed to allow the expression of an encoded protein in the targeted cell (in the sense of a gene therapy) or to mediate RNA interference (RNAi) including small interfering RNAs (siRNAs) or a short hairpin RNA (shRNA), an antisense DNA or RNA, a double stranded RNA (dsRNA) or a micro RNA (miRNA).

In a specific embodiment component C is a siRNA or a linker structure as defined hereinbefore with one or more functionally attached siRNAs of a single specificity or several different specificities. The RNAi mediating compound may be directed against any desired cellular mRNA. The RNA interference (RNAi) mediating compound may be designed to directly or indirectly downregulate the expression of factors that are essential for the survival of the targeted cell (e.g siRNA mediated knock down of elongation factor II (eEFII or a variety of anti-apoptotic factors as BCL2, BCL-xL or other oncogenes) or may be designed to alter the gene expression profile in a targeted cell in a way that has a therapeutic effect.

In a concrete example the complex AB-C comprises an EGFR specific single chain antibody or human EGF fused to the SnapTag, to which a siRNA directed against the human elongation factor II, laminA/C, or GFP modified with BG is coupled.

Component C may further be a prodrug that is activated e.g. by cellular proteases upon entry into the target cell.

The drug may further be a peptide or polypeptide that has toxic activity in the targeted cell.

Examples are the ADP ribosylating enzymes pseudomonas exotoxin A, diphteria-, cholera-, pertussis- and botulinotoxin. The ribosome inactivating proteins diathin, saporin, bryodin, gelonin, ricin, abrin or restrictocin, ribonucleases (Phosphodiesterases) RNAse H, angiogenin, eosinophil-derived neurotoxin (EDN), eosinophilic cationic protein, onconase and bullfrog lektin. Additional proteins that can be represented by C include prodrug activating enzymes as caliceamicin, glucoseoxidase, carboxypeptidase, alkaline phosphatase, cytosindeaminase, beta-glycosidase, beta-glucoronidase, beta-lactamase, nitroreductase, thymidinkinase or purin-nucleosid phosphorylase. Further cathepsines, granzymes and combinations and possible variations of the afore mentioned protein families.

Prefered are validated toxins as ricin A, alpha sarcin (family of lectins), diphteriatoxin and pseudomonas exotoxin A. They have been subject of several clinical studies and their efficasy is well documented.

Component C may also represent toxic peptides as denfensines, anti-fungal peptides or e.g. several peptides isolated from lumpfish or sponges.

Detectable labels are fluorescent dyes such as fluorescein, rhodamine, courmarine, and cyanine and derivatives hereof. Prefered fluorophores emitt in the near infra red (NIR) range between 680 and 950 nm. This wavelength results in very low background fluorescence and excellent tissue penetration and is therefore ideally suited for fluorescence detection *in vivo.* In a specific embodiment a tumour specific antibody or other ligand in fusion with the Snap-tag is labeled with a BG derivative of a NIR dye. The labeled antibody or ligand serves as an imaging tool that can be used to visualize tumor growth and/or treatment *in vivo.*

In a concrete example a BG derivative of an NIR dye emitting at 782nm was coupled to a single chain antibody fragment SNAP-tag fusion protein targeting EGFR. The resulting in vivo imaging probe was used to detect EGFR expression in a pancreatic carcinoma xenograft model. In other concrete examples several fluorophore coupled complexes AB were used for flow cytometry and confocal microscopy applications.

Further the detectable label can be gamma emitting radioisotopes as e.g. iodine-131, lutetium-177, yttrium 90 or any other diagnostically relevant isotope usually combined with a complexing agent as DOTA or DTAP.

Further the detectable label can be a quantum dot composed of heavy metals like CdSe or InGaP. Quantum dots are favourable optical imaging agents due to their high quantum yield and photostability. Another possibility for a fluorescent label represented by component C may be noble metal nanoclusters composed of a few (8-12) gold or silver atoms, or synthetic fluorophores captured in nanoparticles made from silicon dixode.

Further detectable labels are superparamagnetic iron oxid particles for MRI based molecular imaging.

Fluorescent proteins like GFP or dsRED or derivatives hereof can serve as detectable label coupled to the complexes AB. Fluorescent proteins today cover a wide range of the visible spectrum as well as the near infrared.

Further detectable labels can be enzymes like alkaline phosphatase, peroxidases and galactosidases that are commonly applied in a variety of immunoassays.

Component C can also be a solid phase in the sense of a bead, a biochip surface or an ELISA-plate.

As used herein the term "antigen" is describing any target structure being bound by any component A.

As used herein the term "complex" is a chemical entity which may be constructed from different chemical structures forming a chemical compound, the different chemical structures Inked to each other by covalent and/or ionic bonds, as well as hydrophobic and/or hydrophilic interactions.

As used herein the term "therapeutic" represents any use of the complex ABC that leads to at least stabilization of diseases.

As used herein the term "diagnostic" represents any use of the complex ABC which leads to the identification of the nature of problem in medicine, science, engineering, environment, food & feed, business, trade.

The term "target cell" and or "target tissue" refers to cells or tissues carrying an extracellular surface structure to which the component A of the complex actively or passively binds. Target cells and target tissues are thus cells and tissues to which the component A of the complex can bind.

The term "recombinant" refers to the preparation of molecules, in particular the covalent joining of molecules from different sources, by any one of the known methods of molecular biology. As used in the present invention, the term "recombinant" refers in particular to the fusion of the antibody or ligand part A to the enzyme like protein part B by any one of the known methods of molecular biology, such as through production of single chain antibodies. The recombinant DNA molecule encoding the recombinant fusion protein comprising the antibody/ligand part and the enzyme type protein part are recombinantly expressed. Recombinant invention related complexes produced in this way may be isolated by any technique known in the field of recombinant DNA expression technology suitable for this purpose.

The term "derivative" refers to a mutated or modified protein, which has retained its characterizing activity, i.e. binding activity or enzymatic activity. Particular preferred are constitutively active derivatives. The term derivative comprises proteins, which carry at least one amino acid substitution, deletion, addition, a swapping of a single domain or at least one modification of at least one amino acid. In particular derivatives having as many modification as possible but not destroying the function of the compound of the invention are within the scope of the present invention more particularly those proteins which carry about 20 such changes or those with about 10 such changes or those with 1 to 5 such changes.

A further meaning of "derivative" is a chemical modification of a protein in its side chain, e.g. by glycosylation, phosphorylation, modification of carboxyl groups, such as amidation, esterification, modification of thiol or hydroxyl groups, e.g. by alkylation or oxidation or disulfide linking, modification of amino groups which may act as nucleophilic moiety, such as acylation, alkylation or other electrophilic attacks.

Further the term "derivative" refers to chemical structures analogous to a parent structure, which is extended or modified by another more or less complex group, e.g. a fluorophore being the parent structure extended by one or more reactive groups, e.g. a maleimido group.

As used herein, the term "As used herein, the term "vector" comprises DNA and RNA forms of a plasmid, a cosmid, a phage, phagemid, derivatives of them, or a virus. A vector comprises control sequences and coding sequences.

The term "expression of the recombinant genes encoding the recombinant complex", wherein the recombinant complex is a single chain antibody/ligand-enzyme type protein fusion polypeptide, refers to the transformation and/or transfection of a host cell with a nucleic acid or vector encoding such a complex, and culturing said host cells selected from the group of bacteria, such as *E. coli, and*/*or* in yeast, such as in *S. cerevisiae,* and/or in established mammalian or insect cell lines, such as CHO, NS0, COS, BHK, 293T and MDCK cells, and/or in primary cells, such as human cells, non-human vertebrate cells, and/or in invertebrate cells such as insect cells, and the synthesis and translation of the corresponding mRNA, finally giving rise to the recombinant protein, the recombinant complex. In more detail, the term "expression of the recombinant genes encoding the recombinant complex", comprises the following steps:

Transformation of an appropriate cellular host with a recombinant vector, in which a nucleotide sequence coding for the fusion protein had been inserted under the control of the appropriate regulatory elements, particularly a promoter recognized by the polymerases of the cellular host. In the case of a prokaryotic host, an appropriate ribosome-binding site (RBS) also precedes the nucleotide sequence coding for the fusion protein, enabling the translation in said cellular host. In the case of a eukaryotic host any artificial signal sequence or pre/pro sequence may be provided, or the natural signal sequence may be employed. The transformed cellular host is cultured under conditions enabling the expression of said insert.

Also claimed are cells or *in vitro* translation systems, which synthesize complete complexes according to the invention or individual components thereof, after transformation and/or transfection with, or addition of the nucleic acid molecules or vectors according to the invention.

One further embodiment of the present invention is a cellular compartment or an organism except a human being which compartment or organism being transformed or transfected with the nucleic acid according to the invention. The cellular compartment may be of prokaryotic origin in particular from *E. coli, B. subtilis, S. carnosus S. coelicolor,* and/or *Marinococcus sp.,* or a lower eukaryote, such as *Saccharomyces sp., Aspergillus sp., Hansenula polymorpha, Arxula adeninivorans, Spodoptera sp. and*/*or P. pastoris,* a higher non-human eukaryote such as a plant and/or an animal, and the cell is a primary or cultivated mammalian cell, such as a freshly isolated human cell or a eukaryotic cell line such as CHO, NS0, COS, BHK, 293T and MDCK.

Cells or organisms according to the invention are either of prokaryotic origin, especially from *E. coli, B. subtilis, S. carnosus, S. coelicolor, Marinococcus sp.,* or eukaryotic origin, especially from *Saccharomyces sp., Aspergillus sp., Spodoptera sp., P. pastoris,* primary or cultivated mammalian cells, eukaryotic cell lines (e.g., CHO, Cos or 293), plants (e.g. *N. tabacum),* or yeasts (e.g. *S*. *cerevisiae, H. polymorpha, A. adenivorans).*

The invention also relates to medicaments and analytical / diagnostic tools comprising the complex of the present invention and/or the nucleic acid or vectors encoding the complex of present invention. Typically, the complexes according to the invention are administered in physiologically acceptable dosage forms. These include, for example, Tris, NaCl, phosphate buffers and all approved buffer systems, especially including buffer systems, which are characterized by the addition of approved protein stabilizers. The administration is effected, in particular, by parenteral, intravenous, subcutaneous, intramuscular, intratumoral, transnasal administrations, and by transmucosal application.

The dosage of the complexes according to the invention to be administered must be established for each application in each disease to be newly treated by clinical phase I studies (dose-escalation studies).

The complex according to the invention, nucleic acid molecules coding therefore and/or cells or *in vitro* translation systems can be used for the preparation of a medicament for treating tumor diseases, allergies, autoimmune diseases, and chronic/acute inflammation reactions or for the preparation of a diagnostic tool for the same. Furthermore malignant diseases and tissue/graft rejection reactions can be treated.

Further details of recombinant protein engineering are either well known to the skilled person or become evident from Rosenblum in (US 2006/0280749 A1) incorporated herein by reference.

### Examples

The following is an illustration of preferred embodiments for practicing the present invention. However, they are not limiting examples. Other examples and methods are possible in practicing the present invention.

### I Chemical synthesis of component C

### Abbreviations:

BC-NH2 = 2-(4-aminomethylbenzyloxy)-4-aminopyrimidine (aminomethylbenzylcytosine)
BG-PEG4-NH2= 6-(4-((2-(2-(2-(2-aminoethoxy)ethoxy)ethoxy)ethoxy)methyl)benzyloxy)-9H-purin-2-amine (pegylated O⁶-benzylguanine)
CDI = N,N'-carbonyl diimidazole
CoA-SH = coenzyme A
DCC = dicyclohexylcarbodiimide
DCU = dicyclohexylurea
DIPEA = diisopropylethylamine
DMF = dimethylformamide
DMSO = dimethyl sulfoxide
DTT = dithiothreitol
EDC = 1-(3-(dimethylamino)propyl)-3-ethylcarbodiimide
eq = equivalent
ESI-MS = electrospray ionization mass spectrometry
Et₃N = triethylamine
EtOAc = ethyl acetate
EtOH = ethanol
FAB-MS = fast atom bombardment mass spectrometry
HOBT = 1-hydroxybenzotriazole
HPLC = high pressure liquid chromatography
Lys = lysine
MeNH₂ = methylamine
MeOH = methanol
NHS = N-hydroxy succinimide
NMP = N-methylpyrrolidine
PEG12 = -(CH₂CH₂O)₁₂-
PMe₃ = trimethylphosphine
PYBOP = (benzotriazol-1-yloxy)-tripyrrolidino-phosphonium hexafluorophosphate
TFA = trifluoroacetic acid
Tris = tris(hydroxymethyl)methylamine

### Abbreviations for molecular biology related terms :

| | |
|---|---|
| BG | 06-Benzylguanine (derivative) |
| CT | 02-Benzylcytosine (derivative) |
| LB | Luria broth |
| TB | terrific brith |
| IMAC | Immbilized metal affinity chromatography |
| p | Mikro |
| M | Milli |
| M | Molar |
| mAk | monoclonal antibody |
| Min | Minute |
| mRNA | ("messenger") ribonucleic acid (RNA) |
| siRNA | short interfering ribonucleic acid |
| DNA | desoxyribonucleic acid |
| Mw | molecular weight |
| N | Nano |
| N-term | Amino terminal (for proteins/oligo peptides) |
| C-term | carboxy-terminal (for proteins/oligo peptides) |
| ORF | open reading frame |
| PAA | Polyacrylamid |
| PAGE | Polyacrylamide gelelectrophoresis |
| pAk | polyclonal antibody |
| PBS | phosphate buffered saline |
| PBS-T | PBS + 0,05% (v/v) Tween-20 |
| PCR | polymerase chain reaction |
| PEG | Polyethylenglycol |
| pelB | bacterial leader-peptide for periplasmatic targeting in *E. coli* |
| RT | reverse transkriptase |
| RT-PCR | reverse transkriptase PCR |
| s | Second |
| scFv | single-chain variable fracment |
| SDS | Natriumdodecylsulfat |
| Taq | *Thermus aquaticus* |
| Tris | Tris(hydroxymethyl)aminomethan |
| Tween 20 | Polyoxyethylensorbitanmonolaurate |
| U | Unit |
| o.n. | over night |
| RPM | rounds per minute |
| UV | Ultra-violett |
| V | Volt |
| v/v | volume per volume |
| V_{H}/V_{L} | variable region of heavy (H) or light (L) immunglobuline |
| Vol. 1. | Volume |
| W | Watt |
| w/v | weight per volume |
| scFv H22 | Humanized scFv against human CD64 |
| scFv 40 | Murine antibody against apple scrap spores |
| CD40L | natural ligand for CD40 |
| CD30L | natural ligand for CD30 |
| scFv Ki4 | murine scFv against human CD30 |
| scFvKi3 | murine scFv against human CD30 |
| scFvKi2 | murine scFv against human CD30 |
| scFv 425 (Hai) | murine scFv against human EGF receptor (EGFR) |
| hEGF | Human epidermal growth factor binding to human EGF receptor |
| Adapter3 | Adapter3 consists of an endosomal cleavable+membrane transfer peptide |
| scFv 14.1 | murine scFv against pancreatic cancer cells |
| MOG | Myelin Oligodendrocyte Glycoprotein |
| scFv35 | human scFv against fetal acteylcholine receptor |
| | Trans-Activator of Transcription taken from HIV genome |
| TAT | |
| scFvM12 | human scFv against CEA (carcinoembryogenic antigen) |
| PIGF | Phosphatidylinositol glycan, class F protein |
| | |
| VEGF | Vascular endothelial growth factor |
| mSNAP | SfiI restriction endonuclease recognition site depleted version of SNAP-Tag (SNAP 26m) |
| SNAP | SNAP-Tag (SNAP26m / SNAP26b gene) |
| IL1-IL31 | interleukin 1-interleukin 31 |
| CXCL9 (MIG | Chemokine CXC motif ligand 9 |
| CXCL10 (IP10) | Interferon-gamma-inducible protein 10 |
| CXCL11 | Chemokine CXC motif ligand 11 |
| CXCL13 | Chemokine CXC motif ligand 13 |
| CXCL16 | Chemokine CXC motif ligand 16 |
| CCL11 (Eotaxin-1) | Chemokine CC motif ligand 11 |
| CCL14 | Chemokine CC motif ligand 14 |
| CCL16 | Chemokine CC motif ligand 16 |
| CCL18 | Chemokine CC motif ligand 18 |
| CCL27 | Chemokine CC motif ligand 27 |
| CCL28 | Chemokine CC motif ligand 28 |
| XCL1 (Lymphotatcin) | Chemokine C motif ligand 1 |
| CX3CL1(Neurotactin) | Chemokine CX3C motif ligand 1 |
| TGFbeta | TGF beta receptor, type I |
| G-CSF | Granulocyte-Colony Stimulating Factor |
| NGF | Nerve growth factor |
| HGF | Hepatocyte growth factor/scatter factor |
| sCD64 | soluble CD64 (FC gamma receptor I) |

### Example 1:

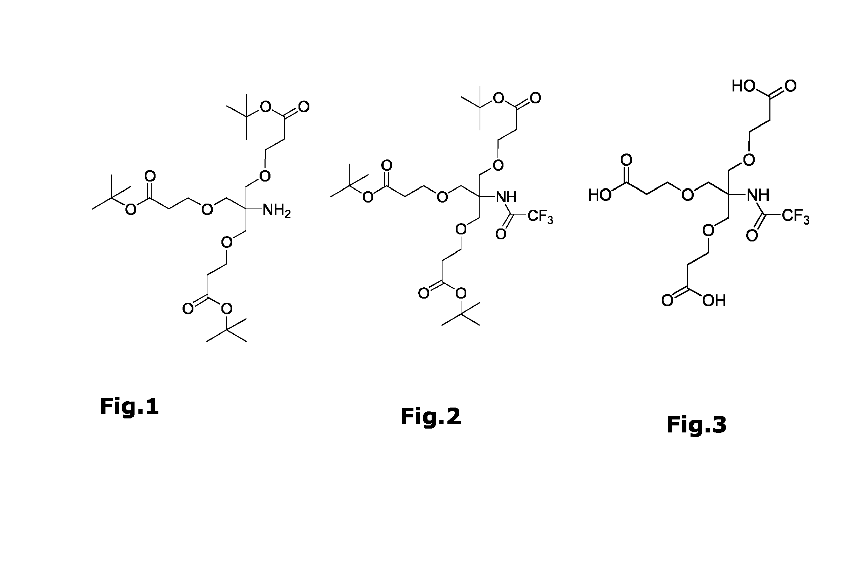

### Chemical synthesis of Tris{[2-(tert-butoxycarbonyl)ethoxy]methyl}methylamine (Fig. 1)

Tris(hydroxymethyl)methylamine (Tris, 2.42 g, 20.0 mmol) in 4.0 mL of a newly opened bottle of DMSO is cooled to 15.0°C. Then, 0.4 mL of 5.0 M NaOH is injected while stirring, followed by tert-butyl acrylate (10.0 mL, 68 mmol), which is injected dropwise. A solvent mixture of 5-10% water in DMSO is optimal for this reaction. The reaction mixture is allowed to reach room temperature and left stirring for 24 h. Then the crude mixture is poured onto water and extracted with ethyl acetate, the organic phase is dried over MgSO₄, and evaporated under reduced pressure to afford **(****Fig. 1****).** The compound is directly used for next step without further purification. FAB-MS: m/z 506 [M+H]⁺.

### Example 2:

### Chemical synthesis of N-Tris{[2-(tert-butoxycarbonyl)ethoxy]methyl}methyl trifluoroacetamide (Fig. 2)

To a solution of tris{[2-(tert-butoxycarbonyl)ethoxy]methyl}methylamine **(****Fig. 1****)** (10 mmol, 5.05 g) in MeOH (30 mL) is added triethylamine (1 eq, 10 mmol, 1.39 mL) at rt. Then, ethyl trifluoroacetate (1.3 eq, 13 mmol, 1.55 mL) is slowly added over 20 min at rt. The reaction mixture is stirred overnight at rt. Then, the solvent is evaporated, the residue is diluted with EtOAc (100 mL) and washed with a saturated solution of NaCl. The organic layer is dried over MgSO₄ and concentrated under reduce pressure. Flash chromatography (cyclohexane /EtOAc, 2/1→1/1) gives the desired compound **(****Fig. 2****).**

ESI-MS: m/z 602.31 [M+H]⁺.

### Example 3:

### Chemical synthesis of N-Tris{[2-(carboxy)ethoxy]methyl}methyl trifluoroacetamide (Fig. 3)

N-Tris{[2-(tert-butoxycarbonyl)ethoxy]methyl}methyl trifluoroacetamide **(****Fig. 2****)** (4.81 g, 8 mmol) is stirred in 80 mL of 96% formic acid for 18 h. Then, the formic acid is removed at reduced pressure at 50°C to produce a colorless oil in quantitative yield.

ESI-MS: m/z 434.12 [M+H]⁺.

### Example 4:

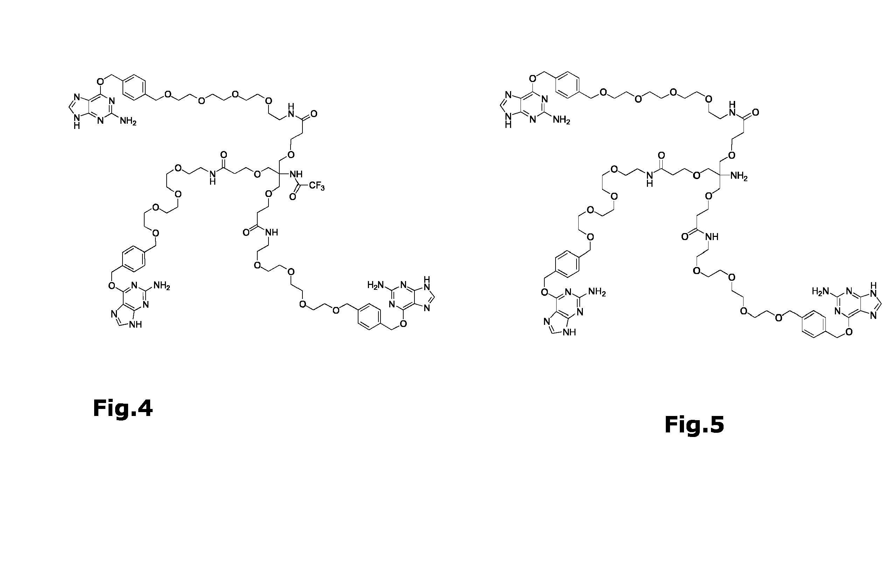

### Chemical synthesis of N-Tris(BG-PEG4-NH-carbonylethyloxymethyl)methyl trifluoroacetamide (Fig. 4)

To a solution of **(****Fig. 3****)** (433 mg, 1 mmol, 1 eq) and BG-PEG4-NH2 (1.34 g, 3 mmol, 3 eq) in DMF (10 mL) are successively added DIPEA (495 µL, 3 mmol, 3 eq), HOBT (1 M in NMP, 3 mL, 3 mmol, 3 eq) and DCC (620 mg, 3 mmol, 3 eq) at rt. The resulting mixture is stirred overnight. Then the solvent is removed under reduced pressure and the mixture is diluted with 250 mL of EtOAc. The organic layer is washed with water, dried over MgSO₄ and evaporated under reduced pressure. Flash chromatography (CH₂Cl₂ / MeOH, 10/1→5/1) gives the desired compound **(****Fig. 4****).** ESI-MS: m/z 1718.77 [M+H]⁺.

### Example 5:

### Chemical synthesis of Tris(BG-PEG4-NH-carbonylethyloxymethyl)methylamine (Fig. 5)

To a solution of compound **(****Fig. 4****)** (1.03 g, 0.6 mmol) in EtOH (15 mL) is added a solution of MeNH₂ (30% in EtOH, 30 mL). The corresponding solution is stirred overnight at rt. A cloudy mixture is obtained. The solid is removed by filtration and evaporation of the resulting clean solution affords the desired compound **(****Fig. 5****).** No further purification is required. ESI-MS: m/z 1621.79 [M+H]⁺.

### Example 6:

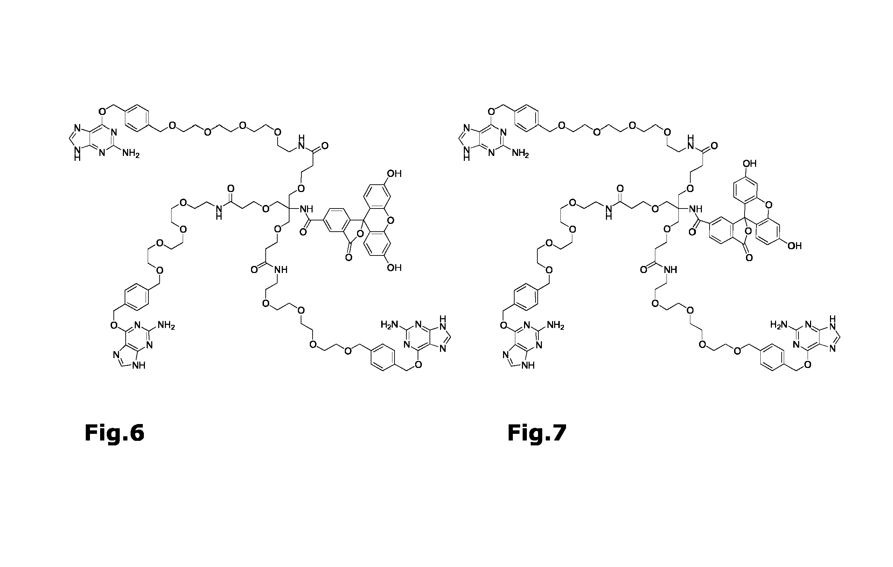

### Chemical synthesis of N-Tris(BG-PEG4-NH-carbonylethyloxymethyl)methyl fluorescein-5-carboxamide (Fig. 6) and corresponding fluorescein-6-carboxamide (Fig. 7)

Compound **(****Fig. 5****)** (29 mg, 0.018 mmol) and 5(6)-carboxyfluorescein succinimidyl ester (8.5 mg, 0.018 mmol) are dissolved in 1 mL of DMF with Et₃N (2.7 µL, 0.018 mmol) and heated overnight at 31°C. The solvent is evaporated under vacuum and the compounds **(****Fig. 6)** and **(Fig. 7****)** isolated by reversed phase HPLC on a C18 column using a linear gradient of water:acetonitrile 95:5 to 20:80 in 20 min, 0.08% TFA). ESI-MS: m/z 1980.84 [M+H]⁺.

### Example 7:

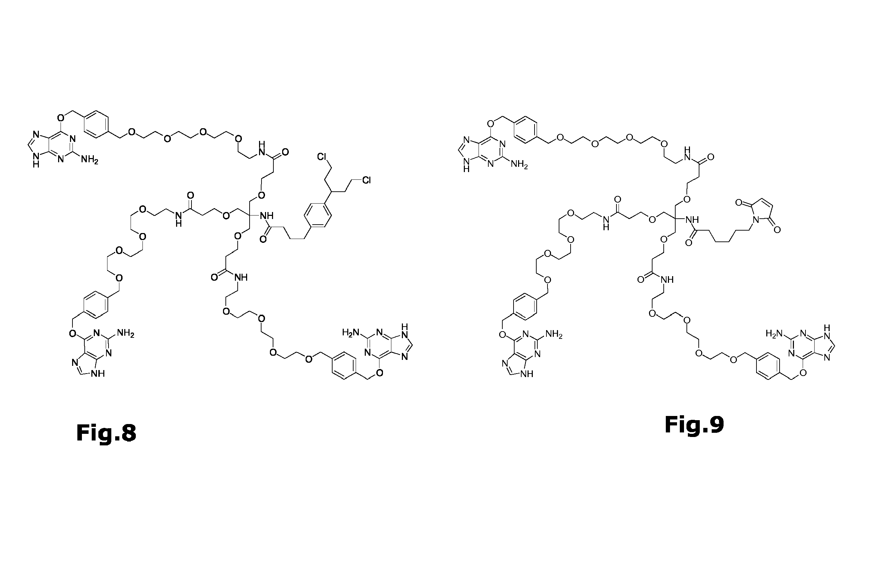

### Chemical synthesis of N-Tris(BG-PEG4-NH-carbonylethyloxymethyl)methyl chlorambucil-carboxamide (Fig.8)

To a solution of chlorambucil (22 mg, 0.072 mmol) in DMF (2 mL) is added PYBOP (38 mg, 0.072 mmol) at rt. The solution is stirred at room temperature for 20 min. Then, compound **(****Fig. 5****)** (116 mg, 0.072 mmol) and DIPEA (12 µL, 0.072 mmol) are added and the solution is heated at 50°C for 5 min. The solution is stirred at room temperature overnight. Then the solvent is removed under reduced pressure and the mixture is diluted with 150 mL of EtOAc. The organic layer is washed with water, dried over MgSO₄ and evaporated under reduced pressure. Flash chromatography (CH₂Cl₂/MeOH, 10/1→5/1) gives the desired compound **(****Fig. 8****).**

ESI-MS: m/z 1906.86 [M+H]⁺.

### Example 8:

### Chemical synthesis of N-Tris(BG-PEG4-NH-carbonylethyloxymethyl)methyl 5-maleimidopentanecarboxamide (Fig. 9)

To a solution of 6-maleimido-hexanoic acid (8 mg, 0.036 mmol) in DMF (2 mL) is added PYBOP (19 mg, 0.036 mmol) at rt. The solution is stirred at room temperature for 20 min. Then compound **(****Fig. 5****)** (58 mg, 0.036 mmol) and DIPEA (6 µL, 0.036 mmol) are added and the solution is heated at 50°C for 5 min. The solution is stirred at room temperature overnight. Then the solvent is removed under reduced pressure and the mixture is diluted with 150 mL of EtOAc. The organic layer is washed with water, dried over MgSO₄ and evaporated under reduced pressure. Flash chromatography (CH₂Cl₂/MeOH, 10/1→5/1) gives the desired compound **(****Fig. 9****).**

ESI-MS: m/z 1815.86 [M+H]⁺.

### Example 9:

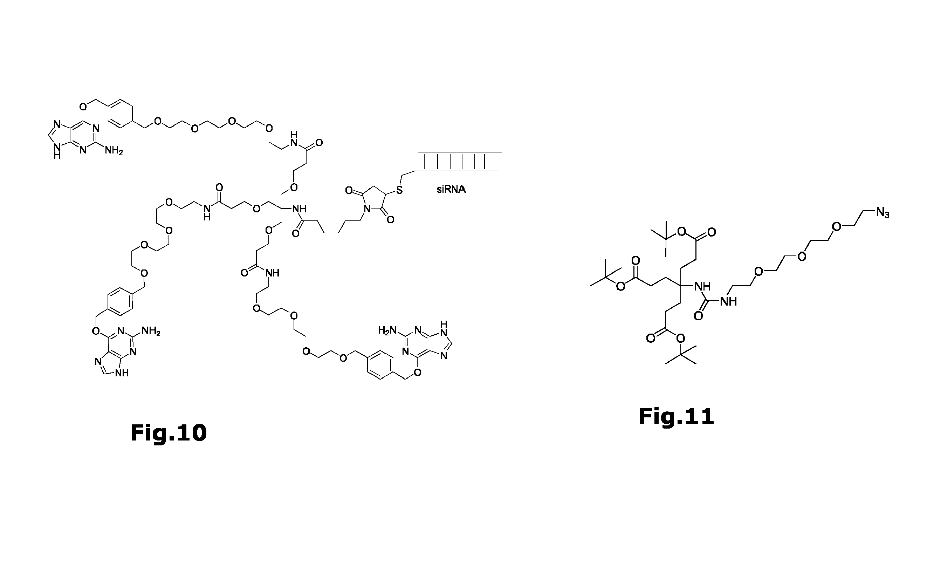

### Chemical synthesis of Tris(BG-PEG4-NH-carbonylethyloxymethyl)methyl 6-maleimido-hexanoic amide siRNA conjugate (Fig. 10)

5'-Thiol modified oligonucleotide (43 nmol) is reduced by incubation for 1 h at room temperature with 200 mM DTT in 200 µL Tris-buffer pH 8.5. The DTT is removed by gel filtration and the oligonucleotide eluted in PBS (pH 7.4). The most concentrated fractions are combined giving a total of 800 µL. 300 µL of a solution of compound **(****Fig. 9****)** (2.5 mM in DMF) is added and the reaction mixture incubated at room temperature for 1 h. The reaction mixture is diluted with water to a total volume of 2 mL and excess maleimide removed by gel filtration. The tris(BG-PEG4-NH-carbonylethyloxymethyl)methylamide-maleimide-oligonucleotide conjugate **(****Fig. 10****)** is then purified by HPLC (solvent A: 0.1 M tetraethylammonium acetate pH 6.9 in water; solvent B: acetonitrile).

### Example 10:

### Chemical synthesis of N-2-(2-(2-(2-azidoethoxy)ethoxy)ethoxy)ethyl-N'-tris[2-(tert-butoxycarbonyl)ethyl]methyl-urea (Fig. 11)

To a solution of 11-azido-3,6,9-trioxaundecan-1-amine (1.55 g, 1 eq, 7.1 mmol) in DMF (2 mL) is added tris[2-(tert-butoxycarbonyl)ethyl]methyl_isocyanate (3.1 g, 1 eq, 7.1 mmol) and Et₃N (988 µL, 1 eq, 7.1 mmol). The solution is stirred overnight at 31°C. Then the crude mixture is poured onto water and extracted with ethyl acetate, the organic phase is dried over MgSO₄, and evaporated under reduced pressure to afford **(****Fig. 11****).** No further purification is required.

FAB-MS: m/z 660.41 [M+H]⁺.

### Example 11:

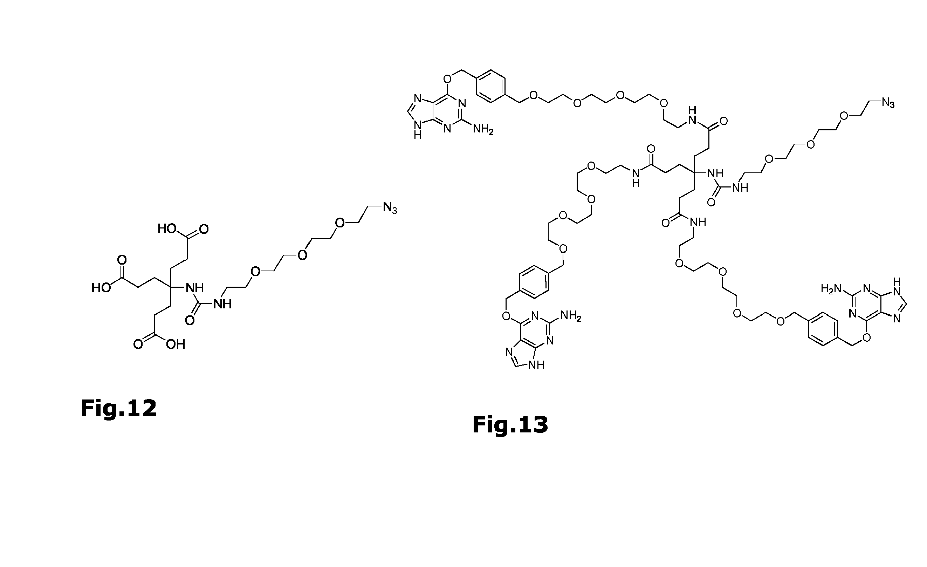

### Chemical synthesis of 4-[2-carboxyethyl]-4-(2-(2-(2-(2-azidoethoxy)ethoxy)-ethoxy)ethylaminocarbonylamino)-1,7-heptanedicarboxylic acid (Fig.12)

Compound **(****Fig. 11****)** (3.3 g, 5 mmol) is stirred in 50 mL of 96% formic acid for 18 h. Then, the formic acid is removed at reduced pressure at 50°C to produce a colorless oil, compound **(****Fig. 12****).** ESI-MS: m/z 492.22 [M+H]⁺.

### Example 12:

### Chemical synthesis of N-Tris(BG-PEG4-NH-carbonylethyl)methyl-N'-2-(2-(2-(2-azidoethoxy)ethoxy)ethoxy)ethyl-urea (Fig. 13)

To a solution of compound **(****Fig. 12****)** (491 mg, 1 mmol, 1 eq) and BG-PEG4-NH2 (1.34 g, 3 mmol, 3 eq) in DMF (50 mL) are successively added DIPEA (495 µL, 3 mmol, 3 eq), HOBT (1 M in NMP, 3 mL, 3 mmol, 3 eq) and DCC (620 mg, 3 mmol, 3 eq) at rt. The resulting mixture is stirred overnight. Then the solvent is removed under reduced pressure and the mixture is diluted with 250 mL of EtOAc. The organic layer is washed with water, dried over MgSO₄ and evaporated under reduced pressure. Flash chromatography (CH₂Cl₂/MeOH, 10/1→5/1) gives the desired compound **(****Fig. 13****).** ESI-MS: m/z 1776.87 [M+H]⁺.

### Example 13:

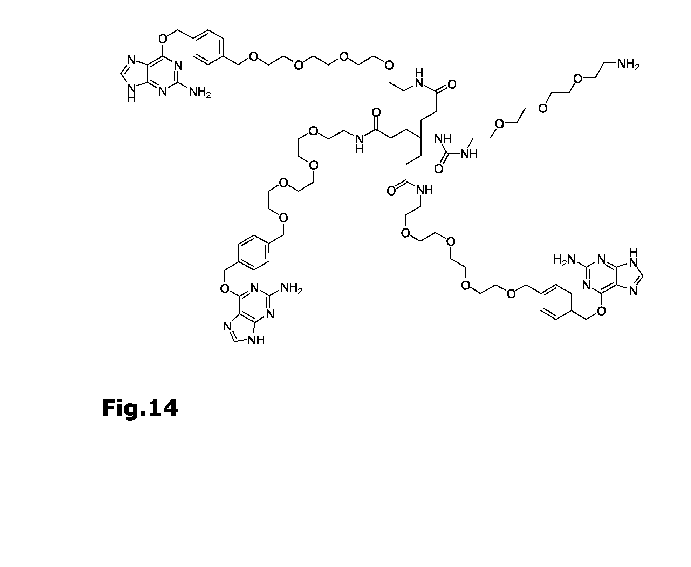

### Chemical synthesis of N-Tris-(BG-PEG4-NH-carbonylethyl)methyl-N'-2-(2-(2-(2-aminoethoxy)ethoxy)ethoxy)ethyl-urea (Fig. 14)

To a solution of compound **(****Fig. 13****)** (708 mg, 0.4 mmol) in dioxane (10 mL) is added water (1 mL). Then PMe₃ (2.40 mL 1 M in THF solution, 6 eq) is added and the solution is stirred at room temperature for 2 h. The solvent is removed under reduced pressure, and the compound **(****Fig. 14****)** is obtained by purification with preparative HPLC. ESI-MS: m/z 1750.88 [M+H]⁺.

### Example 14:

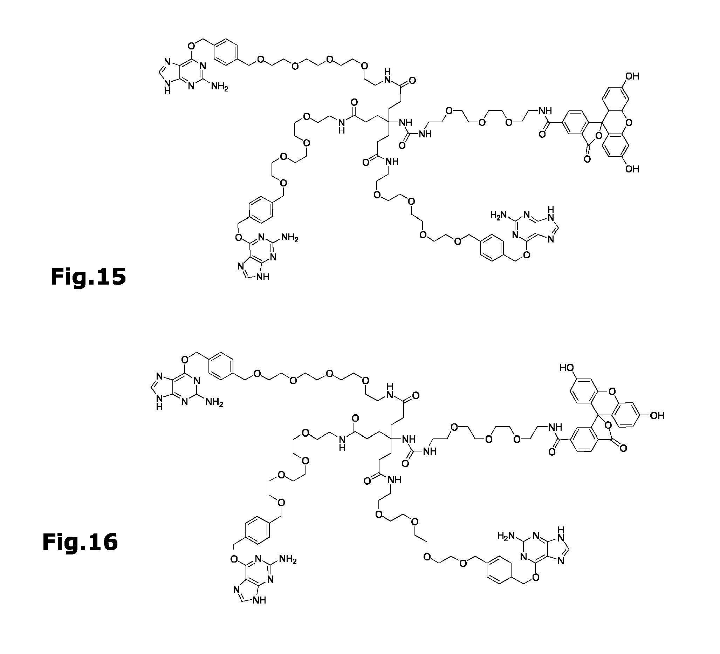

### Chemical synthesis of N-Tris-(BG-PEG4-NH-carbonylethyl)methyl-N'-2-(2-(2-(2-fluorescein-5-carboxamido-ethoxy)ethoxy)ethoxy)ethyl-urea (Fig. 15) and corresponding 6-fluorescein derivative (Fig. 16)

Compound **(****Fig. 14****)** (18 mg, 0.01 mmol) and 5(6)-carboxyfluorescein succinimidyl ester (5 mg, 0.01 mmol) are dissolved in 800 µL DMF with Et₃N (1.6 µL, 0.01 mmol) and heated overnight at 31°C. The solvent is evaporated under vacuum and the compounds **(****Fig. 15)** and **(Fig. 16****)** isolated by reversed phase HPLC on a C18 column using a linear gradient of water:acetonitrile (from 95:5 to 20:80 in 20 min, 0.08% TFA). ESI-MS: m/z 2108.93 [M+H]⁺.

### Example 15:

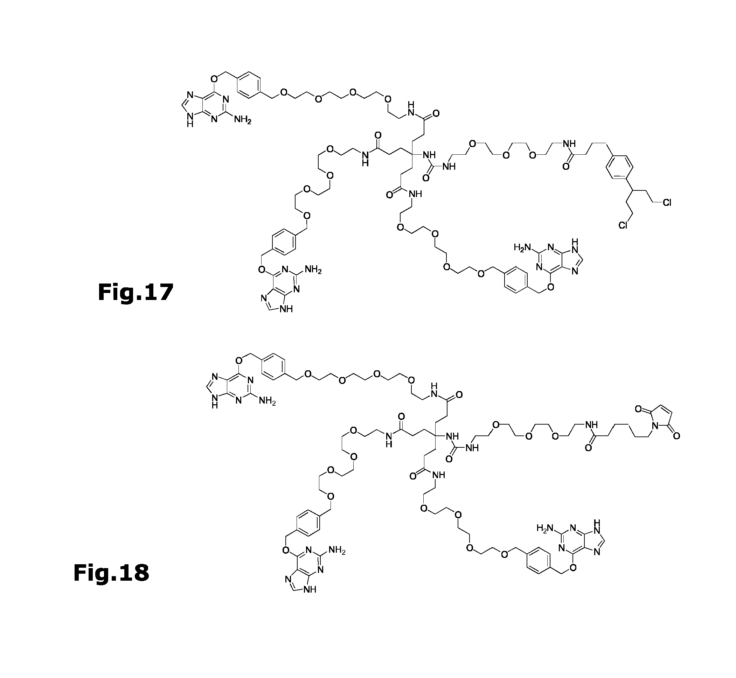

### Chemical synthesis of N-Tris(BG-PEG4-NH-carbonylethyl)methyl-N'-2-(2-(2-(2-chlorambucil-carboxamido-ethoxy)ethoxy)ethoxy)ethyl-urea (Fig. 17)

To a solution of chlorambucil (18 mg, 0.06 mmol) in DMF (3 mL) is added PYBOP (31 mg, 0.06 mmol) at rt. The solution is stirred at room temperature for 20 min. Then compound **(****Fig. 14****)** (103 mg, 0.06 mmol) and DIPEA (10 µL, 0.06 mmol) are added and the solution is heated at 50°C for 5min. The solution is stirred at room temperature overnight. Then the solvent is removed under reduced pressure and the mixture is diluted with 150 mL of EtOAc. The organic layer is washed with water, dried over MgSO₄ and evaporated under reduced pressure. Flash chromatography (CH₂Cl₂/MeOH, 10/1→5/1) gives the desired compound **(****Fig. 17****).** ESI-MS: m/z 2050.99 [M+H]⁺.

### Example 16:

### Chemical synthesis of N-Tris(BG-PEG4-NH-carbonylethyl)methyl-N'-2-(2-(2-(2-(6-maleimido-hexanoylamido)ethoxy)ethoxy)ethoxy)ethyl-urea (Fig. 18)

To a solution of 6-maleimidohexanoic acid (10 mg, 0.046 mmol) in DMF (2 mL) is added PYBOP (24 mg, 0.046 mmol) at rt. The solution is stirred at room temperature for 20 min. Then compound **(****Fig. 14****)** (80 mg, 0.046 mmol) and DIPEA (7.7 µL, 0.046 mmol) are added and the solution is heated at 50°C for 5min. The solution is stirred at room temperature overnight. Then the solvent is removed under reduced pressure and the mixture is diluted with 150 mL of EtOAc. The organic layer is washed with water, dried over MgSO₄ and evaporated under reduced pressure. Flash chromatography (CH₂Cl₂/MeOH, 10/1→5/1) gives the desired compound **(****Fig. 18****).**

ESI-MS: m/z 1959.99 [M+H]⁺.

### Example 17:

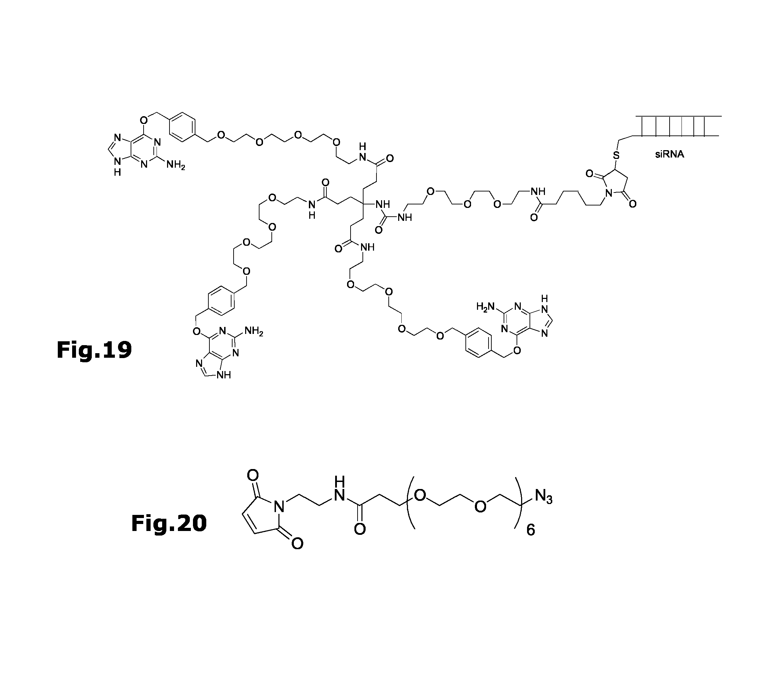

### Chemical synthesis of N-Tris(BG-PEG4-NH-carbonylethyl)methyl-N'-2-(2-(2-(2-(6-maleimidohexanoylamido)ethoxy)ethoxy)ethoxy)ethyl-urea siRNA conjugate (Fig. 19)

The 5'-thiol modified oligonucleotide (43 nmol) is reduced by incubation for 1 h at room temperature with 200 mM DTT in 200 µL Tris-buffer pH 8.5. The DTT is removed by gel filtration and the oligonucleotide eluted in PBS (pH 7.4). The most concentrated fractions are combined giving a total of 800 µL. 300 µL of a solution of compound **(****Fig. 18****)** (2.5 mM in DMF) is added and the reaction mixture incubated at room temperature for 1 h. The reaction mixture is diluted with water to a total volume of 2 mL and excess maleimide removed by gel filtration. The siRNA conjugate **(****Fig. 19****)** is then purified by HPLC (solvent A: 0.1 M tetraethylammonium acetate pH 6.9 in water; solvent B: acetonitrile).

### Example 18:

### Chemical synthesis of Azido-PEG12-propionic acid 2-maleimidoethylamide (Fig. 20)

N-(2-aminoethyl)maleimide trifluoroacetate (343 mg, 1.35 mmol) and azido-PEG12-propionic NHS ester (1 g, 1.35 mmol) are dissolved in 5 mL DMF with Et₃N (188 µL, 1.35 mmol) and heated overnight at 31°C. The solvent is evaporated under vacuum and the product is isolated by reversed phase HPLC on a C18 column using a linear gradient of water:acetonitrile (from 95:5 to 20:80 in 20 min, 0.08% TFA). ESI-MS: m/z 766.40 [M+H]⁺.

### Example 19:

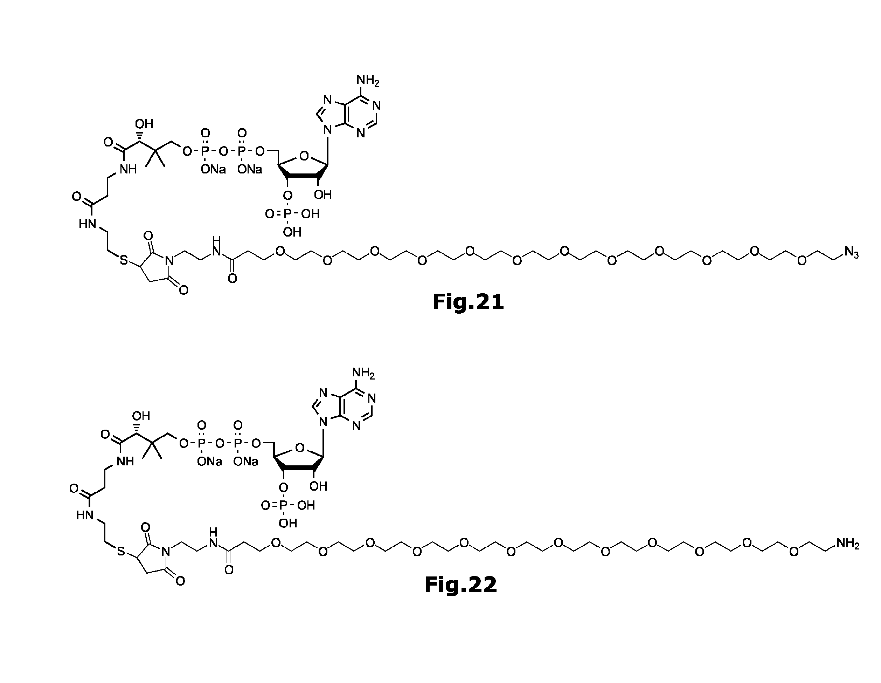

### Chemical synthesis of Azido-PEG12-propionic acid 2-maleimidoethylamide CoA-SH conjugate (Fig. 21)

A solution of maleimide derivative **(****Fig. 20****)** (192 mg, 1 eq, 252 µmol) in DMF (2 mL) is added to a solution of CoA-SH (248 mg, 1.2 eq, 304 µmol) in Tris-buffer (pH 7.5, 200 µL). The reaction mixture is shaken overnight at 31°C. Then the solvent is removed under vacuum and the crude mixture is purified via preparative HPLC. ESI-MS: m/z 1554.48 [M-Na]⁻.

### Example 20:

### Chemical synthesis of Amino-PEG12-propionic acid 2-maleimidoethylamide CoA-SH conjugate (Fig. 22)

To a solution of compound **(****Fig. 21****)** (204 mg, 0.13 mmol) in dioxane (3 mL) is added water (450 µL). Then PMe₃ (800 µL 1 M in THF solution, 6 eq) is added and the solution is stirred at room temperature for 2 h. The solvent is removed under reduced pressure the compound is obtained by purification with preparative HPLC.

ESI-MS: m/z 1527.48 [M-Na]⁻.

### Example 21:

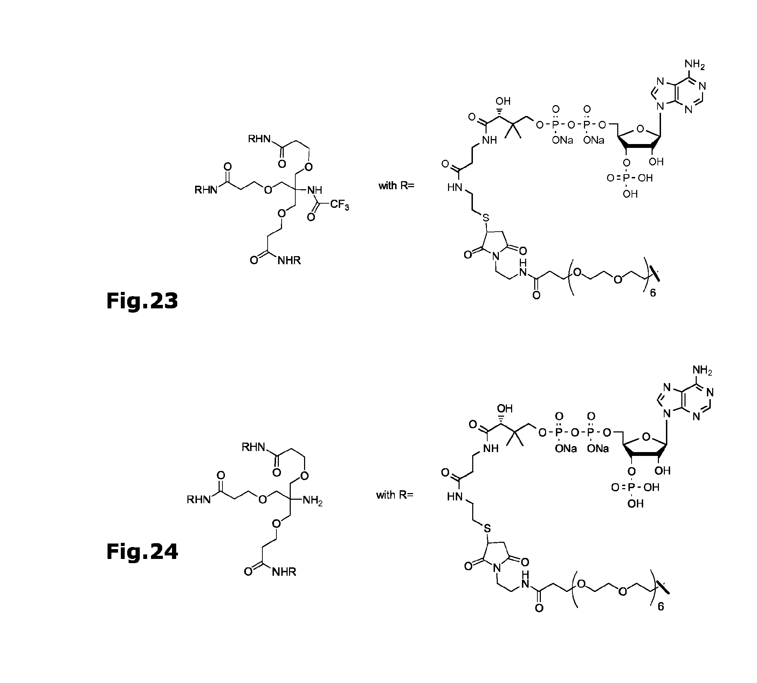

### Chemical synthesis of N-Tris{2-(2-(2-(CoA-S-succinimido)ethylaminocarbonyl-PEG12)ethylaminocarbonyl)ethoxymethyl}methyl trifluoroacetamide (Fig. 23)

To a solution of **(****Fig. 3****)** (21 mg, 0.05 mmol, 1 eq) and **(****Fig. 22****)** (232 mg, 0.15 mmol, 3 eq) in DMF (1 mL) are successively added DIPEA (25 µL, 0.15 mmol, 3 eq), HOBT (1 M in NMP, 150 µL, 0.3 mmol, 3 eq) and DCC (31 mg, 0.15 mmol, 3 eq) at rt. The resulting mixture is stirred overnight. The solvent is removed under reduced pressure, and the compound **(****Fig. 23****)** is obtained by purification with preparative HPLC. ESI-MS: m/z 5010.4 [M-Na]⁻.

### Example 22:

### Chemical synthesis of N-Tris{2-(2-(2-(CoA-S-succinimido)ethylaminocarbonyl-PEG12)ethylaminocarbonyl)ethoxymethyl}methylamine (Fig. 24)

To a solution of compound **(****Fig. 21****)** (100 mg, 0.02 mmol) in EtOH (1.5 mL) is added a solution of MeNH₂ (3 mL, 30% in EtOH). The corresponding solution is stirred overnight at rt. Evaporation of the solvent affords the desired compound **(****Fig. 24****).** No further purification is required. ESI-MS: m/z 4914.4 [M-Na]⁻.

### Example 23:

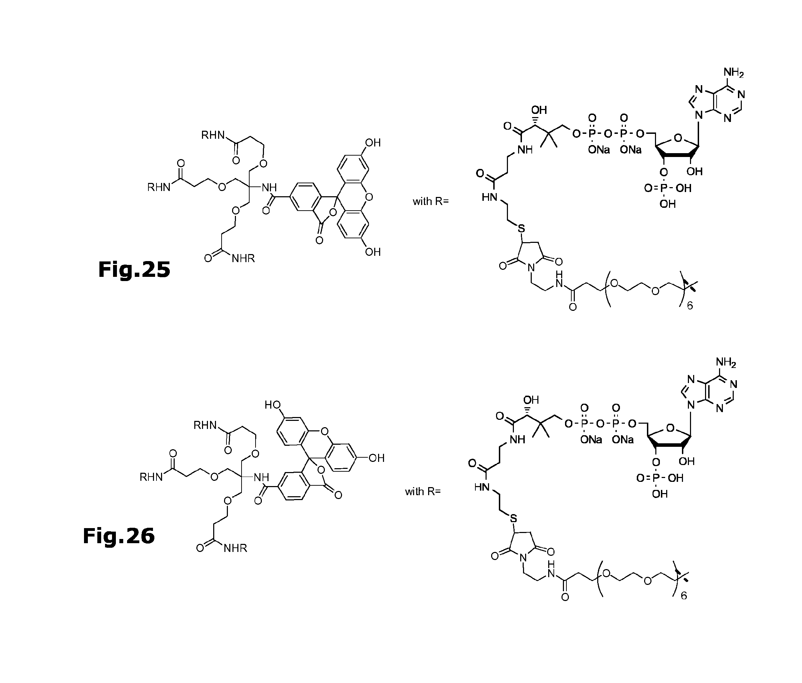

### Chemical synthesis of N-Tris{2-(2-(2-(CoA-S-succinimido)ethylaminocarbonyl-PEG12)ethylaminocarbonyl)ethoxymethyl}methyl fluorescein-5-carboxamide (Fig. 25) and corresponding fluorescein-6-carboxamide (Fig. 26)

Compound **(****Fig. 24****)** (19 mg, 0.004 mmol) and 5(6)-carboxyfluorescein NHS ester (2 mg, 0.004 mmol) are dissolved in 600 µL DMF with Et₃N (0.6 µL, 0.004 mmol) and heated overnight at 31°C. The solvent is evaporated under vacuum and the compounds **(****Fig. 25)** and **(Fig. 26****)** isolated by reversed phase HPLC on a C18 column using a linear gradient of water:acetonitrile (from 95:5 to 20:80 in 20 min, 0.08% TFA). ESI-MS: m/z 5272.7 [M-Na]⁻.

### Example 24:

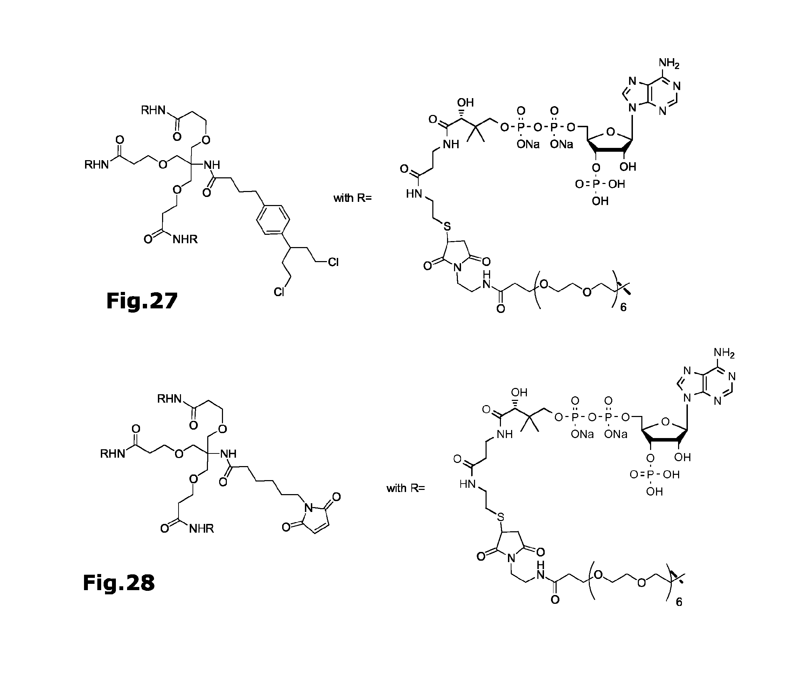

### Chemical synthesis of N-Tris{2-(2-(2-(CoA-S-succinimido)ethylaminocarbonyl-PEG12)ethylaminocarbonyl)ethoxymethyl}methyl chlorambucil-carboxamide (Fig. 27)

To a solution of chlorambucil (1.8 mg, 0.006 mmol) in DMF (1 mL) is added PYBOP (3 mg, 0.006 mmol) at rt. The solution is stirred at room temperature for 20 min. Then compound **(****Fig. 24****)** (29 mg, 0.006 mmol) and DIPEA (0.9 µL, 0.006 mmol) are added and the solution is heated at 50°C for 5 min. Then the solution is stirred at room temperature overnight. The solvent is removed under reduced pressure. Compound **(****Fig. 27****)** is isolated by reversed phase HPLC on a C18 column using a linear gradient of water:acetonitrile (from 95:5 to 20:80 in 20 min, 0.08% TFA).

ESI-MS: m/z 5200.6 [M-Na]⁻.

### Example 25:

### Chemical synthesis of N-Tris{2-(2-(2-(CoA-S-succinimido)-ethyl-aminocarbonyl-PEG12)ethylamino-carbonyl)ethoxymethyl}methyl 6-maleimido-hexanoyl-amide (Fig. 28)

To a solution of 6-maleimido-hexanoic acid (0.844 mg, 0.004 mmol) in DMF (1 mL) is added PYBOP (2.08 mg, 0.004 mmol) at rt. The solution is stirred at room temperature for 20 min. Then compound **(****Fig. 24****)** (19 mg, 0.004 mmol) and DIPEA (0.6 6µL, 0.004 mmol) are added and the solution is heated at 50°C for 5 min. The solution is stirred at room temperature overnight. Then the solvent is removed under reduced pressure. The compound is isolated by reversed phase HPLC on a C18 column using a linear gradient of water:acetonitrile (from 95:5 to 20:80 in 20 min, 0.08% TFA). ESI-MS: m/z 5107.7 [M-Na]⁻.

### Example 26:

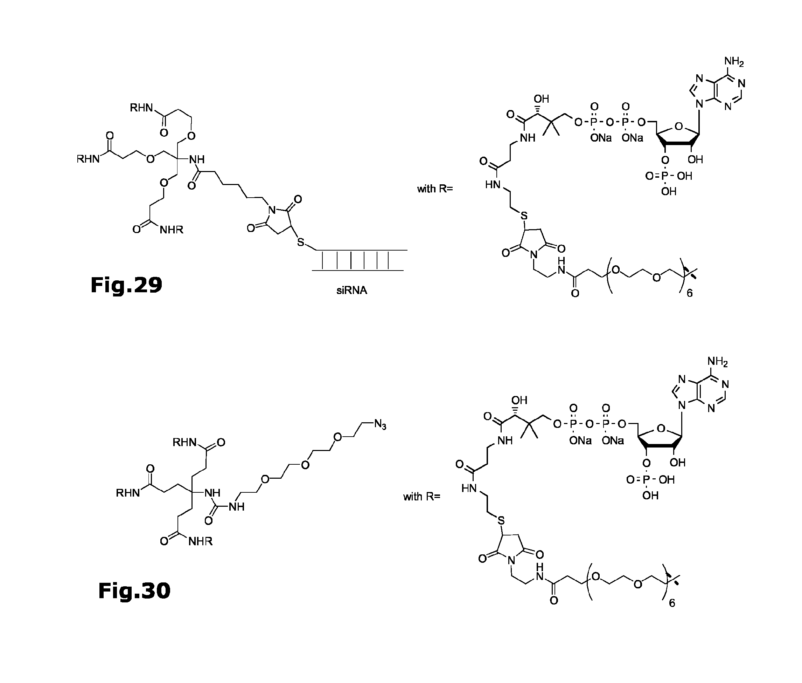

### Chemical synthesis of N-Tris{2-(2-(2-(CoA-S-succinimido)ethylamino-carbonyl-PEG12)-ethylamino-carbonyl)ethoxymethyl}methyl 6-maleimidohexanoylamide siRNA conjugate (Fig. 29)

The 5'-thiol modified oligonucleotide (43 nmol) is reduced by incubation for 1 h at room temperature with 200 mM DTT in 200 µL Tris-buffer pH 8.5. The DTT is removed by gel filtration and the oligonucleotide eluted in PBS (pH 7.4). The most concentrated fractions are combined giving a total of 800 µL. 300 µL of a solution of compound **(****Fig. 28****)** (2.5 mM in DMF) is added and the reaction mixture incubated at room temperature for 1 h. The reaction mixture is diluted with water to a total volume of 2 mL and excess maleimide removed by gel filtration. The conjugate **(****Fig. 29****)** is then purified by HPLC (solvent A: 0.1 M tetraethylammonium acetate pH 6.9 in water; solvent B: acetonitrile).

### Example 27:

### Chemical synthesis of N-Tris{2-(2-(2-(CoA-S-succinimido)ethylaminocarbonyl-PEG12)ethylaminocarbonyl)ethoxymethyl}methyl-N'-2-(2-(2-(2-azidoethoxy)-ethoxy)ethoxy)ethyl-urea (Fig. 30)

To a solution of compound **(****Fig. 12****)** (49 mg, 0.1 mmol, 1 eq) and compound **(****Fig. 22****)** (134 mg, 0.3 mmol, 3 eq) in DMF (5 mL) are successively added DIPEA (49 µL, 0.3 mmol, 3 eq), HOBT (1 M in NMP, 0.3 mL, 0.3 mmol, 3 eq) and DCC (62 mg, 0.3 mmol, 3 eq) at rt. The resulting mixture is stirred overnight. The solvent is removed under reduced pressure. The compound **(****Fig. 30****)** is isolated by reversed phase HPLC on a C18 column using a linear gradient of water:acetonitrile (from 95:5 to 20:80 in 20 min, 0.08% TFA). ESI-MS: m/z 5068.6 [M-Na]⁻.

### Example 28:

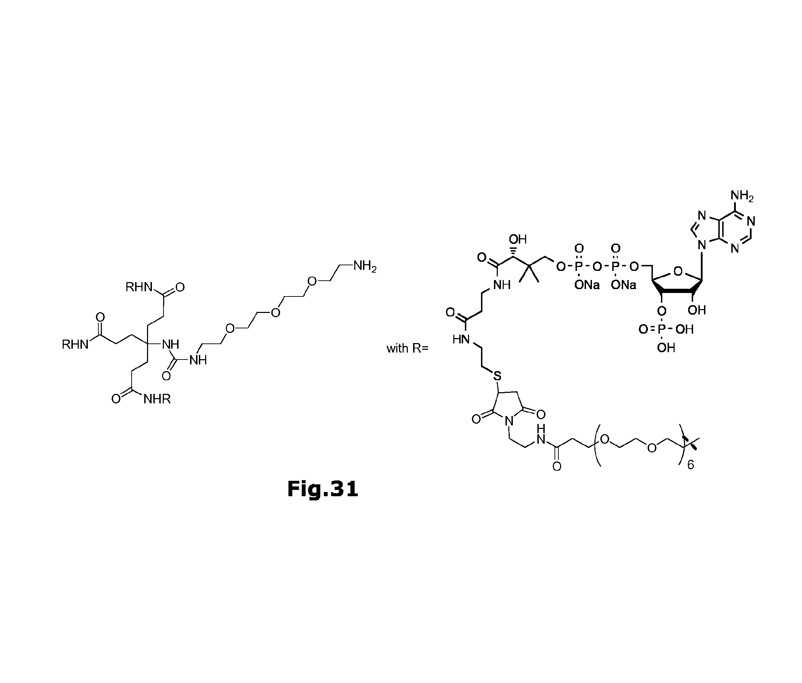

### Chemical synthesis of N-Tris{2-(2-(2-(CoA-S-succinimido)ethylaminocarbonyl-PEG12)ethylaminocarbonyl)ethoxymethyl}methyl-N'-2-(2-(2-(2-aminoethoxy)-ethoxy)ethoxy)ethyl-urea (Fig. 31)

To a solution of compound **(****Fig. 30****)** (127 mg, 0.025 mmol) in dioxane (3 mL) is added water (450 µL). Then PMe₃ (154 µL of 1 M THF solution, 6 eq) is added and the solution is stirred at room temperature for 2 h. The solvent is removed under reduced pressure, and the compound **(****Fig. 31****)** is obtained by purification with preparative HPLC on a C18 column using a linear gradient of water:acetonitrile (from 95:5 to 20:80 in 20 min, 0.08% TFA). ESI-MS: m/z 5042.5 [M-Na]⁻.

### Example 29:

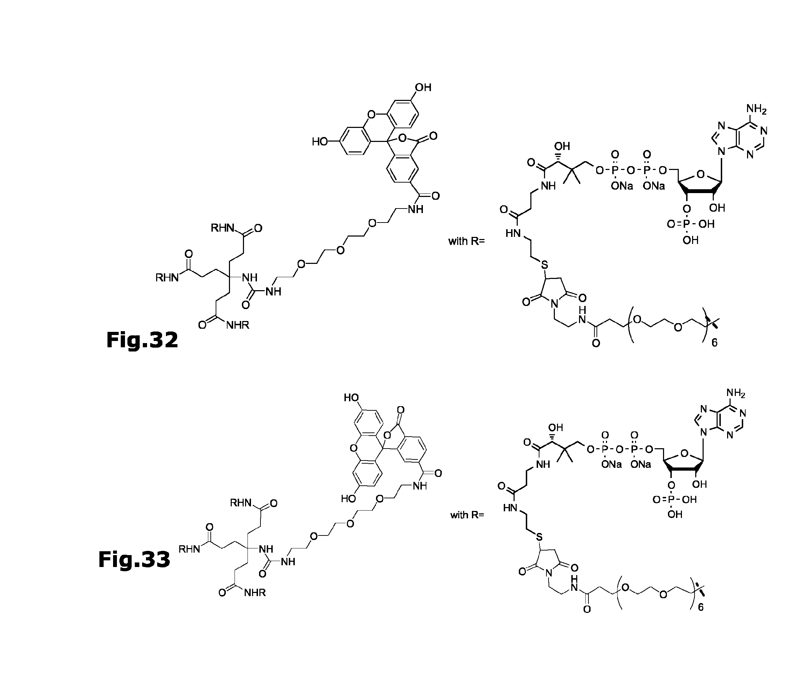

### Chemical synthesis of N-Tris{2-(2-(2-(CoA-S-succinimido)ethyl-aminocarbonyl-PEG12)ethylaminocarbonyl)ethoxymethyl}methyl N'-2-(2-(2-(2-fluorescein-5-carboxamidoethoxy)ethoxy)ethoxy)ethyl-urea (Fig. 32) and corresponding 6-fluorescein derivative (Fig. 33)

Compound **(****Fig. 31****)** (20 mg, 0.004 mmol) and 5(6)-carboxyfluorescein NHS ester (2 mg, 0.004 mmol) are dissolved in 600 µL DMF with Et₃N (0.6 µL, 0.004 mmol) and heated overnight at 31°C. The solvent is evaporated under vacuum and the compounds **(****Fig. 32)** and **(Fig. 33****)** isolated by reversed phase HPLC on a C18 column using a linear gradient of water:acetonitrile (from 95:5 to 20:80 in 20 min, 0.08% TFA). ESI-MS: m/z 5400.9 [M-Na]⁻.

### Example 30:

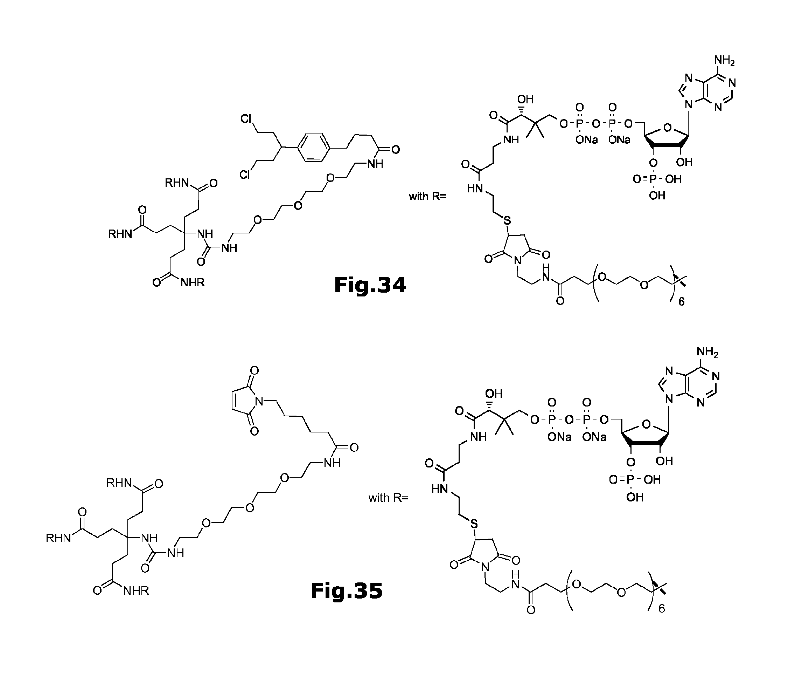

### Chemical Synthesis of N-Tris{2-(2-(2-(CoA-S-succinimido)ethylaminocarbonyl-PEG12)ethylaminocarbonyl)ethoxymethyl}methyl-N'-2-(2-(2-(2- chlorambucil-carboxamido-ethoxy)ethoxy)ethoxy)ethyl-urea (Fig. 34)

To a solution of chlorambucil (2.1 mg, 0.007 mmol) in DMF (1 mL) is added PYBOP (3.5 mg, 0.007 mmol) at rt. The solution is stirred at room temperature for 20 min. Then compound **(****Fig. 31****)** (35 mg, 0.007 mmol) and DIPEA (1.1 µL, 0.007 mmol) are added and the solution is heated at 50°C for 5min. The solution is stirred at room temperature overnight. Then the solvent is removed under reduced pressure. Compound **(****Fig. 34****)** is isolated by reversed phase HPLC on a C18 column using a linear gradient of water:acetonitrile (from 95:5 to 20:80 in 20 min, 0.08% TFA).

ESI-MS: m/z 5327.8 [M-Na]⁻.

### Example 31:

### Chemical Synthesis of N-Tris-{2-(2-(2-(CoA-S-succinimido)ethylaminocarbonyl-PEG12)ethylaminocarbonyl)ethoxymethyl}methyl-N'-2-('2-('2-('2-('6-maleimido-hexanoylamido)ethoxy)ethoxy)ethoxy)ethyl-urea (Fig. 35)

To a solution of 6-maleimido-hexanoic acid (1 mg, 0.005 mmol) in DMF (1 mL) is added PYBOP (2.5 mg, 0.005 mmol) at rt. The solution is stirred at room temperature for 20 min. Then compound **(****Fig. 31****)** (24 mg, 0.005 mmol) and DIPEA (0.8 µL, 0.005 mmol) are added and the solution is heated at 50°C for 5min. The solution is stirred at room temperature overnight. Then the solvent is removed under reduced pressure. Compound **(****Fig. 35****)** is isolated by reversed phase HPLC on a C18 column using a linear gradient of water:acetonitrile (from 95:5 to 20:80 in 20 min, 0.08% TFA). ESI-MS: m/z 5235.7 [M-Na]⁻.

### Example 32:

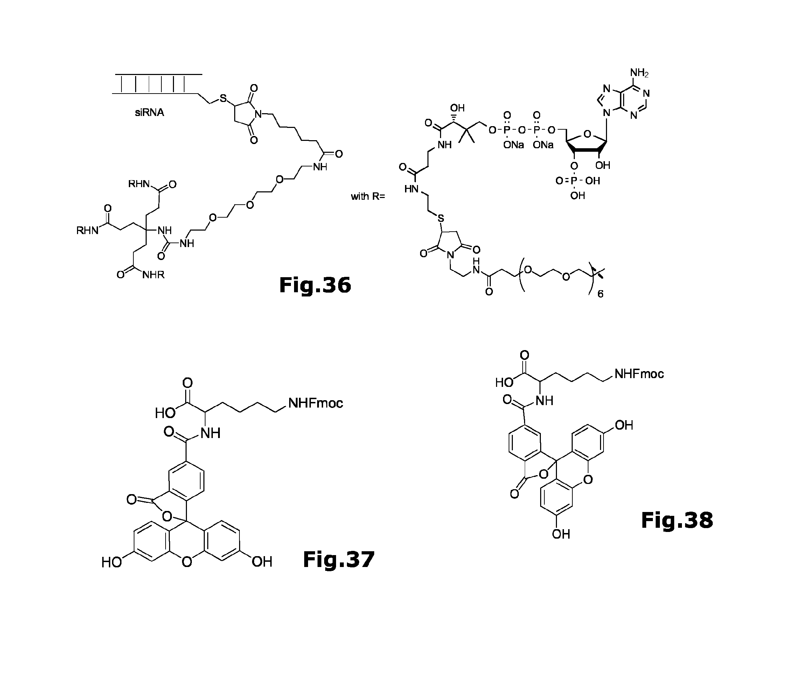

### Chemical synthesis of N-Tris{2-(2-(2-(CoA-S-succinimido)ethylaminocarbonyl-PEG12)ethylaminocarbonyl)ethoxymethyl}methyl-N'-2-(2-(2-(2-(6-maleimido-hexanoylamido)ethoxy)ethoxy)ethoxy)ethyl-urea siRNA conjugate (Fig. 36)

The 5'-thiol modified oligonucleotide (43 nmol) is reduced by incubation for 1 h at room temperature with 200 mM DTT in 200 µL Tris-buffer pH 8.5. The DTT is removed by gel filtration and the oligonucleotide eluted in PBS (pH 7.4). The most concentrated fractions are combined giving a total of 800 µL. 300 µL of a solution of compound **(****Fig. 35****)** (2.5 mM in DMF) is added and the reaction mixture incubated at room temperature for 1 h. The reaction mixture is diluted with water to a total volume of 2 mL, and excess maleimide removed by gel filtration. The conjugate **(****Fig. 36****)** is then purified by HPLC (solvent A: 0.1 M tetraethylammonium acetate pH 6.9 in water; solvent B: acetonitrile).

### Example 33:

### Chemical synthesis of 5-Fluorescein-Lys-Fmoc-OH (Fig. 37) and 6-fluorescein-Lys-Fmoc-OH (Fig. 38)

Fmoc-Lys-OH (184 mg, 0.5 mmol) and 5(6)-carboxyfluorescein NHS ester (237 mg, 0.5 mmol) are dissolved in 5 mL of DMF with Et₃N (70 µL, 0.5 mmol) and heated overnight at 31°C. Then the crude mixture is poured onto water (100 mL). The aqueous is basified (pH = 9) with NaOH (1 M). The aqueous phase is washed with ethyl acetate. Upon acidification of the aqueous phase with acetic acid, a yellowish precipitate is formed. The solid is collected via filtration to afford the desired compound as a mixture of isomers **(****Fig. 37)** and **(Fig. 38****).**

ESI-MS: m/z 727.7 [M+H]⁺.

### Example 34:

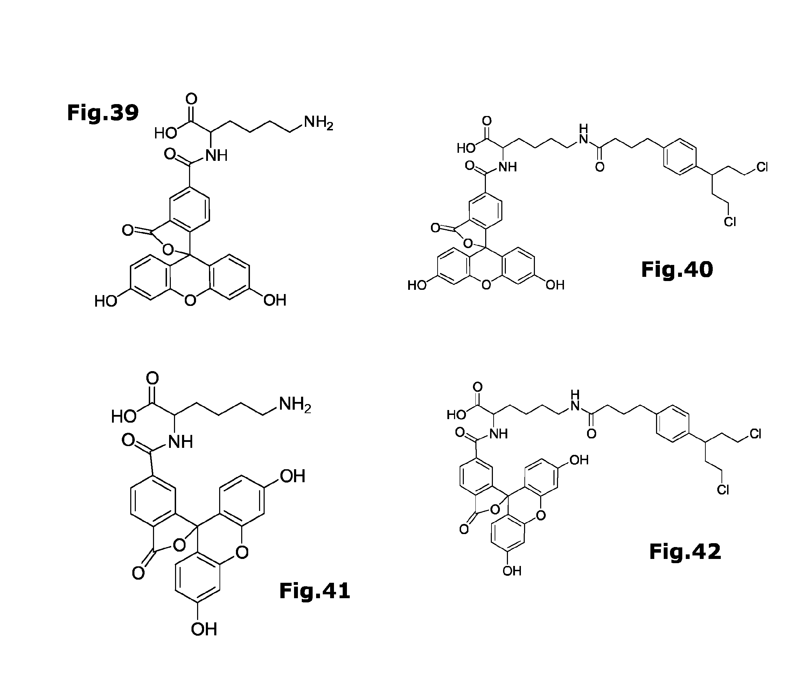

### Chemical synthesis of 5-Fluorescein-Lys-OH (Fig. 39) and 6-fluorescein-Lys-OH (Fig. 40)

To a solution of mixture of compounds **(****Fig. 37)** and **(Fig. 38****)** (300 mg, 0.4 mmol) in DMF (3 mL) is added diethylamine (600 µL) at rt. The solution is stirred at room temperature for 3 h. The solvent is removed under reduced pressure and the desired mixture of compounds **(****Fig. 39)** and **(Fig. 40****)** is directly used for next step.

ESI-MS: m/z 505.15 [M+H]⁺.

### Example 35:

### Chemical synthesis N-5-Fluorescein-N'-chlorambucil-Lys-OH (Fig. 41) and N-6-fluorescein-N'-chlorambucil-Lys-OH (Fig. 42)

To a solution of chlorambucil (106 mg, 0.35 mmol) in DMF (3 mL) is added PYBOP (182 mg, 0.35 mmol) at rt. The solution is stirred at room temperature for 20 min. Then the mixture of isomers **(****Fig. 39)** and **(Fig. 40****)** (176 mg, 0.35 mmol) and DIPEA (58 µL, 0.35 mmol) are added and the solution is heated at 50°C for 5 min. The solution is stirred at room temperature overnight. Then the crude mixture is poured onto water (60 mL). The aqueous solution is basified (pH = 9) with NaOH (1 M). The aqueous phase is washed with ethyl acetate. Upon acidification of the aqueous phase with acetic acid, a yellowish precipitate is formed. The solid is collected via filtration to afford the desired compound as a mixture of isomers **(****Fig. 41)** and **(Fig. 42****).**

ESI-MS: m/z 789.23 [M+H]⁺.

### Example 36:

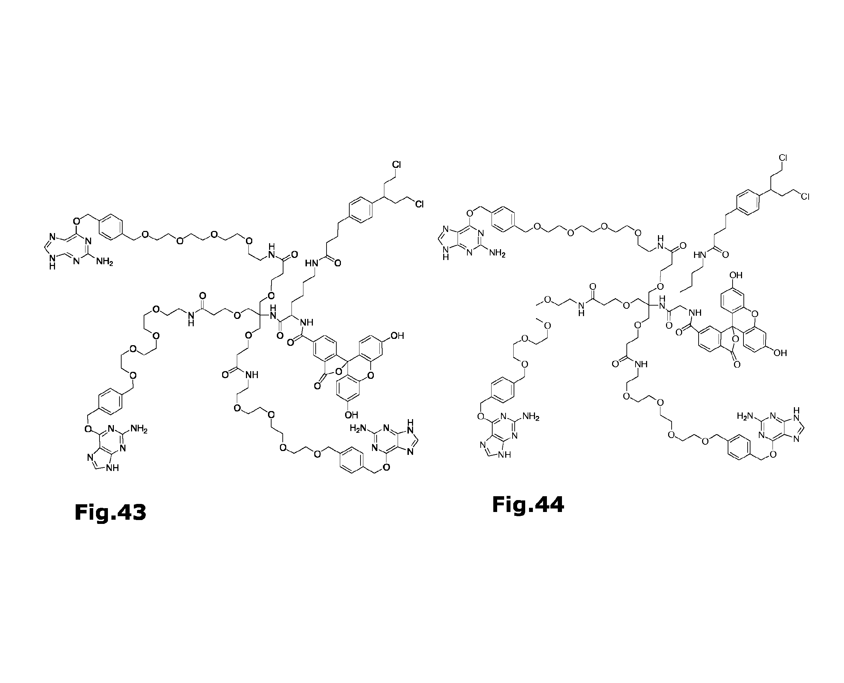

### Chemical synthesis of N-Tris(BG-PEG4-NH-carbonylethyloxymethyl)-methyl N'-5-fluorescein-N"-chlorambucil-Lys-amide (Fig. 43) and corresponding 6-fluorescein derivative (Fig. 44)

To a solution of a mixture of isomers **(****Fig. 41)** and **(Fig. 42****)** (15 mg, 0.02 mmol) in DMF (2 mL) is added PYBOP (10 mg, 0.02 mmol) at rt. The solution is stirred at room temperature for 20 min. Then compound **(****Fig. 5****)** (32 mg, 0.02 mmol) and DIPEA (3.3 µL, 0.02 mmol) are added and the solution is heated at 50°C for 5 min. The solution is stirred at room temperature overnight. The solution is poured onto water, the precipitate is filtered and washed with water. The desired compounds **(****Fig. 43)** and **(Fig. 44****)** are obtained as a solid. ESI-MS: m/z 2393.01 [M+H]⁺.

### Example 37:

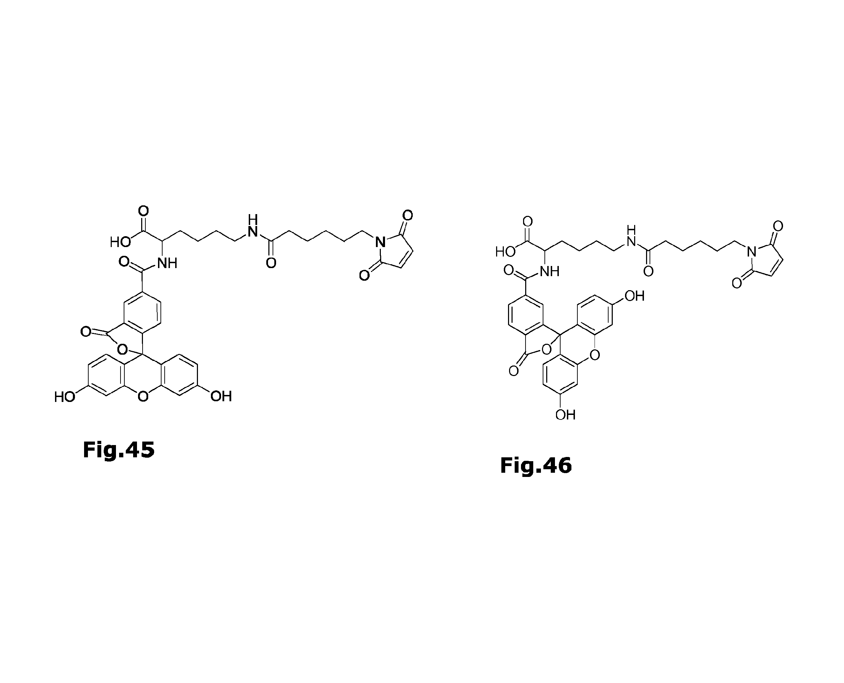

### Chemical synthesis of N-5-Fluorescein-N'-6-maleimidohexanoyl-Lys-OH (Fig. 45) and N-6-fluorescein-N'-6-maleimidohexanoyl-Lys-OH (Fig. 46)

To a solution of 6-maleimido-hexanoic acid (66 mg, 0.31 mmol) in DMF (3 mL) is added PYBOP (161 mg, 0.31 mmol) at rt. The solution is stirred at room temperature for 20 min. Then the mixture of compounds **(****Fig. 39)** and **(Fig. 40****)** (156 mg, 0.31 mmol) and DIPEA (51 µL, 0.31 mmol) is added and the solution is heated at 50°C for 5 min. The solution is stirred at room temperature overnight. Then the crude mixture is poured onto water (60 mL). The aqueous is basified (pH = 9) with NaOH (1 M). The aqueous phase is washed with ethyl acetate. Upon acidification of the aqueous phase with acetic acid, a yellowish precipitate is formed. The solid is collected via filtration to afford the desired compound as a mixture of isomers **(****Fig. 45)** and **(Fig. 46****).** ESI-MS: m/z 699.23 [M+H]⁺.

### Example 38:

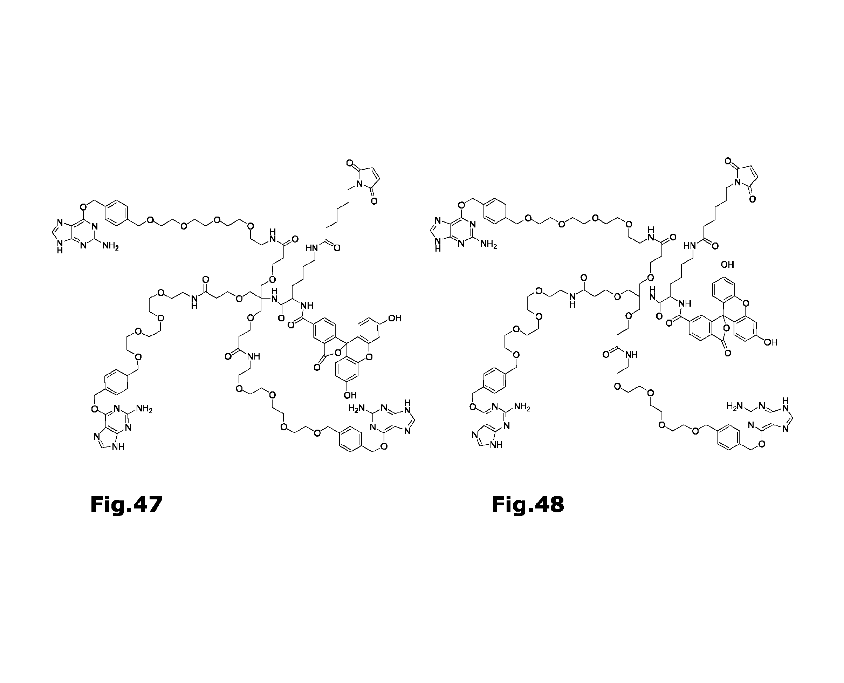

### Chemical synthesis of N-Tris(BG-PEG4-NH-carbonylethyloxymethyl)methyl N'-5-fluorescein-N"-6-maleimidohexanoyl-Lys-amide (Fig. 47) and corresponding 6-fluorescein derivative (Fig. 48)

To a solution of mixture of isomers **(****Fig. 45)** and **(Fig. 46****)** (9 mg, 0.013 mmol) in DMF (2 mL) is added PYBOP (6.5 mg, 0.013 mmol) at rt. The solution is stirred at room temperature for 20 min. Then compound **(****Fig. 5****)** (21 mg, 0.013 mmol) and DIPEA (2.1 µL, 0.013 mmol) are added and the solution is heated at 50°C for 5 min. The solution is stirred at room temperature overnight. The solution is poured onto water, the precipitate is filtered and washed with water. The desired compound is obtained as a mixture of isomers **(****Fig. 47)** and **(Fig. 48****)** as a solid.

ESI-MS: m/z 2302.01 [M+H]⁺.

### Example 39:

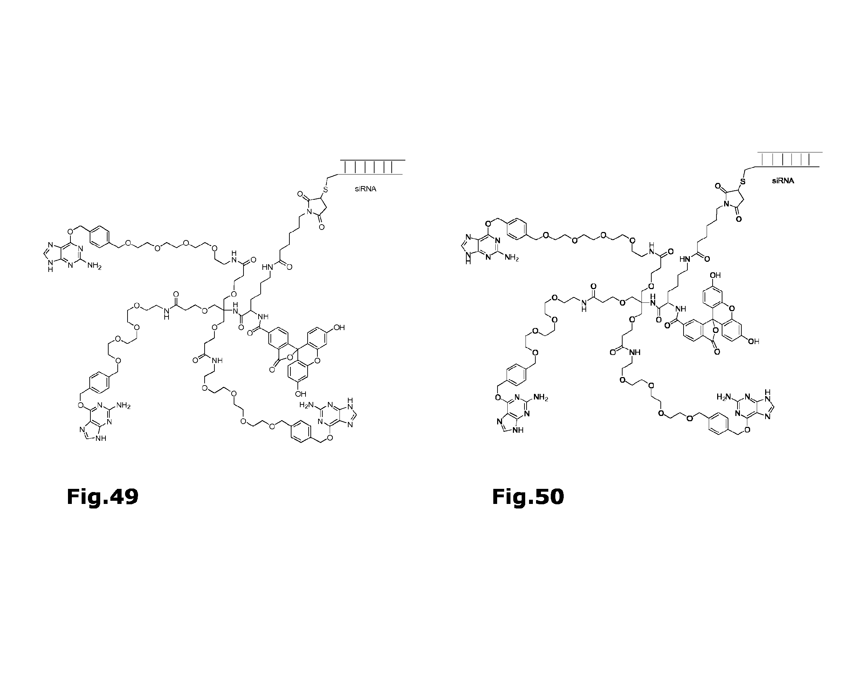

### Chemical synthesis of N-Tris(BG-PEG4-NH-carbonylethyloxymethyl)methyl N'-5-fluorescein-N"-6-maleimidohexanoyl-amide siRNA conjugate (Fig. 49) and corresponding 6-fluorescein derivative (Fig. 50)

The 5'-thiol modified oligonucleotide (43 nmol) is reduced by incubation for 1 h at room temperature with 200 mM DTT in 200 µL Tris-buffer pH 8.5. The DTT is removed by gel filtration and the oligonucleotide eluted in PBS (pH 7.4). The most concentrated fractions are combined giving a total of 800 µL. 300 µL solution of a mixture of isomers **(****Fig. 47)** and **(Fig. 48****)** (2.5 mM in DMF) is added and the reaction mixture incubated at room temperature for 1 h. The reaction mixture is diluted with water to a total volume of 2 mL and excess maleimide removed by gel filtration. The mixture of conjugates **(****Fig. 49)** and **(Fig. 50****)** is then purified by HPLC (solvent A: 0.1 M tetraethylammonium acetate pH 6.9 in water; solvent B: acetonitrile).

### Example 40:

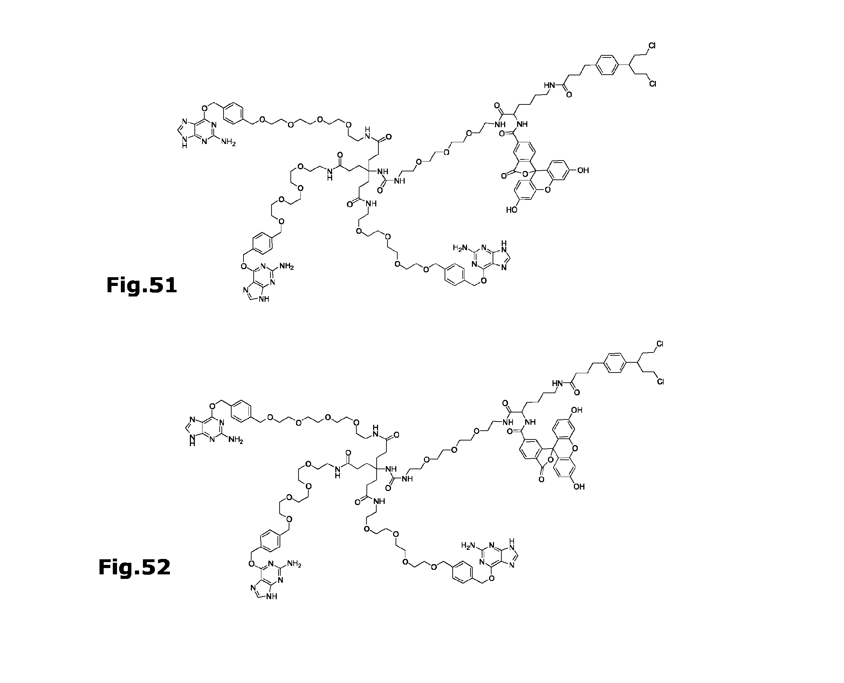

### Chemical synthesis of N-Tris(BG-PEG4-NH-carbonylethyloxymethyl)methyl N'-2-(2-(2-(2-(N"-5-fluorescein-N"'-chlorambucil-Lys-amido)ethoxy)ethoxy)ethoxy)-ethyl-urea (Fig. 51) and corresponding 6-fluorescein derivative (Fig. 52)

To a solution of mixture of isomers **(****Fig. 41)** and **(Fig. 42****)** (19 mg, 0.024 mmol) in DMF (3 mL) is added PYBOP (13 mg, 0.024 mmol) at rt. The solution is stirred at room temperature for 20 min. Then compound **(****Fig. 14****)** (42 mg, 0.024 mmol) and DIPEA (4 µL, 0.024 mmol) are added and the solution is heated at 50°C for 5 min. The solution is stirred at room temperature overnight. The solution is poured onto water, the precipitate is filtered and washed with water. The desired compound is obtained as a mixture of isomers **(****Fig. 51)** and **(Fig. 52****).**

ESI-MS: m/z 2521.10 [M+H]⁺.

### Example 41:

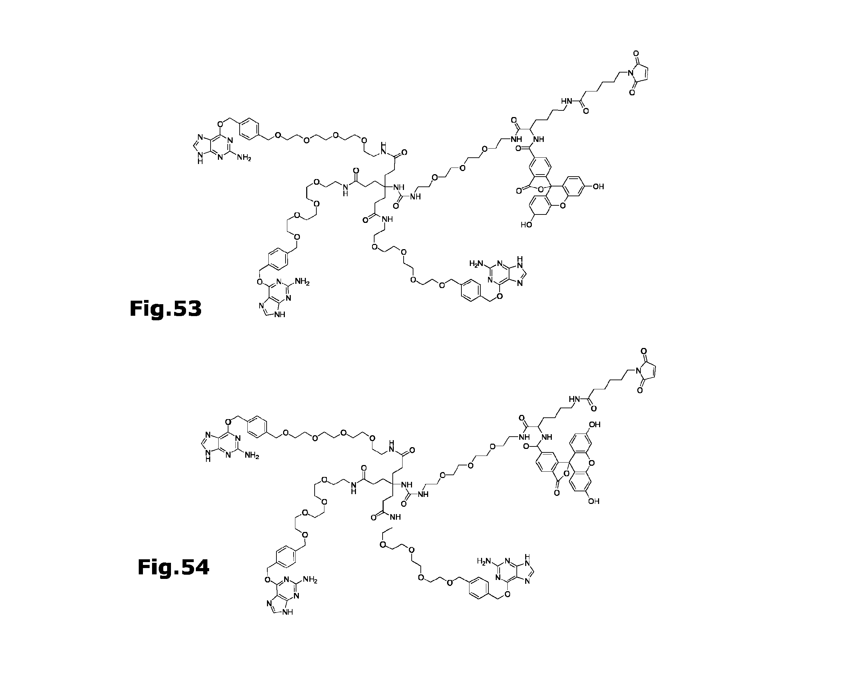

### Chemical synthesis of N-Tris(BG-PEG4-NH-carbonylethyloxymethyl)methyl N'-2-(2-(2-(2-(N"-5-fluorescein-N"'-6-maleimidohexanoyl-Lys-amido)ethoxy)ethoxy)ethoxy)ethyl-urea (Fig. 53) and corresponding 6-fluorescein derivative (Fig. 54)

To a solution of a mixture of isomers **(****Fig. 45)** and **(Fig. 46****)** (21 mg, 0.03 mmol) in DMF (3 mL) is added PYBOP (16 mg, 0.03 mmol) at rt. The solution is stirred at room temperature for 20 min. Then compound **(****Fig. 14****)** (53 mg, 0.03 mmol) and DIPEA (5 µL, 0.03 mmol) are added and the solution is heated at 50°C for 5 min. The solution is stirred at room temperature overnight. The solution is poured onto water, the precipitate is filtered and washed with water. The desired compound is obtained as a mixture of isomers **(****Fig. 53)** and **(Fig. 54****).**

ESI-MS: m/z 2430.10 [M+H]⁺.

### Example 42:

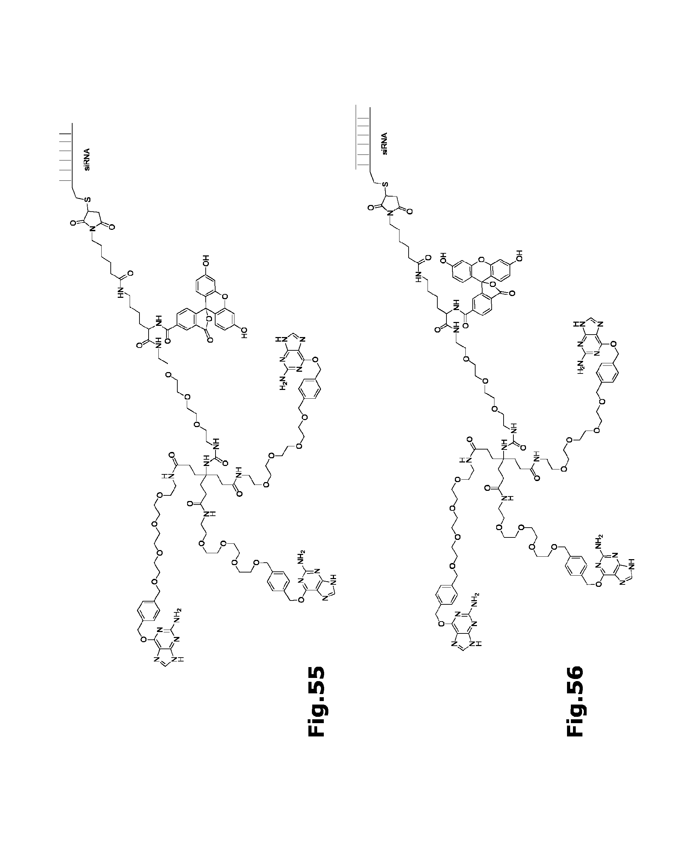

### Chemical synthesis of N-Tris(BG-PEG4-NH-carbonylethyloxymethyl)methyl N'-2-(2-(2-(2-(N"-5-fluorescein-N"'-6-maleimidohexanoyl-Lys-amido)ethoxy)ethoxy)-ethoxy)ethyl-urea siRNA conjugate (Fig. 55) and corresponding 6-fluorescein derivative (Fig. 56)

The 5'-thiol modified oligonucleotide (43 nmol) is reduced by incubation for 1 h at room temperature with 200 mM DTT in 200 µL Tris-buffer pH 8.5. The DTT is removed by gel filtration and the oligonucleotide eluted in PBS (pH 7.4). The most concentrated fractions are combined giving a total of 800 µL. 300 µL solution of a mixture of isomers **(****Fig. 53)** and **(Fig. 54****)** (2.5 mM in DMF) is added and the reaction mixture incubated at room temperature for 1 h. The reaction mixture is diluted with water to a total volume of 2 mL, and excess maleimide removed by gel filtration. The mixture of conjugates **(****Fig. 55)** and **(Fig. 56****)** is then purified by HPLC (solvent A: 0.1 M tetraethylammonium acetate pH 6.9 in water; solvent B: acetonitrile).

### Example 43:

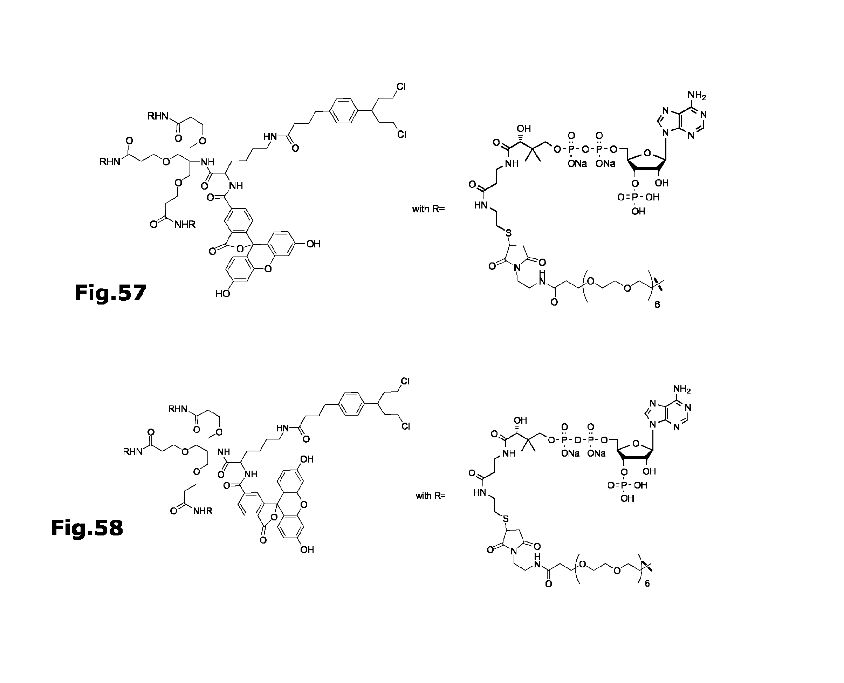

### Chemical synthesis of N-Tris{2-(2-(2-(CoA-S-succinimido)ethylaminocarbonyl-PEG12)ethylaminocarbonyl)ethoxymethyl}-methyl N'-5-fluorescein-N"-chlorambucil-Lys-amide (Fig. 57) and corresponding 6-fluorescein derivative (Fig. 58)

To a solution of mixture of isomers **(****Fig. 41)** and **(Fig. 42****)** (12 mg, 0.015 mmol) in DMF (2 mL) is added PYBOP (8 mg, 0.015 mmol) at rt. The solution is stirred at room temperature for 20 min. Then compound **(****Fig. 24****)** (73 mg, 0.015 mmol) and DIPEA (2.5 µL, 0.015 mmol) are added and the solution is heated at 50°C for 5 min. The solution is stirred at room temperature overnight. The solvent is removed under reduced pressure, and the compound is obtained as a mixture of isomers **(****Fig. 57)** and **(Fig. 58****)** by purification with preparative HPLC.

ESI-MS: m/z 5686.1 [M-Na]⁻.

### Example 44:

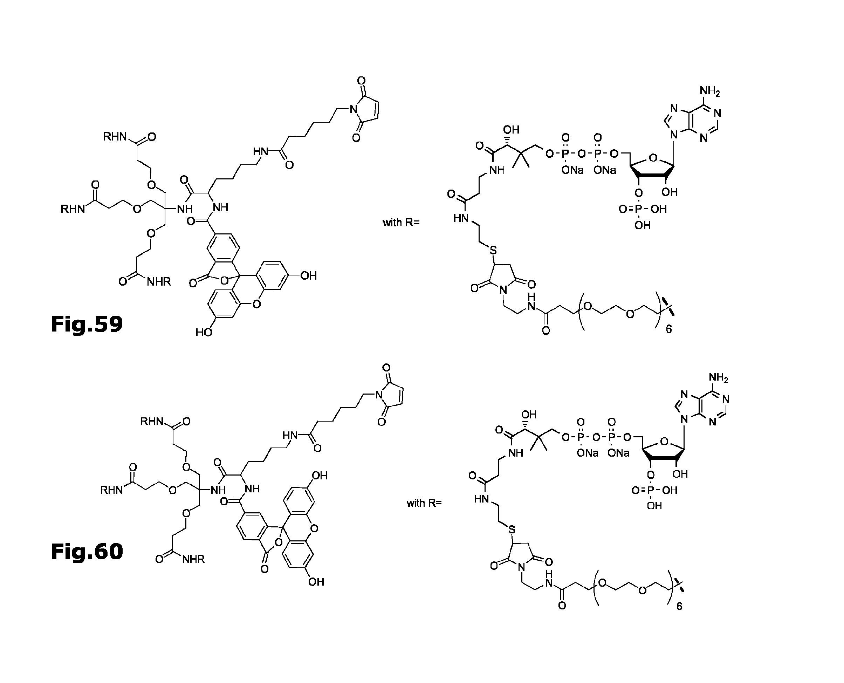

### Chemical synthesis of N-Tris{2-(2-(2-(CoA-S-succinimido)ethylaminocarbonyl-PEG12)ethylaminocarbonyl)ethoxymethyl}methyl N'-5-fluorescein-N"-6-maleimidohexanoyl-Lys-amide (Fig. 59) and corresponding 6-fluorescein derivative (Fig. 60)

To a solution of mixture of isomers **(****Fig. 45)** and **(Fig. 46****)** (7 mg, 0.01 mmol) in DMF (2 mL) is added PYBOP (5 mg, 0.01 mmol) at rt. The solution is stirred at room temperature for 20 min. Then compound **(****Fig. 24****)** (50 mg, 0.01 mmol) and DIPEA (1.65 µL, 0.01 mmol) are added and the solution is heated at 50°C for 5 min. The solution is stirred at room temperature overnight. The solvent is removed under reduced pressure, and the compound is obtained as a mixture of isomers **(****Fig. 59)** and **(Fig. 60****)** by purification with preparative HPLC.

ESI-MS: m/z 5594.1 [M-Na]⁻.

### Example 45:

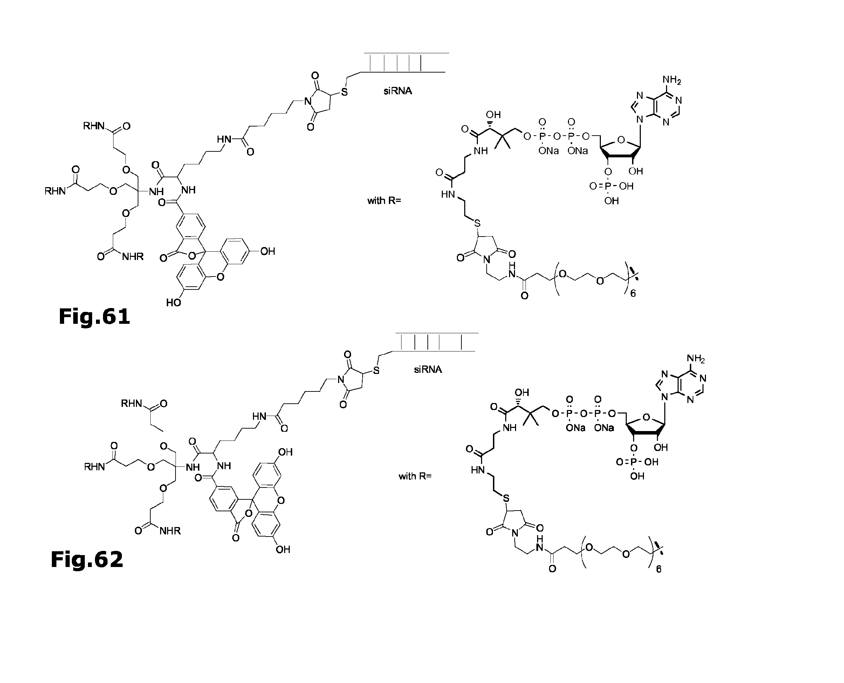

### Chemical synthesis of N-Tris{2-(2-(2-(CoA-S-succinimido)ethylaminocarbonyl-PEG12)ethylaminocarbonyl)ethoxymethyl}methyl N'-5-fluorescein-N"-6-maleimidohexanoyl-Lys-amide siRNA conjugate (Fig. 61) and corresponding 6-fluorescein derivative (Fig. 62)

The 5'-thiol modified oligonucleotide (43 nmol) is reduced by incubation for 1 h at room temperature with 200 mM DTT in 200 µL Tris-buffer pH 8.5. The DTT is removed by gel filtration and the oligonucleotide eluted in PBS (pH 7.4). The most concentrated fractions are combined giving a total of 800 µL. 300 µL of a solution of mixture of isomers **(****Fig. 59)** and **(Fig. 60****)** (2.5 mM in DMF) is added and the reaction mixture incubated at room temperature for 1 h. The reaction mixture is diluted with water to a total volume of 2 mL, and excess maleimide removed by gel filtration. The mixture of conjugates **(****Fig. 61)** and **(Fig. 62****)** is then purified by HPLC (solvent A: 0.1 M tetraethylammonium acetate pH 6.9 in water; solvent B: acetonitrile).

### Example 46:

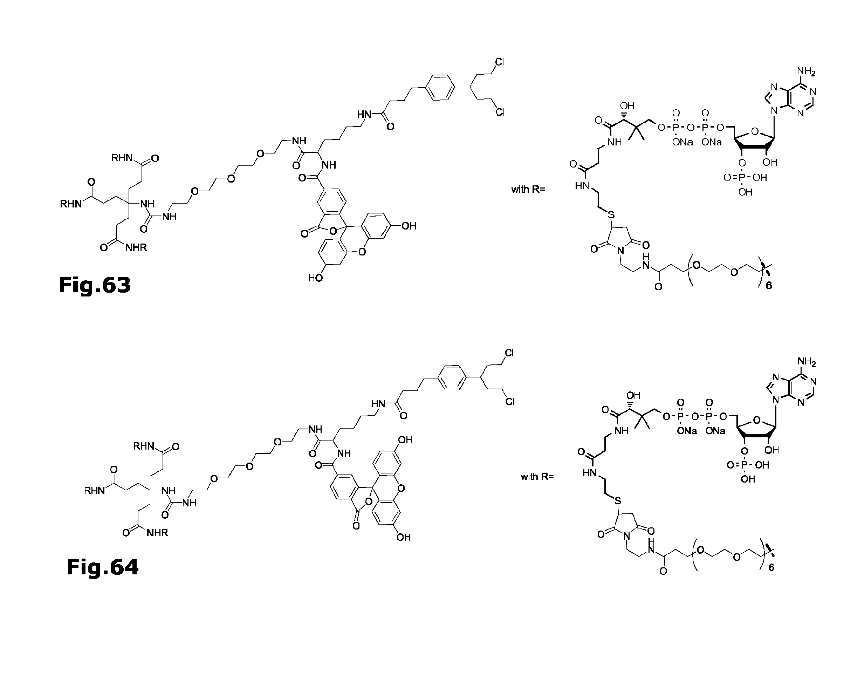

### Chemical synthesis of N-Tris{2-(2-(2-(CoA-S-succinimido)ethylaminocarbonyl-PEG12)ethylaminocarbonyl)ethoxymethyl}methyl N'-2-(2-(2-(2-(N"-5-fluorescein-N'"-chlorambucil-Lys-amido)ethoxy)ethoxy)ethoxy)ethyl-urea (Fig. 63) and corresponding 6-fluorescein derivative (Fig. 64)

To a solution of mixture of isomers **(****Fig. 41)** and **(Fig. 42****)** (5 mg, 0.006 mmol) in DMF (1 mL) is added PYBOP (3 mg, 0.006 mmol) at rt. The solution is stirred at room temperature for 20 min. Then compound **(****Fig. 31****)** (30 mg, 0.006 mmol) and DIPEA (1 µL, 0.006 mmol) are added and the solution is heated at 50°C for 5 min. The solution is stirred at room temperature overnight. The solvent is removed under reduced pressure, and the compound is obtained as a mixture of isomers **(****Fig. 63)** and **(Fig. 64****)** by purification with preparative HPLC.

ESI-MS: m/z 5814.9 [M-Na]⁻.

### Example 47:

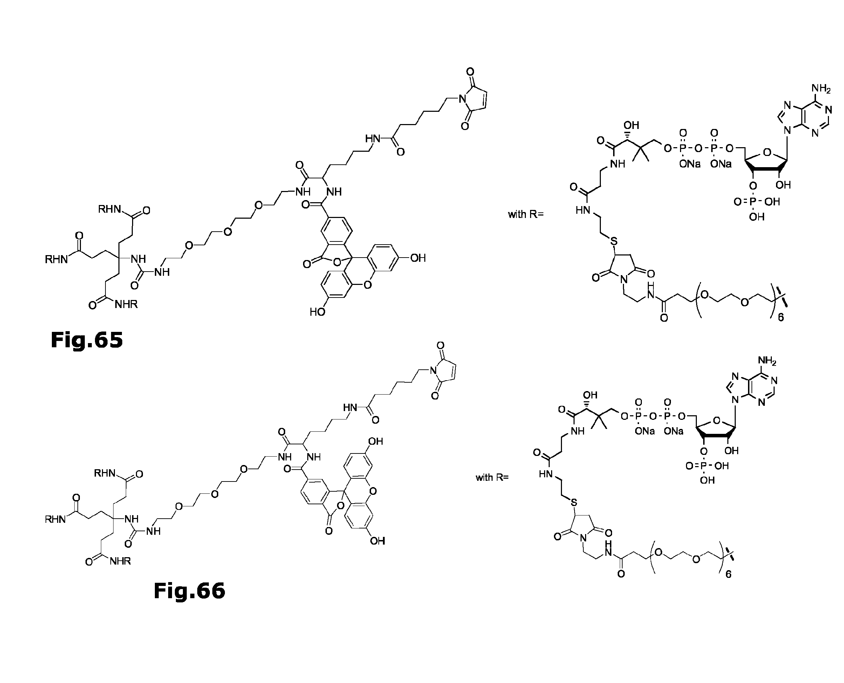

### Chemical synthesis of N-Tris{2-(2-(2-(CoA-S-succinimido)ethylaminocarbonyl-PEG12)ethylaminocarbonyl)ethoxymethyl}methyl N'-2-(2-(2-(2-(N"-5-fluorescein-N"'-6-maleimidohexanoyl-Lys-amido)ethoxy)ethoxy)ethoxy)ethyl-urea (Fig. 65) and corresponding 6-fluorescein derivative (Fig. 66)

To a solution of mixture of isomers **(****Fig. 45)** and **(Fig. 46****)** (14 mg, 0.02 mmol) in DMF (2 mL) is added PYBOP (10 mg, 0.02 mmol) at rt. The solution is stirred at room temperature for 20 min. Then compound **(****Fig. 31****)** (100 mg, 0.02 mmol) and DIPEA (3.3 µL, 0.02 mmol) are added and the solution is heated at 50°C for 5 min. The solution is stirred at room temperature overnight. The solvent is removed under reduced pressure, and the compound is obtained as a mixture of isomers **(****Fig. 65)** and **(Fig. 66****)** by purification with preparative HPLC.

ESI-MS: m/z 5722.2 [M-Na]⁻.

### Example 48:

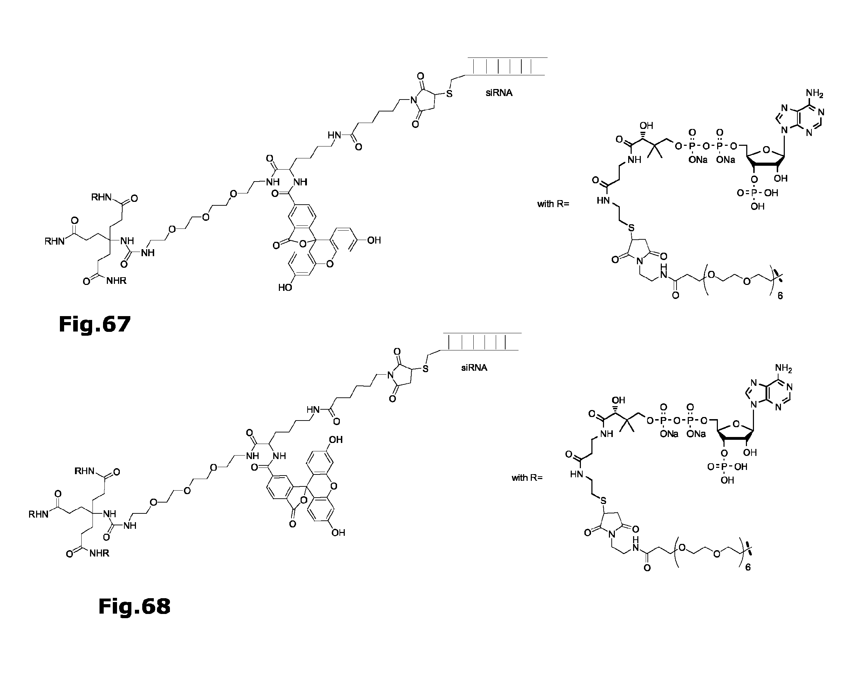

### Chemical synthesis of N-Tris{2-(2-(2-(CoA-S-succinimido)ethylaminocarbonyl-PEG12)ethylaminocarbonyl)ethoxymethyl}methyl N'-2-(2-(2-(2-(N"-5-fluorescein-N"'-6-maleimidohexanoyl-Lys-amido)ethoxy)ethoxy)ethoxy)ethyl-urea siRNA conjugate (Fig. 67) and corresponding 6-fluorescein derivative (Fig. 68)

The 5'-thiol modified oligonucleotide (43 nmol) is reduced by incubation for 1 h at room temperature with 200 mM DTT in 200 µL Tris-buffer pH 8.5. The DTT is removed by gel filtration and the oligonucleotide eluted in PBS (pH 7.4). The most concentrated fractions are combined giving a total of 800 µL. 300 µL solution of a mixture of isomers **(****Fig. 65)** and **(Fig. 66****)** (2.5 mM in DMF) is added and the reaction mixture incubated at room temperature for 1 h. The reaction mixture is diluted with water to a total volume of 2 mL and excess maleimide removed by gel filtration. The mixture of conjugates **(****Fig. 67)** and **(Fig. 68****)** is then purified by HPLC (solvent A: 0.1 M tetraethylammonium acetate pH 6.9 in water; solvent B: acetonitrile).

### Example 49:

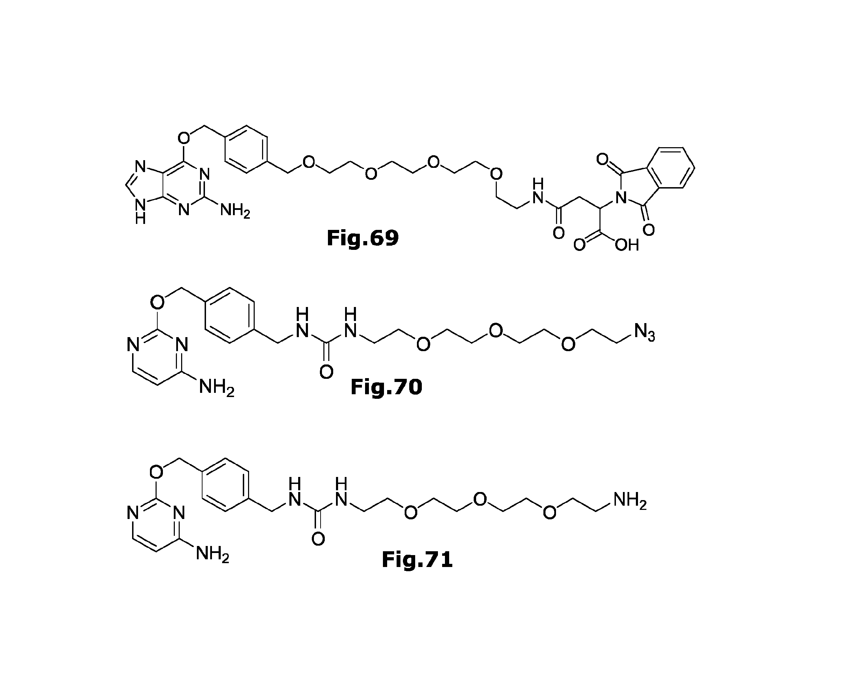

### Chemical synthesis of 2-Phthalimido-N-(BG-PEG4)-succinic acid monoamide (Fig. 69)

To a solution of BG-PEG4-NH2 (620 mg, 1.3 mmol, 1 eq) in DMF (15 mL) is added 2-phthalimido-succinic anhydride (340 mg, 1.39 mmol, 1 eq) at rt. The reaction mixture is stirred at room temperature for 4 h, then the crude mixture is poured into water (225 mL). The pH of the water phase is adjusted to 8 with NaOH (1 M), and the precipitate disappears. The aqueous layer is washed with EtOAc (2 times 100 mL). Then the pH is adjusted to 4 and the precipitate is collected. ESI-MS: m/z 692.69 [M+H]⁺.

### Example 50:

### Chemical synthesis of N-4-((4-Aminopyrimidin-2-yloxy)methyl)benzyl-N'-2-(2-(2-(2-azidoethoxy)ethoxy)ethoxy)ethyl-urea (Fig. 70)

To a solution of 11-azido-3,6,9-trioxaundecan-1-amine (73 µL, 1 eq, 0.37 mmol) in DMF (3 mL) is added CDI (60 mg, 1 eq, 0.37 mmol). The solution is stirred overnight at rt. To the solution is added BC-NH2 (85 mg, 1 eq, 0.37 mmol) and the mixture is heated at 65°C for 3 h. Then the crude mixture is poured onto water and extracted with ethyl acetate, the organic phase is dried over MgSO₄, and evaporated under reduced pressure to afford the desired compound. No further purification is required.

TLC (CH₂Cl₂/MeOH 10:1). ESI-MS: m/z 475.51 [M+H]⁺.

### Example 51:

### Chemical synthesis of N-4-((4-Aminopyrimidin-2-yloxy)methyl)benzyl-N'-2-(2-(2-(2-aminoethoxy)ethoxy)ethoxy)ethyl-urea (Fig. 71)

To a solution of compound **(****Fig. 70****)** (54 mg, 0.12 mmol) in dioxane (3 mL) is added water (360 µL). Then PMe₃ (720 µL 1 M in THF solution, 6 eq) is added and the solution is stirred at room temperature for 2 h. The solvent is removed under reduced pressure, and the compound **(****Fig. 71****)** is obtained by purification with preparative HPLC. ESI-MS: m/z 449.52 [M+H]⁺.

### Example 52:

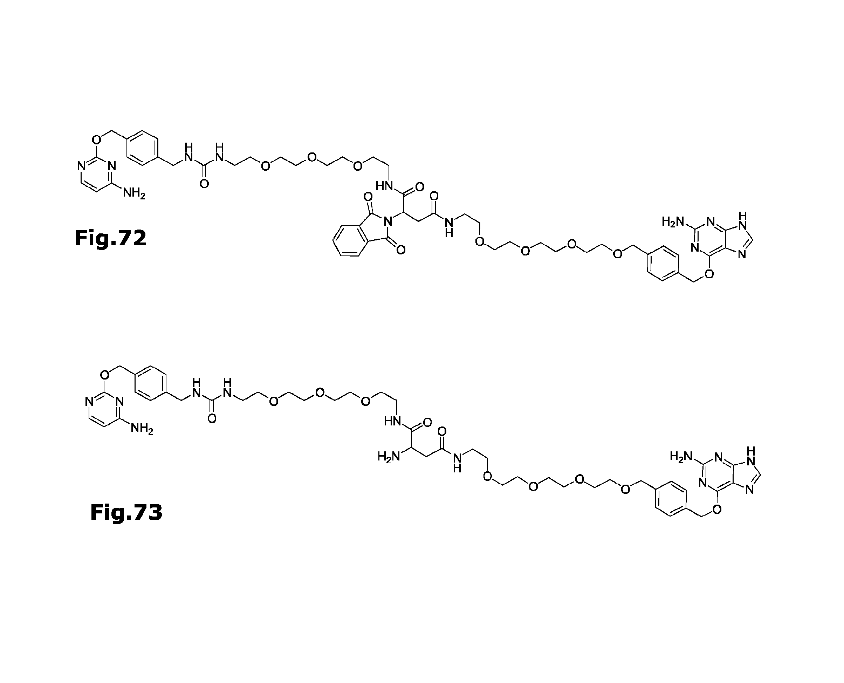

### Chemical synthesis N-4-((4-Aminopyrimidin-2-yloxy)methyl)benzyl-N'-2-(2-(2-(2-(3-BG-PEG4-NH-carbonyl-2-phthalimido-propionylaminoethoxy)ethoxy)-ethoxy)ethyl-urea (Fig. 72)

To a solution of compound **(****Fig. 71****)** (45 mg, 0.1 mmol, 1 eq) and compound **(****Fig. 69****)** (69 mg, 0.1 mmol, 1 eq) in DMF (2 mL) are successively added DIPEA (17 µL, 0.1 mmol, 1 eq), HOBT (1 M in NMP, 0.1 mL, 0.1 mmol, 1 eq) and DCC (21 mg, 1 mmol, 1 eq) at rt. The resulting mixture is stirred overnight. Then the solvent is removed under reduced pressure and the mixture is diluted with 50 mL EtOAc. The organic layer is washed with water, dried over MgSO₄ and evaporated under reduced pressure. Flash chromatography (CH₂Cl₂/MeOH, 10/1→5/1) gives the desired compound **(****Fig. 72****).**

ESI-MS: m/z 1123.19 [M+H]⁺.

### Example 53:

### Chemical synthesis of N-4-((4-Aminopyrimidin-2-yloxy)methyl)benzyl-N'-2-(2-(2-(2-(3-BG-PEG4-NH-carbonyl-2-amino-propionylaminoethoxy)ethoxy)ethoxy)ethyl-urea (Fig. 73)

To a solution of compound **(****Fig. 72****)** (45 mg, 0.04 mmol) in EtOH (3 mL) is added methylamine (300 µl), and the solution is stirred at room temperature for 12 h. The solvent is removed under reduced pressure and the compound **(****Fig. 73****)** is obtained by purification with preparative HPLC. ESI-MS: m/z 993.09 [M+H]⁺.

### Example 54:

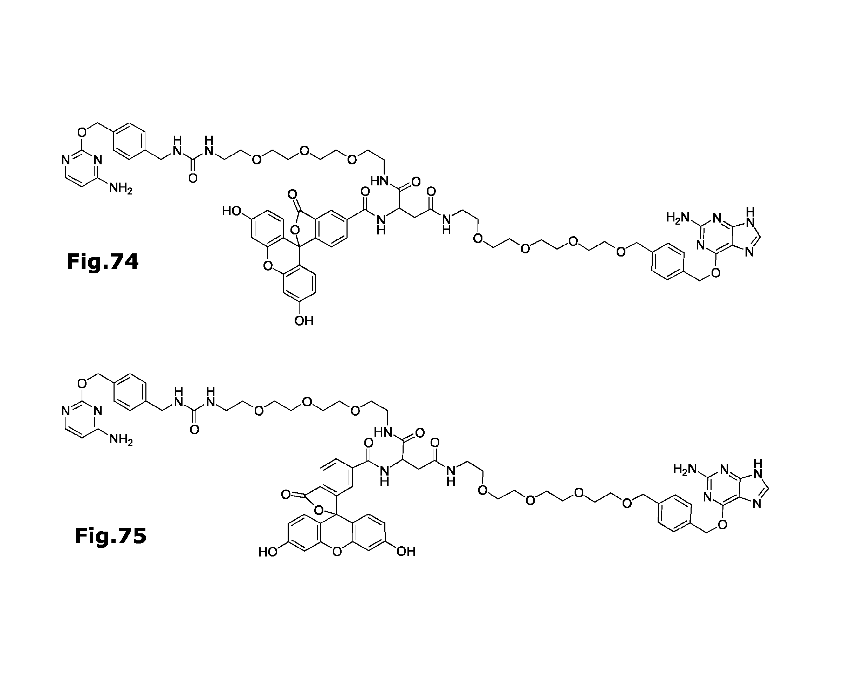

### Chemical synthesis of N-4-((4-Aminopyrimidin-2-yloxy)methyl)benzyl-N'-2-(2-(2-(2-(3-BG-PEG4-NH-carbonyl-2-(fluorescein-5-carboxamido)propionylamino-ethoxy)ethoxy)ethoxy)ethyl-urea (Fig. 74) and corresponding fluorescein-6-carboxamide (Fig. 75)

Compound **(****Fig. 73****)** (9 mg, 0.009 mmol) and 5(6)-carboxyfluorescein NHS ester (4 mg, 0.009 mmol) are dissolved in 800 µL DMF with Et₃N (1.35 µL, 0.009 mmol) and heated overnight at 31°C. The solvent is evaporated under vacuum and the compounds **(****Fig. 74)** and **(Fig. 75****)** isolated by reversed phase HPLC on a C18 column using a linear gradient of water:acetonitrile (from 95:5 to 20:80 in 20 min, 0.08% TFA).

ESI-MS: m/z 1351.39 [M+H]⁺.

### Example 55:

### Chemical synthesis of N-4-((4-Aminopyrimidin-2-yloxy)methyl)benzyl-N'-2-(2-(2-(2-(3-BG-PEG4-NH-carbonyl-2-(6-maleimidohexanoylamino)propionyl-amino-ethoxy)ethoxy)ethoxy)ethyl-urea (Fig. 72)

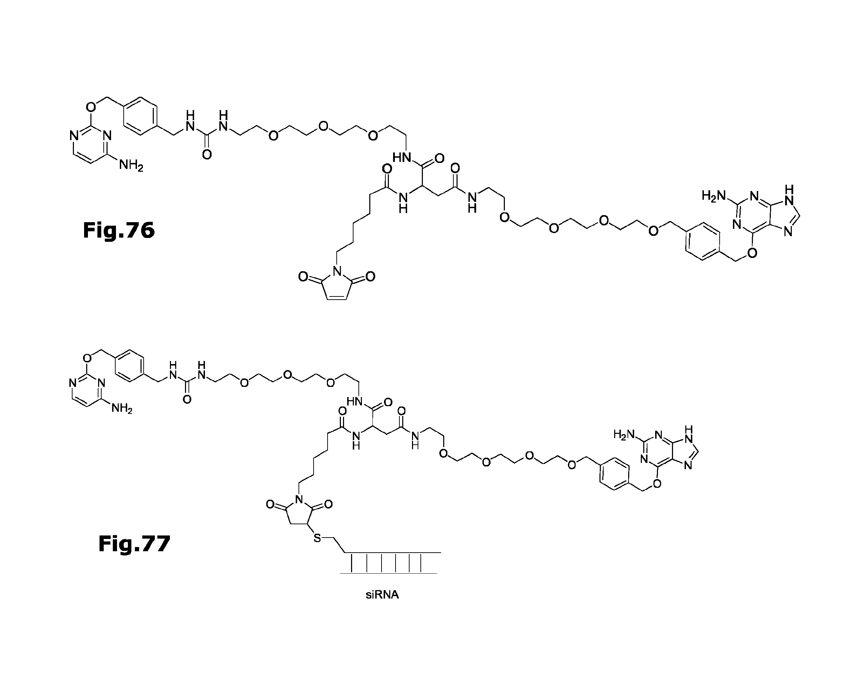

To a solution of 6-maleimido-hexanoic acid (4.4 mg, 0.02 mmol) in DMF (1 mL) is added PYBOP (10 mg, 0.02 mmol) at rt. The solution is stirred at room temperature for 20 min. Then compound **(****Fig. 73****)** (20 mg, 0.02 mmol) and DIPEA (3.3 µL, 0.02 mmol) are added and the solution is heated at 50°C for 5 min. The solution is stirred at room temperature overnight. Then the solvent is removed under reduced pressure and the mixture is diluted with 150 mL EtOAc. The organic layer is washed with water, dried over MgSO₄ and evaporated under reduced pressure. Flash chromatography (CH₂Cl₂/MeOH, 10/1→5/1) gives the desired compound **(****Fig. 76****).** ESI-MS: m/z 1186.29 [M+H]⁺.

### Example 56:

### Chemical synthesis N-4-((4-Aminopyrimidin-2-yloxy)methyl)benzyl-N'-2-(2-(2-(2-(3-BG-PEG4-NH-carbonyl-2-(6-maleimidohexanoylamino)propionylamino-ethoxy)ethoxy)ethoxy)ethyl-urea siRNA conjugate (Fig. 77)

The 5'-thiol modified oligonucleotide (43 nmol) is reduced by incubation for 1 h at room temperature with 200 mM DTT in 200 µL Tris-buffer pH 8.5. The DTT is removed by gel filtration and the oligonucleotide eluted in PBS (pH 7.4). The most concentrated fractions are combined giving a total of 800 µL. 300 µL solution of compound **(****Fig. 76****)** (2.5 mM in DMF) is added and the reaction mixture incubated at room temperature for 1 h. The reaction mixture is diluted with water to a total volume of 2 mL and excess maleimide removed by gel filtration. The conjugate **(****Fig. 77****)** is then purified by HPLC (solvent A: 0.1 M tetraethylammonium acetate pH 6.9 in water; solvent B: acetonitrile).

### Example 57:

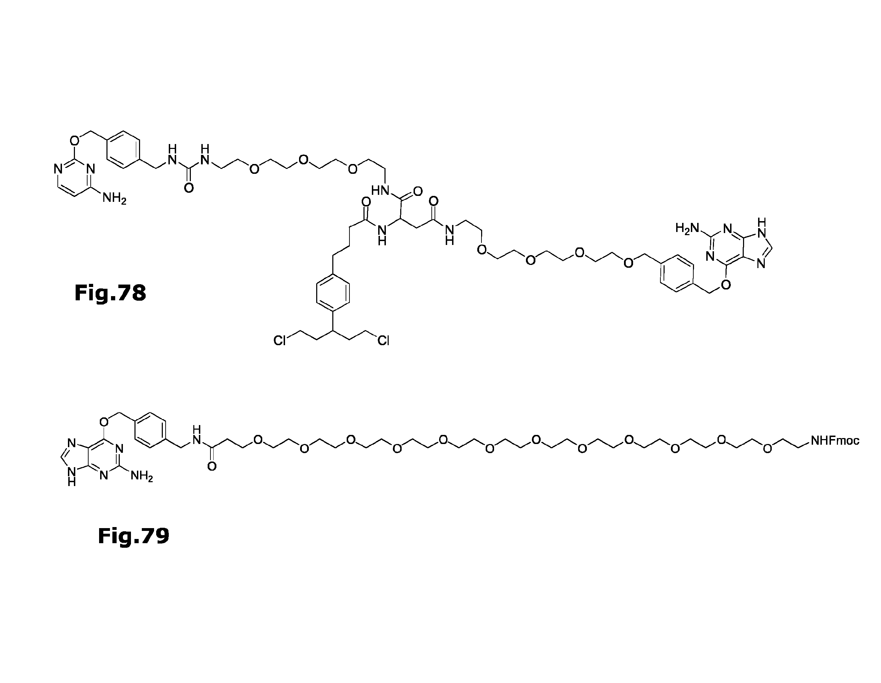

### Chemical synthesis N-4-((4-Aminopyrimidin-2-yloxy)methyl)benzyl-N'-2-(2-(2-(2-(3-BG-PEG4-NH-carbonyl-2-chlorambucilcarboxamino-propionylaminoethoxy)ethoxy)ethoxy)ethyl-urea (Fig. 78)

To a solution of chlorambucil (6 mg, 0.02 mmol) in DMF (1 mL) is added PYBOP (10 mg, 0.02 mmol) at rt. The solution is stirred at room temperature for 20 min. Then compound **(****Fig. 73****)** (20 mg, 0.02 mmol) and DIPEA (3.3 µL, 0.02 mmol) are added and the solution is heated at 50°C for 5 min. The solution is stirred at room temperature overnight. Then the solvent is removed under reduced pressure and the mixture is diluted with 150 mL of EtOAc. The organic layer is washed with water, dried over MgSO₄ and evaporated under reduced pressure. Flash chromatography (CH₂Cl₂/MeOH, 10/1→5/1) gives the desired compound **(****Fig. 78****).** ESI-MS: m/z 1276.56 [M+H]⁺.

### Example 58:

### Chemical synthesis of BG-PEG12-NHFmoc (Fig. 79)

To a solution of Fmoc-amido-PEG12-acid (1 g, 1.19 mmol) in DMF (2 mL) is added PYBOP (619 mg, 1.19 mmol) at rt. The solution is stirred at room temperature for 20 min. Then O⁶-aminomethylbenzyl guanine (320 mg, 1.19 mmol) and DIPEA (196 µL, 1.19 mmol) are added and the solution is heated at 50°C for 5 min. Then the solution is stirred at room temperature overnight. The crude mixture is poured onto diethyl ether. The precipitate is collected and washed with diethyl ether. The obtained solid is dissolved in MeOH and the solvent is concentrated until dryness. No further purification is required. MS (ESI) m/z 1093 [M+H]⁺.

### Example 59:

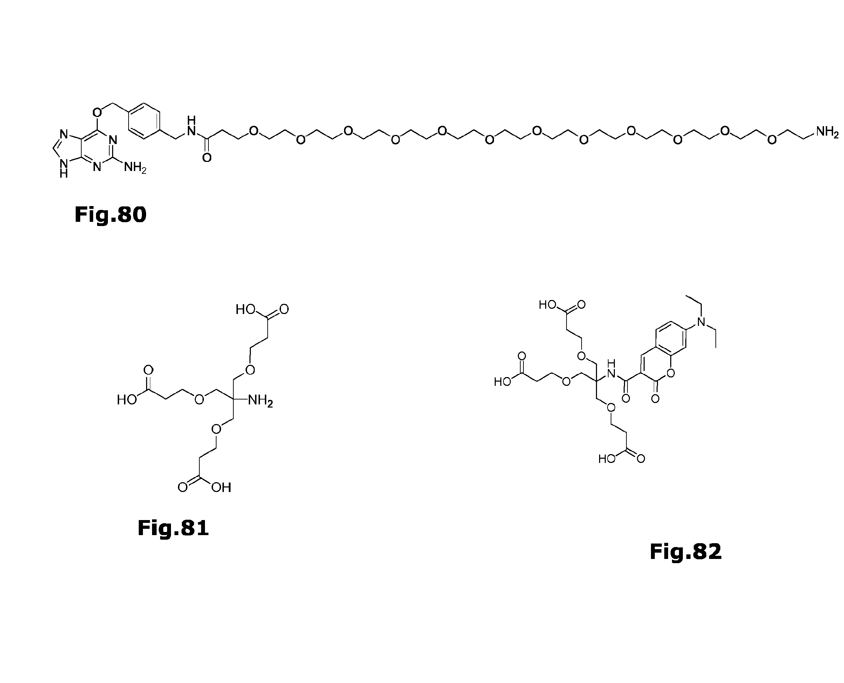

### Chemical synthesis of BG-PEG12-NH₂ (Fig. 80)

To a solution of compound **(****Fig. 79****)** (1.5 g, 1.72 mmol) in dioxane (10 mL) is added diethylamine (2.5 mL) at rt. The solution is stirred at room temperature for 3 h. Then the solvent is removed under reduced pressure. The crude mixture is dissolved in DMF (1.5 mL) and poured into diethyl ether (10 mL). The resulting precipitate is collected. No further purification is required. MS (ESI) m/z 871 [M+H]⁺.

### Example 60:

### Chemical synthesis of Tris{[2-carboxyethoxy]methyl}methylamine (Fig. 81)

Tris{[2-tert-butoxycarbonyl)ethoxy]methyl}methylamine **(****Fig. 1**)(4.3g, 8 mmol) is stirred in 80 mL of 96% formic acid for 18 h. Then the formic acid is removed at reduced pressure at 50°C to produce a colorless oil in quantitative yield. ¹H NMR ((CD₃)₂SO, 400 MHz): 8.2 (m, 2H), 7.45 (m, 3H), 3.6 (m, 6H), 3.4 (m, 6H), 2.45 (m, 6H).

### Example 61:

### Chemical synthesis of N-Tris[(2-carboxyethoxy)methyl]methyl 7-(diethylamino)coumarin-3-carboxamide (Fig. 82)

Compound **(****Fig. 81****)** (66 mg, 0.195 mmol) and 7-(diethylamino)coumarin-3-carboxylic acid N-succinimidyl ester (70 mg, 0.195 mmol) are dissolved in 2 mL of DMF with Et₃N (28 µL, 0.195 mmol) and heated overnight at 40°C. The solvent is evaporated under vacuum and the compound **(****Fig. 82****)** isolated by reversed phase HPLC on a C18 column using a linear gradient of water:acetonitrile (from 95:5 to 20:80 in 20 min, 0.08% TFA). MS (ESI) m/z 581 [M+H]⁺.

### Example 62:

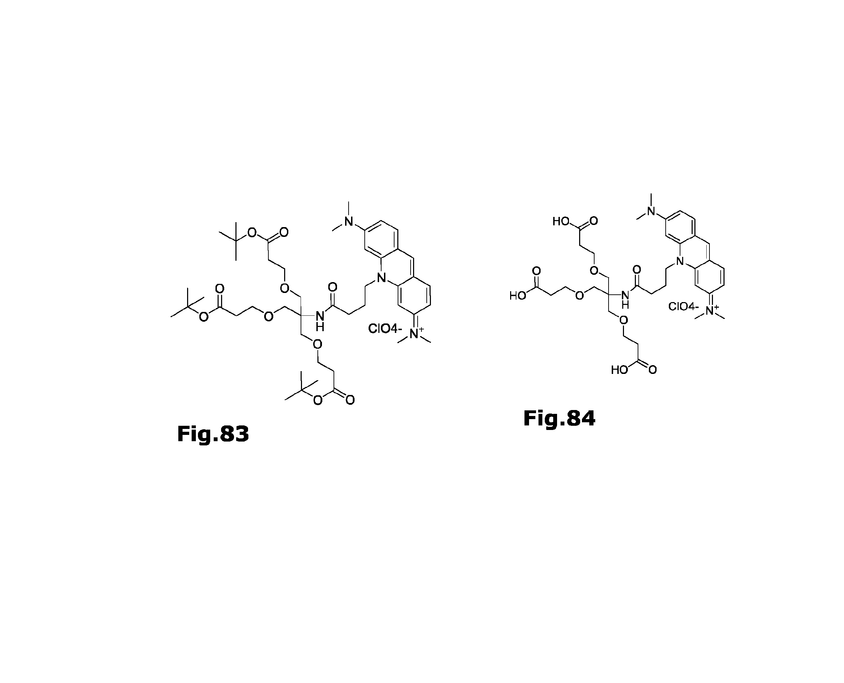

### Chemical synthesis of N-Tris{[2-(tertbutoxycarbonyl)ethoxy]methyl}methyl ATTO-495-carboxamide (Fig. 83)

Tris{[2-(tert-butoxycarbonyl)ethoxy]methyl}methylamine **(****Fig. 1****)** (4.8 mg, 9.45 µmol) and ATTO-495 N-succinimidyl ester (5.2 mg, 9.45 µmol) are dissolved in 2 mL DMF with Et₃N (1.3 µL, 9.45 µmol) and heated overnight at 40°C. The solvent is evaporated under vacuum and the compound **(****Fig. 83****)** isolated by reversed phase HPLC on a C18 column using a linear gradient of water:acetonitrile (from 95:5 to 20:80 in 20 min, 0.08% TFA). MS (ESI) m/z 841 [M+H]⁺.

### Example 63: Chemical synthesis of

### N-Tris[(2-carboxyethoxy)methyl]methyl ATTO-495-carboxamide (Fig. 84)

Compound **(****Fig. 83****)** (210 mg, 0.25 mmol) is stirred in 250 µL of 96% formic acid for 18 h. Then the formic acid is removed at reduced pressure at 5 °C to produce a colorless oil in quantitative yield. MS (ESI) m/z 672 [M+H]⁺.

### Example 64:

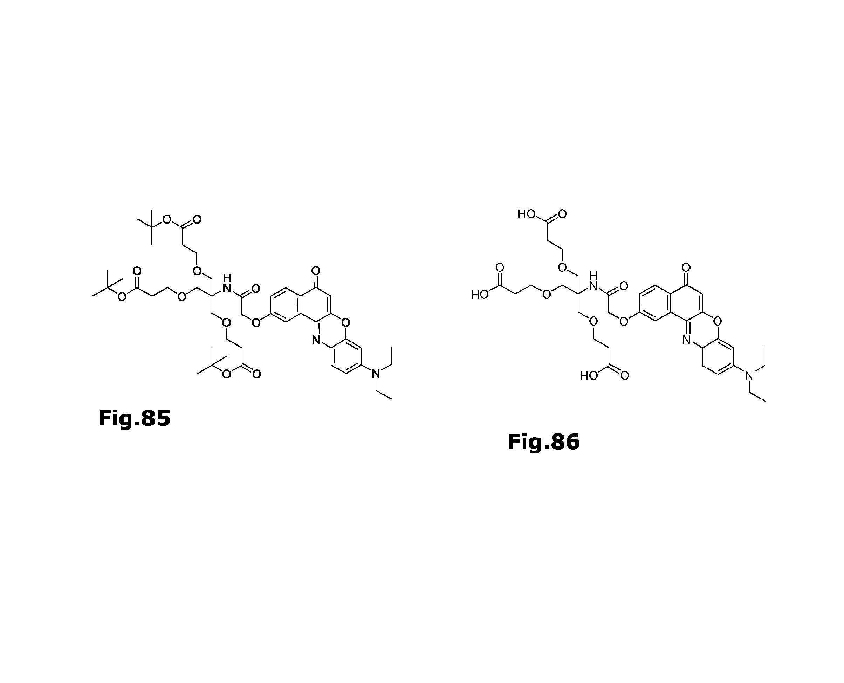

### Chemical synthesis of N-Tris{[2-(tert-butoxycarbonyl)ethoxy]methyl}methyl nile red-oxyacetamide (Fig. 85)

To a solution of nile red-oxyacetic acid (9-diethylamino-5-oxo-benzo[a]phenoxazin-2-oxyacetic acid, 100 mg, 0.255 mmol, 1 eq) in DMF (50 mL) are successively added DCC (160 mg, 0.765 mmol, 3 eq) and NHS (90 mg, 0.765 mmol, 3 eq). The resulting mixture is stirred overnight. Then DCU salts are removed by centrifugation. Compound **(****Fig. 1****)** (130 mg, 0.255 mmol, 1 eq) and DIPEA (42 µL, 0.255 mmol, 1 eq) are added to the solution at rt. The resulting mixture is stirred overnight. Then the solvent is removed under reduce pressure. Flash chromatography (CH₂Cl₂/MeOH, 10/1→5/1) gives the desired compound **(****Fig. 85****).** MS (ESI) m/z 881 [M+H]⁺.

### Example 65:

### Chemical synthesis of N-Tris[(2-carboxyethoxy)methyl]methyl nile red-oxyacetamide (Fig. 86)

Compound **(****Fig. 85****)** (70 mg, 0.08 mmol) is stirred in 250 µL of 96% formic acid for 18 h. Then the formic acid is removed under reduced pressure at 50°C to produce a colorless oil in quantitative yield. MS (ESI) m/z 712 [M+H]⁺.

### Example 66:

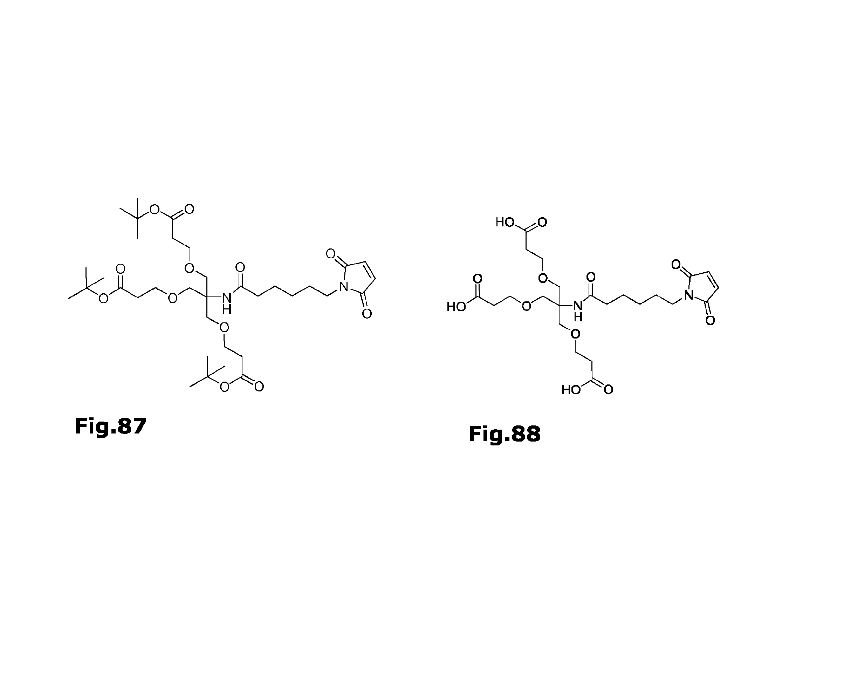

### Chemical synthesis of N-Tris{[2-(tert-butoxycarbonyl)ethoxy]methyl}methyl 5-maleimidopentanecarboxamide (Fig. 87)

To a solution of 6-maleimido-hexanoic acid (106 mg, 0.5 mmol) in DMF (5 mL) is added PYBOP (260 mg, 0.5 mmol) at rt. The solution is stirred at room temperature for 20 min. Then compound **(****Fig. 1****)** (253 mg, 0.5 mmol) and DIPEA (83 µL, 0.5 mmol) are added and the solution is heated at 50°C for 5 min. The solution is stirred at room temperature overnight. Then the solvent is removed under reduced pressure. Flash chromatography (cyclohexane/ethyl acetate, 1/1) gives the desired compound **(****Fig. 87****).** ¹H NMR ((CD₃)₂SO, 400 MHz): 6.7 (s, 2H), 3.7 (s, 6H), 3.65 (m, 6H), 3.5 (m, 2H), 2.45 (m, 6H), 2.1 (m, 2H), 1.6 (m, 4H), 1.45 (m, 27H), 1.35 (m, 2H).

### Example 67:

### Chemical synthesis of N-Tris[(2-carboxyethoxy)methyl]methyl 5-maleimidopentanecarboxamide (Fig. 88)

Compound **(****Fig. 87****)** (214 mg, 0.305 mmol) is stirred in 3 mL of 96% formic acid for 18 h. Then the formic acid is removed at reduced pressure at 50°C to produce a colorless oil in quantitative yield. The compound is directly used for next step.

### Example 68:

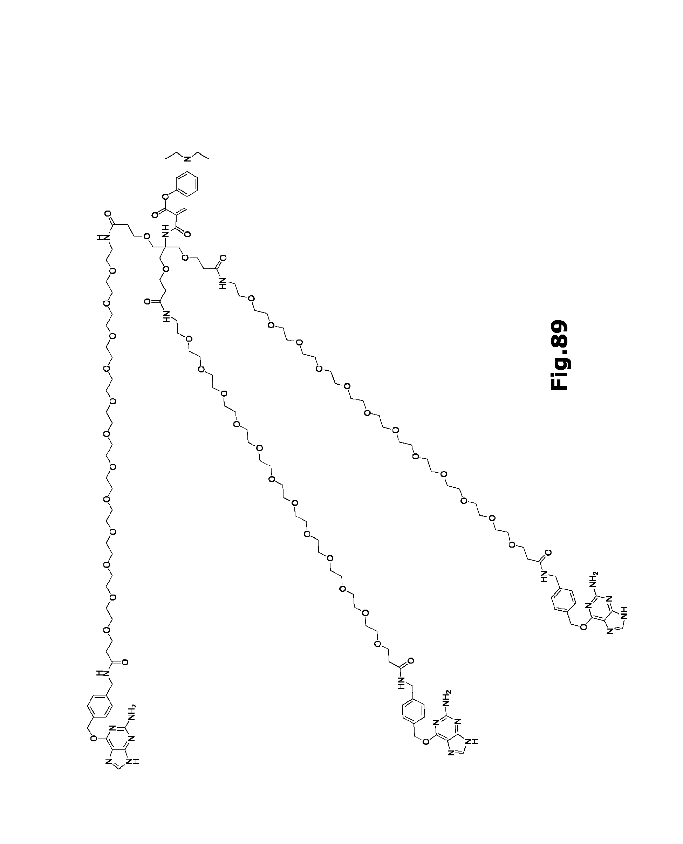

### Chemical synthesis of N-Tris-{[2-(BG-PEG12-NH)-carbonylethoxy]methyl}methyl 7-(diethylamino)coumarin-3-carboxamide (Fig. 89)

To a solution of N-Tris[(2-carboxyethoxy)methyl]methyl 7-(diethylamino)-coumarin-3-carboxamide **(****Fig. 82****)** (10 mg, 0.018 mmol) and BG-PEG12-NH₂ **(****Fig. 80****)** (54 mg, 0.062 mmol, 3.6 eq) in DMF (1 mL) are successively added DIPEA (8 µL, 0.062 mmol, 3.6 eq), HOBT (1 M in NMP, 18 µL, 0.018 mmol, 1 eq) and EDC (12 mg, 0.062 mmol, 3.6 eq) at rt. The resulting mixture is stirred overnight. The solvent is evaporated under vacuum and the compound **(****Fig. 89****)** isolated by reversed phase HPLC on a C18 column using a linear gradient of water:acetonitrile (from 95:5 to 20:80 in 20 min, 0.08% TFA). The structural ability of compound **(****Fig. 89****)** to trigger the formation of a protein trimer is confirmed by *in vitro* experiments using the fusion protein SNAP-FKBP according to Example 76. The formation of the protein trimer is visualized by SDS-PAGE followed by coomassie staining of the proteins.

### Example 69:

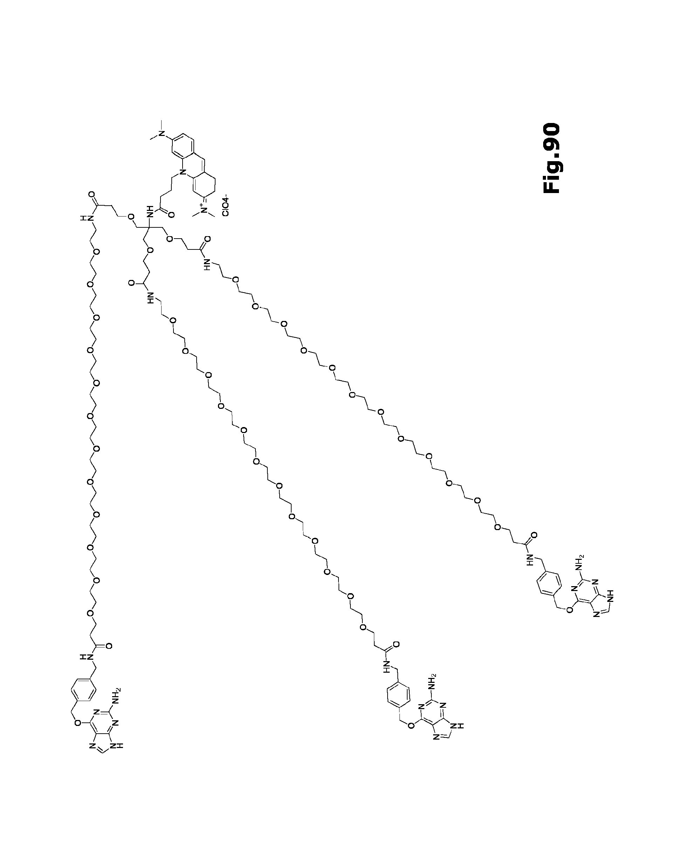

### Chemical synthesis of N-Tris-{[2-(BG-PEG12-NH)-carbonylethoxy]methyl}-methyl ATTO-495-carboxamide (Fig. 90)

To a solution of N-Tris[(2-carboxyethoxy)methyl]methyl ATTO-495-carboxamide **(****Fig. 84****)** (4 mg, 0.005 mmol) and BG-PEG12-NH₂ **(****Fig. 80****)** (15 mg, 0.0175 mmol, 3.6 eq) in DMF (1 mL) are successively added DIPEA (3 µL, 0.0175 mmol, 3.6 eq), HOBT (1 M in NMP, 5 µL, 0.005 mmol, 1 eq) and EDC (4 mg, 0.0175 mmol, 3.6 eq) at rt. The resulting mixture is stirred overnight. The solvent is evaporated under vacuum and the compound **(****Fig. 90****)** isolated by reversed phase HPLC on a C18 column using a linear gradient of water:acetonitrile (from 95:5 to 20:80 in 20 min, 0.08% TFA). The structural ability of compound **(****Fig. 90****)** to trigger the formation of a protein trimer is confirmed by *in vitro* experiments using the fusion protein SNAP-FKBP according to Example 76.

### Example 70:

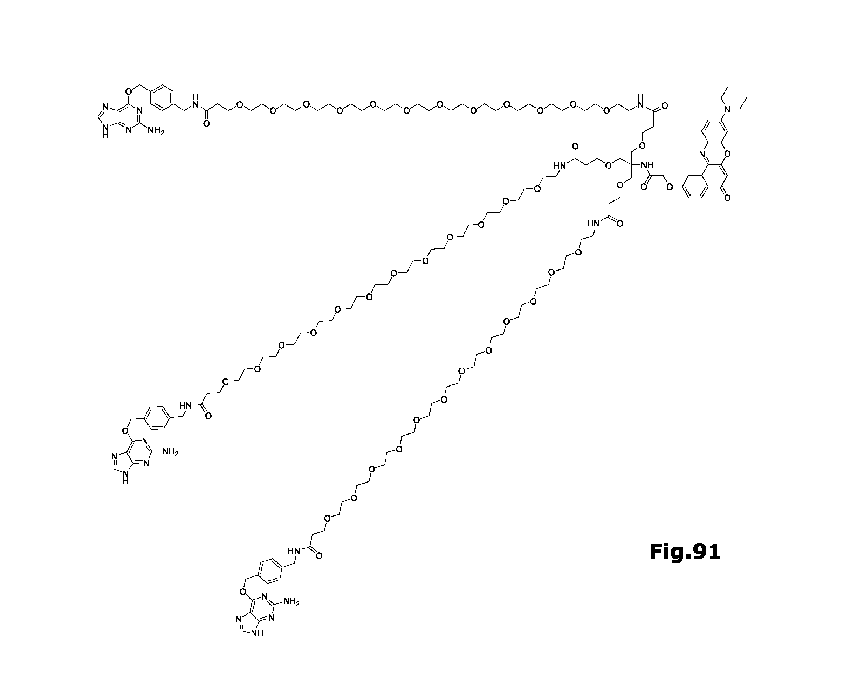

### Chemical synthesis of N-Tris-{[2-(BG-PEG12-NH)-carbonylethoxy]methyl}-methyl nile red-oxyacetamide (Fig. 91)

To a solution of N-Tris[(2-carboxyethoxy)methyl]methyl nile red-oxyacetamide **(****Fig. 86****)** (8 mg, 0.011 mmol) and BG-PEG12-NH₂ **(****Fig. 80****)** (34 mg, 0.039 mmol, 3.6 eq) in DMF (1 mL) are successively added DIPEA (7 µL, 0.039 mmol, 3.6 eq), HOBT (1 M in NMP, 11 µL, 0.01 mmol, 1 eq) and EDC (8 mg, 0.039 mmol, 3.6 eq) at rt. The resulting mixture is stirred overnight. The solvent is evaporated under vacuum and the compound **(****Fig. 91****)** isolated by reversed phase HPLC on a C18 column using a linear gradient of water:acetonitrile (from 95:5 to 20:80 in 20 min, 0.08% TFA). The structural ability of compound **(****Fig. 91****)** to trigger the formation of a protein trimer is confirmed by *in vitro* experiments using the fusion protein SNAP-FKBP according to Example 76.

### Example 71:

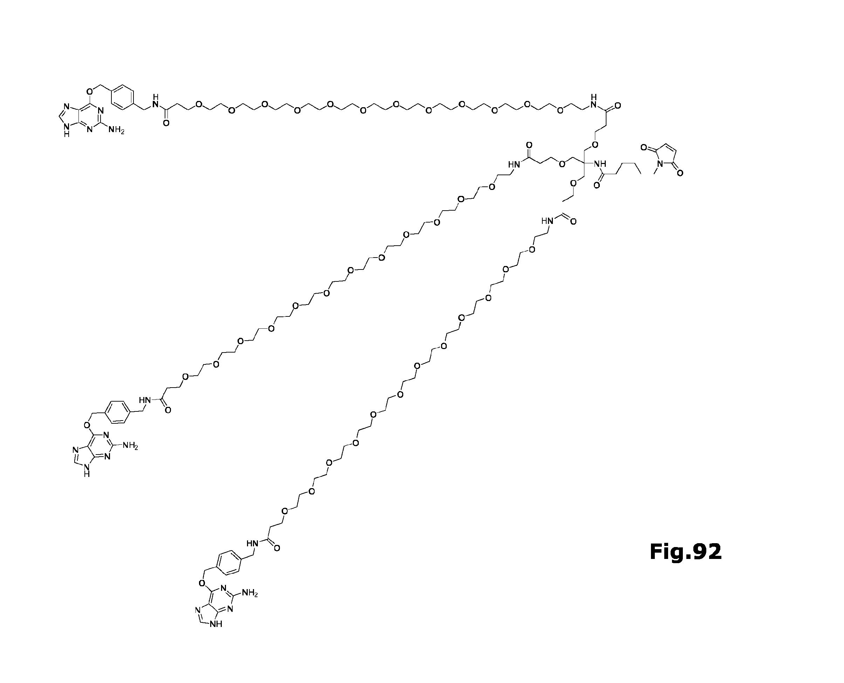

### Chemical synthesis of N-Tris-{[2-(BG-PEG12-NH)-carbonylethoxy]methyl}-methyl 5-maleimidopentanecarboxamide (Fig. 92)

To a solution of N-Tris[(2-carboxyethoxy)methyl]methyl 5-maleimidopentanecarboxamide **(****Fig. 88****)** (8 mg, 0.016 mmol) and BG-PEG12-NH₂ **(****Fig. 80****)** (50 mg, 0.057 mmol, 3.6 eq) in DMF (1 mL) are successively added DIPEA (10 µL, 0.057 mmol, 3.6 eq), HOBT (1 M in NMP, 16 µL, 0.016 mmol, 1 eq) and EDC (2 mg, 0.057 mmol, 3.6 eq) at rt. The resulting mixture is stirred overnight. The solvent is evaporated under vacuum and the compound **(****Fig. 92****)** isolated by reversed phase HPLC on a C18 column using a linear gradient of water:acetonitrile (from 95:5 to 20:80 in 20 min, 0.08% TFA). The structural ability of compound **(****Fig. 92****)** to trigger the formation of a protein trimer is confirmed by *in vitro* experiments using the fusion protein SNAP-FKBP according to Example 76.

### Example 72:

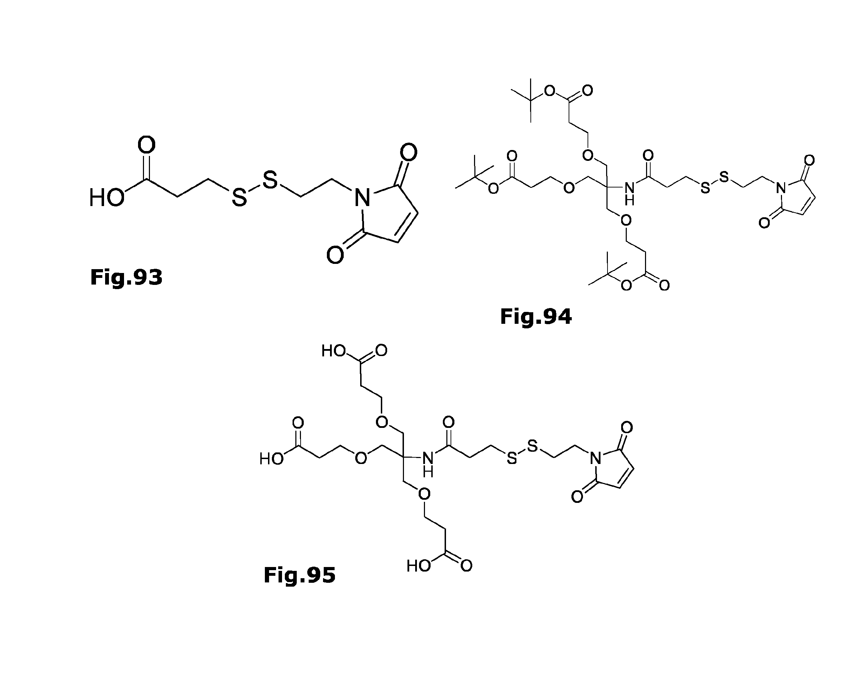

### Chemical synthesis of 3-[2-(2-maleimidoethyl)disulfanyl]propanoic acid (Fig. 93)

A solution of 3-[2-(2-aminoethyl)disulfanyl]propanoic acid (250 mg, 1.38 mmol) and maleic anhydride (272 mg, 2.76 mmol) in a mixture of acetic acid/toluene (3/1, 3 mL) is heated overnight at 120°C. Then the crude mixture is cooled down to rt, and further cooled in an ice bath to 0°C. Pentane (50 mL) is added, and a precipitate is formed. Diethyl ether is added to this precipitate, and the white solid formed is removed. The ether solution is concentrated under vacuum to yield the product **(****Fig. 93****).** No further purification is required. ¹H NMR ((CD₃)₂SO, 400 MHz): 7.4 (s, 1H), 6.7 (s, 2H), 3.7 (m, 2H), 2.9 (m, 4H), 2.6 (m, 2H).

### Example 73:

### Chemical synthesis of N-Tris{[2-(tert-butoxycarbonyl)ethoxy]methyl}methyl 3-[2-(2-maleimidoethyl)disulfanyl]propanoylamide (Fig. 94)

To a solution of 3-[2-(2-maleimidoethyl)disulfanyl]propanoic acid **(****Fig. 93****)** (188 mg, 0.72 mmol) in DMF (2 mL) is added PYBOP (376 mg, 0.72 mmol) at rt. The solution is stirred at room temperature for 20 min. Then tris{[2-tert-butoxycarbonyl)ethoxy]methyl}methylamine **(****Fig. 1****)** (364 mg, 0.72 mmol) and DIPEA (119 µL, 0.72 mmol) are added and the solution is heated at 50°C for 5 min. The solution is stirred at room temperature overnight. Then the solvent is removed under reduced pressure. Flash chromatography (cyclohexane/ethyl acetate, 2/1) gives the desired compound **(****Fig. 94****).** ¹H NMR ((CD₃)₂SO, 400 MHz): 6.6 (s, 2H), 3.8 (m, 2H), 3.6 (m, 6H), 3.55 (m, 6H), 2.8 (m, 4H), 2.5 (m, 2H), 2.35 (m, 6H), 1.4 (m, 27H).

### Example 74:

### Chemical synthesis of N-Tris[(2-carboxyethoxy)methyl]methyl 3-[2-(2-male-imidoethyl)disulfanyllpropanoylamide (Fig. 95)

Compound **(****Fig. 94****)** (112 mg, 0.15 mmol) is stirred in 1.5 mL of 96% formic acid for 18 h. Then formic acid is removed at reduced pressure at 50°C to produce a colorless oil in quantitative yield. The compound is directly used for the next step. ¹H NMR ((CD₃)₂SO, 400 MHz): 7.0 (s, 2H), 3.7 (m, 2H), 3.55 (m, 12H), 2.75 (m, 4H), 2.45 (m, 6H).

### Example 75:

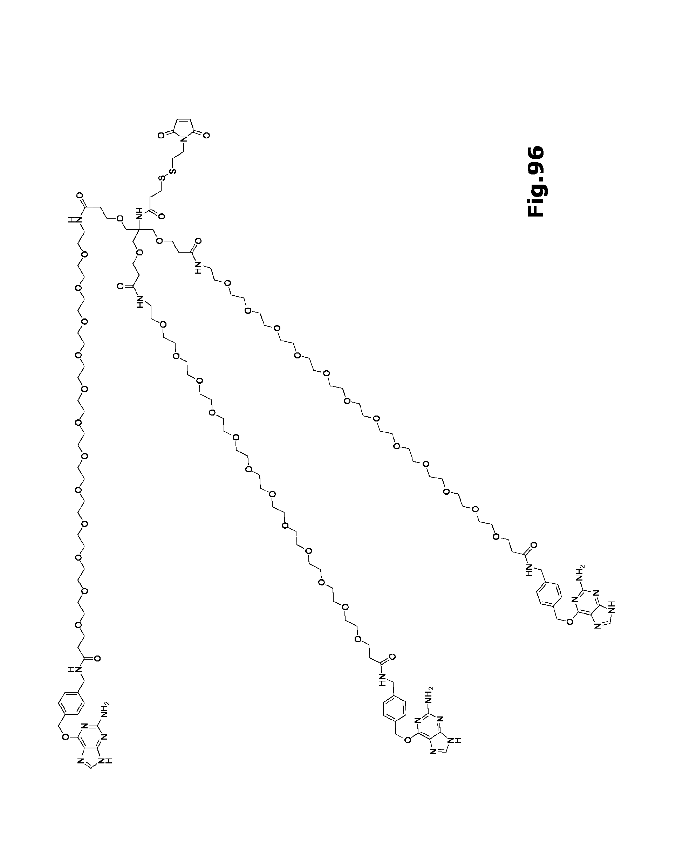

### Chemical synthesis of N-Tris-{[2-(BG-PEG12-NH)-carbonylethoxy]methyl}-methyl-3-[2-(2-maleimidoethyl)disulfanyl]propanoylamide (Fig. 96)

To a solution of compound **(****Fig. 95****)** (10 mg, 0.017 mmol) and BG-PEG12-NH₂ **(****Fig. 80****)** (120 mg, 0.138 mmol, 8 eq) in DMF (1 mL) are successively added DIPEA (17 µL, 0.069 mmol, 4 eq), HOBT (1 M in NMP, 17 µL, 0.017 mmol, 1 eq) and EDC (14 mg, 0.069 mmol, 4 eq) at rt. The resulting mixture is stirred overnight. The solvent is evaporated under vacuum and the compound **(****Fig. 96****)** isolated by reversed phase HPLC on a C18 column using a linear gradient of water:acetonitrile (from 95:5 to 20:80 in 20 min, 0.08% TFA). The structural ability of compound **(****Fig. 96****)** to trigger the formation of a protein trimer is confirmed by *in vitro* experiments using the fusion protein SNAP-FKBP according to Example 76.

### Example 76:

### Determination of the reactivity of compound (Fig. 89), (Fig. 90), (Fig. 91), (Fig. 92) and (Fig. 96) with FKBP-AGT fusion protein

1 µL of a 591 µM solution of FKBP protein fused to a variant of AGT available from Covalys as SNAP26™ and 1 µL of a 100 µM solution of compound **(****Fig. 89****), (****Fig. 90****), (****Fig. 91****), (****Fig. 92****)** or **(****Fig. 96****)_are** added to 8 µL of a solution of 50 mM Tris-HCl pH 7.5; 100 mM NaCl; 0.1% Tween20™; 1 mM DTT. Following a 4 h incubation at rt, 15 µL of a solution of 100 mM Tris-HCl pH 6.8; 2% SDS; 35% glycerol; 10 mM EDTA; 20 mM DTT is added. Then the mixture is boiled for 5 min at 95°C. After cooling to rt, 25 µL of this solution is loaded on a 4-20% linear gradient SDS-PAGE gel. After electrophoresis, the proteins are coomassie stained in gel to visualize protein trimer.

### II Assembly and expression of components A and B

### Construction of the expression vectors:

### Eukaryotic expression vectors

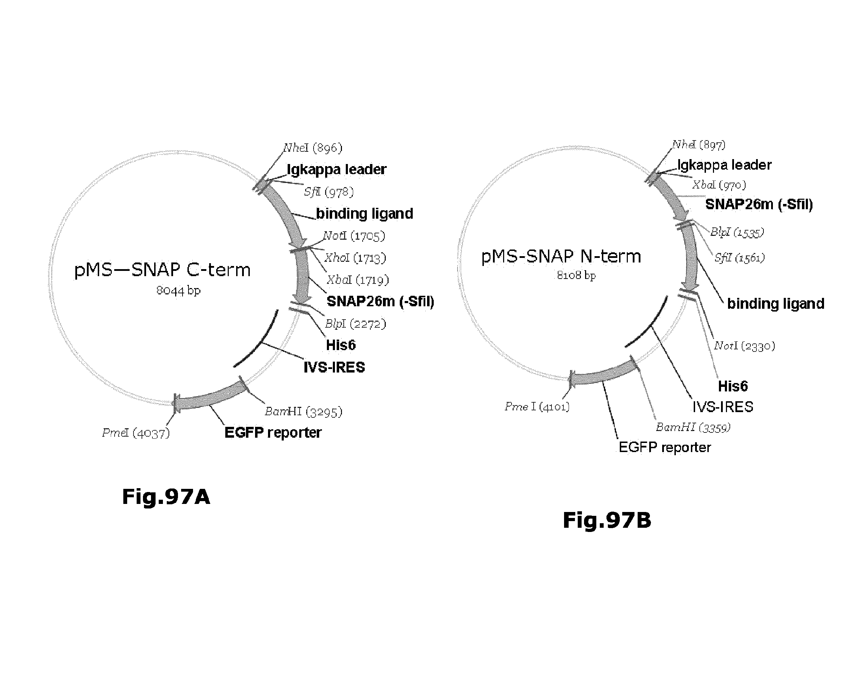

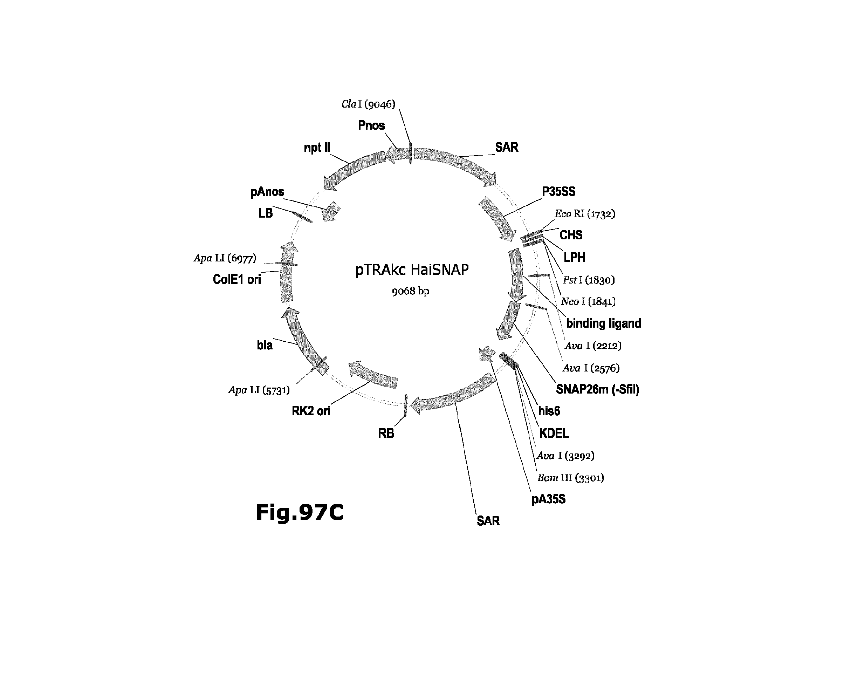

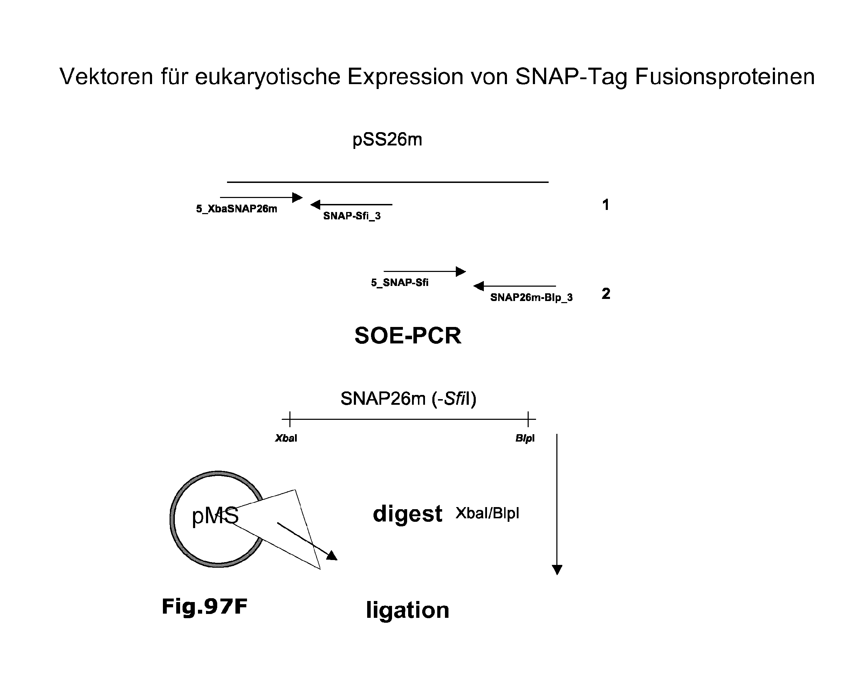

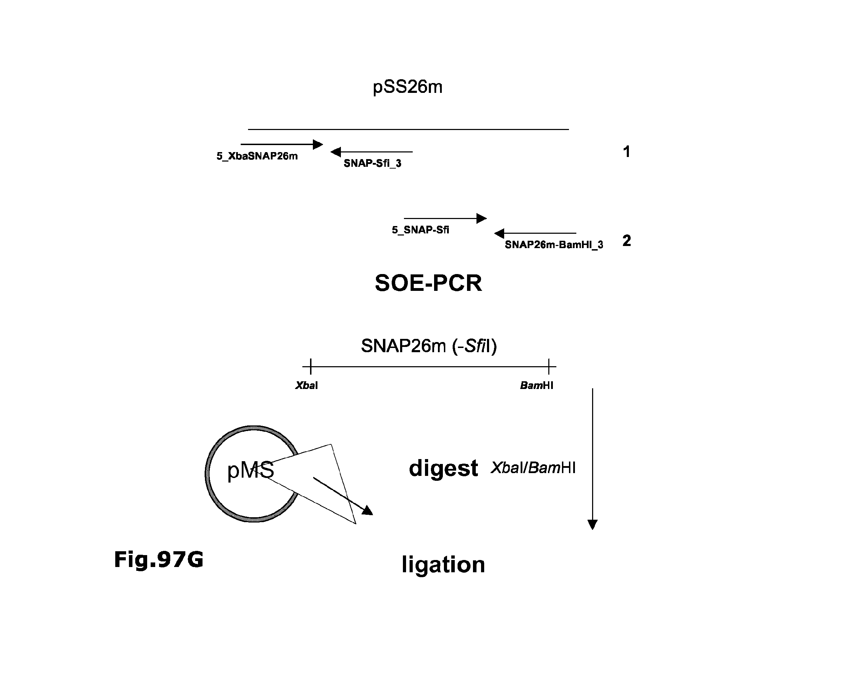

For the construction of a vector encoding a recombinant complex AB, a modified pSecTac based mammalian expression vector (pMS, Stöcker et al., 2003) was provided with the SNAP 26m gene by PCR cloning from the storage vector pSS26m (COVALYS AG). Two versions are available allowing to link component A to the N-terminus of component B or to the C-terminus of component B which are depicted in Figures **(97A****+B).** In a further version of these vectors the internal *Sfi*I endonuclease restriction site of the SNAP26m gene (Covalys) was removed to allow rapid exchange of scFv fusion partners by common *Sfi*I/NotI cloning **(****Fig. 97F****+G).** The SfiI depleted version of the SNAP-Tag is further on named as mSNAP.

The expression cassette of the vector comprises of the following key features: the human cytomegali virus promoter sequence (CMV), a bovine growth hormone polyadenylation signal (BGH pA) and an internal IVS ribosome entry site (IRES). The SNAP-tag fusion protein is secreted through a Igkappa leader peptide whereas the reporter EGFP gene in 3'of the IRES site is lacking a secretion signal, therefore accumulating in cytoplasm.

### Plant expression vectors

A plant expression vector system designed for transient and stable expression of SNAP-tag fusion proteins in plants is shown in figure **(97C****).** The vector comprises the following features:

KDEL: plant ER retention signal; LPH: codon optimized murine signal peptide; Bla: ampicillin resistance (*E. coli*), cabenicillin resistance *(A. tumefaciens*); nptII: Kanamycin resistance plant; SAR: scaffold attachment region of tobacco RB7 gene; P35SS: transcription start; CHS: 5'UTR from chalcon synthase; pA35S: polyadenylation signal from CaMV; RK3 ori: ori for *A. tumefaciens;* ColE1 ori: ori for *E. coli;* LB/RB: elft/right border; pAnos: nopaline synthase polyadenylation signal; Pnos: nopaline synthase gene promoter.

### Procaryotic expression vectors

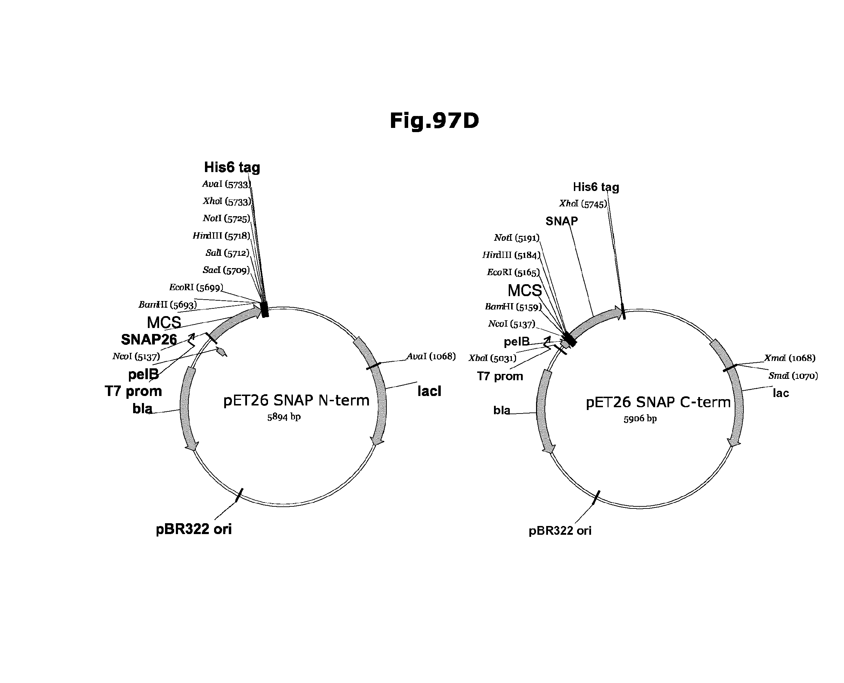

Procaryotic expression plasmids exemplified here are based on the pET26b™ system (Novagen) and designed for periplasmatic expression of C/N-terminal SNAP-tag fusion proteins in *E*. *coli* **(****Fig. 97D****).** The SNAP-tag version (26b) is codon optimized for and was PCR amplified from the storage vector pSET7-26b from Covalys. The expression is regulated through the T7 promoter; together with a host-encoded T7 polymerase the regulation of expression is extremely tight. The kanamycin resistance gene allows selection of transformed bacteria. The pelB leader is directing the recombinant protein into the periplasmic space.

### Yeast expression vectors

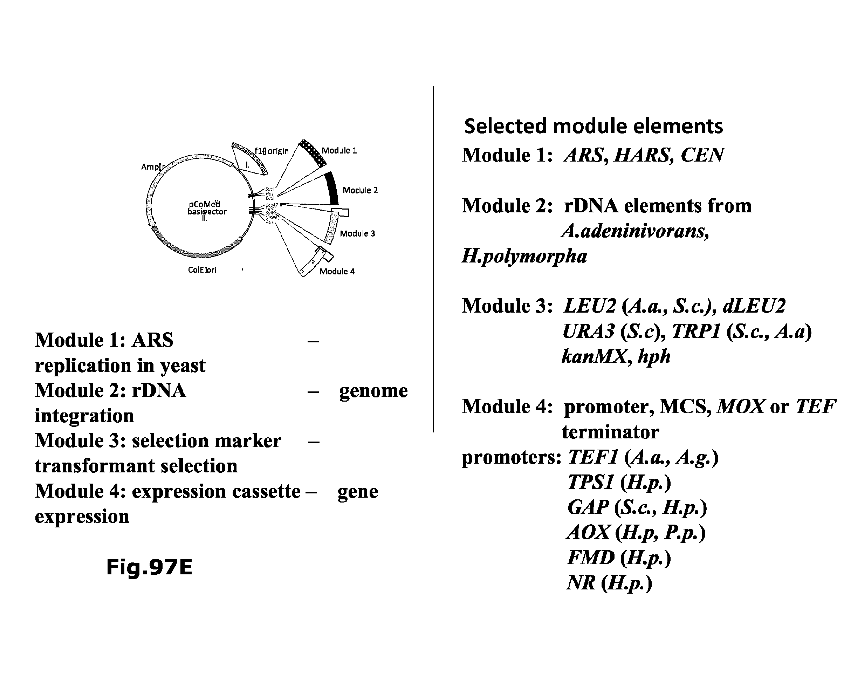

Yeast expression plasmid based on the CoMedTM system provided by Pharmedartis (Aachen, Germany) **(****Fig. 97E****).** The vector backbone is a derivative of a standard *E*. *coli* vector combining a ColE1 ori and an ampicillin resistance (bla) sequence. A variant I contains an f1(-)origin, a variant II is without that sequence. A multiple cloning site (MCS) has been engineered for the uptake of various modules. For insertion ARS/CEN modules (module 1) are flanked by SacII/BcuI restriction sites, rDNA segments (module 2) by BcuI/Eco47III sites, selection marker modules (module 3) by Eco47III/SalI sites and expression cassettes (module 4) by SalI/ApaI sites. In variant II additional SphI and BsiWI cloning sites are present.

The Plasmid is designed to work with a variety of yeast strains:

### Yeast strains (selection)

| **Species** | **auxotrophies** |
|---|---|
| *Arxula adeninivorans* (LS3) | wild type; *leu2* |
| *Arxula adeninivorans* (CBS7350) | wild type; *leu2* |
| *Arxula adeninivorans* (CBS1738) | wild type; *leu2* |
| *Hansenula polymorpha* (CBS4732) | wild type; *ura3; leu2 ura3; arg1 leu2 ura; ade1 leu2 ura3* |
| *Kluyveromyces lactis* | *met- ura3* |
| *Pichia pastoris* | wild type; *ura3; ura3 his3* |
| *Saccharomyces cerevisiae* | wild type; *ura3; leu2 ura3 trp1 lys2* |
| *Yarrowia lipolytica E150* | wild type; *ura3; leu2; ura3 leu2* |

The key features are subsegmented into four modules, whereas the modules of a concrete vector construct may contain one or more of the following features:
Module 1consists of ARS/CEN sequences for replication in yeasts:
   HARS1 (H. polymorpha-derived autonomously replication sequence)
   ARS (S. cerevisiae)
   CEN (S. cerevisiae)
Module 2 consists of rDNA targeting sequences for yeast genomic integration
   NTS2-ETS-18SrDNA-ITS1 (H. polymorpha, Arxula adeninivorans)
Module 3 consists of selection markers for transformant selection
   1. dominant selection markers:
      TEF promoter (A. gossypii; A. adeninivorans) - hph (E.coli) - TEF terminator (hygromycin resistance)
      TEF promoter (A. gossypii; A. adeninivorans) - kanMX (E. coli) - TEF terminator (gentamycin resistance)
   2. complementation selection markers:
      URA3 (S. cerevisiae)
      LEU2 (S. cerevisiae, A. adeninivorans)
      dLEU2 (A. adeninivorans) (deficient promoter)
      TRP1 (S. cerevisiae)
Module 4 comprises the SNAP-tag fusion protein expression cassette consisting of promoter - cloning site - terminator whereas the terminator sequence is mostly from MOX but also from TEF and PHO5.

### Construction of open reading frames for the component A fused to B

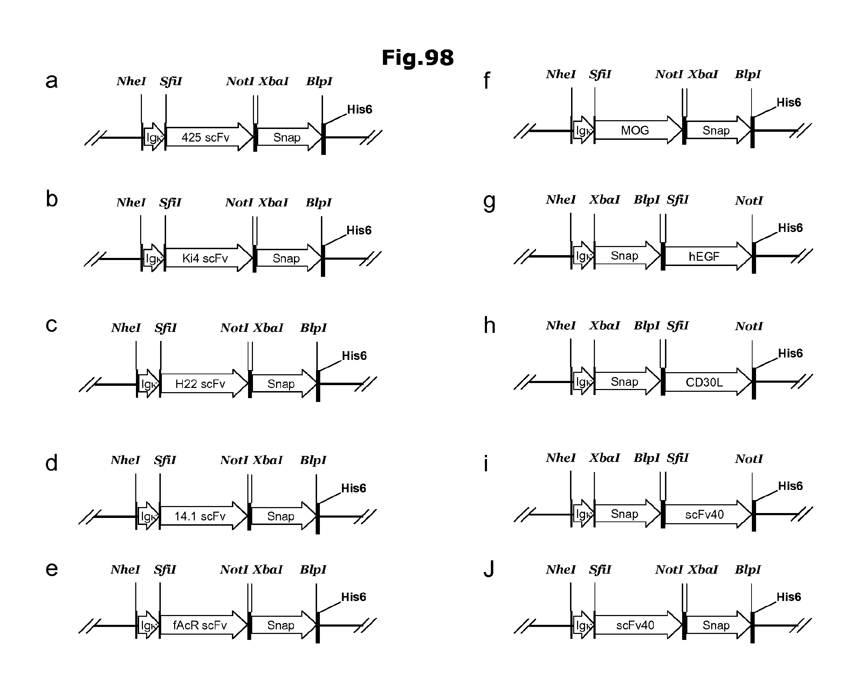

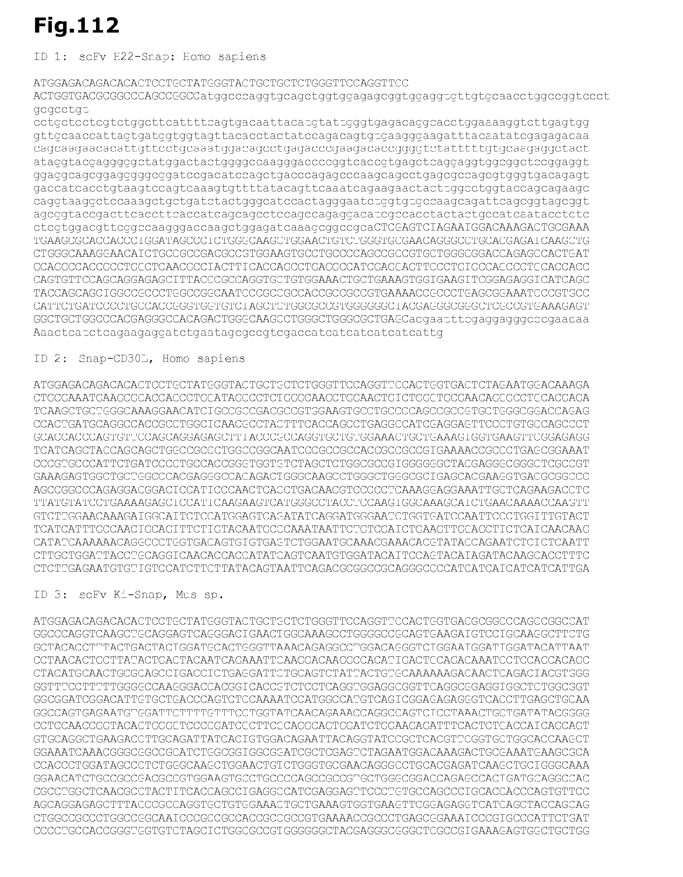

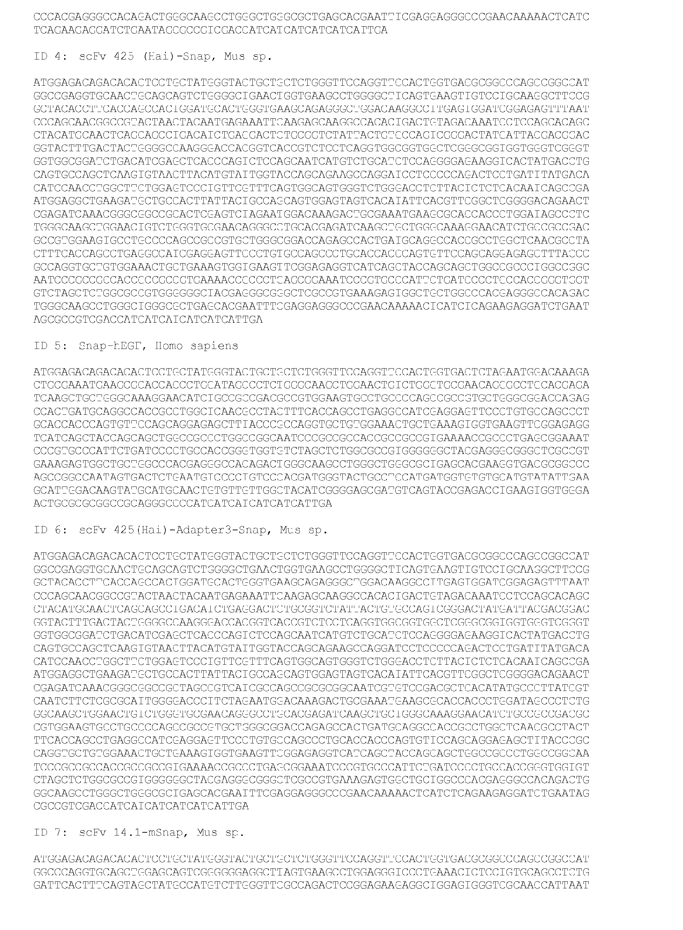

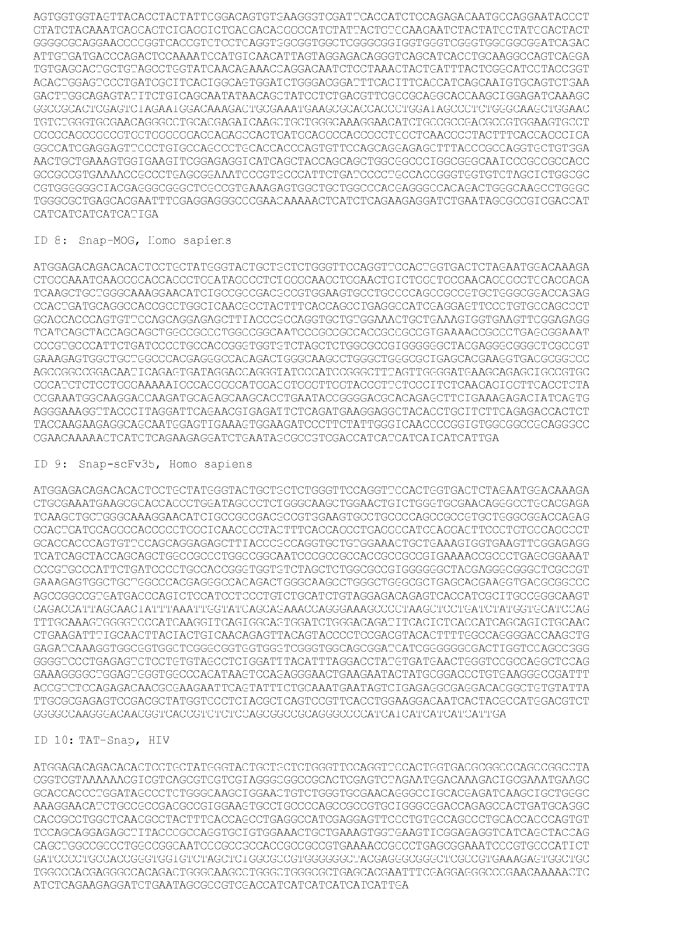

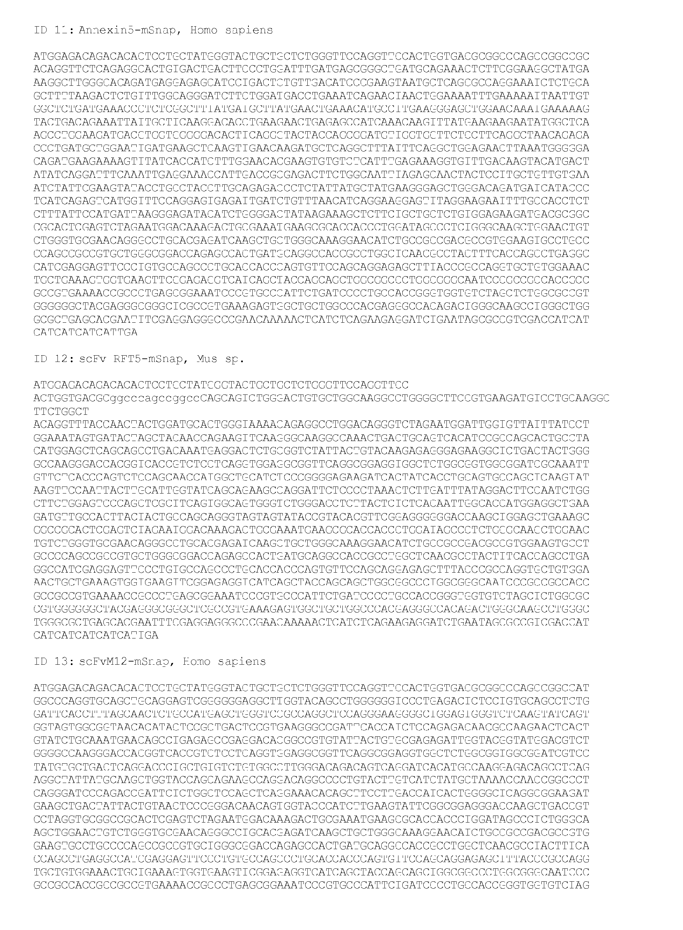

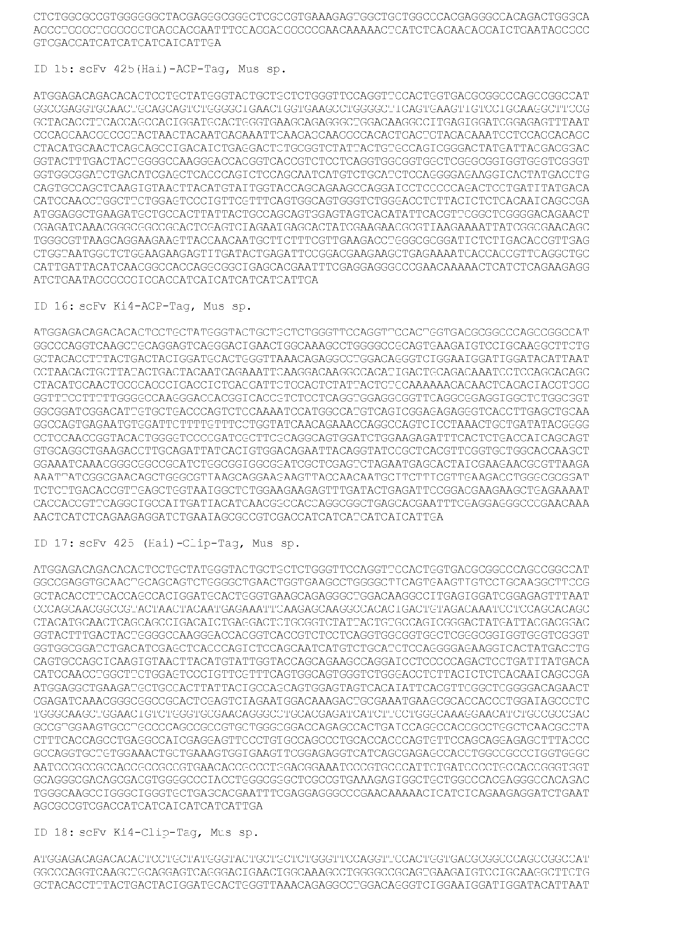

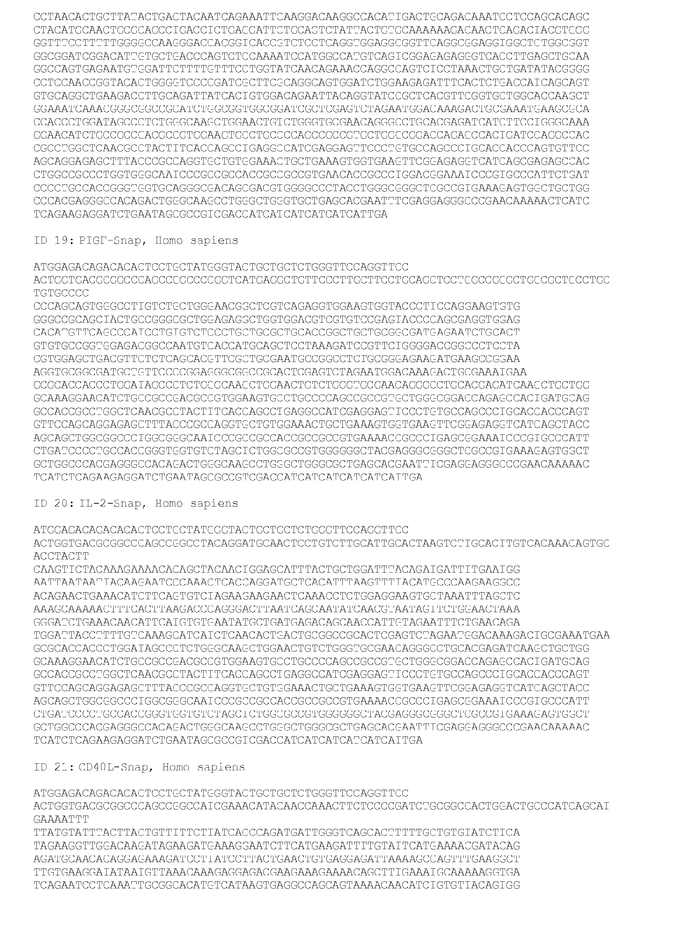

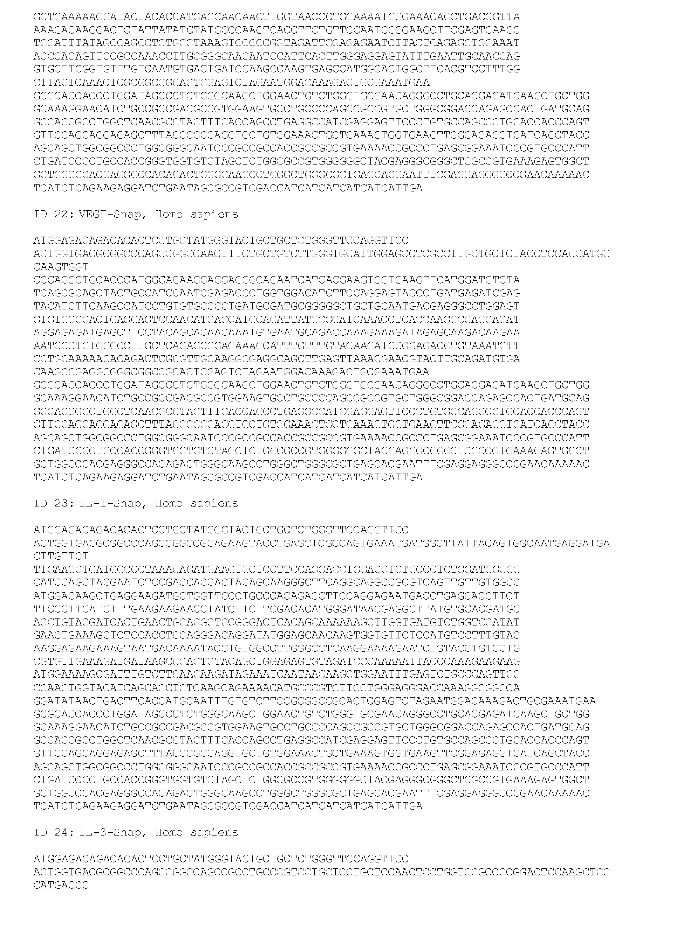

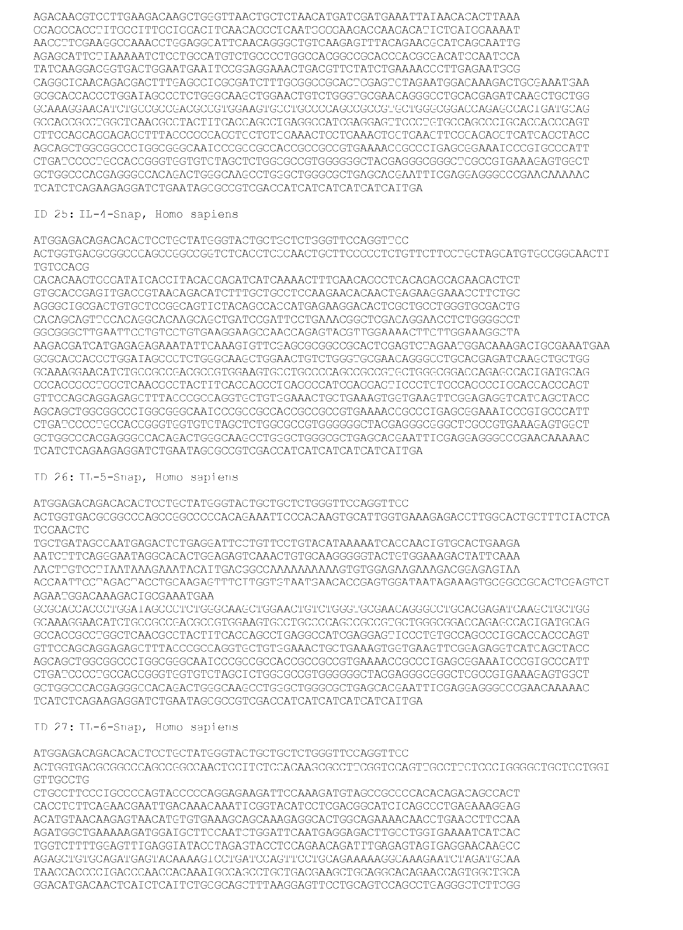

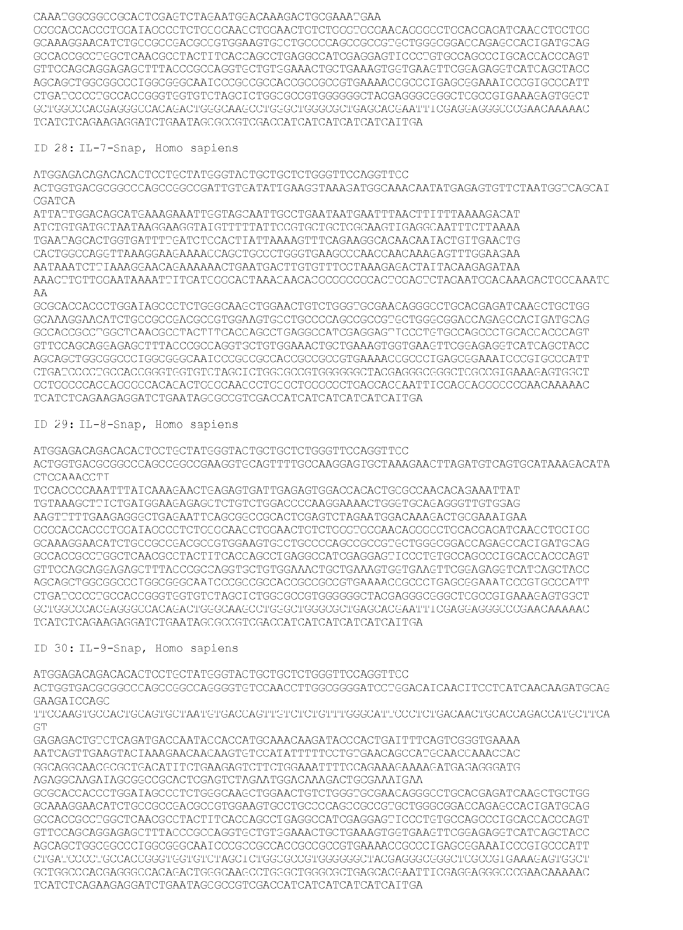

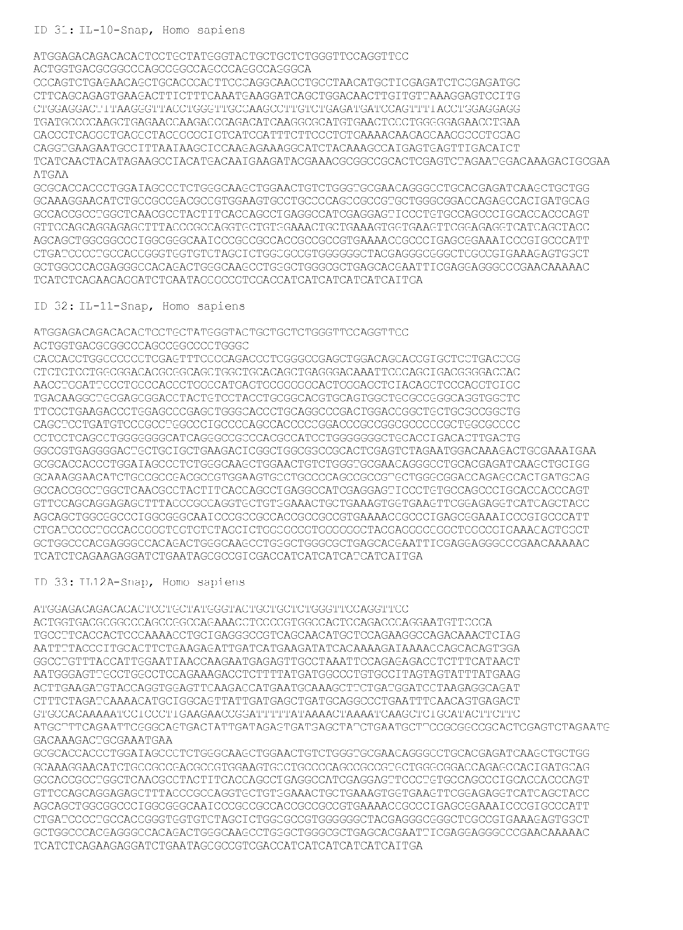

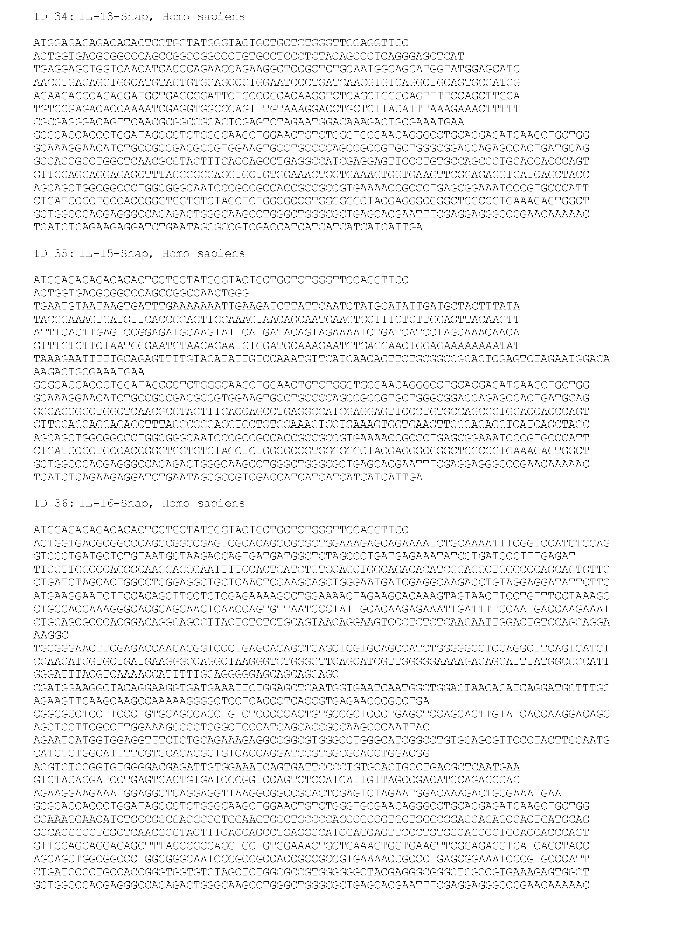

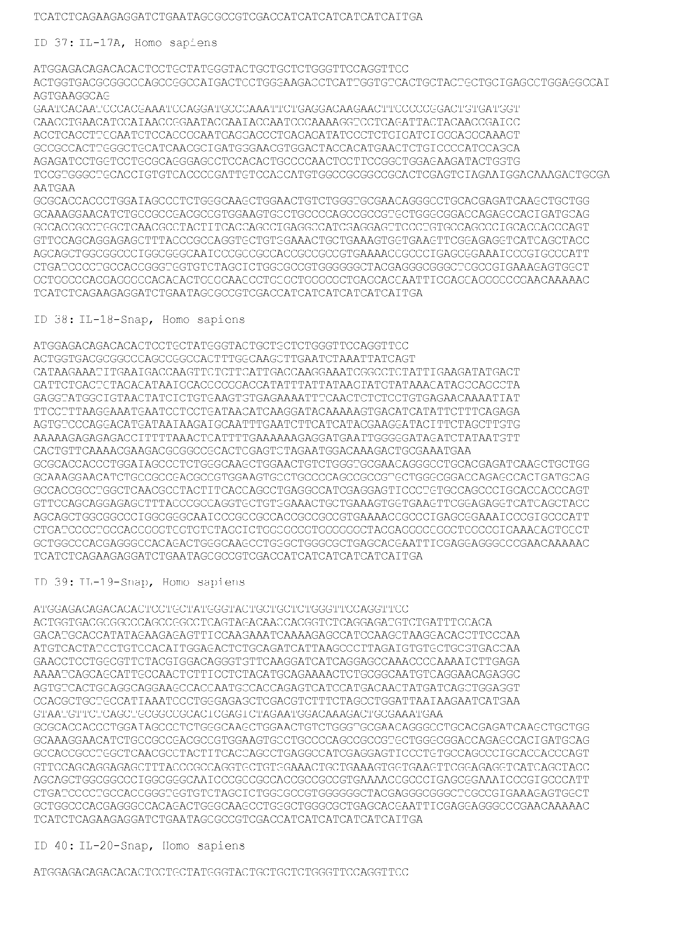

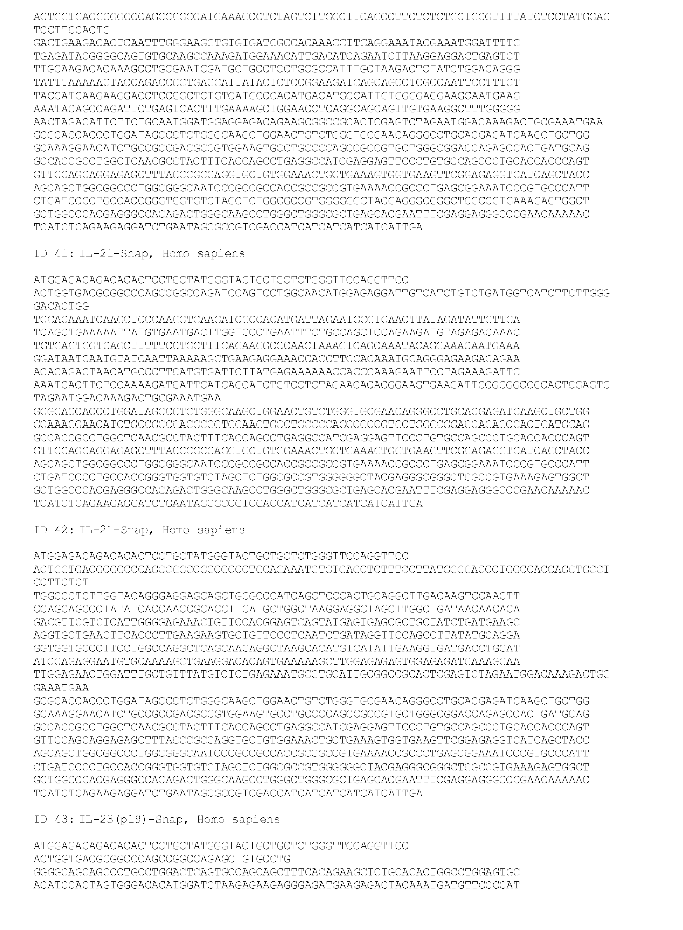

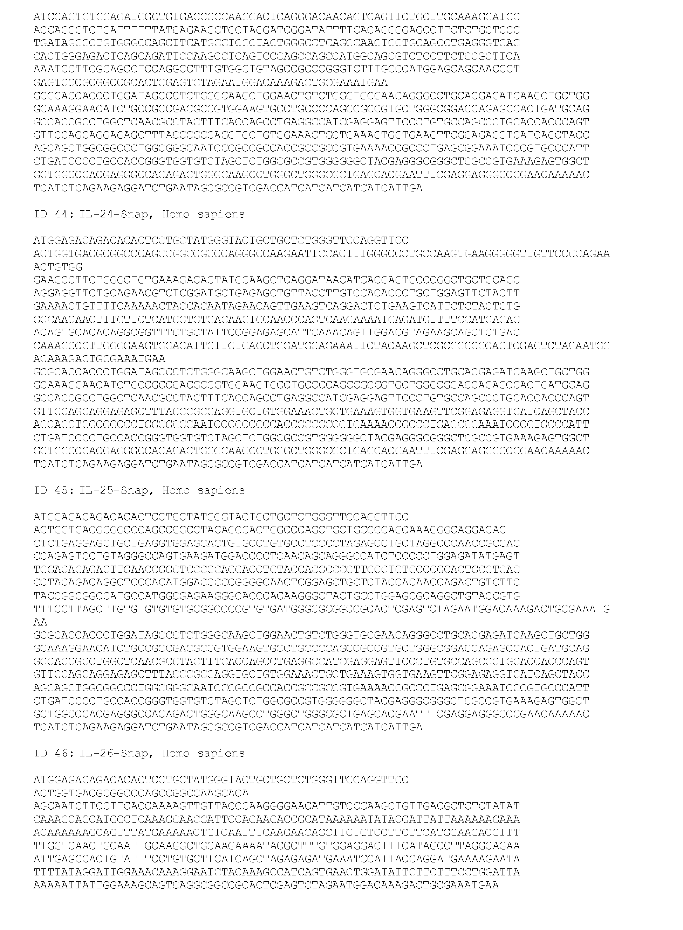

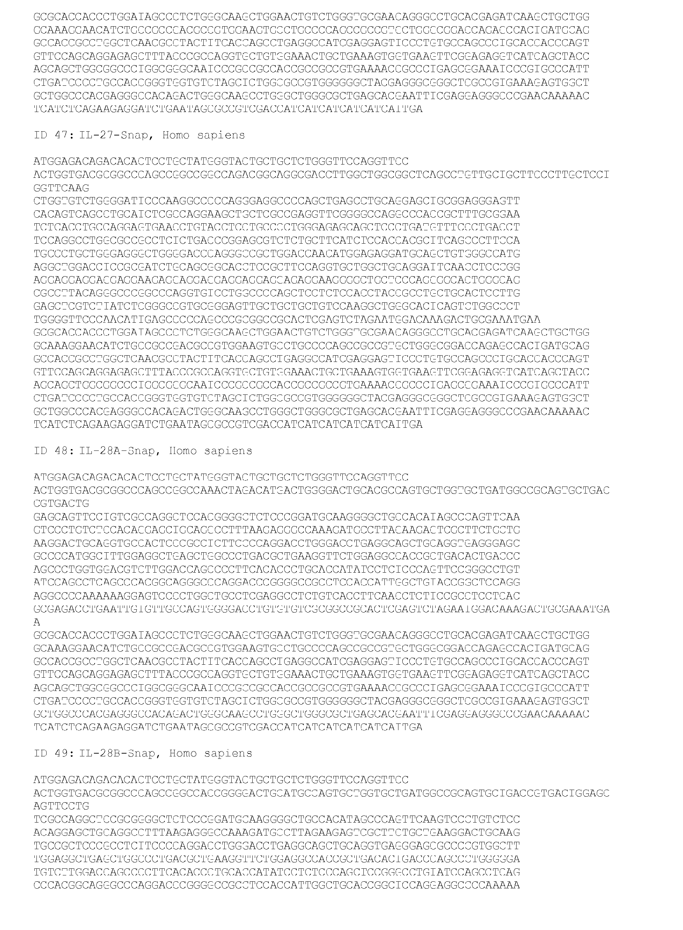

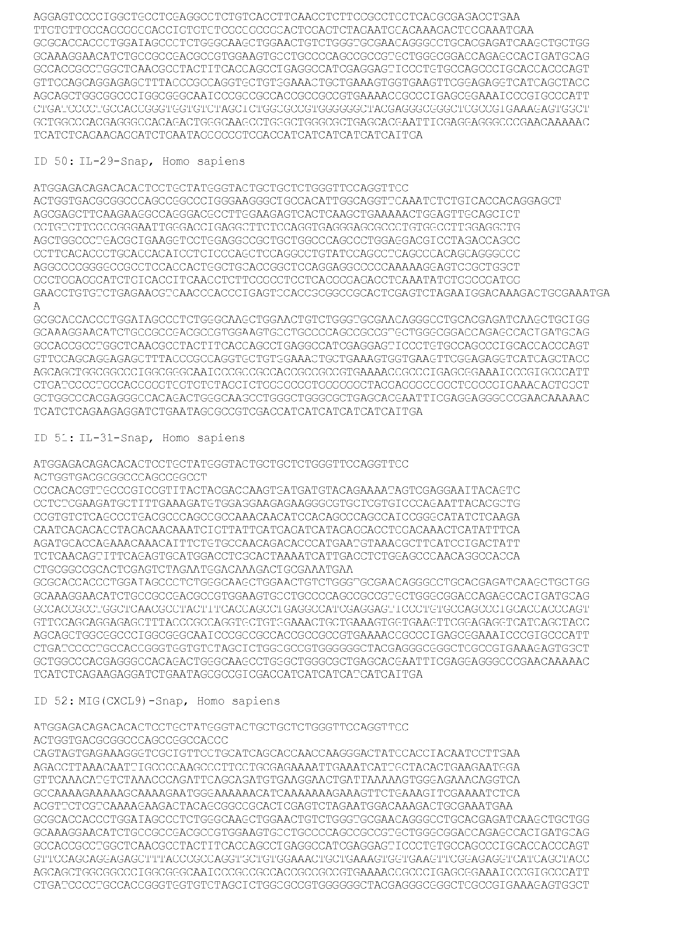

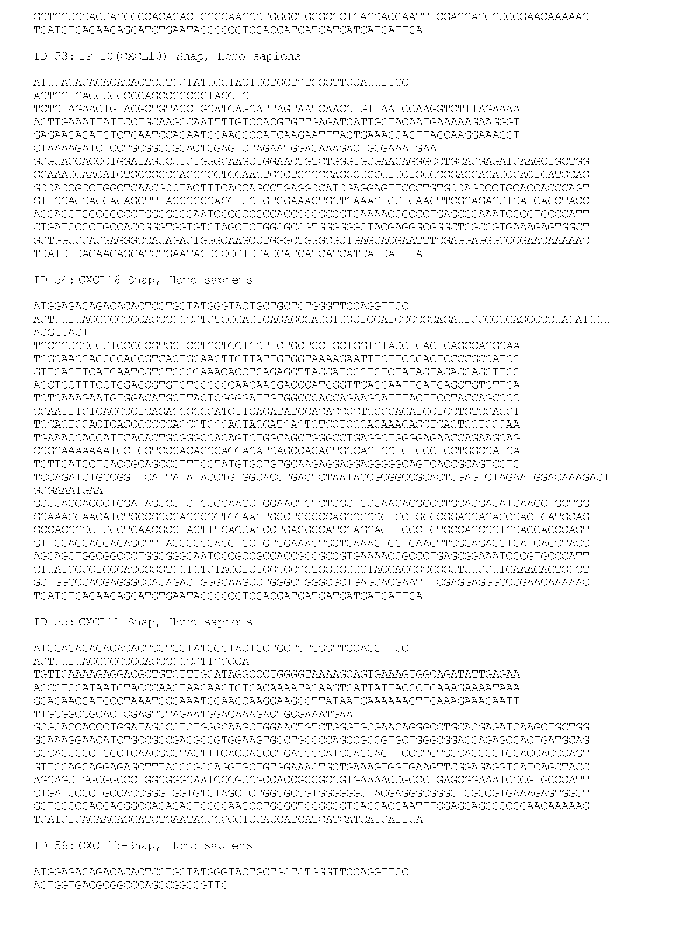

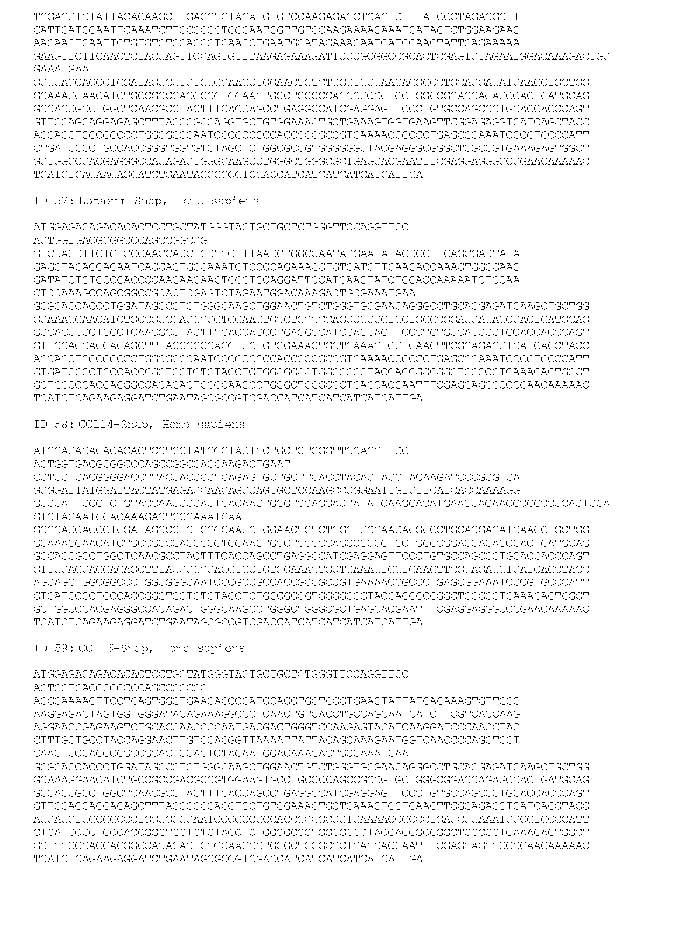

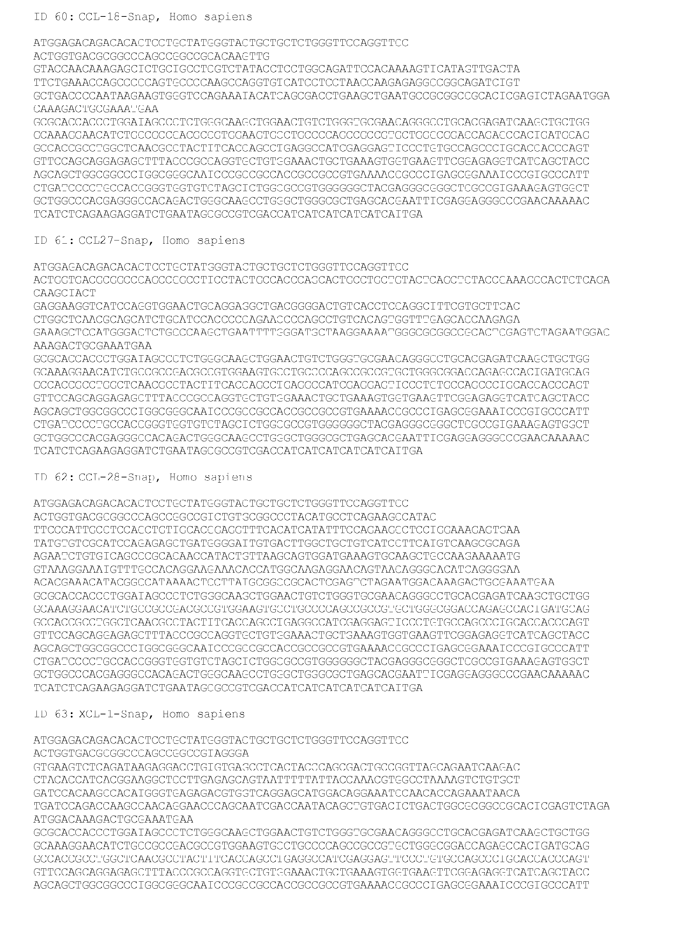

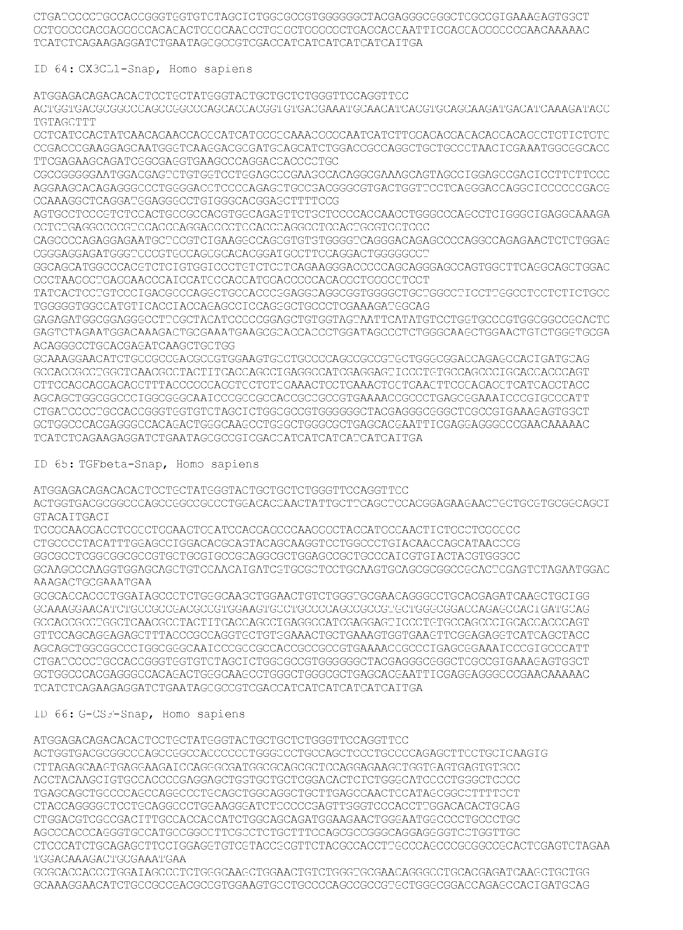

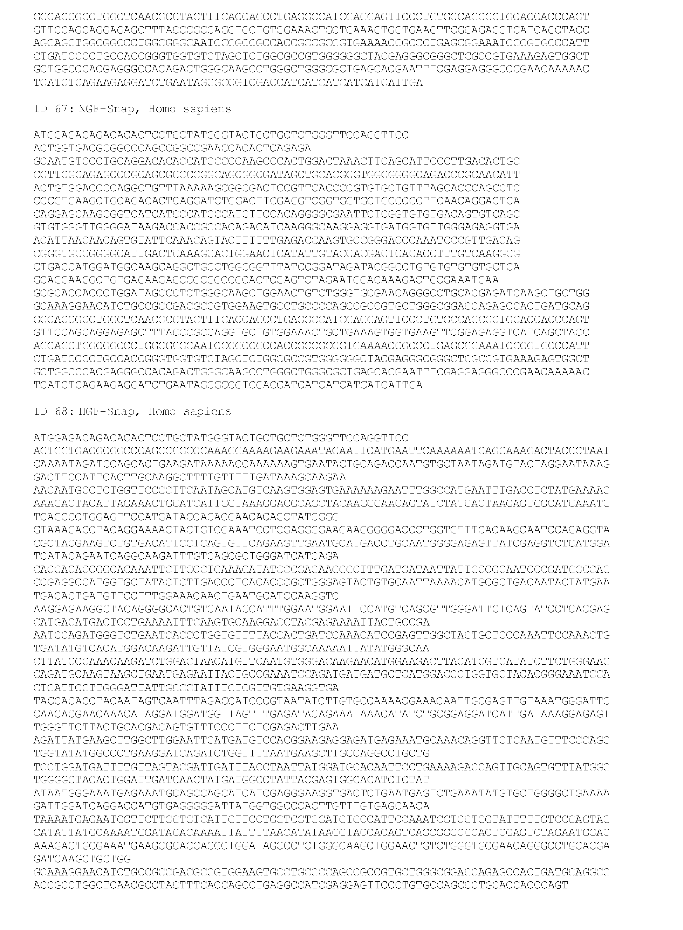

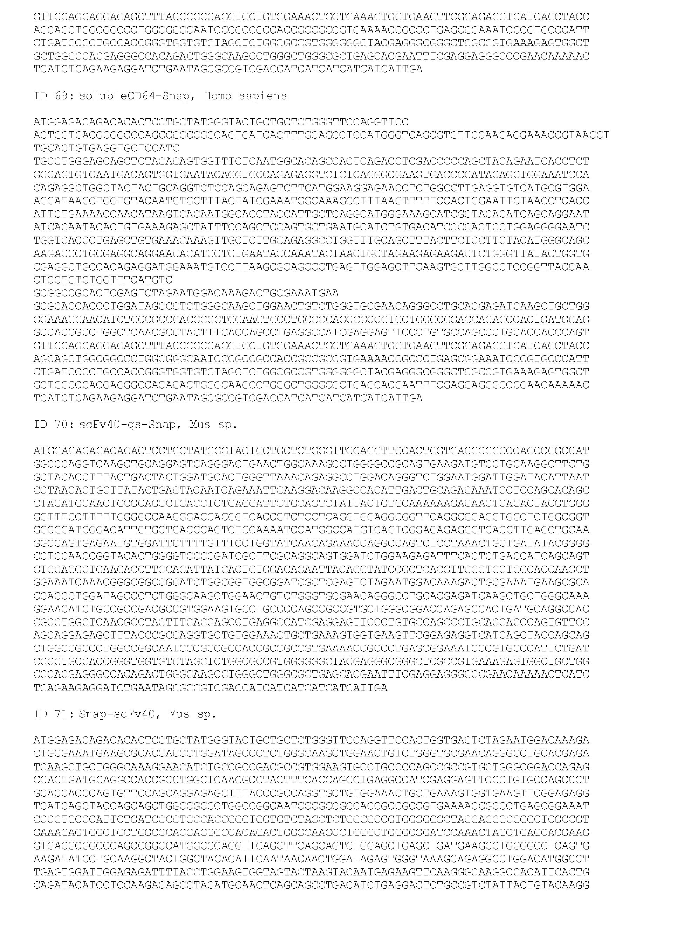

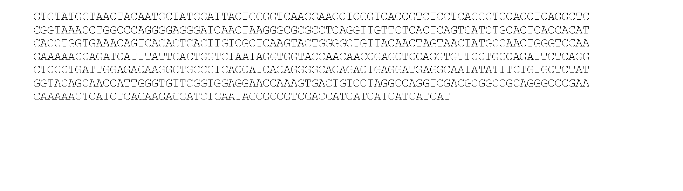

Different components A like antibody fragments and natural ligands for receptors including soluble ligands, receptors, chemokines, growth factors or interleukins or fragments therof were cloned in an open reading frame (ORF) together with the SNAP-tag. The exemplified ORFs are listed by their sequences (expression was exemplified after cloning into the mammalian pMS vector constructs) **(****Fig. 98** **and** **112****).** See list of sequences ID 1-71 in Fig. 112.

### Mammalian expression of SNAP-tag fusion proteins

After *TransFast-*mediated (Promega, Mannhein, Germany) transformation into 293T-cells, the recombinant SNAP-tag fusion proteins were expressed as described by Stöcker M. *et al.,* 2003. Briefly, one µg plasmid-DNA (like Ki4-SNAP (anti-CD30 scFv); SNAP-EGF (EGFR ligand); Hai-SNAP (anti-EGFR scFv 425); H22-SNAP (anti-CD64 scFv) or SNAP-CD30L (CD30 ligand) and 3µl TransFast have been used according to the manufactures protocol for 12 well cell culture plates. Transfection efficiency was between 75 and 95% determined by counting green fluorescent cells. 3 days after initial transfection, cell culture supernatants were analyzed for recombinant protein. Subsequently, transfected cells were transferred into medium-sized cell culture flasks (Nunc; 85m²) and grown in RPMI complex medium supplemented with 100 µg/ml Zeocin. One to two weeks productively transfected clones were green fluorescing and hence could be detected by fluorescence microscopy. Transfected cell populations were established by subcultivation of these clones.

### Plant expression of SNAP-tag fusion proteins

For transient expression of SNAP-tag fusion proteins in plant (e.g. tobacco-*Nicotiana benthamiana),* an *Agrobacterium tumefaciens (A. tumefaciens)* mediated transformation method is chosen.

Therefore *A. tumefaciens* (e.g. strain GV3101 : : pMP90RK) is made electrocompetent (Shen & Forde, 1989) and 100 µl of the competent cells are mixed with 50-200 ng of a binary vector (pTRAkc based) containing the expression cassette for the SNAP-Tag fusion protein in a 0.1 cm electrogap cuvette (BioRad). The cells are transformed by a electric pulse using a GenePulser (BioRad) set at 1.8 kV, 25 mF and 200 V. Electroporated cells are incubated in 1 ml Luria-Bertani (LB) broth for 2 h prior to plating on LB medium containing 50 mg carbenicillin ml⁻¹, 50 mg rifampicin ml⁻¹ and 30 mg kanamycin ml⁻¹_{.}

For *A. tumefaciens* mediated transient expression of SNAP-tag fusion proteins in tobacco plants *A. tumefaciens* cultures containing pTRAkc vector bearing the SNAP-tag fusion protein expression cassette clones are supplemented with 50 mg carbenicillin ml⁻¹ and 50 mg rifampicin ml⁻¹. Cultures are grown with shaking at 27 °C to exponential phase (OD600 approx. 0.8) in LB broth containing the appropriate antibiotics. Cells are collected by centrifugation at 4000 g, resuspended in induction medium (LB broth at pH 5.6 containing 10 mM MES, 20 mM acetosyringone and 2 mM MgSO₄) with the appropriate antibiotics, and grown as above. The cells are collected by centrifugation at 4000 g and resuspended in infiltration medium (10 mM MgCl2, 10 mM MES, 2% sucrose and 150 mg acetosyringone ml⁻¹, pH 5.6). The Agrobacterium suspensions are diluted in infiltration medium to an OD600 of 1.0 and are stored at 22 °C for 2-3 h.

There are two infiltration methods: direct injection and vacuum infiltration. For injection, the *Agrobacterium* suspensions are diluted and combined in infiltration medium, both to a final OD600 of 0.25. When Agrobacterium (pTRAkc) was co-infiltrated with the above suspension, it was used at a final OD600 of 0.0125. Leaves from 2-4-week-old *Nicotiana benthamiana* plants are infiltrated by injecting the bacterial suspension into the abaxial air spaces from the underside of the leaf. E.g. six leaves are agroinfiltrated with each bacterial mixture (three plants, two leaves per plant). The plants are grown for 5-6 days under conditions of 16 h light, 8 h dark, 22 °C.

For vacuum infiltration, *Agrobacterium* cultures are grown overnight in induction medium. The cells from are resuspended in 1-8 I infiltration medium to a final OD600 of 0.25 per culture. Whole *Nicotiana tabacum* L. 'Petite Havana' SR1 plants with roots removed are submerged into the bacterial suspension and subjected to a vacuum of 290 kPa for 5-10 min, with occasional agitation to release trapped air bubbles. The vacuum is released rapidly (approx. 10 kPa s⁻¹). The plant stalks are placed in water-saturated floral foam. The plants are grown for 3 days under conditions of 16 h light, 8 h dark, 22 °C.

For recombinant protein extraction *N. tabacum* leaf discs (cut by using the cap of a microfuge tube) are harvested from agroinfiltrated leaves and ground in 250 ml high-salt phosphate buffer (0.5 M NaCl) per disc. The extract is centrifuged at 13 000 r.p.m. for 5 min, supernatant is collected and the centrifugation is repeated.

For Western blot analysis, plant extracts were incubated at 95 °C for 2 min in loading buffer (Sambrook et al., 1989), separated by SDS-PAGE (10% gel) and then transferred onto a nitrocellulose membrane by semi-dry electroblotting. Recombinant SNAP-tag fusion protein protein is detected with anti His-tag mAb horsereadiish peroxdase coupled (1 : 5000). The detection reaction is done with DAB ragent (SIGMAFAST, Sigma).

The SNAP-tag fusion protein is alternatively detected in cell extracts by adding appropriate amounts of the BG stain SNAP-vista green. The manufacturers protocoll is followed regarding the staining conditions and reaction conditions. The recombinant SNAP-tag fusion proteins stained by SNAP-vista green can be visualized in a standard UV transilluminator used for gel documentation.

A scientist skilled in the art may recognize that different *A. tumefaciens* strains together with other binary *A. tumefaciens* plasmid vectors than pTRAkc may also lead to successful transformation of tobacco plants and therefore functional expression of SNAP-Tag fusion proteins.

A skilled artisan may further recognize the possibility of transformation of a variety of different hosts plants with the here described *A. tumefaciens* based method.

### Yeast expression of SNAP-tag fusion proteins

Yeast strains like *A. adeninivorans* LS3, *A. adeninivorans* 135, *A. adeninivorans* G1211 ([aleu2-), *D.hansenii* H158, *D. polymorphus* H120, *P. pastoris* GS115 (his4 -) and the *H. polymorpha* MedHp1 (odc1 -), as well as *S. cerevisiae* C13ABYS86 (MATα leu2 ura3 his pra1 prb1 prc1 cps -) are used as possible hosts (Steinborn,G. et al., 2006). All strains are grown either under non-selective conditions in complex medium (YEPD) or under selective conditions in a yeast minimal medium (YMM) supplemented with 2% of a selected carbon source (Steinborn,G. et al., 2006). Cultivation is performed at 30°C. *A.adeninivorans* LS3, *A.adeninivorans* 135, *A. adeninivorans* G1211, *D.hansenii* H158, *D.polymorphus* H120, *H. polymorpha* MedHp1, *P. pastoris* GS115 and S. *cerevisiae* C13ABYS86 are transformed according to Rösel H. et al., 1998; and Dohmen RJ et al.,1991. Stable transformants are obtained after a sequence of passages on selective and non-selective media. After transformation of plasmids with the hph selection marker, hygromycin B-resistant colonies are selected on YEPD agar plates supplemented with 150 - 400 mg I-1 hygromycin B (200 mg I⁻¹ for *A.adeninivorans* LS3 and 135, 250 mg I⁻¹ for *D.hansenii* H158 and *D.polymorphus* H120, 400 mg I⁻¹ for *H. polymorpha* MedHp1, 150 mg I⁻¹ for P. *pastoris* GS115 and *S. cerevisiae* C13ABYS86. Single colonies are isolated and grown on YEPD medium and hygromycin B at 30°C for 2 days. This step is repeated three times before the cells are plated on non-selective YEPD agar and grown for 3-5 days at 30°C. A single colony from each transformant is then isolated and defined as a strain.

In case of auxothrophy complementation the transformants are selected on YMM agar plates lacking the respective amino acid.

Intracellular and extracellular expression levels of SNAP-tag fusion proteins are analyzed by Western blot experiments with anti-His-Tag antibodies for the newly generated expression yeast cell lines.

For this purpose, five transformants per yeast species are cultured in YMM12% glucose at 30°C for 72 h. The SNAP-tag fusion protein is alternatively detected in cell extracts by adding appropriate amounts of the BG stain SNAP-vista green. The manufacturers protocoll is followed regarding the staining conditions and reaction conditions. The recombinant SNAP-tag fusion proteins stained by Vista green can be visualized in a standard UV transilluminator used for gel documentation.

### Bacterial expression of SNAP-tag fusion proteins

For bacterial expression of SNAP-tag fusion proteins the desired fusion partners are cloned into the pET26b+ derived bacterial periplasmic expression vectors described in "construction of expression vectors".

Heat shock competent bacteria of the appropriate *E. coli* strain (e.g. ROSETTA, EMD Biosciences, Darmstadt, Germany) are transformed by e.g. heat shock transformation. Seleced clones growing on agar plates with Kanamycin (pET encoded Kan^{R} provides bacteria with resistance gene) are taken for expression.

The expression of the plasmid encoded SNAP-Tag fusion proteins is done using the osmotic stress expression protocol described in Barth et al., 2000.

Recombinant RFT5-SNAP-tag fusion proteins are expressed under the control of the IPTG inducible T7 lac promoter in *E. coli* ROSETTA (DE3). Bacteria are grown overnight at 26°C in Terrific Broth (TB) (Sambrook&Maniatis, 1989) containing 50 mg of kanamycin/ml and 0.5 mM ZnCl2, since it has been shown earlier that periplasmic proteolysis can be dramatically reduced upon addition of this salt (Baneyx, F., and G. Georgiou. 1992. ). The shaking culture is diluted 30-fold in 200 ml of the same medium. At an optical density at 600 nm (OD600) of 2, it is supplemented with 0.5 M sorbitol, 4% NaCl, and 10 mM glycine betaine and is then incubated at 26°C for additional 30 to 60 min. Thereafter, SNAP-tag fusion protein production is induced by the addition of 2 mM IPTG at 26°C.

Fifteen hours later, cells are harvested by centrifugation at 3,700 3 g for 10 minat 4°C. For all the following steps, tubes are chilled on ice. The bacterial pellet is centrifuged, and its wet weight is determined. Cells are frozen at -80°C until further processing.

The expression and purification of RFT5-SNAP by IMAC is performed as described in section "IMAC purification of SNAP-Tag fusion proteins fromm mammalian expression".

### IMAC purification of SNAP-Tag fusion proteins from mammalian expression

Purifications of the His-tagged proteins were accomplished by the Ni-NTA metal-affinity method (Hochuli, V., 1989, Porath, J. *et al.,* 1975). The protein purification followed a modified protocol for the purification of native protein from Qiagen *(The Expressionist* 07/97). For protein mini-preparation, 900 µl centrifugation-cleared cell culture supernatant was supplemented with 300 µl of 4x incubation buffer (200 mM NaH₂PO₄, pH 8.0; 1.2 M NaCl; 40 mM Imidazol) and 30 µl 50% Ni-NTA. Following 1 h incubation, the Ni-NTA resin was pelleted by centrifugation. After washing the sediment twice in 175 µl 1x incubation buffer, bound protein was eluted with 30 µl of elution buffer (50 mM NaH₂PO₄, pH 8.0; 1.2 M NaCl; and 40 mM imidazol) and 30 µl 50% Ni-NTA. Following an 1 h incubation, the Ni-NTA resin was pelleted by centrifugation. After washing the sediment twice in 175 µl 1x incubation buffer, bound protein was eluted with 30 µl of elution buffer (50 mM NaH₂PO₄, pH 8.0; 300 mM NaCl; 250 mM Imidazol) for 20 min at RT. Larger scale purification of eukaryotically-expressed proteins up to 500 ml cell culture supernatant was performed on a AEKTA FPLC system (Amersham-Pharmacia, USA). Cell culture supernatants were loaded onto a Ni-NTA column and following elution of the His-tagged proteins were made under the conditions described above.

Figure **(101****)** shows a 12 % SDS-PAGE gel (A: UV light, B: Coomassie stained) which was loaded with 5 µg of the different mammalian expressed and IMAC (Immobilized Metal Affinity Chromatography) purified. The Gel contains: 1:Ki4-SNAP; 2: SNAP-EGF; 3: Hai-SNAP; 4: H22-SNAP; 5: SNAP-CD30L; M: prestained portein marker (NEB).

### III Complex ABC and its use

### Labeling of SNAP-tag fusion proteins with BG derivatives of organic fluorophores

In a first step the SNAP-tag fusion protein (Ki4-SNAP) is Ni-NTA purified as decribed in section "IMAC purification of SNAP-Tag fusion proteins from mammalian expression". While still bound on the resin via His-Tag-Nickel interaction the Ki4-SNAP protein can be labeled with one of the SNAP-tag specific BG substrates like e.g BG505 as seen in figure **(99c****).**

A labeling solution of BG-505 2 µM is prepared in 1x Ni-NTA wash buffer (300 mM NaCl, 50 mM sodium phosphate, pH=7,5). As much solution as the estimated void volume of the Ni-NTA resin is prepared and added to the column. The incubation is done at room temperature for 30 minutes in the dark. The resin is washed twice with 5 bed volumes of Ni-NTA wash buffer. The elution of CT-fluorophor labeled His-tagged protein is done with a Ni-NTA elution buffer (300 mM NaCl, 50 mM sodium phosphate, 500 mM immidazole, pH=7,5).

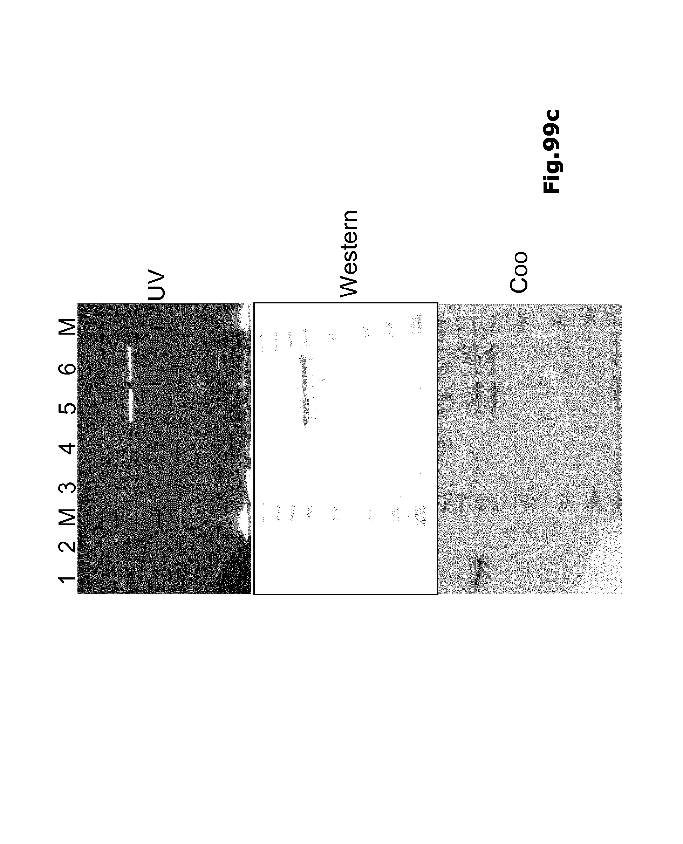

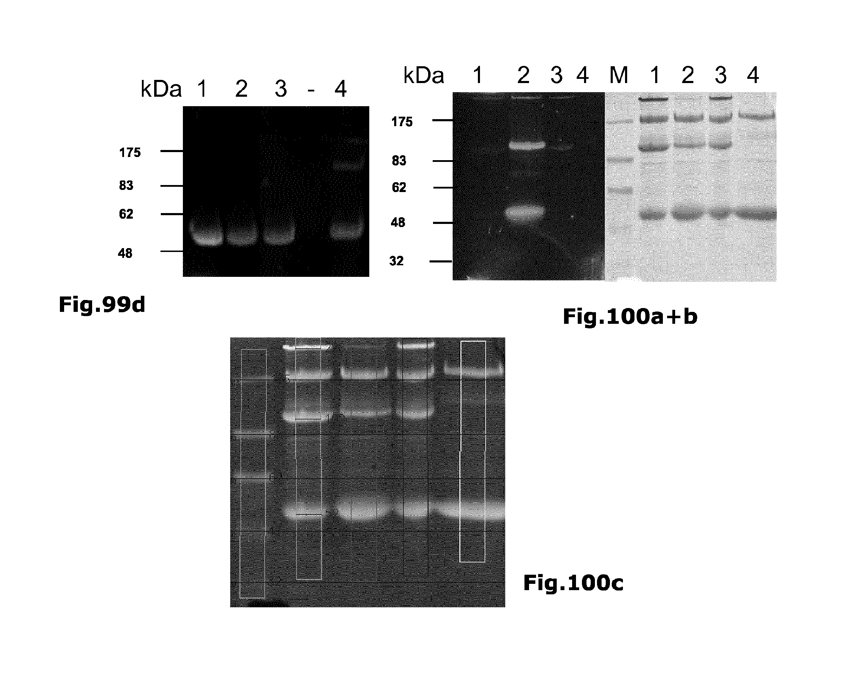

The success of the labeling reaction is documented by SDS-PAGE followed by analysis under a UV transilluminator (BioRad Gel Doc XR gel documentation) **(****Fig. 99c****).**

Furthermore the labeling success is documented with a Intas CRI-Maestro *In vivo* imager.

### Labeling of CLIP-tag fusion proteins with CT derivatives

CLIP-tag fusion proteins are Ni-NTA purified as decribed for SNAP-tag constructs. While still bound on the resin via His-Tag-Nickel interaction the CLIP-Tag fusion proteins can be labeled with one of the CLIP-tag specific CT substrates like CT-360,CT-430, CT-FL/CT-PF, CT-488, CT-505, CT547, CT-TMR, CT-647 CT-Biotin, CLIP-vista Green.

A labeling solution of CT-505 2 µM is prepared in 1x Ni-NTA wash buffer (300 mM NaCl, 50 mM sodium phosphate, pH=7,5). As much solution as the estimated void volume of the Ni-NTA resin is prepared and added to the column. The incubation is done at room temperature for 30 minutes in the dark. The resin is washed twice with 5 bed volumes of Ni-NTA wash buffer. The elution of CT-fluorophor labeled His-tagged protein is done with a Ni-NTA elution buffer (300 mM NaCl, 50 mM sodium phosphate, 500 mM immidazole, pH=7,5).

The success of the labeling reaction is documented by SDS-PAGE followed by analysis under a UV transilluminator (BioRad Gel Doc XR gel documentation).

Furthermore the labeling success is documented with a Intas CRI-Maestro *In vivo* imager.

### Labeling of ACP-/MCP-tag fusion proteins with CoA-derivatives in living cells

Wash the ACP-Tag-Eotaxin /MCP-Tag-CXCL9 expressing HEK293 cells three times with tissue culture medium with serum. One vial of ACP-tag substrate is dissolved in 25 µL of DMSO to give a labeling stock solution of 1 mM in DMSO. After 10 minutes of mixing all the ACP-tag substrate is dissolved.

The 1 mM ACP-tag substrate stock solution is diluted 1:200 in medium to give a labeling medium of 5 µM. Afterwards MgCl2 to a final concentration of 10 mM is supplemented. Finally, the ACP-Synthase is added to a final concentration of 1 µM.

The culture medium on the cells expressing an ACP-tag fusion protein located in or on the cell membrane with the ACP-tag facing the outside of the cell is exchanged with the labeling medium and incubated for 30 minutes.

Afterwards the labeling medium is removed and exchanged by fresh cell culture medium and incubated for another 20 minutes to remove unreacted ACP-tag substrate. The medium is exchanged again and the cells are ready for microscopy, flow cytometric analysis or FACS sorting.

The same procedure can be operated with cells expressing a MCP-Tag fusion protein. Therefore the labeling substrate is the same, a CoA derivative but instead of the ACP-Synthase the SFP-Synthase is taken for catalyzing the labeling reaction.

### Labeling of purified ACP-/MCP-tag fusion proteins with CoA derivatives

ACP/MCP-tag fusion proteins are Ni-NTA purified as decribed for SNAP-Tag constructs. While still bound on the resin via His-Tag-Nickel interaction the ACP/MCP-Tag fusion proteins can be labeled with one of the ACP/MCP-tag specific CoA based substrates like CoA-488, CoA-547, CoA-647and CoA-Biotin.

One vial of ACP-tag substrate is dissolved in 25 µL of DMSO to give a labeling stock solution of 1 mM in DMSO. After 10 minutes of mixing all the ACP-tag substrate is dissolved.

As much solution as the estimated void volume of the Ni-NTA resin is prepared and added to the column. The incubation is done at room temperature for 30 minutes in the dark. The resin is washed twice with 5 bed volumes of Ni-NTA wash buffer. The elution of BG-fluorophor labeled His-tagged protein is done with a Ni-NTA elution buffer (300 mM NaCl, 50 mM sodium phosphate, 500 mM immidazole, pH=7,5).

The same procedure can be performed with cells expressing a MCP-Tag fusion protein. Therefore the labeling substrate is the same, a CoA derivative but instead of the ACP-Synthase the SFP-Synthse is taken for catalyzing the labeling reaction.

The success of the labeling reaction is documented by SDS-PAGE followed by analysis under a UV transilluminator (BioRad Gel Doc XR gel documentation).

Furthermore the labeling success is documented with a Intas CRI-Maestro *In vivo* imager.

### Homo-/Hetero bivalent antibody-SNAP-tag conjugates

The moldular structure of the invention related complex allows the combination of two SNAP-tag constructs with (antibody) fusion partners of different/same binding specificity via a linker structure containing two or more BG residues, resulting in a bispecific molecule.

For the construction of heterobivalent (bispecific) constructs the whole process consists of two steps to maximize the amount of built heterodimers.

In a first step a recombinant SNAP-tag fusion protein with specificity 1 was bound on the resin via His-Tag-Nickel interaction.

A solution of 2µM of the desired homobifunctional BG-crosslinker (Fig. 3b) was prepared in 1x Ni-NTA wash buffer (300mM NaCl, 50mM sodium phosphate, pH= 7,5). As much solution as the estimated void volume of the Ni-NTA resin was prepared and added to the column. The incubation was done at room temperature for 30 minutes in the dark. The resin was washed twice with 5 bed volumes of Ni-NTA wash bufferto remove unreacted crosslinker.

The elution of BG-crosslinker labeled His-tagged protein was done with a Ni-NTA elution buffer (300 mM NaCl, 50 mM sodium phosphate, 500 mM immidazole, pH=7,5).

In a second step the recombinant SNAP-tag fusion protein with specificity 2 was added in the same molar ratio than the prelabeled protein 1. The crosslinking reaction was then performed at 4°C over night in solution.

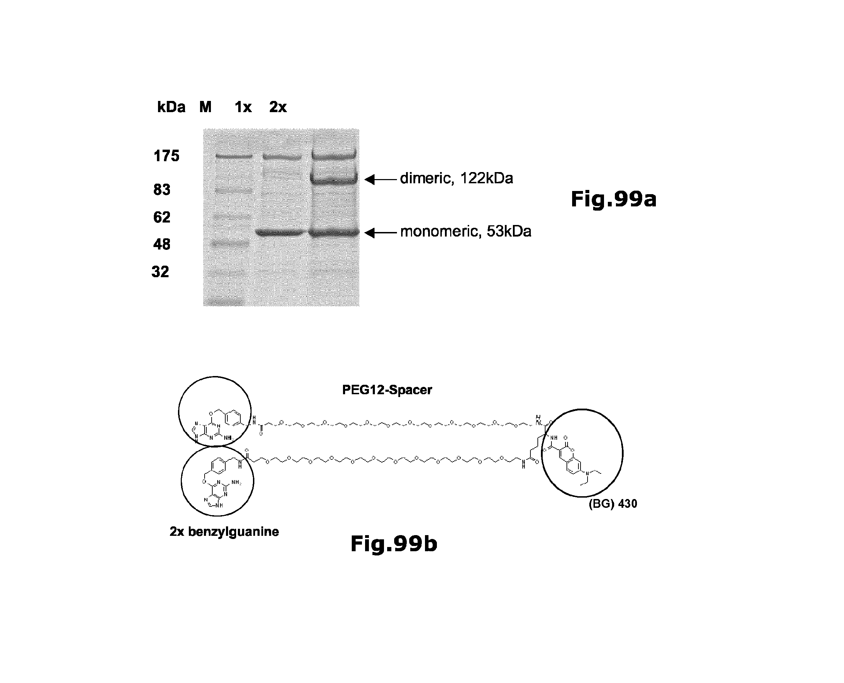

The success of the crosslinking reaction was documented by SDS-PAGE and Coomassie staining **(****Fig. 99a****)**.The gel shows the Ki4-SNAP (lane **1x**) and its crosslinked version (lane **2x)** together with a molecular weight marker (lane **M).** The molecular sizes determined by densitometric analysis are given as 53 kDa for the single Ki4-SNAP and 122kDa for the crosslinked version. Crosslinking was realized with a homobifunctional crosslinker, SV 305, containing a PEG 12 spacer **(****Fig. 99b****).**Crosslinkers like in Fig. **(99b****)** comprising a flourophor were additionaly documented with a *CRi*-Maestro *In vivo* Imager (INTAS, Göttingen, Germany) which is able to excite and detect all kinds of fluorophors from 430 nm up to 800 nm. It is able to assess emissions wavelengths from 500 up to 900 nm.

Successfully coupled SNAP-tag fusion proteins were detctable down to amounts of 50 ng depending on the quantum yield of the fluorophor.

### Bi-/multimeric SNAP-tag fusion proteins conjugates

Analogous to the in "bispecific antibody-SNAP-tag conjugates" described procedure di- or multimeric complexes with one binding specificity can be produced by crosslinkers having two or more BG moieties.

The reaction can also be done IMAC matrix assisted like for bispecifics but also without in a single step reaction.

For a single step reaction the IMAC purified SNAP-tag fusion protein is mixed with the crosslinker in the following ratio: for a crosslinker with with a given number of BG residues BGₙ the mixture formula is:

### (n) Mol SNAP fusion+ 1M BGₙ

The reaction mix is incubated for at least 12h at 4°C in the dark.

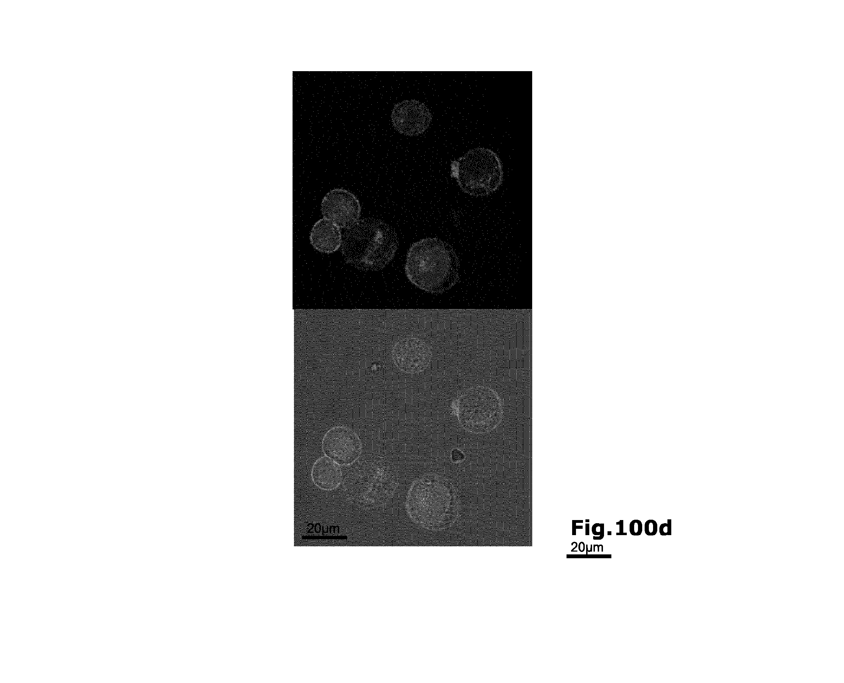

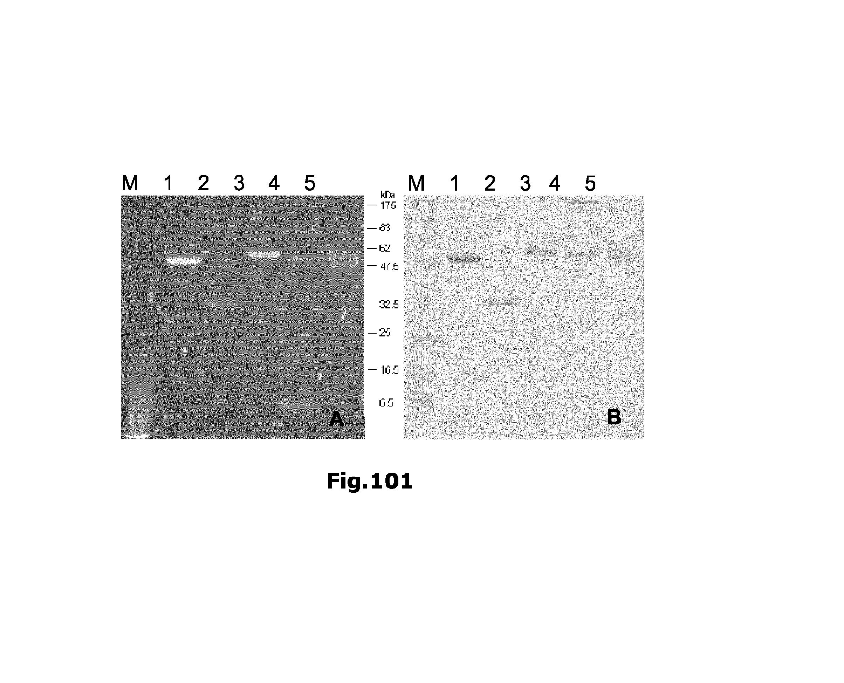

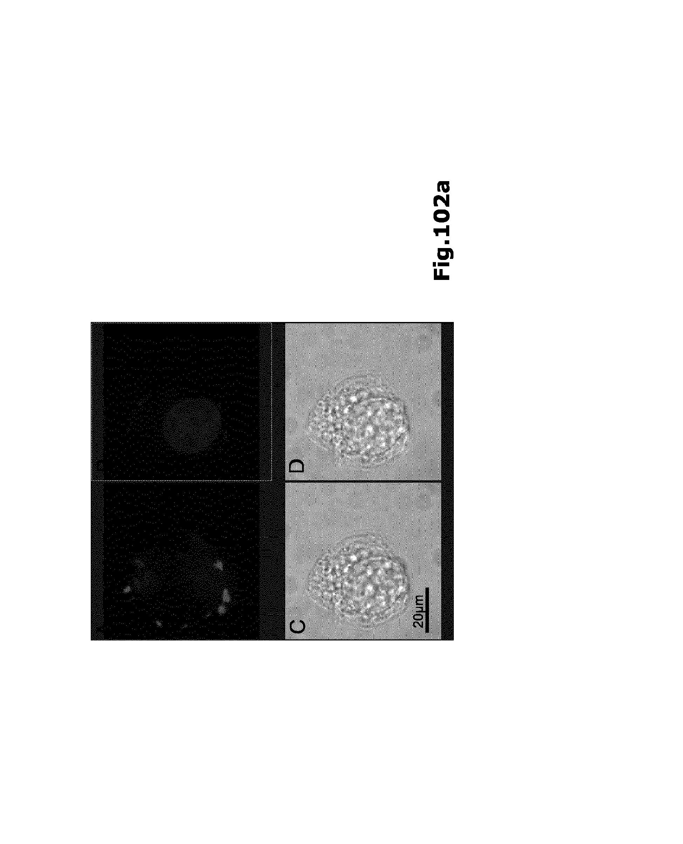

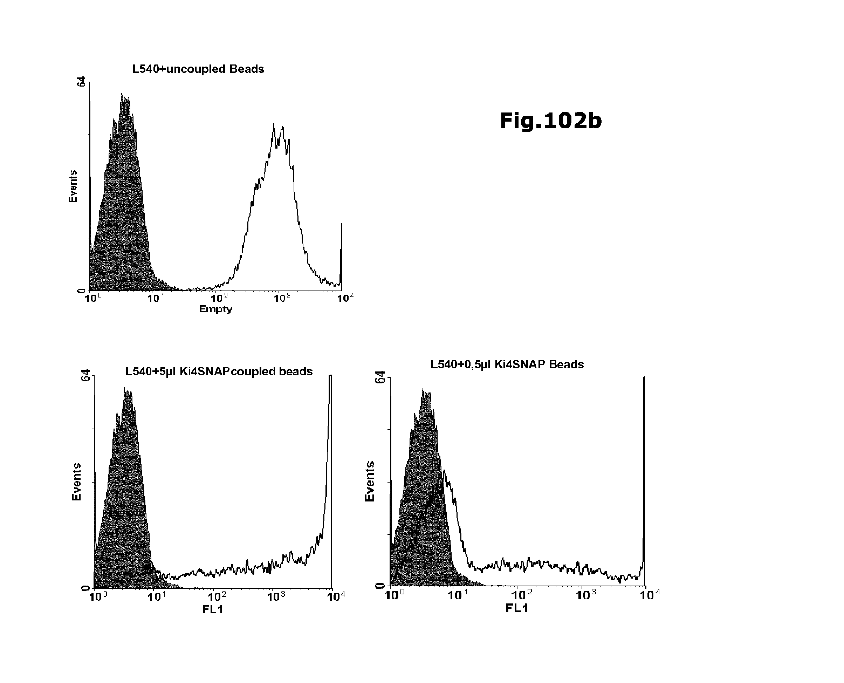

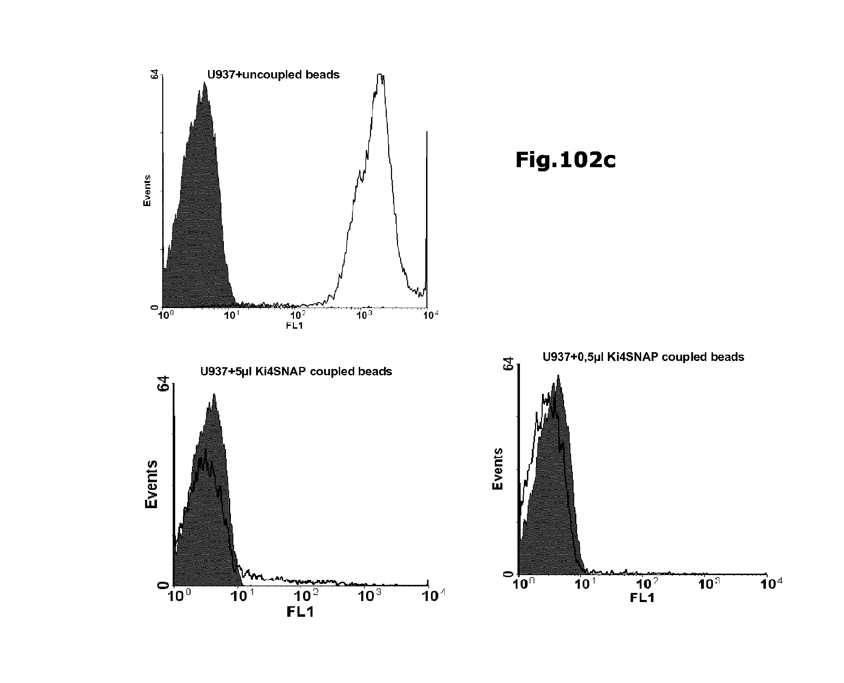

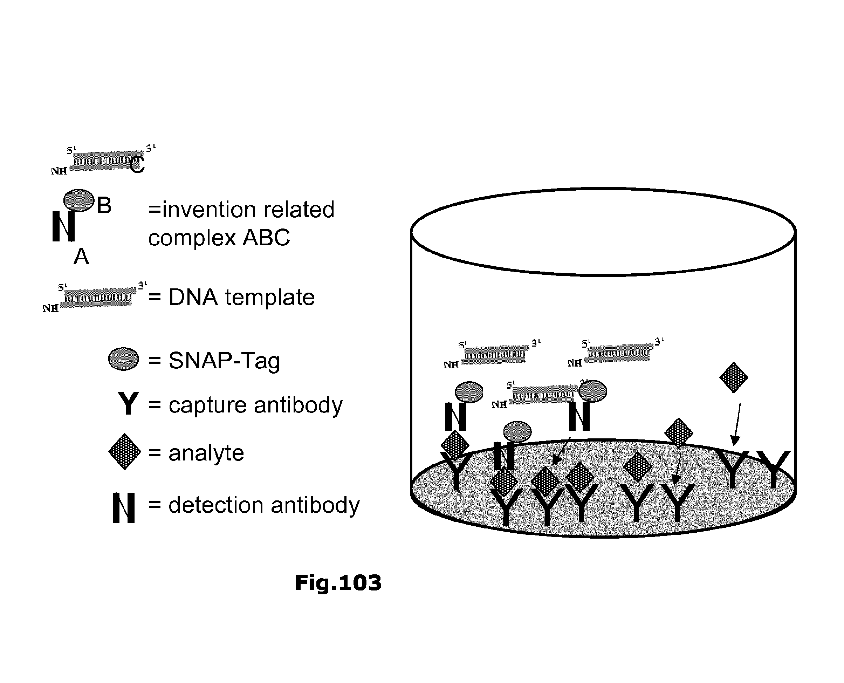

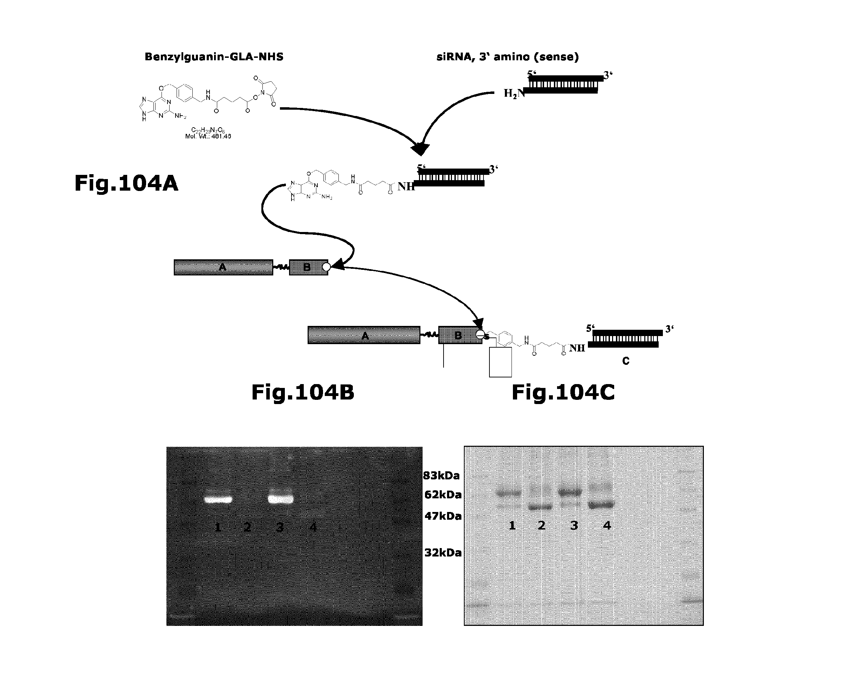

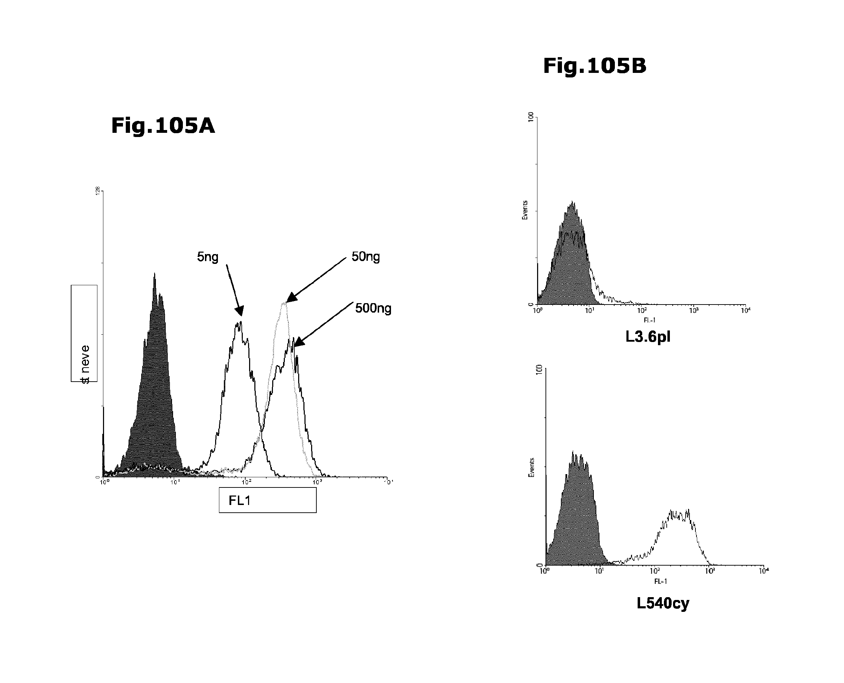

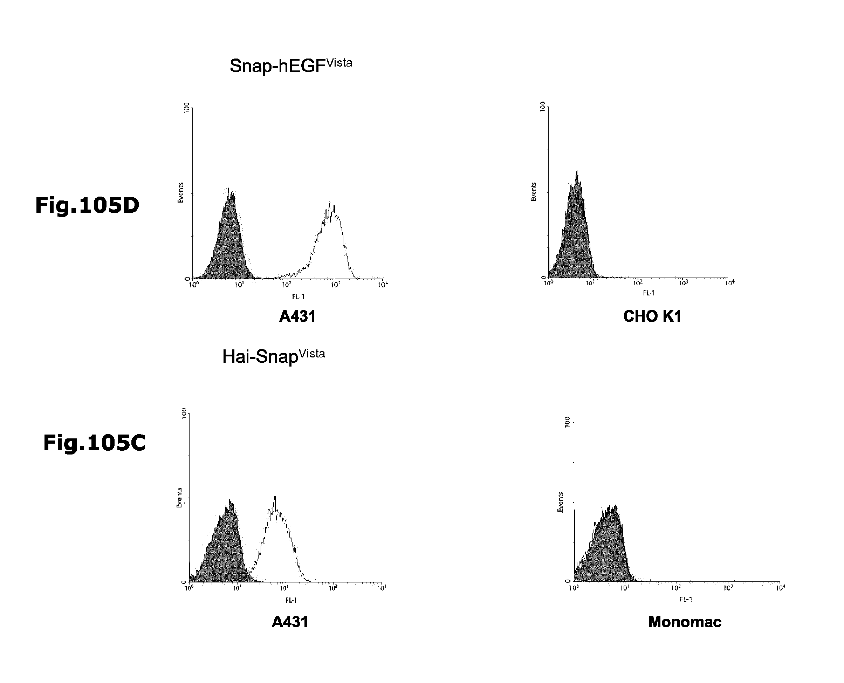

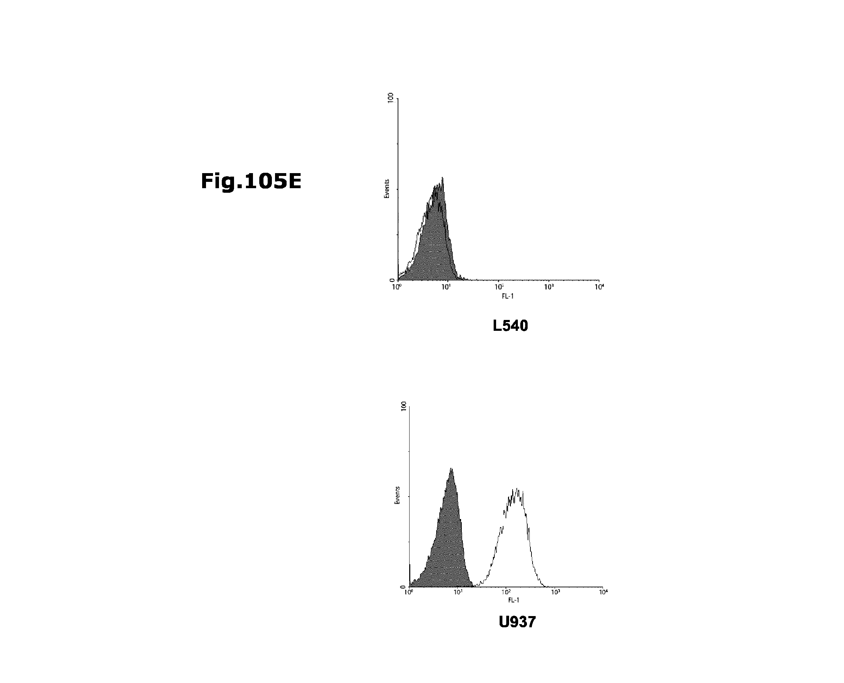

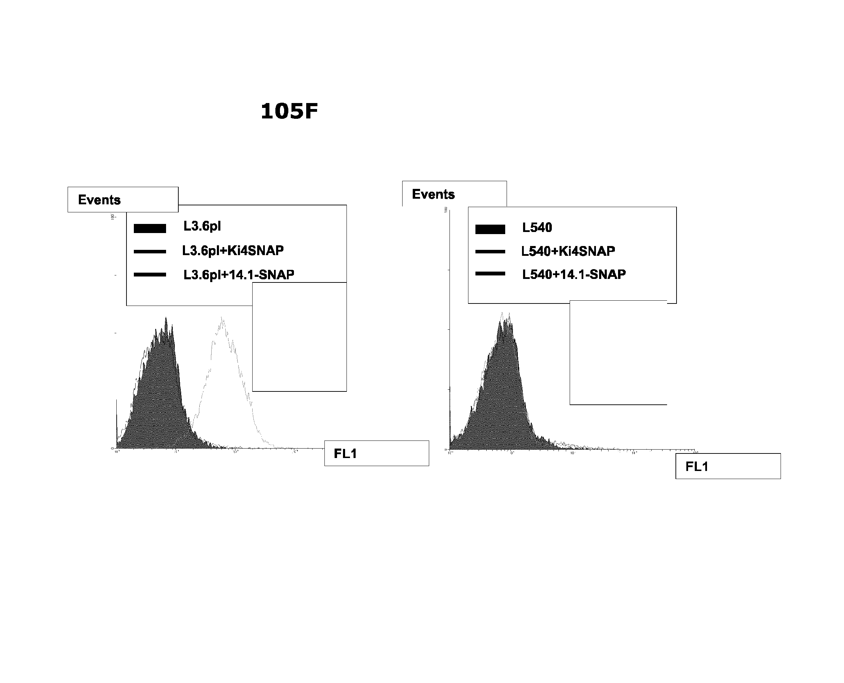

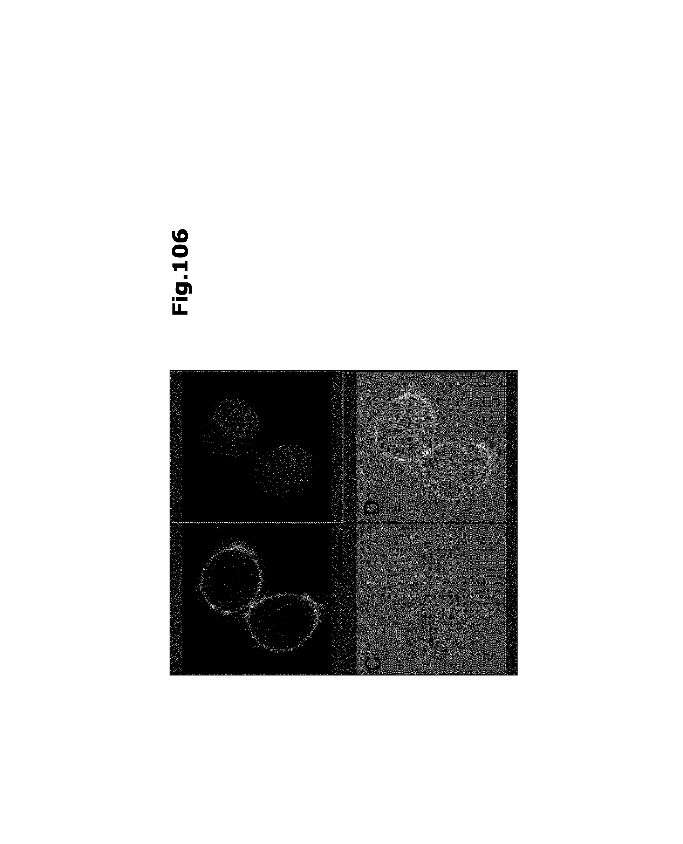

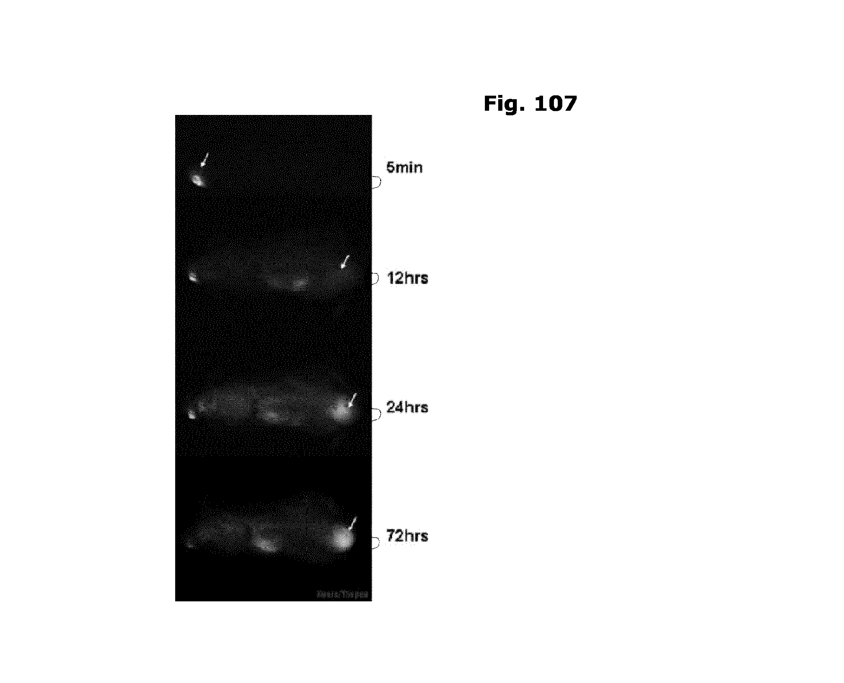

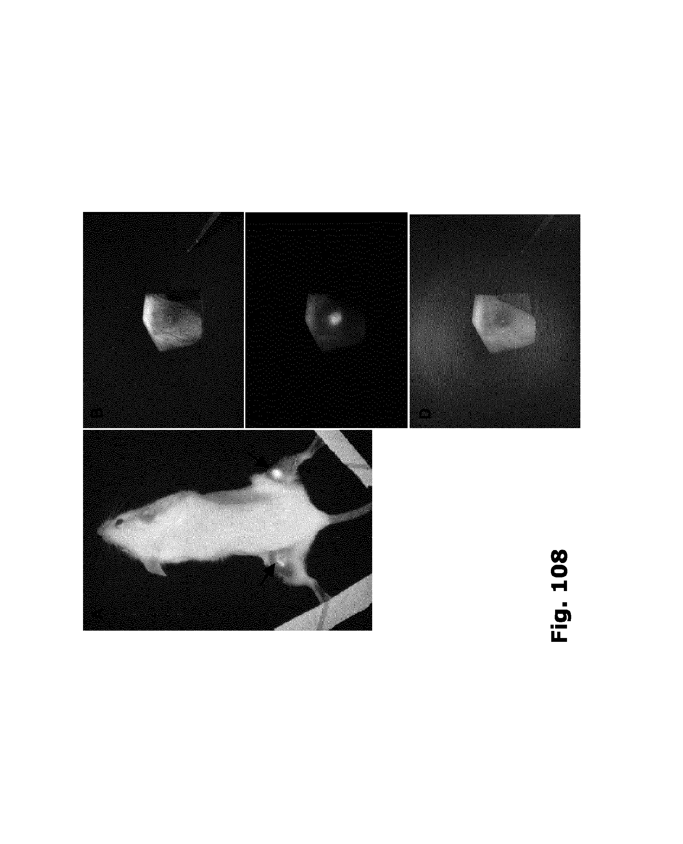

Figure **(100a****-d)** shows: **(****Fig. 100a****):** composite picture of Hai-SNAP fusion protein labelled with three different fluorophor labeled homotrimeric crosslinkers and visualized by Cri- MAESTRO *In vivo* Imager. **(****Fig. 100b****):** the same gel coomassie stained and **(****Fig. 100c****):** the same coomassie stained gel analyzed with a densitometric analysis software. The HaiSNAP was mixed with the crosslinkers in a molar ratio of 3:1. The fluorophors can be well detected using implemented conventional emission filter sets. Samples 1: C1776-4 labelled with BG430 ( Ex 421nm, Em 444nm and 484nm), 2: C1884-4 labelled with Atto 495 (Ex: 495, Em:527); 3: C1883-4 labelled with nile red (ex.: 554nm; ex: 638). For chemical structures see figures15,16 and 17. The chemical structure formulas are depicted in figures **(89-91****).**

Figure **(100d****)** shows a confocal microscopy done with the SV305 crosslinked version of Hai-SNAP. The crosslinked protein was separated from non crosslinked version by 100kDA MWCO spin columns (Pall Nanosep). In brief 25µg of the crosslinked sample were added to the column and cenrifuged at 10.000 g for 10 minutes. Afterwards 500µl 1xPBS were added and the sample centrifuged again until the volume was reduced to 50µl. This step was repeated and the residue in the column was taken for microscopic analysis.

The staining of 5x105 L3.6pl cells was done as described in section "Confocal microscopy applications of SNAP-tag fusion proteins".

### Antibody-Nucleic acid conjugates (RNA)

Optimized siRNAs were synthesised by Dharmacon with an amino-group and C(6) spacer on the 3' or 5' end of the sense strand. The siRNA duplexes are solubilised in PBS and reacted with a 50fold molar excess of BG-GLA-NHS (Covalys) solubilized in water free DMF for 4h at RT. In the next step the siRNA is ethanol precipitated and residual BG-GLA-NHS is removed by passing through a gel filtration column (Centri-spin 10, Princeton separations). Analogously thiol-modified RNA can be used together with a BG-maleimide after reduction of the thiol with DTT. Figure **(104A****)** shows te schematic procedure for si-RNA coupling to SNAP-tag fusion proteins.

The results of a coupling reactions of anti eEFII siRNA to H22-SNAP and to Hai-SNAP were separated on a 10% SDS-PAGE. The gel shows the following samples: 1: H22-SNAP + a eEFII-BG; 2: H22-SNAP; 3: Hai-SNAP + a eEFII-BG and 4: Hai-SNAP. Figure **(104B****)** is an ethidiumbromide stained gel analyzed under a standard UV transilluminator (BioRad). Figure **(104C)** is the same gel subsequently coomassie stained. The siRNA coupled SNAP-tag fusion proteins show a clear electromobility shift in comparison to their uncoupled versions. The siRNA coupled complexes run as exprected around 15 kDa. higher in size (65 kDa instead of 50 kDa).

### Antibody-Nucleic acid conjugates (DNA)

For targeted delivery of DNA molecules the DNA is modified with Benzylguanine either by direkt modifications of oligonukleotides with a terminal benzylguanine (BG) or benzylcytosine (BC). For longer DNA stretches or whole plasmids the DNA fragment is amplified by PCR using a BG or BC modified oligonucleotide as one of the two primers.

The PCR product is purified from unreacted BG or BC modified oligonucleotides via a commercial plasmid preparation kit (EndoFree Plasmid Maxi Kit QIAGEN, Hilden Germany).

The purified PCR product is then incubated with the SNAP-tag fusion protein in a molar ratio of (RNA:SNAP-tag fusion protein) 2:1 over night at 4°C. The success of the coupling reaction is monitored via agarose gel electrophoresis followed by ethidiumbromide staining where only the DNA labeled SNAP-tag fusion proteins are stained whereas the SNAP-tag fusion protein alone is not stained. A discrimination between DNA labeled and non labeled fusion proteins is also possible via the electromobility shift of labeled protein.

The successful DNA labeled Ki4-SNAP-tag fusion protein is able to target cancer cells via their overexpressed cell surface marker CD30.

After binding of the protein-DNA complex it is internalized via receptor mediated endocytosis processes. An alternative route of internalization is the electroporation of cells after binding of the complex with a nucleofector (AMAXA).

In another embodiment the SNAP-tag fusion protein-DNA complexes are at first coupled via specific DNA-DNA interaction on a DNA loaded surface. After coupling the complexes are able to fix CD30 overexpressing cells on certain spots, where the Protein-DNA complex was immobilized beforehand.

### Directed immobilization of SNAP-tag fusion proteins on particles

In this certain embodiment silica nanobeads with a size distribution between 20 and 80nm and encapsulated rhodamine fluorophor were taken. The beads were concentrated at 9.5 mg/ml with 5.3x10¹⁵ amino (NH₂) groups (8.76 x 10⁻³ µmol/ml).

500 µl beads (containing 4.38 nmol NH² groups) were pelleted with 1500 g for 2 min, washed 2x with dry DMF and resuspended in 80 µl DMF.

Beads in DMF were added to an excess of BG-GLA-NHS (415 nmol ⇒ 95fold molar excess) and incubated 1 h at 25°C with shaking.

Beads were washed twice with 800 µl PBS, resuspended with 200 ml Ki4-SNAP (100 µg, 2 nmol) in PBS/1 mM DTE and incubated for one hour at room temperature.

Beads were washed two times with 800 µl PBS and resuspended in 100 µl PBS prior to use.

Figure **(102A****)** shows a confocal microscopy of Ki4-SNAP functionalized Nanobeads binding CD30-positive L540 cells. Rhodamine based emission of beads in red (A) and Draq5 emission in blue pseudocolour (B), overlay (D) with grayscale picture (C).

Figure **(102B****)** shows the flow cytometric analysis of cD30 overexpressing L540cy cells incubated with different amounts (0,5 and 5µl) of Ki4-SNAP coupled rhodamine doted nanobeads. As control 5µl uncoupled beads were applied to L540cy cells.

Figure **(102C****)** shows the flow cytometric analysis of the CD30 negative U937 cells incubated with different amounts (0,5 and 5µl) of Ki4-SNAP coupled rhodamine doted nanobeads. As control 5µl uncoupled beads were applied to the U937 cells.

### Direct ELISA with SNAP-tag fusion proteins

A 96 well ELISA plate is coated with the analyte by pipetting 25 µl of coating buffer (100 mM Sodiumcarbonate, pH 9,6) in each well and then mixing with 25µl analyte solution per well.

After 2 hours of coating at room temperature the plate is washed twice with 1xPBS and then 50 µl of the detection antibody solution (SNAP-tag fusion protein, 50-100ng) is added. The SNAP-tag fusion protein was labeled beforehand with the SNAP-vista green fluorophor (Covalys) as described in "Labeling of SNP-tag fusion proteins with BG derivatives of organic fluorophores". The detection antibody solution is incubated for 1 hour at room temperature and the plate is washed twice with 1xPBS. Afterwards the plate is analyzed in a fluorescence ELISA reader using s filter set suited for the SNAP-tag coupled fluorophor.

### Sandwich ELISA with SNAP-tag fusion proteins

ELISA plate surfaces can be modified with BG-PEG-NH2 so that they will covalently immobilize SNAP-tag fusion proteins. Surface activation is done using standard amino-coupling procedures (such as exposure to NHS and EDC). This surface is then modified as follows: 1.4 mg (0.0031 mmol) BG-PEG-NH2 is dissolved in 10 mL HBSbuffer and centrifuged (20,000 x g, room temperature,20 min). 100 µL of this solution is pipetted into each well of a 96 well ELISA plate with carboxylated surfaces. After 30 minutes of incubation excess reactive groups are quenched by adding ethanolamin (10mmol) and further 10 minutes incubation. After three times of washing with 1x PBS the ELISA plate surface is ready to use for the direct immobilization of SNAP-tag fusion protein from samples. Therefore 100 ng of purified Ki2 mab (in 50µl PBS) are pipetted into each well and incubated for 2 hours at room temperature or alternatively over night at 4°C.

After washing two times with 1x PBS the sample (50µl) containing the analyte (secreted CD30) is pipetted into the wells and incubated for 2hours at room temperature. The Plate is then washed twice with 1x PBS before 50µl of the detection antibody solution (scFv Ki3, 200ng/well) is applied. The detection antibody consists either of a scFv-GFP fusion or a fluorophore labeled scFv-SNAP-tag fusion protein for fluorescent readout.

### Immuno-PCR

This protocol is basically a modified version of the quantitative immuno-PCR (qIPCR) method described by Niemeyer et al.,2007.

The basic principle of the assay relies upon a sandwich immunoassay followed by qPCR 1:captured of antigen by a non labeled antibody (Ki2 mab) coated on the 96 well PCR/ELISA plate and 2: sCD30 antigen capture from blood serum and 3: the detection of this captured sCD30 by a fusion protein of Ki3 scFv and SNAP-tag which was beforehand covalently labeled with a dsDNA PCR template and finaly 4: a qPCR step for signal amplification and readout.

Part 1 -3 represent a typical sandwich ELISA protocol as previously described. A schematic overview is given in figure **(103****).**

The assay has to be performed in thin-walled polycarbonate plates suited for immunoassay as well as for thermocycling based applications (e.g. Nunc TopYield starter kit).

To avoid contamination by PCR product, handling of immuno-PCR product DNA is strictly separated from earlier immuno-PCR set-up steps, by performing both in different, well-separated laboratories.

The protocol is performed as follows:

The initial working step is mostly performed as described in the "Sandwich ELISA" protocol. The only difference is the use of thin walled polycarbonate PCR grade 96 well plates or strips instead of conventional ELISA plates.

Instead of fluorophor labeled detection antibody (SNAP-tag fusion protein) a DNA template coupled detection antibody complex is used.

To remove as much unbound target DNA the plates are rigorously washed five times with PBS+Tween (0.01%), soaking wells for 3 min with wash buffer during each cycle, followed by two washes with ultrapure, 0.2 mm filtered water. After addition of PCR reagents, the plates are subjected to 30 cycles of PCR amplification, using a 96-well real-time PCR cycler, for example the ABIprism 7000 (Applied Biosystems) system.

The TaqMan Universal PCR Mastermix is prepared according to the manufacturer's instructions using the described concentrations of primer-1, primer-2 and probe. 30µl of the PCR Mastermix are pipetted in each well. The modules are sealed with an adhesive foil. The plate or PCR stripes are placed into the precleaned real time PCR machine and a typical PCR program is run: initial denaturation: 5 min 95 °C followed by 30 cycles consisting of denaturation step: 30 s, 50 °C , synthesis step: 30 s, 72 °C and denaturation step: 12 s, 95 °C. After the run the acquired data are evaluated by software.

### Flow cytometric applications of SNAP-tag fusion proteins

The cell-binding activity of the SNAP-tag fusion proteins containing a targeting component A was evaluated using a FACS Calibur flow cytometer and CellQuest software (Becton Dickinson, Heidelberg, Germany) or the free software WinMDI 2.8. The SNAP-tag fusion protein was labeled ahead of application as described in "Labeling of SNAP-tag fusion proteins with BG derivatives of organic fluorophores". Cells were labeled with the fluorophor labeled SNAP-tag complex by incubation for 30 minutes on ice. Cells were then washed twice with 500 µl cold PBS in an automated cell washer (Dade Serocent; Baxter). As alternative to the direct staining of the complex, the binding of the SNAP-tag fusion protein (only AB) was detected via the polyhistidine tag by using a Penta-His Alexa Fluor 488 antibody (Qiagen, Hilden, Germany).

In certain embodiments of this procedure antibody SNAP-tag fusion constructs targeting the human cell surface molecules CD30 (Hodgkin lymphoma), the CD64 *((Fc gamma RI* ) on activated macrophages) and the EGF receptor (on pancreatic-, breast-, lung-and non small cell lung cancer) were employed.

### a) targeting CD30:

The scFv Ki4, a monomeric recombinant version of the parental CD30 specific monoclonal antibody (Barth e al. 2000) and its counterpart the CD30 ligand were cloned as fusion proteins to SNAP-tag. The choice of positions (N- or C-terminal) was done in respect of maintaining full functionality of both fusion partners.

Figure **(105A****)** shows an evaluation of flow cytometric analysis of Ki-SNAP labeled with SNAP-vista Green (Covalys) binding on the CD30 overexpressing cell line L540cy.

Briefly 5x10⁵ L540cy cells were mixed with different amounts of SNAP-vista Green labeled Ki4-SNAP (5, 50 and 500 ng) in 500µl PBS. The binding reaction was allowed to proceed for 20 minutes on ice in the dark. Afterwards the cells were washed twice with PBS and analyzed in a FACS Calibur (Becton&Dickinson) flow cytometer.

Figure **(105B****)** shows an evaluation of flow cytometric analysis of SNAP-CD30L labeled with SNAP-vista Green (Covalys) binding on the CD30 overexpressing cell line L540cy.

Briefly 5x10⁵ L540cy cells were mixed with 500 ng of Vista Green labeled SNAP-CD30L in 500 µl PBS. The binding reaction was allowed to proceed for 20 minutes on ice in the dark. Afterwards the cells were washed twice with PBS and analyzed in a FACS Calibur (beton&Dickinson) flow cytometer. As negative control the CD30 negative cell line L3.6pl was stained and analyzed in the same manner.

### b) targeting EGFR:

The scFv 425 (further named Hai), a monomeric recombinant version of the parental EGFR specific monoclonal antibody (Haisma et. al., 2000) and the natural EGFR ligand EGF were cloned as fusion proteins to SNAP-tag. The choice of positions (N- or C-terminal) was done in respect of maintaining full functionality of both fusion partners.

Figure **(105C****)** shows an evaluation of flow cytometric analysis of Hai-SNAP labeled with SNAP-vista Green (Covalys) binding on the EGFR overexpressing cell line A431.

Briefly 5x10⁵ A431 cells were mixed with 500 ng of SNAP-vista Green labeled HAi-SNAP in 500 µl PBS. The binding reaction was allowed to proceed for 20 minutes on ice in the dark. Afterwards the cells were washed twice with PBS and analyzed in a FACS Calibur (Becton&Dickinson) flow cytometer. As negative control the same staining was performed with the EGFR negative cell line Monomac.

Figure **(105D****)** shows an evaluation of flow cytometric analysis of SNAP-EGF labeled with SNAP-vista Green (Covalys) binding on the EGFR overexpressing cell line A431.

Briefly 5x10⁵ A431 cells were mixed with 500 ng of Vista Green labeled SNAP-EGF in 500 µl PBS. The binding reaction was allowed to proceed for 20 minutes on ice in the dark. Afterwards the cells were washed twice with PBS and analyzed in a FACS Calibur (Becton&Dickinson) flow cytometer. As negative control the same staining was performed with the EGFR negative cell line CHO K1.

### c) targeting CD64:

The H22, a monomeric recombinant version of the parental anti CD64 specific monoclonal antibody (Tur et. al., 2003) was cloned as fusion proteins N-terminal to SNAP-tag.

Briefly 5x10⁵ U937 cells (CD64 positive) were mixed with 500 ng of SNAP-vista Green labeled H22-SNAP in 500 µl PBS. The binding reaction was allowed to proceed for 20 minutes on ice in the dark. Afterwards the cells were washed twice with PBS and analyzed in a FACS Calibur (beton&Dickinson) flow cytometer. As negative control the same staining was performed with the CD64 negative cell line L540.

Figure **(105E****)** shows an evaluation of flow cytometric analysis of H22-SNAP labeled with SNAP-vista Green (Covalys) binding on the CD64 overexpressing cell line U937 and not binding on the CD64 negative cell line L540.

### d) targeting pancreatic cancer:

To destinguish between inflammatory pancreatitis and pancratic cancer a immunized murine scFv Phage Display library was depleted on pancreatitis derived cellular material followed by three rounds of Phage Display selection. one pancreatic cancer specific scFv clone (clone 14.1) was selected as specific binder for pancreatic cancer derived cell lines L3.6pl and A431 and being negative on pancreatitis cell membranes.

In brief 5x10⁵ A431/L3.6pl cells were mixed with SNAP-vista Green labeled 14.1-SNAP in 500 µl PBS. The binding reaction was allowed to proceed for 20 minutes on ice in the dark. Afterwards the cells were washed twice with PBS and analyzed in a FACS Calibur (Becton&Dickinson) flow cytometer. As negative control the same staining was performed with the EGFR negative cell line L540 see figure **(105F****).**

### Confocal microscopy applications of SNAP-tag fusion proteins

The target cells were prepared as described in "Flow cytometric applications of SNAP-tag fusion proteins" but were fixed with formaldehyde after the last washing step. Therefore 300 µl of an icecold 0,4 % formaldehyde-PBS solution were added to the cells on ice and incubated for 30 minutes. The cells were washed once again with PBS in an automated cell washer. For counterstaining of nuclei, the cells were mixed with 2 µl of a 1/100 diluton of Draq5 (BioStatus, Leicestershire, UK). After 5 minutes incubation 10 µl of the cell suspension were mounted on a glass slide covered with glass coverslips and investigated with a Leica fluorescence (DMR) and Confocal microscope (TSC SP).

Figure **(106****)** shows confocal pictures of L540cy cells stained with BG505 (Covalys) labeled Ki4-SNAP.

### In vivo imaging

*In vivo* imaging of L3.6pl (EGFR⁺) tumor xenograft was done with the Intas *Cri Maestro In vivo* imager.

L3.6pl pancreatic carcinoma 5x10⁵ cells were injected intravenously in a female 6 week old SCID mouse and visualized after 1 week growth by retrobulbic injection of 70 µg Hai-SNAP labeled with BG-782 NIR dye. The labeling reaction was done beforehand as described in section "Labeling of SNP-tag fusion proteins with BG derivatives of organic fluorophores". The imaging was done with anesthetized mice after 5 minutes, 12, 24 and 72 hours after injection of the Hai-SNAP-BG782 imaging agent.

Figure **(107****)** shows infrared pictures from the whole mouse that were taken and analyzed by spectral unmixing the signal from background with the Intas Cri-Maestro *In vivo* imager. A large tumor in the abdomen of the mouse could be well visualized by accumulation of Hai-SNAP. There is a clear movement of the injected tumor imaging substrate from the place of injection towards the tumor detectable within a time range of 24 hours.

Figure **(108****)** shows infrared pictures from the whole mouse that were taken and analyzed by spectral unmixing the signal from background with the Intas Cri-Maestro *In vivo* imager. In contrast to picture 10 stably EGFP expressing L3.6pl pancreatic carcinoma 5x10⁵ cells were injected under the skin at the right and left femoral region of a female 6 week old SCID mouse and visualized after 1 week growth by retrobulbic injection of 70 µg Hai-SNAP labled with BG-782 NIR dye. The labeling reaction was done beforehand as described in section "Labeling of SNP-tag fusion proteins with BG derivatives of organic fluorophores". The imaging was done with anesthetized mouse after 24 past injection of the Hai-SNAP-BG782 imaging agent.

The green fluorescence and the infrared fluorescence signal clearly overlap when overlaying the corresponding pictures taken by the Intas Cri-Maestro In vivo imager.

### Receptor internalization studies using SNAP-tag fusion proteins

The EGFR-positive target cells were prepared as described in "Confocal microscopy applications of SNAP-tag fusion proteins" but were fixed with formaldehyde after different time points of SNAP-tag fusion protein application. Therefore 300 µl of an icecold 0,4 % formaldehyde-PBS solution were added after 15, 30 and 60 minutes of incubation at 4°C/37° to the cells on ice and incubated for 30 minutes. The cells were washed once again with PBS in an automated cell washer. For counterstaining of nuclei, the cells were mixed with 2 µl of a 1/100 diluton of Draq5 (BioStatus, Leicestershire, UK). After 5 minutes incubation 10µl of the cell suspension were mounted on a glass slide covered with glass coverslips and investigated with a Leica fluorescence (DMR) and Confocal microscope (TSC SP).

Figure **(109****)** shows confocal pictures of L3.6pl cells stained with BG505 (Covalys) labeled Hai-SNAP. There is a clear higher internalization rate of bound Hai-SNAP into the cells when incubated at 37° in comparison to the 4°C sample. EGFR negative cell lines like L540 and U937 were not stained under the same conditions.

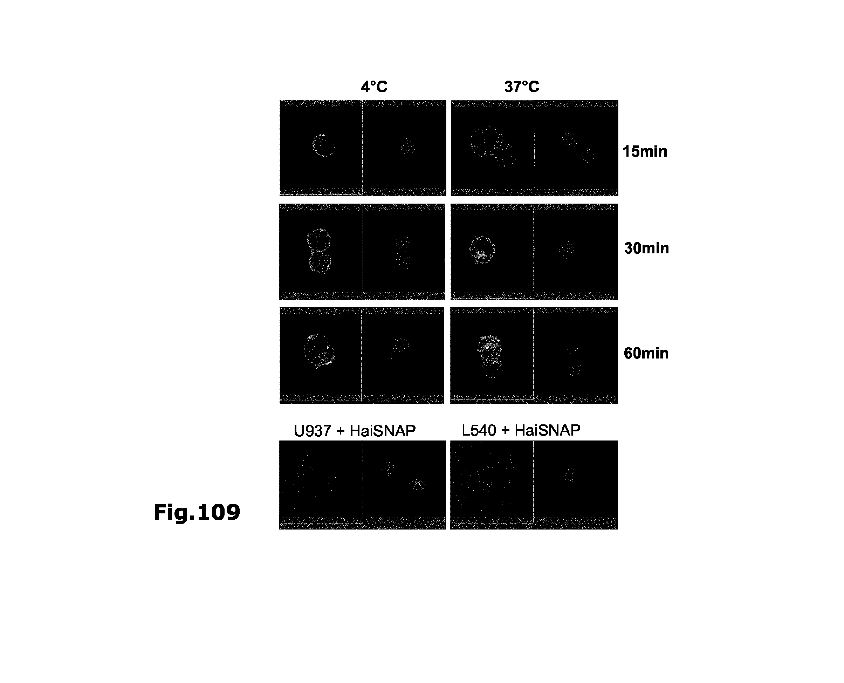

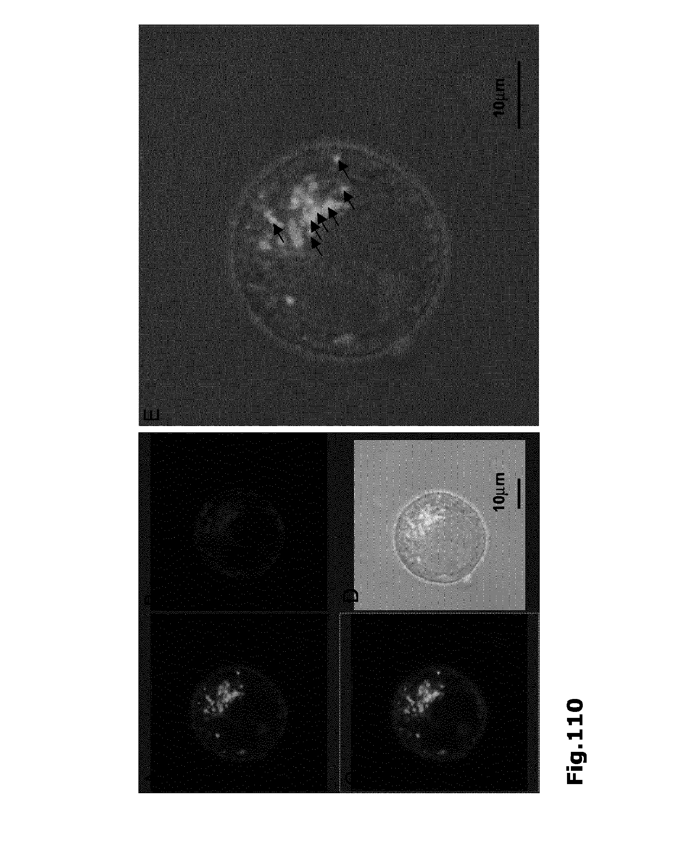

Figure **(110****)** shows the colocalization of Hai-SNAP BG505 labeled and transferrin ALEXA 594 labeled after internalization (see black arrows in **(Fig. 109E)).** Figure **(109A)** shows internalized HaiSNAP-BG505 in green; **(Fig. 109B)** clathrin-mediated internalization of transferrin-ALEXA594 in blue, **(Fig. 109C)** an overlay of A and B and D is an overlay of C with transmission light picture; **(Fig. 109E):** magnification of **(Fig. 109D):** arrows depict vesicles harboring both labeled transferrin and HaiSNAP-BG505. There is a high degree of overlapping localization of transferrin and HaiSNAP-BG505.

Transferrin is known to be internalized via clathrin supported internalization. This is also reported for EGFR internalization. The colocalization of Hai-SNAP and transferrin therefore indicates that the original internalization route of EGFR is not affected by bound Hai-SNAP.

### Use of SNAP-tag fusion proteins for flow cytometry based high producing strain selection

Cell permeable SNAP-tag staining substrates like BG-430, BG-505, BG-DAF and TMR-Star (Covalys) can be used to specifically label SNAP-tag fusion proteins in living mammalian cells. In order to detect the expression rate of transiently transfected HEK 293 or CHO cells (with pMS based vectors, see figures 1A+B and 5) the cells were incubated with cell permeable TMRstar.

The TMS-Star substrate was dissolved in DMSO according to the manufacturers (Covalys) instructions. 5 µM, TMR-Star was diluted to a final working concentration of 1 µM. Cells were labeled in the dark for 30 min at 37°C, then washed twice in medium and incubated for a further 30 min prior to imaging to allow diffusion of non-reacted substrate out of the cell. All steps were performed under a laminar flow and with sterile filtrated solutions.

For microscopic preevaluation 1x10⁵ TMR stained cells were counterstained with a 1:2000 dilution of DRAQ5™ solution for 2 min followed by a washing step with PBS. Thhe staining solution concentration and washing conditions affter TMR staining were carefully determined by microscopy until the TMR background in non or mock transfected cell lines was low enough to get a good signal to background ratio in the cells expressing SNAP-tag fusion protein.

The rest of the properly stained cells were sorted according to the strength of the TMR signal. Ten percent of the cells with strongest TMR signal were sorted and afterwards transfered into a new culture flask.

This procedure was repeated on demand to get a homogenous high producing cell population for scale up of the mammalian expression.

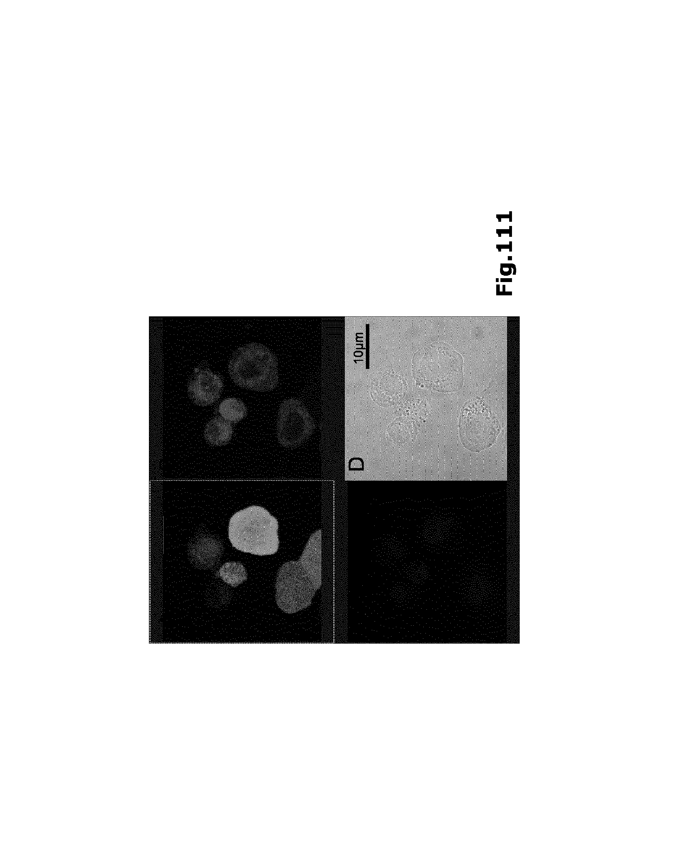

Figure **(111****)** shows the TMR staining of HEK293 cells expressing the Hai-SNAP fuison protein together with the EGFP reporter protein which is aencoded 3' on the biscistronic mRNA. Figure **(111A)** shows the signal of the EGFP reporter, **(Fig. 111B)** the TMR signal belonging to the SNAP-Tag fusion protein, **(Fig. 111C)** the Draq5 nuclear counterstain and **(Fig. 111D)** the transmission light picture of the same cells.

### Targeted delivery of interfering RNA via SNAP-tag fusion proteins

The coupling of anti eEFII siRNA and the Hai-SNAP fusion protein is basically done like described in section "Antibody-Nucleic acid conjugates (RNA)".

The complex was applied to target cells in concentrations ranging from 10ng/well (1x10⁵ target cells) to 100 ng/well (EGFR overexpressing cell line L3.6pl) and the cells are tested for specific knockdown of target genes 62 hours after application of the siRNA complex by westen blot and quantitatve PCR.

This approach can readily be adapted to other ligands and ribonucleic acids based molecules e.g. miRNAs/shRNA of different specificity.

In a further application the coupling of RNA molecules is realized over a homotrifunctional or heterotrifunctional and homobifunctional/heterobifunctional crosslinkers containing a maleimide function, by which a RNA molecule (preferably having RNA interference properties like siRNA) containing a terminal primary SH-group is coupled.

The preformed crosslinker-RNA complex is then added in a molar ratio of 2:1 to the pre- purified SNAP-/CLIP-tag fusion proteins and reaced as decribed above.

An example for heterobifunctional RNA bearing crosslinkers (one BG and on BC residue for crosslinking one SNAP-tag and one CLIP-tag fusion protein) is given in figure **(77****).**

Examples for homotrifunctional RNA bearing crosslinkers (three BG residues for crosslinking of three SNAP-tag fusion proteins) is given in figure **(10****,** **19****,** **50****).** This crosslinker **(****Fig. 50****)** additionally contains a fluorecein residue which enables tracing of the complex by e.g. microscopy.

### Targeted delivery of cytotoxic/cytostatic agents via SNAP-tag fusion proteins

In a specific embodiment of the invention the targeting complex AB is consisting of a EGFR targeting antibody (Hai) or natural lingand (EGF) whereas the SNAP-tag is coupled to Benzylguanine modified cytotoxic agents like Paclitaxel. This Paclitaxel is representing the invention related component C and is delivering in complex with AB a cytotoxic payload to targeted cells (EGFR overexpressing).

To increase the toxic payload per bound complex AB the toxic moiety is loaded on dendrimeric structures like described for Paclitaxel in Jongdoo Lim et al.,2007. or methothrexate in Gong Wu et al.,2006.

In brief the benzylguanine-modified dendrimers carrying a high number of cytotoxic moieties are coupled to the Hai-SNAP like described in section "Labeling of SNAP-tag fusion proteins with BG derivatives of organic fluorophores".

The purified (dialysis) dendrimer-Hai-SNAP complex is then applied to target cells and tested for specific cytotoxicity in a XTT based cell viability assay.

In a further imbodyment of the envention a toxic molecule like Chlorambucil is coupled to a homotrifunctional crosslinker. The preformed crosslinker-Chlorambucil complex is then added in a molar ratio of 3:1 to the pre- purified SNAP-/CLIP-tag fusion proteins and reaced as decribed above.

An example for heterotrifunctional Chlorambucil bearing crosslinker (three BG residues for crosslinking three SNAP-tag proteins) is given in figures **(17****,****43****,****51****).**

### Purification of multitag fusion proteins using SNAP-/CLIP-tag and ACP-tag technology

The general structure of the protein complex is SNAP-/CLIP-tag-Protease cleavage site-Target protein (+His-Tag) -ACP-tag.

All steps are performed in a FPLC system to better monitor the protein concentrations of every protocol step. In brief, the protein is primarily covalently bound via SNAP-/CLIP-tag to SNAP-/CLIP-Capture purification resin. After this step, the resin is intensively washed until no protein signal can be detected in the wash fraction. By adding the desired protease the target protein is then cleaved off and released from the resin. The eluted protein is then directly bound to a IMAC (Ni-NTA) column to re-bind the target protein via His-Tag and remove the protease by simple washing steps. The Protein can then be labeled at the ACP-tag site with fluorophors etc. on the column. Afterwards unreacted ACP substrate is washed away and a labeled highly pure target protein can be eluted from the Ni-NTA column.

All of the methods and compositions disclosed and claimed herein can be made and executed without undue experimentations in light of the present disclosure. While the compositions and methods of this invention have been described in terms of preferred embodiments, it will be apparent to those of skill in the art that variations may be applied to the methods and the steps or in the sequence of the steps of the method described herein without departing from the concept, spirit and scope of the invention. More specifically, it will be apparent that certain agents that are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

**Table 1**

| **CD molecule** | **Alternate Names** | **Entrez Gene** |
|---|---|---|
| CD1a | R4; HTA1 | 909 |
| CD1b | R1 | 910 |
| CD1c | M241; R7 | 911 |
| CD1d | R3 | 912 |
| CD1e | R2 | 913 |
| CD2 | CD2R; E-rosette receptor; T11; LFA-2 | 914 |
| CD3delta | CD3d | 915 |
| CD3epsilon | CD3e | 916 |
| CD3gamma | CD3g | 917 |
| CD4 | L3T4; W3/25 | 920 |
| CD5 | Leu-1; Ly-1; T1; Tp67 | 921 |
| CD6 | T12 | 923 |
| CD7 | gp40 | 924 |
| CD8alpha | Leu2; Lyt2; T cell co-receptor; T8 | 925 |
| CD8beta | Leu2; CD8; Lyt3 | 926 |
| CD9 | DRAP-27; MRP-1; p24 | 928 |
| CD10 | EC 3.4.24.11; neprilysin; CALLA; enkephalinase; gp100; NEP | 4311 |
| CD11a | AlphaL integrin chain; LFA-1alpha | 3683 |
| CD11b | AlphaM integrin chain; AlphaM-beta2; C3biR; CR3; Mac-1; Mo1 | 3684 |
| CD11c | AlphaX integrin chain; Axb2; CR4; leukocyte surface antigen p150,95 | 3687 |
| CDw12 | p90-120 | 23444 |
| CD13 | APN; EC 3.4.11.2; gp150 | 290 |
| CD14 | LPS-R | 929 |
| CD15u | Sulphated CD15 | |
| CD16a | FCRIIIA | 2214 |
| CD16b | FCRIIIB | 2215 |
| CDw17 | LacCer | |
| CD18 | CD11a beta subunit; CD11b beta subunit; CD11c beta subunit; beta-2 integrin chain | 3689 |
| CD19 | B4 | 930 |
| CD20 | B1; Bp35 | 931 |
| CD21 | C3d receptor; CR2; EBV-R | 1380 |
| CD22 | BL-CAM; Lyb8 | 933 |
| CD23 | B6; BLAST-2; FceRII; Leu-20; Low affinity IgE receptor | 2208 |
| CD24 | BA-1; HSA | 934 |
| CD25 | IL-2R alpha chain; IL-2R; Tac antigen | 3559 |
| CD26 | EC 3.4.14.5; ADA-binding protein; DPP IV ectoenzyme | 1803 |
| CD27 | S152; T14 | 939 |
| CD28 | T44; Tp44 | 940 |
| CD29 | Platelet GPIIa; VLA-beta chain; beta-1 integrin chain | 3688 |
| CD30 | Ber-H2 antigen; Ki-1 antigen | 943 |
| CD31 | GPiia'; endocam; PECAM-1 | 5175 |
| CD32 | FCR II; Fc gamma RII | 2212 |
| CD33 | gp67; p67 | 945 |
| CD34 | gp105-120 | 947 |
| CD35 | C3bR; C4bR; CR1; Immune Adherence Receptor | 1378 |
| CD36 | GPIIIb; GPIV; OKM5-antigen; PASIV | 948 |
| CD37 | gp52-40 | 951 |
| CD38 | T10; cyclic ADP-ribose hydrolase | 952 |
| CD39 | | 953 |
| CD40 | Bp50 | 958 |
| CD41 | GPIIb; alpha IIb integrin chain | 3674 |
| CD42a | GPIX | 2815 |
| CD42b | GPIbalpha; Glycocalicin | 2811 |
| CD42c | GPIb-beta | 2812 |
| CD42d | GPV | 2814 |
| CD43 | gpL115; leukocyte sialoglycoprotein; leukosialin; sialophorin | 6693 |
| CD44 | ECMR III; H-CAM; HUTCH-1; Hermes; Lu, In-related; Pgp-1; gp85 | 960 |
| CD44R | CD44v; CD44v9 | 960 |
| CD45 | B220; CD45R; CD45RA; CD45RB; CD45RC; CD45RO; EC 3.1.3.4; LCA; T200; Ly5 | 5788 |
| CD46 | MCP | 4179 |
| CD47R | Rh-associated protein; gp42; IAP; neurophilin; OA3; MEM-133; formerly CDwl49 | 961 |
| CD48 | BCM1; Blast-1; Hu Lym3; OX-45 | 962 |
| CD49a | Alpha-1 integrin chain; VLA-1 alpha chain | 3672 |
| CD49b | Alpha-2 integrin chain; GPIa; VLA-2 alpha chain | 3673 |
| CD49c | Alpha-3 integrin chain; VLA-3 alpha chain | 3675 |
| CD49d | Alpha-4 integrin chain; VLA-4 alpha chain | 3676 |
| CD49e | Alpha-5 integrin chain; FNR alpha chain; VLA-5 alpha chain | 3678 |
| CD49f | Alpha-6 integrin chain; Platelet gpI; VLA-6 alpha chain | 3655 |
| CD50 | ICAM-3 | 3385 |
| CD51 | VNR-alpha chain; alpha V integrin chain; vitronectin receptor | 3685 |
| CD52 | | 1043 |
| CD53 | | 963 |
| CD54 | ICAM-1 | 3383 |
| CD55 | DAF | 1604 |
| CD56 | Leu-19; NKH1; NCAM | 4684 |
| CD57 | HNK1; Leu-7 | 964 |
| CD58 | LFA-3 | 965 |
| CD59 | 1F-5Ag; H19; HRF20; MACIF; MIRL; P-18; Protectin | 966 |
| CD60a | GD3 | |
| CD60b | 9-O-acetyl-GD3 | |
| CD60c | 7-O-acetyl-GD3 | |
| CD61 | CD61A; GPIIb/IIIa; beta 3 integrin chain | 3690 |
| CD62E | E-selectin; ELAM-1; LECAM-2 | 6401 |
| CD62L | L-selectin; LAM-1; LECAM-1; Leu-8; MEL-14; TQ-1 | 6402 |
| CD62P | P-selectin; GMP-140; PADGEM | 6403 |
| CD63 | LIMP; MLA1; PTLGP40; gp55; granulophysin; LAMP-3; ME491; NGA | 967 |
| CD64 | FC gammaRI; FCR I | 2209 |
| CD65 | Ceramide-dodecasaccharide; VIM-2 | |
| CD65s | Sialylated-CD65; VIM2 | |
| CD66a | NCA-160; BGP | 634 |
| CD66b | CD67; CGM6; NCA-95 | 1088 |
| CD66c | NCA; NCA-50/90 | 4680 |
| CD66d | CGM1 | 1084 |
| CD66e | CEA | 1048 |
| CD66f | Pregnancy specific b1 glycoprotein; SP-1; PSG | 5669 |
| CD68 | gp110; macrosialin | 968 |
| CD69 | AIM; EA 1; MLR3; gp34/28; VEA | 969 |
| CD70 | CD27-ligand; Ki-24 antigen | 970 |
| CD71 | T9; transferrin receptor | 7037 |
| CD72 | Ly-19.2; Ly-32.2; Lyb-2 | 971 |
| CD73 | Ecto-5'-nucleotidase | 4907 |
| CD74 | Class II-specific chaperone; Ii; Invariant chain | 972 |
| CD75 | Lactosamines | |
| CD75s | Alpha-2,6-sialylated lactosamines (formerly CDw75 and CDw76) | |
| CD77 | Pk blood group antigen; BLA; CTH; Gb3 | |
| CD79a | Ig alpha; MB1 | 973 |
| CD79b | B29; Ig beta | 974 |
| CD80 | B7; BB1 | 941 |
| CD81 | TAPA-1 | 975 |
| CD82 | 4F9;C33;IA4;KAI1; R2 | 3732 |
| CD83 | HB15 | 9308 |
| CD84 | | 8832 |
| CD85 | ILT/LIR family | 10859 |
| CD86 | B7-2;B70 | 942 |
| CD87 | uPAR | 5329 |
| CD88 | C5aR | 728 |
| CD89 | Fcalpha-R; IgA Fc receptor;IgA receptor | 2204 |
| CD90 | Thy-1 | 7070 |
| CD91 | ALPHA2M-R; LRP | 4035 |
| CD92 | CTL1; formerly CDw92 | 23446 |
| CDw93 | | 23447 |
| CD94 | Kp43 | 3824 |
| CD95 | APO-1; Fas; TNFRSF6; APT1 | 355 |
| CD96 | TACTILE | 10225 |
| CD97 | | 976 |
| CD98 | 4F2; FRP-1; RL-388 | 4198 |
| CD99 | CD99R; E2; MIC2 gene product | 4267 |
| CD100 | SEMA4D | 10507 |
| CD101 | IGSF2; P126; V7 | 9398 |
| CD102 | ICAM-2 | 3384 |
| CD103 | ITGAE; HML-1; integrin alphaE chain | 3682 |
| CD104 | beta 4 integrin chain; TSP-1180; beta 4 | 3691 |
| CD105 | endoglin | 2022 |
| CD106 | INCAM-110; VCAM-1 | 7412 |
| CD107a | LAMP-1 | 3916 |
| CD107b | LAMP-2 | 3920 |
| CD108 | SEMA7A; JMH human blood group antigen; formerly CDw108 | 8482 |
| CD109 | 8A3; E123; 7D1 | |
| CD110 | MPL; TPO-R; C-MPL | 4352 |
| CD111 | PVRL1; PRR1; HevC; nectin-1; HIgR | 5818 |
| CD112 | HVEB; PRR2; PVRL2; nectin 2 | 5819 |
| CDw113 | PVRL3, Nectin3; poliovirus receptor-related 3; nectin-3 | 25945 |
| CD114 | CSF3R; HG-CSFR; G-CSFR | 1441 |
| CD115 | c-fms; CSF-1R; M-CSFR | 1436 |
| CD116 | GM-CSF receptor alpha chain | 1438 |
| CD117 | c-KIT; SCFR | 3815 |
| CD118 | LIFR; leukemia inhibitory factor receptor | 3977 |
| CDw119 | IFNgR; IFNgRa | 3459 |
| CD120a | TNFRI; p55 | 7132 |
| CD120b | TNFRII; p75; TNFR p80 | 7133 |
| CD121a | IL-1R; type 1 IL-1R | 3554 |
| CDw121b | IL-1R, type 2 | 7850 |
| CD122 | IL-2Rbeta | 3560 |
| CD123 | IL-3Ralpha | 3563 |
| CD124 | IL-4R | 3566 |
| CDw125 | IL-5Ralpha | 3568 |
| CD126 | IL-6R | 3570 |
| CD127 | IL-7R; IL-7R alpha; p90 117 R | 3575 |
| CDw128a | CXCR1; IL-8RA | 3577 |
| CDw128b | CXCR2; IL-8RB | 3579 |
| CD129 | Reserved | |
| CD130 | gp130 | 3572 |
| CD131 | common beta subunit | 1439 |
| CD132 | IL2RG; common cytokine receptor gamma chain; common gamma chain | 3561 |
| CD133 | PROML1; AC133; hematopoietic stem cell antigen; prominin-like 1 | 8842 |
| CD134 | OX40 | 7293 |
| CD135 | flt3; Flk-2; STK-1 | 2322 |
| CDw136 | msp receptor; ron; p158-ron | 4486 |
| CDw137 | 4-1BB; ILA | 3604 |
| CD138 | heparan sulfate proteoglycan; syndecan-1 | 6382 |
| CD139 | | 23448 |
| CD140a | PDGF-R; PDGFRa | 5156 |
| CD140b | PDGFRb | 5159 |
| CD141 | fetomodulin; TM | 7056 |
| CD142 | F3; coagulation Factor III; thromboplastin; TF | 2152 |
| CD143 | EC 3.4.15.1; ACE; kininase II; peptidyl dipeptidase A | 1636 |
| CD144 | cadherin-5; VE-Cadherin | 1003 |
| CDw145 | | |
| CD146 | MCAM; A32; MUC18; Mel-CAM; S-endo | 4162 |
| CD147 | 5A11; Basigin; CE9; HT7; M6; Neurothelin; OX-47; EMMPRIN; gp42 | 682 |
| CD148 | HPTP-eta; DEP-1; p260 | 5795 |
| CDw149 | new designation is CD47R | |
| CD150 | SLAM; IPO-3; fomerly CDw150 | 6504 |
| CD151 | PETA-3; SFA-1 | 977 |
| CD152 | CTLA-4 | 1493 |
| CD153 | CD30L | 944 |
| CD154 | CD40L; T-BAM; TRAP; gp39 | 959 |
| CD155 | PVR | 5817 |
| CD156a | ADAM8; MS2 human; fomerly CD156 | 101 |
| CD156b | ADAM17; TACE; cSVP | 6868 |
| CDw156C | ADAM10; a disintegrin and metalloproteinase domain 10 | 102 |
| CD157 | BP-3/IF-7; BST-1; Mo5 | 683 |
| CD158 | KIR family | |
| CD159a | NKG2A | 3821 |
| CD159c | NKG2C; killer cell lectin-like receptor subfamily C, member 2 | 3822 |
| CD160 | BY55 antigen; NK1; NK28 | 11126 |
| CD161 | KLRB1; NKR-P1A; killer cell lectin-like receptor subfamily B, member 1 | 3820 |
| CD162 | PSGL-1, PSGL | 6404 |
| CD162R | PEN5 (a post-translational modification of PSGL-1) | 6404 |
| CD163 | GHI/61; M130; RM3/1 | 9332 |
| CD164 | MUC-24; MGC-24v | 8763 |
| CD165 | AD2; gp37 | 23449 |
| CD166 | BEN; DM-GRASP; KG-CAM; Neurolin; SC-1; ALCAM | 214 |
| CD167a | trkE; trk6; cak; eddr1; DDR1; MCK10; RTK6; NTRK4 | 780 |
| CD168 | HMMR; IHABP; RHAMM | 3161 |
| CD169 | sialoadhesin; siglec-1 | 6614 |
| CD170 | Siglec-5 | 8778 |
| CD171 | L1; L1CAM; N-CAM L1 | 3897 |
| CD172a | SIRP alpha | 8194 |
| CD172b | SIRPbeta; signal-regulatory protein beta 1 | 10326 |
| CD172g | SIRPgamma; signal-regulatory protein beta 2 | 55423 |
| CD173 | Blood group H type 2 | |
| CD174 | Lewis y | 2525 |
| CD175 | Tn | |
| CD175s | Sialyl-Tn | |
| CD176 | TF | |
| CD177 | NB1 | |
| CD178 | fas-L; TNFSF6; APT1LG1; CD95-L | 356 |
| CD179a | VpreB; VPREB1; IGVPB | 7441 |
| CD179b | IGLL1; lambda5; immunoglobulin omega polypeptide; IGVPB; 14.1 chain | 3543 |
| CD180 | LY64; RP105 | 4064 |
| CD181 | CXCR1; (was CDw128A), IL8Ralpha | 3577 |
| CD182 | CXCR2; (was CDw128B), IL8Rbeta | 12765 |
| CD183 | CXCR3; GPR9; CKR-L2; IP10-R; Mig-R | 2833 |
| CD184 | CXCR4; fusin; LESTR; NPY3R; HM89; FB22 | 7852 |
| CD185 | CXCR5; Chemokine (C-X-C motif) Receptor 5, Burkitt lymphoma receptor 1 | 643 |
| CDw186 | CXCR6; Chemokine (C-X-C motif) Receptor 6 | 10663 |
| CD191 | CCR1; Chemokine (C-C motif) Receptor 1, RANTES Receptor | 1230 |
| CD192 | CCR2; Chemokine (C-C motif) Receptor 2, MCP-1 receptor | 1231 |
| CD193 | CCR3; Chemokine (C-C motif) Receptor 3, eosinophil eotaxin receptor | 1232 |
| CD195 | CCR5 | 1234 |
| CD196 | CCR6; Chemokine (C-C motif) Receptor 6 | 1235 |
| CD197 | CCR7; (was CDw197) Chemokine (C-C motif) Receptor 7 | 1236 |
| CDw198 | CCR8; Chemokine (C-C motif) Receptor 8 | 1237 |
| CDw199 | CCR9; Chemokine (C-C motif) Receptor 9 | 10803 |
| CDw197 | CCR7 | 1236 |
| CD200 | OX2 | 4345 |
| CD201 | EPC R | 10544 |
| CD202b | tie2; tek | 7010 |
| CD203c | NPP3; PDNP3; PD-Ibeta; B10; gp130RB13-6; ENPP3; bovine intestinal phosphodiesterase | 5169 |
| CD204 | macrophage scavenger R | 4481 |
| CD205 | DEC205 | 4065 |
| CD206 | MRC1; MMR | 4360 |
| CD207 | Langerin | 50489 |
| CD208 | DC-LAMP | 27074 |
| CD209 | DC-SIGN | 30385 |
| CDw210 | IL-10 R | 3587; 3588 |
| CD212 | IL-12 R | 3594 |
| CD213a1 | IL-13 R alpha 1 | 3597 |
| CD213a2 | IL-13 R alpha 2 | 3598 |
| CDw217 | IL-17 R | 23765 |
| CDw218a | IL18Ralpha; IL18Ralpha | |
| CDw218b | IL18Rbeta; IL18Rbeta | |
| CD220 | Insulin R | 3643 |
| CD221 | IGF1 R | 3480 |
| CD222 | Mannose-6-phosphate/IGF2 R | 3482 |
| CD223 | LAG-3 | 3902 |
| CD224 | GGT; EC2.3.2.2 | 2678 |
| CD225 | Leu13 | 8519 |
| CD226 | DNAM-1; PTA1; TLiSA1 | 10666 |
| CD227 | MUC1; episialin; PUM; PEM; EMA; DF3 antigen; H23 antigen | 4582 |
| CD228 | melanotransferrin | 4241 |
| CD229 | Ly9 | 4063 |
| CD230 | Prion protein | 5621 |
| CD231 | TM4SF2; A15; TALLA-1; MXS1; CCG-B7; TALLA | 7102 |
| CD232 | VESP R | 10154 |
| CD233 | band 3; erythrocyte membrane protein band 3;AE1; SLC4A1; Diego blood group; EPB3 | 6521 |
| CD234 | Fy-glycoprotein; Duffy antigen | 2532 |
| CD235a | Glycophorin A | 2993 |
| CD235b | Glycophorin B | 2994 |
| CD235ab | Glycophorin A/B crossreactive mabs | |
| CD236 | Glycophorin C/D | |
| CD236R | Glycophorin C | 2995 |
| CD238 | Kell | 3792 |
| CD239 | B-CAM | 4059 |
| CD240CE | Rh30CE | 6006 |
| CD240D | Rh30D | 6007 |
| CD240DCE | Rh30D/CE crossreactive mabs | |
| CD241 | RhAg | 6005 |
| CD242 | ICAM-4 | 3386 |
| CD243 | MDR-1 | 5243 |
| CD244 | 2B4; NAIL; p38 | 51744 |
| CD245 | p220/240 | |
| CD246 | Anaplastic lymphoma kinase | 238 |
| CD247 | Zeta chain | 919 |
| CD248 | TEM1, Endosialin; CD164 sialomucin-like 1, tumor endothelial marker 1 | 57124 |
| CD249 | Aminopeptidase A; APA, gp160 | 2028 |
| CD252 | OX40L; TNF (ligand) superfamily member 4, CD134 ligand | 7292 |
| CD253 | TRAIL; TNF (ligand) superfamily member 10, APO2L | 8743 |
| CD254 | TRANCE; TNF (ligand) superfamily member 11, RANKL | 8600 |
| CD256 | APRIL; TNF (ligand) superfamily member 13, TALL2 | 8741 |
| CD257 | BLYS; TNF (ligand) superfamily, member 13b, TALL1, BAFF | 10673 |
| CD258 | LIGHT; TNF (ligand) superfamily, member 14 | 8740 |
| CD261 | TRAIL-R1; TNFR superfamily, member 10a, DR4, APO2 | 8797 |
| CD262 | TRAIL-R2; TNFR superfamily, member 10b, DR5 | 8795 |
| CD263 | TRAIL-R3; TNFR superfamily, member 10c, DCR1 | 8794 |
| CD264 | TRAIL-R4; TNFR superfamily, member 10d, DCR2 | 8793 |
| CD265 | TRANCE-R; TNFR superfamily, member 11a, RANK | 8792 |
| CD266 | TWEAK-R; TNFR superfamily, member 12A, type I transmembrane protein Fn14 | 51330 |
| CD267 | TACI; TNFR superfamily, member 13B, transmembrane activator and CAML interactor | 23495 |
| CD268 | BAFFR; TNFR superfamily, member 13C, B cell-activating factor receptor | 115650 |
| CD269 | BCMA; TNFR superfamily, member 17, B-cell maturation factor | 608 |
| CD271 | NGFR (p75); nerve growth factor receptor (TNFR superfamily, member 16) | 4804 |
| CD272 | BTLA; B and T lymphocyte attenuator | 151888 |
| CD273 | B7DC, PDL2; programmed cell death 1 ligand 2 | 80380 |
| CD274 | B7H1, PDL1; programmed cell death 1 ligand 1 | 29126 |
| CD275 | B7H2, ICOSL; inducible T-cell co-stimulator ligand (ICOSL) | 23308 |
| CD276 | B7H3; B7 homolog 3 | 80381 |
| CD277 | BT3.1; B7 family: butyrophilin, subfamily 3, member A1 | 11119 |
| CD278 | ICOS; inducible T-cell co-stimulator | 29851 |
| CD279 | PD1; programmed cell death 1 | 5133 |
| CD280 | ENDO180; uPARAP, mannose receptor, C type 2, TEM22 | 9902 |
| CD281 | TLR1; TOLL-like receptor 1 | 7096 |
| CD282 | TLR2; TOLL-like receptor 2 | 7097 |
| CD283 | TLR3; TOLL-like receptor 3 | 7098 |
| CD284 | TLR4; TOLL-like receptor 4 | 7099 |
| CD289 | TLR9; TOLL-like receptor 9 | 54106 |
| CD292 | BMPR1A; Bone Morphogenetic Protein Receptor, type IA | 657 |
| CDw293 | BMPR1B; Bone Morphogenetic Protein Receptor, type IB | 658 |
| CD294 | CRTH2; PGRD2; G protein-coupled receptor 44, | 11251 |
| CD295 | LEPR; Leptin Receptor | 3953 |
| CD296 | ART1; ADP-ribosyltransferase 1 | 417 |
| CD297 | ART4; ADP-ribosyltransferase 4; Dombrock blood group glycoprotein | 420 |
| CD298 | ATP1B3; Na+/K+ -ATPase beta 3 subunit | 483 |
| CD299 | DCSIGN-related; CD209 antigen-like, DC-SIGN2, L-SIGN | 10332 |
| CD300a | CMRF35 FAMILY; CMRF-35H | 11314 |
| CD300c | CMRF35 FAMILY; CMRF-35A | 10871 |
| CD300e | CMRF35 FAMILY; CMRF-35L1 | |
| CD301 | MGL; CLECSF14, macrophage galactose-type C-type lectin | 10462 |
| CD302 | DCL1; Type I transmembrane C-type lectin receptor DCL-1 | 9936 |
| CD303 | BDCA2; C-type lectin, superfamily member 11 | 170482 |
| CD304 | BDCA4; Neuropilin 1 | 8829 |
| CD305 | LAIR1; Leukocyte-Associated Ig-like Receptor 1 | 3903 |
| CD306 | LAIR2; Leukocyte-Associated Ig-like Receptor 2 | 3904 |
| CD307 | IRTA2; Immunoglobulin superfamily Receptor Translocation Associated 2 | 83416 |
| CD309 | VEGFR2; KDR (a type III receptor tyrosine kinase) | 3791 |
| CD312 | EMR2 ; EGF-like module containing, mucin-like, hormone receptor-like 2 | 30817 |
| CD314 | NKG2D; Killer cell lectin-like receptor subfamily K, member 1 | 22914 |
| CD315 | CD9P1; Prostaglandin F2 receptor negative regulator | 5738 |
| CD316 | EWI2; Immunoglobulin superfamily, member 8 | 93185 |
| CD317 | BST2; Bone Marrow Stromal cell antigen 2 | 684 |
| CD318 | CDCP1; CUB domain-containing protein 1 | 64866 |
| CD319 | CRACC; SLAM family member 7 | 57823 |
| CD320 | 8D6; 8D6 Antigen; FDC | 51293 |
| CD321 | JAM1; F11 receptor | 50848 |
| CD322 | JAM2; Junctional Adhesion Molecule 2 | 58494 |
| CD324 | E-Cadherin; cadherin 1, type 1, E-cadherin (epithelial) | 999 |
| CDw325 | N-Cadherin; cadherin 2, type 1, N-cadherin (neuronal) | 1000 |
| CD326 | Ep-CAM; tumor-associated calcium signal transducer 1 | 4072 |
| CDw327 | siglec6; sialic acid binding Ig-like lectin 6 | 946 |
| CDw328 | siglec7; sialic acid binding Ig-like lectin 7 | 27036 |
| CDw329 | siglec9; sialic acid binding Ig-like lectin 9 | 27180 |
| CD331 | FGFR1; Fibroblast Growth Factor Receptor 1 | 2260 |
| CD332 | FGFR2; Fibroblast Growth Factor Receptor 2 (keratinocyte growth factor receptor) | 2263 |
| CD333 | FGFR3; Fibroblast Growth Factor Receptor 3 (achondroplasia, thanatophoric dwarfism) | 2261 |
| CD334 | FGFR4; Fibroblast Growth Factor Receptor 4 | 2264 |
| CD335 | NKp46; NCR1, (Ly94); natural cytotoxicity triggering receptor 1 | 9437 |
| CD336 | NKp44; NCR2, (Ly95); natural cytotoxicity triggering receptor 2 | 9436 |
| CD337 | NKp30; NCR3 | 259197 |
| CDw338 | ABCG2; ATP-binding cassette, sub-family G (WHITE), member 2 | 9429 |
| CD339 | Jagged-1; Jagged 1 (Alagille syndrome) | 182 |

**Table 2**

| Extracted from R. Thorpe et al., Cytokine 21 (2003) 48-49 | | | | |
|---|---|---|---|---|
| Systematic name | Human chromosome | Human ligand | Mouse ligand | Chemokine receptor(s) |
| CXC *chemokine*/*receptor family* | | | | |
| CXCL1 | 4q21.1 | GROα/MGSA-α | GRO/MIP-2/KC? | CXCR2 > CXCR1 |
| CXCL2 | 4q21.1 | GROβ/MGSA-β | GRO/MIP-2/KC? | CXCR2 |
| CXCL3 | 4q21.1 | GROγ/MGSA-γ | GRO/MIP-2/KC? | CXCR2 |
| CXCL4 | 4q21.1 | PF4 | PF4 | Unknown |
| CXCL5 | 4q21.1 | ENA-78 | GCP2/LIX? | CXCR2 |
| CXCL6 | 4q21.1 | GCP-2 | GCP-2/LIX? | CXCR1, CXCR2 |
| CXCL7 | 4q21.1 | NAP-2 | Unknown | CXCR2 |
| CXCL8 | 4q21.1 | IL-8 | Unknown | CXCR1, CXCR2 |
| CXCL9 | 4q21.1 | Mig | Mig | CXCR3^{a} |
| CXCL10 | 4q21.1 | IP-10 | IP-10/CRG-2 | CXCR3^{a} |
| CXCL11 | 4q21.1 | I-TAC | I-TAC | CXCR3^{a} |
| CXCL12 | 10q11.21 | SDF-1 α/β | SDF-1/PBSF | CXCR4^{b} |
| CXCL13 | 4q21.1 | BCA-1 | BLC | CXCR5 |
| CXCL14 | 5q31.1 | BRAK/bolekine | BRAK | Unknown |
| (CXCL15) | | Unknown | Lungkine/WECHE | Unknown |
| CXCL16 | 17p13 | | | CXCR6 |

| *C chemokine*/*receptor family* | | | | |
|---|---|---|---|---|
| XCL1 | 1q24.2 | Lymphotactin/SCM-1α/ ATAC | Lymphotactin | XCR1 |
| XCL2 | 1q24.2 | SCM-1β | Unknown | XCR1 |

| CX₃*C chemokine*/*receptor family* | | | | |
|---|---|---|---|---|
| CX3CL1 | 16q13 | Fractalkine | Neurotactin/ABCD-3 | CX3CR1 |

| *CC chemokine*/*receptor family* | | | | |
|---|---|---|---|---|
| CCL1 | 17q11.2 | I-309 | TCA-3/P500 | CCR8 |
| CCL2 | 17q11.2 | MCP-1/MCAF/TDCF | JE? | CCR2 |
| CCL3 | 17q12 | MIP-1α/LD78α | MIP-1α | CCR1, CCR5 |
| CCL3L1 | 17q12 | LD78β | Unknown | CCR1, CCR5 |
| CCL4 | 17q12 | MIP-1β | MIP-1β | CCR5³ |
| CCL5 | 17q12 | RANTES | RANTES | CCR1, CCR3, CCR5^{c} |
| (CCL6) | | Unknown | C10/MRP-1 | Unknown |
| CCL7 | 17q11.2 | MCP-3 | MARC? | CCR1, CCR2, CCR3 |
| CCL8 | 17q11.2 | MCP-2 | MCP-2? | CCR3, CCR5^{c} |
| (CCL9/10) | | Unknown | MRP-2/CCF18/MIP-1γ | CCR1 |
| CCL11 | 17q11.2 | Eotaxin | Eotaxin | CCR3 |
| (CCL12) | | Unknown | MCP-5 | CCR2 |
| CCL13 | 17q11.2 | MCP-4 | Unknown | CCR2, CCR3 |
| CCL14 | 17q12 | HCC-1 | Unknown | CCR1, CCR5 |
| CCL15 | 17q12 | HCC-2/Lkn-1/MIP-1 | Unknown | CCR1, CCR3 |
| CCL16 | 17q12 | HCC-4/LEC/LCC-1 | Unknown | CCR1, CCR2 |
| CCL17 | 16q13 | TARC | TARC/ABCD-2 | CCR4 |
| CCL18 | 17q12 | DC-CK1/PARC/AMAC-1 | Unknown | Unknown |
| CCL19 | 9p13.3 | MIP-3β/ELC/exodus-3 | MIP-3β/ELC/exodus-3 | CCR7^{d} |
| CCL20 | 2q36.3 | MIP-3α/LARC/exodus-1 | MIP-3α/LARC/exodus-1 | CCR6 |
| CCL21 | 9p13.3 | 6Ckine/SLC/exodus-2 | 6Ckine/SLC/exodus-2/ | CCR7^{d} |
| | | | TCA-4 | |
| CCL22 | 16q13 | MDC/STCP-1 | ABCD-1 | CCR4 |
| CCL23 | 17q12 | MPIF-1/CKβ8/CKβ8-1 | Unknown | CCR1 |
| CCL24 | 7q11.23 | Eotaxin-2/MPIF-2 | MPIF-2 | CCR3 |
| CCL25 | 19p13.3 | TECK | TECK | CCR9 |
| CCL26 | 7q11.23 | Eotaxin-3 | Unknown | CCR3 |
| CCL27 | 9p13.3 | CTACK/ILC | ALP/CTACK/ILC/ESkine | CCR10 |
| CCL28 | 5p12 | MEC | | CCR3/CCR10 |

| | | | | |
|---|---|---|---|---|
| ^{a} CD183. ^{b} CD184. ^{c} CD195. ^{d} CD_{w} 197. | | | | |

**Table 3**

| **Name** | **Source** | **Target receptors** | **Target cells** | **Function** |
|---|---|---|---|---|
| IL-1 | macrophages, B cells, monocytes, dendritic cells | CD121a/IL1R1, CD121b/IL1R2 | T helper cells | co-stimulation |
| | | | B cells | Maturation & proliferation |
| | | | Nk cells | activation |
| | | | macrophages, endothelium, other | inflammation, small amounts induce acute phase reaction, large amounts induce fever |
| IL-2 | TH1-cells | CD25/IL2RA, CD122/IL2RB, CD132/IL2RG | activated T cells and B cells, NK cells, macrophages, oligodendrocytes | stimulates growth and differentiation of T cell response. Can be used in immunotherapy to treat cancer or suppressed for transplant patients. |
| IL-3 | activated T helper cells[3], mast cells, NK cells, endothelium, eosinophils | CD123/IL3RA, CD131/IL3RB | hematopoietic stem cells | growth and differentiation to e.g. erythrocytes, granulocytes |
| | | | mast cells | growth and histamine release |
| IL-4 | TH2-cells, just activated naive CD4+ cell, memory CD4+ cells, mast cells, macrophages | CD124/IL4R, CD132/IL2RG | activated B cells | proliferation and differentiation, IgG1 and IgE synthesis. Important role in allergic response (IgE) |
| | | | T cells | proliferation |
| IL-5 | TH2-cells, mast cells, eosinophils | CD125/IL5RA, CD131/IL3RB | eosinophils | production |
| | | | B cells | differentiation, IgA production |
| IL-6 | macrophages, TH2-cells, B cells, astrocytes, endothelium | CD126/IL6RA, CD130/IR6RB | activated B cells | differentiation into plasma cells |
| | | | plasma cells | antibody secretion |
| | | | hematopoietic stem cells | differentiation |
| | | | T cells, others | induces acute phase reaction, hematopoiesis, differentiation, inflammation |
| IL-7 | bone marrow stromal cells and thymus stromal cells | CD127/IL7RA, CD132/IL2RG | pre/pro-B cell, pre/pro-T cell, NK cells | involved in B, T, and NK cell survival, development, and homeostasis, ↑ proinflammatory cytokines |
| IL-8 | macrophages, lymphocytes, epithelial cells, endothelial cells | CXCR1/IL8RA, CXCR2/IL8RB/CD128 | neutrophils, basophils, lymphocytes | Neutrophil chemotaxis |
| IL-9 | Th2-cells, specifically by CD4+ helper cells | CD129/IL9R | T cells, B cells | Potentiates IgM, IgG, IgE, stimulates mast cells |
| IL-10 | monocytes, TH2-cells, CD8+ T cells, mast cells, macrophages, B cell subset | CD210/IL10RA, CDW210B/IL10RB | macrophages | cytokine production |
| | | | B cells | activation |
| | | | Th1 cells | inhibits Th1 cytokine production (IFN-γ, TNF-β, IL-2) |
| | | | Th2 cells | Stimulation |
| IL-11 | bone marrow stroma | IL11RA | bone marrow stroma | acute phase protein production, osteoclast formation |
| IL-12 | dendritic cells, B cells, T cells, macrophages | CD212/IL12RB1, IR12RB2 | activated [3] T cells, | differentiation into Cytotoxic T cells with IL-2[3], ↑ IFN-γ, TNF-α, ↓ IL-10 |
| | | | NK cells | ↑ IFN-γ, TNF-α |
| IL-13 | activated TH2-cells, mast cells, NK cells | IL13R | TH2-cells, B cells, macrophages | Stimulates growth and differentiation of B-Cells (IgE), inhibits TH1-cells and the production of macrophage inflammatory cytokines (e.g. IL-1, IL-6), ↓ IL-8, IL-10, IL-12 |
| IL-14 | T cells and certain malignant B cells | | activated B cells | controls the growth and proliferation of B cells, inhibits Ig secretion |
| IL-15 | mononuclear phagocytes (and some other cells), especially macrophages following infection by virus(es) | IL15RA | T cells, activated B cells | Induces production of Natural Killer Cells |
| IL-16 | lymphocytes, epithelial cells, eosinophils, CD8+ T cells | CD4 | CD4+ T cells | CD4+ chemoattractant |
| IL-17 | subsets of T cells | CDw217/IL17RA, IL17RB | epithelium, endothelium, other | osteoclastogenesis, angiogenesis, ↑ inflammatory cytokines |
| IL-18 | macrophages | CDw218a/IL18R1 | Th1 cells, NK cells | Induces production of IFNγ, ↑ NK cell activity |
| IL-19 | - | IL20R | | - |
| IL-20 | - | IL20R | | regulates proliferation and differentiation of keratinocytes |
| IL-21 | - | IL21R | | |
| IL-22 | - | IL22R | | Activates STAT1 and STAT3 and increases production of acute phase proteins such as serum amyloid A, Alpha 1-antichymotrypsin and haptoglobin in hepatoma cell lines |
| IL-23 | - | IL23R | | Increases angiogenesis but reduces CD8 T-cell infiltration |
| IL-24 | - | IL20R | | Plays important roles in tumor suppression, wound healing and psoriasis by influencing cell survival. |
| IL-25 | - | LY6E | | Induces the production IL-4, IL-5 and IL-13, which stimulate eosinophil expansion |
| IL-26 | - | IL20R1 | | Enhances secretion of IL-10 and IL-8 and cell surface expression of CD54 on epithelial cells |
| IL-27 | - | IL27RA | | Regulates the activity of B lymphocyte and T lymphocytes |
| IL-28 | - | IL28R | | Plays a role in immune defense against viruses |
| IL-29 | - | | | Plays a role in host defenses against microbes |
| IL-30 | - | | | Forms one chain of IL-27 |
| IL-31 | - | IL31RA | | May play a role in inflammation of the skin |
| IL-32 | - | | | Induces monocytes and macrophages to secrete TNF-α, IL-8 and CXCL2 |
| IL-33 | - | | | Induces helper T cells to produce type 2 cytokine |
| IL-35 | regulatory T cells | | | Suppression of T helper cell activation |

### Literature:

1. Baneyx F, Georgiou G: Degradation of secreted proteins in Escherichia coli. Ann N Y Acad Sci 1992, 665:301-308.
2. Baskin JM, Prescher JA, Laughlin ST, Agard NJ, Chang PV, Miller IA, Lo A, Codelli JA, Bertozzi CR: Copper-free click chemistry for dynamic in vivo imaging. Proc Natl Acad Sci U S A 2007, 104:16793-16797.
3. Dyba M, Tarasova NI, Michejda CJ: Small molecule toxins targeting tumor receptors. Curr Pharm Des 2004, 10:2311-2334.
4. GAUTIER A, JOHNSSON, K., KINDERMANN, M., JUILLERAT, A., BEAUFILS, F. PCT/EP2007/057597: **LABELLING OF FUSION PROTEINS WITH SYNTHETIC PROBES.** 2008.
5. Hochuli E: Large-scale chromatography of recombinant proteins. J Chromatogr 1988, 444:293-302.
6. JACCARD H, JOHNSSON, K., KINDERMANN, M., SIELAFF, I. C. PCT/EP2005/050900: **SPECIFIC SUBSTRATES FOR O6-ALKYLGUANINE-DNA ALKYLTRANSFERASE.** 2005.
7. JOHNSSON K, GEORGE, N. PCT/IB2004/001733: **METHODS FOR PROTEIN LABELING BASED ON ACYL CARRIER PROTEIN.** 2004.
8. KINDERMANN M, SCHWAB, M. PCT/EP2006/061798: **PYRIMIDINES REACTING WITH 06-ALKYLGUANINE-DNA ALKYLTRANSFERASE.** 2006.
9. Porath J, Carlsson J, Olsson I, Belfrage G: Metal chelate affinity chromatography, a new approach to protein fractionation. Nature 1975, 258:598-599.
10. Steinborn G, Boer E, Scholz A, Tag K, Kunze G, Gellissen G: Application of a wide-range yeast vector (CoMed) system to recombinant protein production in dimorphic Arxula adeninivorans, methylotrophic Hansenula polymorpha and other yeasts. Microb Cell Fact 2006, 5:33.
11. Stocker M, Tur MK, Sasse S, Krussmann A, Barth S, Engert A: Secretion of functional anti-CD30-angiogenin immunotoxins into the supernatant of transfected 293T-cells. Protein Expr Purif 2003, 28:211-219.
12. Thorpe, R., et al., Cytokine 21 (2003) 48-49

## Claims

1. A compound comprising three components A, B, and C, which components are covalently bound forming the compound having the structure A-B-C wherein
component A has a specific binding affinity for antigens,
component B is covalently linked to component A
component C is a compound having an alkylated purin or pyrimidin moiety such as guanin, cytosin or a Coenzyme A moiety and linked thereto a moiety having a physiological effect with the proviso that component B has an catalytical or acceptor activity to couple component C with covalently coupled components A-B.

2. The compound according to claim 1 wherein the compound is a heterologous complex comprising at least one recombinant fusion protein comprising at least one binding component A, in particular cell-specific binding component and one enzyme type protein B and at least one additional component C that is covalently coupled to B or
wherein the compound is a heterologous complex comprising at least one recombinant fusion protein comprising at least one component A binding to a soluble antigen and one enzyme type protein B and at least one additional component C that is covalently coupled to B.

3. The compound of claims 1 or 2 having a covalent modification of component A with component C through component B and/or
wherein component A is a chemical moiety having an antigen binding structure of polypeptidic structure, and component B is an enzyme type protein linked to component A and/or
wherein component B is capable of reacting with component C in a substrate specific manner, thereby connecting covalently the complex AB with component C and/or
wherein component A belongs to the group of antigen binding polypeptides/proteins targeting celltype specific markers, in particular component A is directed against disease specific structures of pathogenic substances or pathogenic matter or component A is binding to soluble markers of disease/environment/food and feed safety or biodefense (e.g. toxins) and/or
wherein component A comprises moieties which are affinity moieties from affinity substances or affinity substances in their entirety selected from the group consisting of antibodies, receptor ligands, enzyme substrates, lectins, cytokines, lymphokines, interleukins, angiogenic or virulence factors, allergens, peptidic allergens, recombinant allergens, allergen-idiotypical antibodies, autoimmune-provoking structures, tissue-rejection-inducing structures, immunoglobulin constant regions and their derivatives, mutants or combinations thereof.

4. The compound of any one of the claims 1 to 3 wherein
● component B is a polypeptide that reacts covalently with a specific substrate and/or
● component B is a derivative of the human DNA repair protein O⁶-alkylguanine-DNA alkyltransferase (AGT) and/or
● component B is a derivative of the Acyl Carrier Protein (ACP) and/or
● the substrate for component B consists of 06-benzylguanine, 02-benzylcytosine or a coenzyme A (CoA).

5. The compound of any one of the claims 1 to 4 wherein the covalently coupled components A-B are polypeptides.

6. The compound of any one of the claims 1 to 5 wherein component C is a drug, a detectable label or other components mediating biological activity in a targeted cell or organism and/or
wherein component C is a solid phase or a support and/or
wherein component C comprises a moiety which serves as a substrate for component B and/or
wherein component C can have the structure (X)ₙ₁-(Y) -(Z)ₙ₂ with X being a component B specific substrate and n1 being one or more, in particular 1-3, Z being a drug, a detectable label or other components mediating biological activity in a targeted cell or organism and n2 being 1 or more and Y is a linker structural element designed to functionally connect X and Z preferably
● wherein Y is a spacer between X and Z for ensuring the functionality of each component within the assembled compound and/or
● wherein the linker structural element comprises structural elements enabeling a controlled release of Z under reaction conditions such as pH sensitive or reducable structures for release in endosomes or the cytosol and/or
● wherein the structural element Y may also have linear, branched, tree like or polymeric structure.

7. A nucleic acid molecule coding for polypeptides of claim 5.

8. A vector comprising the nucleic acid of claim 7.

9. A method for the expression of the recombinant genes encoding the recombinant compounds of any one of the claims 1 to 6, wherein a host cell comprising a vector of claim 8 and culturing the host cell under conditions suitable for the expression of the compounds of any one of the claims 1 to 6, preferably wherein the host cell is a procaryotic or a eucaryotic cell.

10. A cellular compartment or an organism except a human being which compartment or organism being transformed or transfected with the nucleic acid of claim 7, preferably wherein the cellular compartment is of prokaryotic origin in particular from *E. coli, B. subtilis, S. carnosus S. coelicolor,* and/or *Marinococcus sp.,* or a lower eukaryote, such as *Saccharomyces sp., Aspergillus sp., Hansenula polymorpha, Arxula adeninivorans, Spodoptera sp. and*/*or P. pastoris,* a higher non-human eukaryote such as a plant and/or an animal, and the cell is a primary or cultivated mammalian cell, such as a freshly isolated human cell or a eukaryotic cell line such as CHO, NS0, COS, BHK, 293T and MDCK.

11. A method of manufacturing a compound of any one of the claims 1 to 6 by reacting a component AB or BA with a component C comprising one ore more enzyme substrates for which B is specific and further carrying one or more copies of a drug, a detectable label or other components mediating biological activity in a targeted cell or organism.

12. A method of preparing and administering the compounds of any one of the claims 1 to 6 to cells *in vitro* and *in vivo,* with component C carrying one or more copies of a drug, a detectable label or other components mediating biological activity in a targeted cell or organism.

13. A method of using the compounds of any one of the claims 1 to 6 for in vitro and in vivo diagnostic applications in the field of human and animal disorders as well as analytic applications in the field of environmental monitoring, ecotoxicology and biosensor applications, with component C as a detectable label and/or in therapy for human and animal disorders, with component C as a drug or elements mediating biological activity in a targeted cell or organism.

14. A method for immobilizing component A via component C directly to a surface of a support such as a planar surface or a bead, allowing to enrich the marker at a distinct location.

15. A medicament comprising the compound according to any one of the claims 1 to 6, the nucleic acid of claim 7, the vector of claim 8 and/or the cellular compartment or organism of claim 10.

16. Use of the compound of any one of the claims 1 to 6, the nucleic acid of claim 7, the vector of claim 8 and/or the cellular compartment or organism of claim 10 for manufacturing of a medicament for the treatment of malignant diseases, allergic diseases, auto immune reactions, chronic inflammatory reactions or tissue/graft rejection reactions.
